(19) Europäisches Patentamt
European Patent Office
Office européen des brevets

(11) **EP 4 074 808 B1**

(12) **EUROPEAN PATENT SPECIFICATION**

(45) Date of publication and mention of the grant of the patent:
**24.07.2024 Bulletin 2024/30**

(21) Application number: **22167733.9**

(22) Date of filing: **12.04.2022**

(51) International Patent Classification (IPC):
***C09K 19/34*** *(2006.01)* ***C09K 19/30*** *(2006.01)*
***C09K 19/04*** *(2006.01)* ***C09K 19/12*** *(2006.01)*

(52) Cooperative Patent Classification (CPC):
**C09K 19/3444; C09K 19/3098; C09K 19/3491;**
C09K 2019/0466; C09K 2019/122; C09K 2019/123; C09K 2019/124; C09K 2019/3004; C09K 2019/301; C09K 2019/3422

(54) **LIQUID CRYSTALLINE MEDIUM**

FLÜSSIGKRISTALLINES MEDIUM

SUPPORT À CRISTAUX LIQUIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.04.2021 CN 202110393837**

(43) Date of publication of application:
**19.10.2022 Bulletin 2022/42**

(73) Proprietor: **Merck Patent GmbH**
**64293 Darmstadt (DE)**

(72) Inventors:
- **Laut, Sven Christian**
  **SHANGHAI, 201206 (CN)**
- **Lee, Hee-Kyu**
  **SHANGHAI, 201206 (CN)**
- **Yang, Minghui**
  **SHANGHAI, 201206 (CN)**

(74) Representative: **Merck Patent Association**
**Merck Patent GmbH**
**64271 Darmstadt (DE)**

(56) References cited:
**WO-A1-2016/146240**
**DE-A1- 102008 016 053**
**DE-A1- 102013 016 399**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The present invention relates to novel liquid crystalline media, in particular for use in liquid-crystal displays, and to these liquid-crystal displays, particularly to liquid-crystal displays which use the IPS (in-plane switching) or, preferably, the FFS (fringe field switching) effect using dielectrically positive liquid crystals. The last one is also called XB-FFS (extra brightness FFS) effect occasionally.

For this effect dielectrically positive liquid crystals are used, which comprise one or more compounds having at the same time a high dielectric constant parallel to the molecular director and perpendicular to the molecular director, leading to a large average dielectric constant and a high dielectric ratio and, preferably, to a relatively small dielectric anisotropy at the same time. The liquid crystalline media optionally additionally comprise dielectrically negative, dielectrically neutral compounds or both. The liquid crystalline media are used in a homogeneous (i.e. planar) initial alignment. The liquid-crystal media according to the invention have a positive dielectric anisotropy and comprise compounds having at the same time large dielectric constants parallel and perpendicular to the molecular director.

The media are distinguished by a particularly good representation of the black state, primarily attributable to their very low scattering. This in turn is brought about especially by their high elastic constants.

High elastic constants may lead to higher values of the rotational viscosity ($\gamma_1$), too. And consequently to higher values of the ratio $\gamma_1$ /$K_{22}$ of the rotational viscosity ($\gamma_1$) to the elastic constant for the twist deformation($K_{22}$), which then leads to higher response times. Since $K_{22}$ is approximately proportional to the elastic constant $K_{11}$ for splay deformation (the value of $K_{22}$ is typically about half the value of $K_{11}$), this can easily and conveniently be approximated determining $\gamma_1$ and $K_{11}$.

The contrast of an LC Display can be improved, at one hand by a higher transmittance, which can be achieved by a higher $\varepsilon_\perp$ in an FFS cell layout. And, on the other hand, it can be improved by a better dark state. The latter, i.e. the dark state, is strongly influenced, amongst others, by the scattering parameter. Here liquid crystalline media having high elastic constants lower the scattering and therefore improve the contrast of an LCD. This also leads to their excellent performance in the displays according to the invention.

IPS and FFS displays using dielectrically positive liquid crystals are well known in the field and have been widely adopted for various types of displays like e.g. desk top monitors and TV sets, but also for mobile applications.

However, recently, IPS and in particular FFS displays using dielectrically negative liquid crystals are widely adopted. The latter ones are sometimes also called UB-FFS (ultra bright FFS). Such displays are disclosed e.g. in US 2013/0207038 A1. These displays are characterized by a markedly increased transmission compared to the previously used IPS- and FFS displays, which have been dielectrically positive liquid crystals. These displays using conventional, dielectrically negative liquid crystals, however, have the severe disadvantage of requiring a higher operation voltage than the respective displays using dielectrically positive liquid crystals. Liquid crystalline media used for UB-FFS have a dielectric anisotropy of -0.5 or less and preferably of -1.5 or less.

Liquid crystalline media used for HB-FFS (high brightness FFS) have a dielectric anisotropy of 0.5 or more and preferably of 1.5 or more. Liquid crystalline media used for HB-FFS comprising both dielectrically negative and dielectrically positive liquid crystalline compounds, respectively mesogenic compounds are disclosed e.g. in US 2013/0207038 A1. These media feature rather large values of $\varepsilon_\perp$ and of $\varepsilon_{av.}$ already, however, their ratio of ($\varepsilon_\perp$ / $\Delta\varepsilon$) is relatively small.

According to the present application, however, the IPS or the FFS effect with dielectrically positive liquid crystalline media in a homogeneous alignment are preferred.

Industrial application of this effect in electro-optical display elements requires LC phases which have to meet a multiplicity of requirements. Particularly important here are chemical resistance to moisture, air and physical influences, such as heat, radiation in the infrared, visible and ultraviolet regions, and direct (DC) and alternating (AC) electric fields.

Furthermore, LC phases which can be used industrially are required to have a liquid-crystalline mesophase in a suitable temperature range and low viscosity.

None of the series of compounds having a liquid-crystalline mesophase that have been disclosed hitherto includes a single compound which meets all these requirements. Mixtures of two to 25, preferably three to 18, compounds are therefore generally prepared in order to obtain substances which can be used as LC phases.

Matrix liquid-crystal displays (MLC displays) are known. Non-linear elements which can be used for individual switching of the individual pixels are, for example, active elements (i.e. transistors). The term "active matrix" is then used, where in general use is made of thin-film transistors (TFTs), which are generally arranged on a glass plate as substrate.

A distinction is made between two technologies: TFTs comprising compound semiconductors, such as, for example, CdSe, or metal oxides like ZnO or TFTs based on polycrystalline and, inter alia, amorphous silicon. The latter technology currently has the greatest commercial importance worldwide.

The TFT matrix is applied to the inside of one glass plate of the display, while the other glass plate carries the transparent counter electrode on its inside. Compared with the size of the pixel electrode, the TFT is very small and has virtually no adverse effect on the image. This technology can also be extended to fully colour-capable displays, in which

a mosaic of red, green and blue filters is arranged in such a way that a filter element is located opposite each switchable pixel.

The TFT displays mostly used so far usually operate with crossed polarisers in transmission and are backlit. For TV applications, ECB (or VAN) cells or FFS cells are used, whereas monitors usually use IPS cells or TN (twisted nematic) cells, and notebooks, laptops and mobile applications usually use TN, VA or FFS cells.

The term MLC displays here encompasses any matrix display having integrated non-linear elements, i.e., besides the active matrix, also displays with passive elements, such as varistors or diodes (MIM = metal-insulator-metal).

MLC displays of this type are particularly suitable for TV applications, monitors and notebooks or for displays with a high information density, for example in automobile manufacture or aircraft construction. Besides problems regarding the angle dependence of the contrast and the response times, difficulties also arise in MLC displays due to insufficiently high specific resistance of the liquid-crystal mixtures [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, pp. 141 ff., Paris; STROMER, M., Proc. Eurodisplay 84, Sept. 1984: Design of Thin Film Transistors for Matrix Addressing of Television Liquid Crystal Displays, pp. 145 ff., Paris]. With decreasing resistance, the contrast of an MLC display deteriorates. Since the specific resistance of the liquid-crystal mixture generally drops over the life of an MLC display owing to interaction with the inside surfaces of the display, a high (initial) resistance is very important for displays that have to have acceptable resistance values over a long operating period.

Displays which use the ECB effect have become established as so-called VAN (vertically aligned nematic) displays, besides IPS displays (for example: Yeo, S.D., Paper 15.3: "An LC Display for the TV Application", SID 2004 International Symposium, Digest of Technical Papers, XXXV, Book II, pp. 758 and 759) and the long-known TN displays, as one of the three more recent types of liquid-crystal display that are currently the most important, in particular for television applications.

The most important designs may be mentioned here: MVA (multi-domain vertical alignment, for example: Yoshide, H. et al., Paper 3.1: "MVA LCD for Notebook or Mobile PCs ...", SID 2004 International Symposium, Digest of Technical Papers, XXXV, Book I, pp. 6 to 9, and Liu, C.T. et al., Paper 15.1: "A 46-inch TFT-LCD HDTV Technology ...", SID 2004 International Symposium, Digest of Technical Papers, XXXV, Book II, pp. 750 to 753), PVA (patterned vertical alignment, for example: Kim, Sang Soo, Paper 15.4: "Super PVA Sets New State-of-the-Art for LCD-TV", SID 2004 International Symposium, Digest of Technical Papers, XXXV, Book II, pp. 760 to 763) and ASV (advanced super view, for example: Shigeta, Mitzuhiro and Fukuoka, Hirofumi, Paper 15.2: "Development of High Quality LCDTV", SID 2004 International Symposium, Digest of Technical Papers, XXXV, Book II, pp. 754 to 757). More modern versions of the VA effect, are the so called PAVA (photo-alignment VA) and PSVA (polymer-stabilized VA).

In general form, the technologies are compared, for example, in Souk, Jun, SID Seminar 2004, Seminar M-6: "Recent Advances in LCD Technology", Seminar Lecture Notes, M-6/1 to M-6/26, and Miller, Ian, SID Seminar 2004, Seminar M-7: "LCD-Television", Seminar Lecture Notes, M-7/1 to M-7/32. Although the response times of modern ECB displays have already been significantly improved by addressing methods with overdrive, for example: Kim, Hyeon Kyeong et al., Paper 9.1: "A 57-in. Wide UXGA TFT-LCD for HDTV Application", SID 2004 International Symposium, Digest of Technical Papers, XXXV, Book I, pp. 106 to 109, the achievement of video-compatible response times, in particular in the switching of grey shades, is still a problem which has not yet been solved to a satisfactory extent.

ECB displays, like ASV displays, use liquid-crystalline media having negative dielectric anisotropy ($\Delta\varepsilon$), whereas TN and to date all conventional IPS displays use liquid-crystalline media having positive dielectric anisotropy. However, presently there is an increasing demand for IPS and FFS displays utilizing dielectrically negative liquid crystalline media.

In liquid-crystal displays of this type, the liquid crystals are used as dielectrics, whose optical properties change reversibly on application of an electrical voltage.

Since in displays in general, i.e. also in displays in accordance with these mentioned effects, the operating voltage should be as low as possible, use is made of liquid-crystal media which are generally predominantly composed of liquid-crystal compounds, all of which have the same sign of the dielectric anisotropy and have the highest possible value of the dielectric anisotropy. In general, at most relatively small proportions of neutral compounds and if possible no compounds having a sign of the dielectric anisotropy which is opposite to that of the medium are employed. In the case of liquid-crystal media having negative dielectric anisotropy e.g. for ECB or UB-FFS displays, predominantly compounds having negative dielectric anisotropy are thus employed. The respective liquid-crystalline media employed generally consist predominantly of liquid-crystal compounds having negative dielectric anisotropy.

In the media used in accordance with the present application, significant amounts of dielectrically positive liquid-crystal compounds and generally only very small amounts of dielectrically negative compounds or even none at all are typically employed, since in general the liquid-crystal displays are intended to have the lowest possible addressing voltages. The low addressing voltages enables displays which are energy saving and have fast switching times. At the same time small amounts of dielectrically neutral compounds may be beneficially used.

Liquid crystalline media having a positive dielectric anisotropy for IPS and FFS displays have already been

disclosed.

Compounds of the following formula and similar

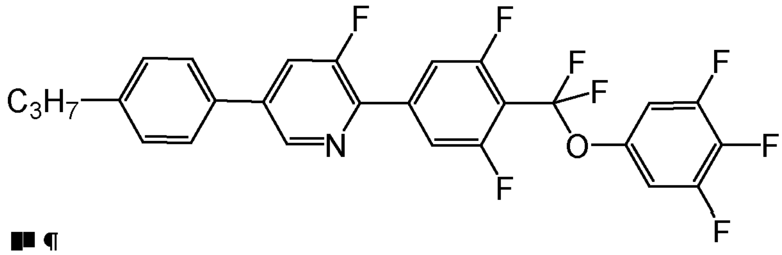

are disclosed in WO 2008/128622 A1 or DE 10 2013 016399 A1.

Liquid crystalline compounds comprising a thiophene ring are disclosed in WO 2012/013281 A1.

The phase range of the liquid-crystal mixture must be sufficiently broad for the intended application of the display. The response times of the liquid-crystal media in the displays should be as low as possible, especially for video, animated simulation and gaming applications. This is particularly important for displays for television or multimedia applications. In order to improve the response times, it has repeatedly been proposed in the past to optimise the rotational viscosity of the liquid-crystal media ($\gamma_1$), i.e. to achieve media having the lowest possible rotational viscosity. However, the results achieved here are inadequate for many applications and therefore make it appear desirable to find further optimisation approaches.

Adequate stability of the media to extreme loads, in particular to UV exposure and heating, is very particularly important. In particular in the case of applications in displays in mobile equipment, such as, for example, mobile telephones, this may be crucial.

Besides their relatively poor transmission and their relatively long response times, the MLC displays disclosed hitherto, they have further disadvantages. These are e.g. their comparatively low contrast, their relatively high viewingangle dependence and the difficulty in the reproduction of grey scales in these displays, especially when observed from an oblique viewing angle, as well as their inadeauate VHR and their inadeouate lifetime. The desired improvements of the transmission of the displays and of their response times are required in order to improve their energy efficiency, respectively their capacity to render rapidly moving pictures.

There thus continues to be a great demand for MLC displays having very high specific resistance at the same time as a large working-temperature range, short response times and a relatively low threshold voltage, with the aid of which various grey shades can be produced and which have, in particular, a good and stable VHR.

The invention has the object of providing MLC displays, not only for monior and TV applications, but also for mobile applications such as e.g. telephones and navigation systems, which are based on the ECB, IPS or FFS effect, do not have the disadvantages indicated above, or only do so to a lesser extent, and at the same time have very high specific resistance values. In particular, it must be ensured for mobile telephones and navigation systems that they also work at extremely high and extremely low temperatures.

Surprisingly, it has been found that it is possible to achieve liquid-crystal displays which have, in particular in IPS and FFS displays, a low threshold voltage with short response times, a sufficiently broad nematic phase, favourable birefringence ($\Delta$n) and, at the same time, a high transmission, high contrast, good stability to decomposition by heating and by UV exposure, and a stable, high VHR if use is made in these display elements of nematic liquid-crystal media, which comprise at least one compound, preferably two or more compounds, of formula Py, preferably additionally one or more compounds, preferably two or more compounds, selected from the group of the compounds of the formulae T, II and III, and/or one or more compounds, preferably two or more compounds selected from the group of formulae IV and/or V.

Media of this type can be used, in particular, for electro-optical displays having active-matrix addressing for IPS - or FFS displays.

The media according to the present invention preferably additionally comprise a one or more compounds selected from the group of compounds of formulae II and III, preferably one or more compounds of formula II, more preferably in addition one or more compounds of formula III and, most preferably, additionally one or more compounds selected from the group of the compounds of formulae IV and V and, again preferably, one or more compounds selected from the group of compounds of formulae VI to IX (all formulae as defined below).

The mixtures according to the invention exhibit very broad nematic phase ranges with clearing points ≥ 70°C, very favourable values for the capacitive threshold, relatively high values for the holding ratio and at the same time good low-temperature stabilities at -20°C and -30°C, as well as very low rotational viscosities. The mixtures according to the invention are furthermore distinguished by a good ratio of clearing point and rotational viscosity and by a relatively high

positive dielectric anisotropy.

Now, it has been found surprisingly that LCDs of the FFS type using liquid crystals with positive dielectric anisotropy may be realised using specially selected liquid crystalline media. These media are characterised by a particular combination of physical properties. Most decisive amongst these are their dielectric properties and here a high average dielectric constant ($\varepsilon_{av.}$), a high dielectric constant perpendicular to the director of the liquid crystal molecules ($\varepsilon_{\perp}$) and, in particular, the relatively high ratio of these latter two values: ($\varepsilon_{\perp}$ / $\Delta\varepsilon$).

Preferably the liquid-crystalline media according to the present invention, on the one hand, have a value of the dielectric anisotropy of 1.5 or more, preferably of 2.5 or more. At the other hand, they preferably have a dielectric anisotropy of 26 or less, preferably of 15 or less and most preferabyl of 10 or less.

The liquid crystalline media according to the present invention in a preferred embodiment have a positive dielectric anisotropy, preferably in the range from 1.5 or more to 20.0 or less, more preferably in the range from 2.0 or more to 15.0 or less and, most preferably in the range from 2.0 or more to 12.0.

The liquid crystalline medium of the present invention, as in claim 1 and defined below, comprises

a) one or more compounds of formula Py, preferably in a concentration in the range from 1 % to 40 %, more preferably in the range from 2 % to 30 %, particularly preferably in the range from 3 % to 20 %,

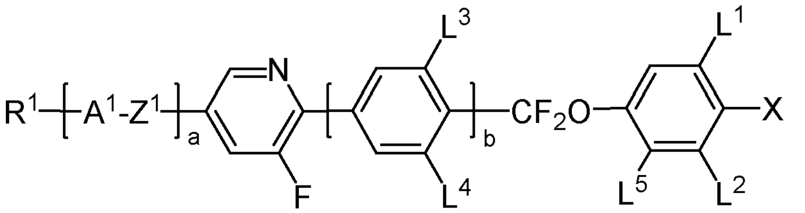 Py

in which

$R^1$ an alkyl radical having 1 to 15 C atoms, wherein one or more $CH_2$ groups, including terminal C atoms, in this radical may each be replaced, independently of one another, by -C=C-, -CH=CH-,

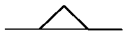 , 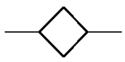 , 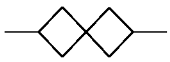 , 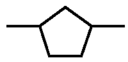 ,

-O-, -S-, -(CO)-O-, -O-(CO)- in such a way that O or S atoms are not linked directly to one another, and in which, in addition, one or more H atoms may be replaced by F or Cl, or H,

$A^1$ on each appearance, independently of one another, denotes

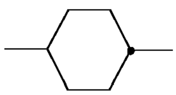 , 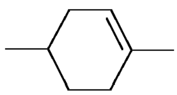 , 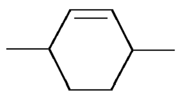 ,

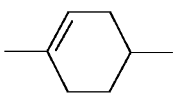 , 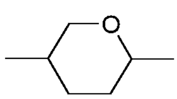 , 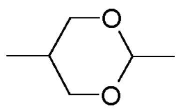 ,

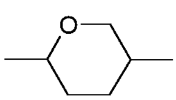 , 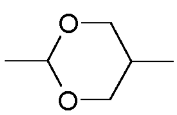 ,

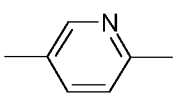 , 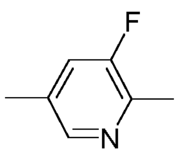 , 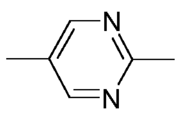 ,

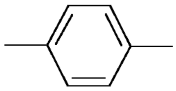, 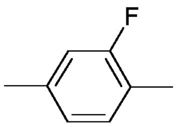, 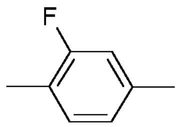,

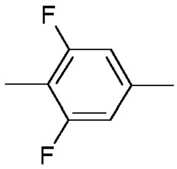 or 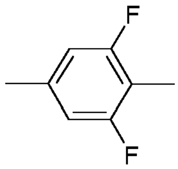,

preferably

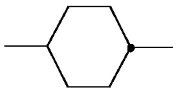, 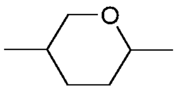, 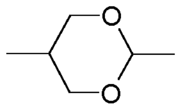,

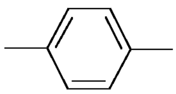, 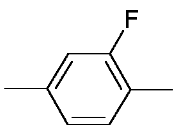, 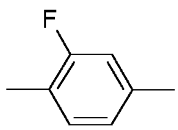,

or 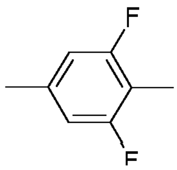,

| | |
|---|---|
| $Z^1$ | independently of one another, identically or differently, denotes a single bond, $-CH_2O-$, -(CO)O-, $-CF_2O-$, -CH2CH2CF$_2$O-, $-CF_2CF_2-$, -CH2CF2-, -CH2CH2-, $-(CH_2)_4-$, -CH=CH-, -CH=CF-, -CF=CF- or -C≡C-, where asymmetrical bridges may be oriented to both sides, |
| $L^1$, $L^2$, $L^3$, $L^4$ | independently H, For Cl, |
| $L^5$ | H, $CH_3$ or $CH_2CH_3$, preferably H, |
| X | F, Cl, $-CF_3$, $-OCF_3$, -CN or -SCN, |
| a | denotes 0, 1 or 2, preferably 1, |
| b | denotes 0, 1 or 2, preferably 1, and |

where a + b is ≤ 3, is preferably equal to 1, 2 or 3, particularly preferably 1 or 2,

and one or more additional compounds, preferably selected from the groups preferably dielectrically neutral, of formula T

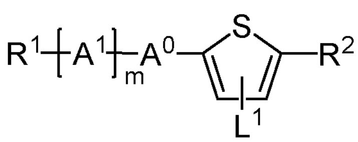 T

in which the individual radicals have the following meanings:

$R^1$ and $R^2$ denote H, F, Cl, Br, -CN, -NCS or straight-chain or branched alkyl having 1 to 12 C atoms, in which, in addition, one or more $CH_2$ groups, including terminal C atoms, may each be replaced, independently of one another, by -CH=CH-, -C≡C-,

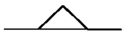 , 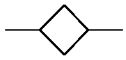 ,

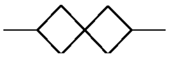 , 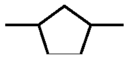 ,

-O-, -S-, -CO-, -CO-O-, -O-CO-, in such a way that O or S atoms are not linked directly to one another, and in which, in addition, one or more H atoms may be replaced by F or Cl,

$L^1$ H or $CH_3$, preferably H,

$A^0$ denotes

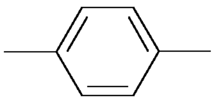 , 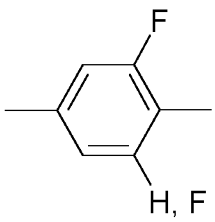 , 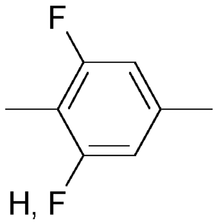 ,

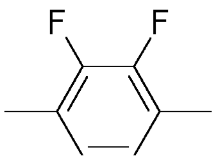 or 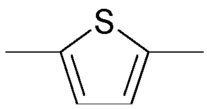

$A^1$, independently of one another, denotes phenylene-1,4-diyl, in which, in addition, one or two CH groups may be replaced by N and one or more H atoms may be replaced by halogen, CN, $CH_3$, $CHF_2$, $CH_2F$, $OCH_3$, $OCHF_2$ or $OCF_3$, cyclohexane-1,4-diyl, in which, in addition, one or two non-adjacent $CH_2$ groups may be replaced, independently of one another, by O and/or S and one or more H atoms may be replaced by F, cyclohexene-1,4-diyl, bicyclo[1.1.1]pentane-1,3-diyl, bicyclo[2.2.2]octane-1,4-diyl, spiro[3.3]heptane-2,6-diyl, tetrahydropyran-2,5-diyl or 1,3-dioxane-2,5-diyl,

m in each case, independently of one another, denotes 0, 1 or 2,

and one or more additional compounds, preferably selected from the groups of compounds according to the following conditions c) to f):

c) one or more, preferably dielectrically positive, compounds selected from the group of compounds of formulae II and III, preferably of compounds having a dielectric anisotropy of greater than 3 each, preferably one or more compounds of formula II:

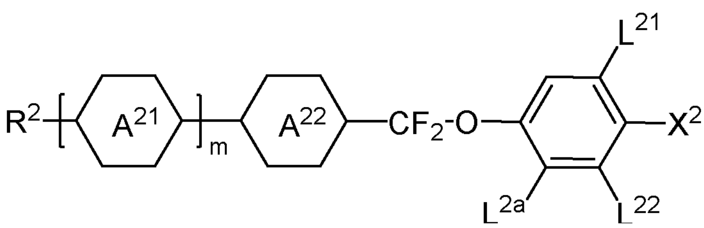

II

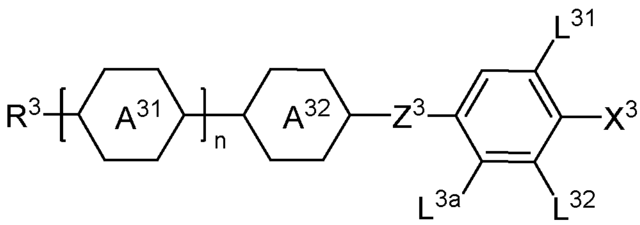

III

in which

$R^2$ an alkyl radical having 1 to 15 C atoms, wherein one or more $CH_2$ groups, including terminal C atoms, in this radical may each be replaced, independently of one another, by -C=C-, -CH=CH-,

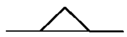 , 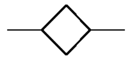 ,

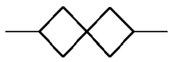 , 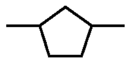 ,

-O-, -S-, -(CO)-O-, -O-(CO)- in such a way that O or S atoms are not linked directly to one another, and in which, in addition, one or more H atoms may be replaced by F or Cl, or H, preferably denotes alkyl, alkoxy, fluorinated alkyl or fluorinated alkoxy having 1 to 7 C atoms, alkenyl, alkenyloxy, alkoxyalkyl, fluorinated alkenyl having 2 to 7 C atoms, cycloalkyl with 3 to 5 C atoms, cyclalkylalkyl, cycloalkylalkoxy and most preferably alkyl, cyclopropyl, cyclopentyl or alkenyl,

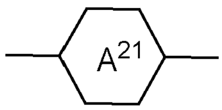 and 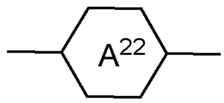

on each appearance, independently of one another, denote

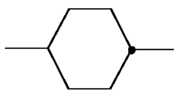 , 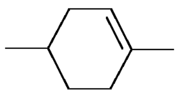 , 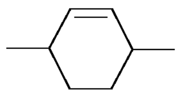 ,

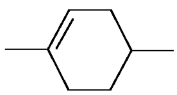 , 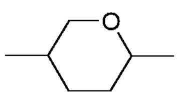 , 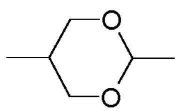 ,

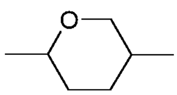 , 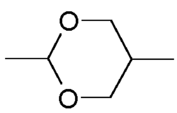 , 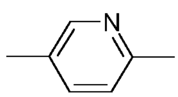 ,

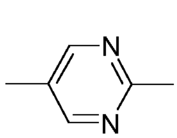 , 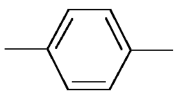 , 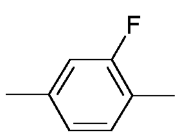 ,

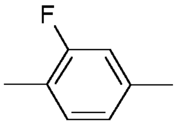, 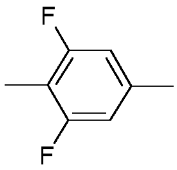 or 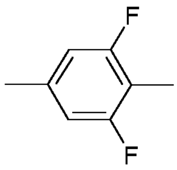,

preferably

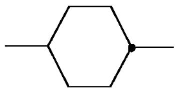, 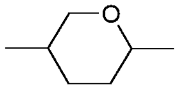, 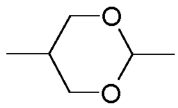,

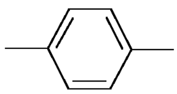, 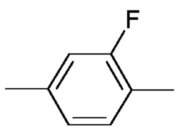, 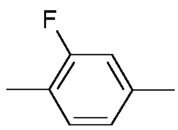,

or 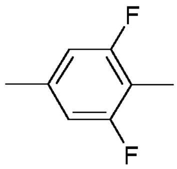,

$L^{21}$ and $L^{22}$ denote H or F,

$L^{2a}$ H or $CH_3$, preferably H,

$X^2$ denotes halogen, halogenated alkyl or alkoxy having 1 to 3 C atoms or halogenated alkenyl or alkenyloxy having 2 or 3 C atoms, preferably F, C!, $-OCF_3$, $-O-CH_2CF_3$, $-O-CH=CH_2$, $-O-CH=CF_2$ or $-CF_3$, very preferably F, Cl, $-O-CH=CF_2$ or $-OCF_3$,

m denotes 0, 1, 2 or 3, preferably 1 or 2 and particularly pref-erably 2,

$R^3$ an alkyl radical having 1 to 15 C atoms, wherein one or more $CH_2$ groups, including terminal C atoms, in this radical may each be replaced, independently of one another, by -C=C-, -CH=CH-,

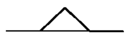, 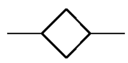,

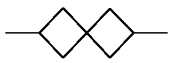, 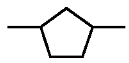,

-O-, -S-, -(CO)-O-, -O-(CO)- in such a way that O or S atoms are not linked directly to one another, and in which, in addition, one or more H atoms may be replaced by F or Cl, or H, preferably denotes alkyl, alkoxy, fluorinated alkyl or fluorinated alkoxy having 1 to 7 C atoms, alkenyl, alkenyloxy, alkoxyalkyl, fluorinated alkenyl having 2 to 7 C atoms, cycloalkyl with 3 to 5 C atoms, cycloalkylalkyl, cycloalkylalkoxy and most preferably alkyl, cyclopropyl, cyclopentyl or alkenyl,

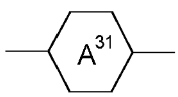 and 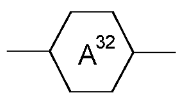

on each appearance, independently of one another, are

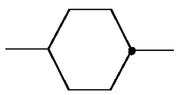, 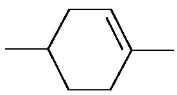, 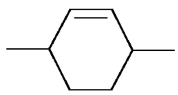,

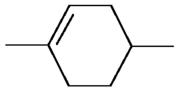, 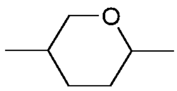, 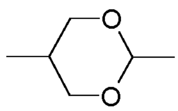,

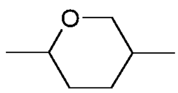, 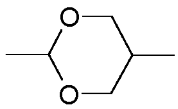,

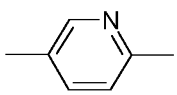, 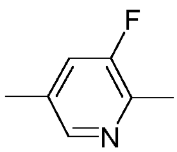, 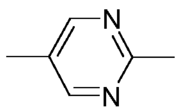,

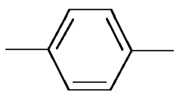, 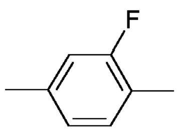, 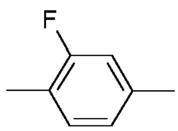,

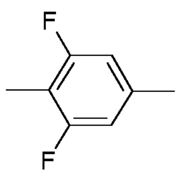 or 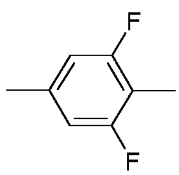,

preferably

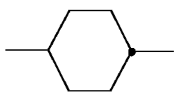, 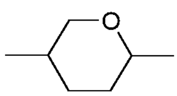, 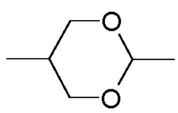,

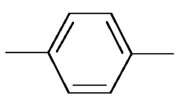, 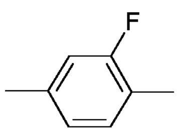, 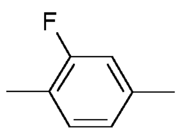,

or 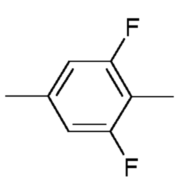,

$L^{31}$ and $L^{32}$, independently of one another, denote H or F, preferably $L^{31}$ denotes F,

$L^{3a}$ H or $CH_3$, preferably H,

$X^3$ denotes halogen, halogenated alkyl or alkoxy having 1 to 3 C atoms or halogenated alkenyl or alkenyloxy having 2 or 3 C atoms, F, Cl, $-OCF_3$, $-OCHF_2$, $-O-CH_2CF_3$, $-O-CH=CF_2$, $-O-CH=CH_2$ or $-CF_3$, very preferably F, Cl, $-O-CH=CF_2$, $-OCHF_2$ or $-OCF_3$,

$Z^3$ denotes $-CH_2CH_2-$, $-CF_2CF_2-$, -COO-, *trans*-CH=CH-, *trans*-CF=CF-, $-CH_2O-$ or a single bond, preferably $-CH_2CH_2-$, -COO-, *trans*-CH=CH- or a single bond and very preferably -COO-, *trans*-CH=CH- or a single bond, and

n denotes 0, 1, 2 or 3, preferably 1, 2 or 3 and particularly preferably 1,

d) one or more dielectrically neutral compounds selected from the group of formulae IV and V:

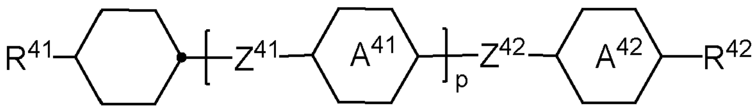

IV

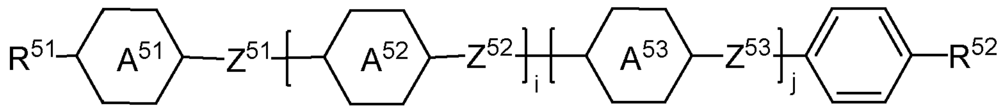

V

in which

$R^{41}$ and $R^{42}$, independently of one another, have the meaning indicated above for $R^3$ under formula III, preferably $R^{41}$ denotes alkyl and $R^{42}$ denotes alkyl, cyclopropyl, cyclopentyl or alkoxy or $R^{41}$ denotes alkenyl and $R^{42}$ denotes alkyl,

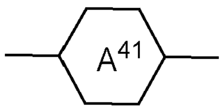 and 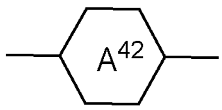

independently of one another and, if

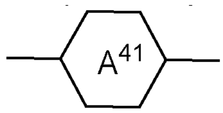

occurs twice, also these independently of one another, denote

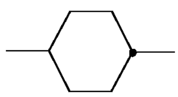 , 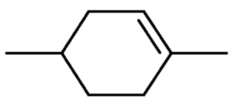 , 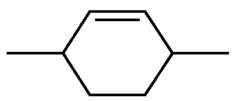 ,

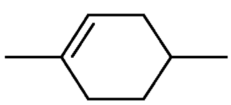 , 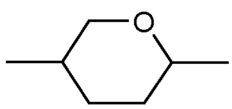 , 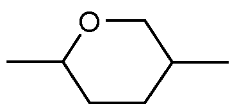 ,

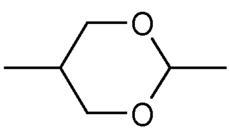 , 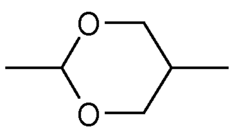 , 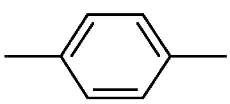 ,

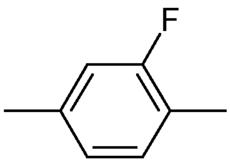, 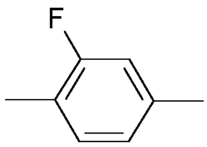, 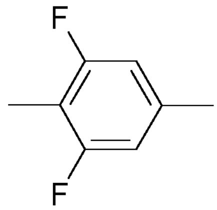,

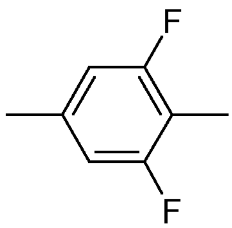 or 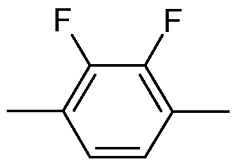,

preferably one or more of

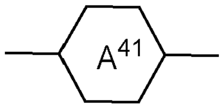 and 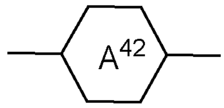

denotes or denote,

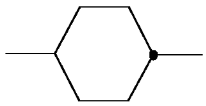

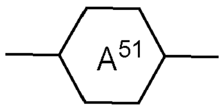 to 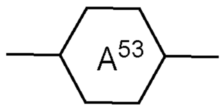,

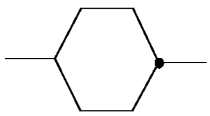, 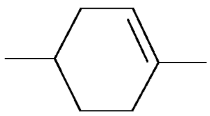, 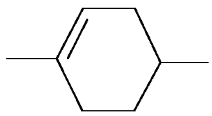,

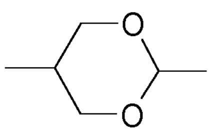, 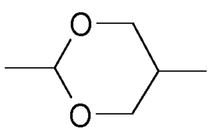, 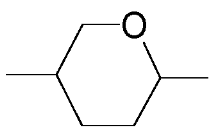,

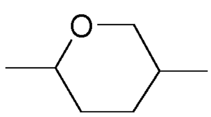, 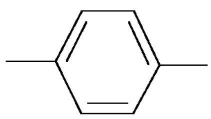,

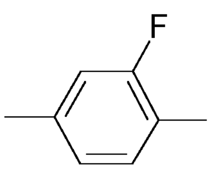 or 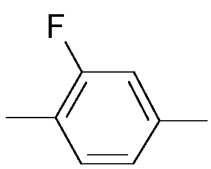,

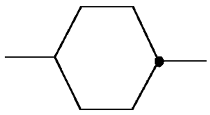, 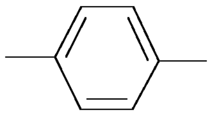, 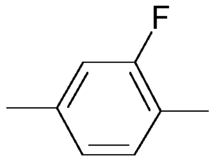

$Z^{41}$ and $Z^{42}$, independently of one another and, if $Z^{41}$ occurs twice, also these independently of one another, denote $-CH_2CH_2-$, -COO-, *trans*-CH=CH-, *trans*-CF=CF-, $-CH_2O-$, $-CF_2O-$, -C=C- or a single bond, preferably one or more thereof denotes/denote a single bond,

p denotes 0, 1 or 2, preferably 0 or 1, and

$R^{51}$ and $R^{52}$, independently of one another, have one of the meanings given for $R^{41}$ and $R^{42}$ and preferably denote alkyl having 1 to 7 C atoms, preferably *n*-alkyl, particularly preferably *n*-alkyl having 1 to 5 C atoms, alkoxy having 1 to 7 C atoms, preferably *n*-alkoxy, particularly preferably *n*-alkoxy having 2 to 5 C atoms, alkoxyalkyl, alkenyl or alkenyloxy having 2 to 7 C atoms, preferably having 2 to 4 C atoms, preferably alkenyloxy, preferably

or 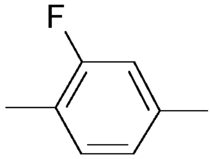,

$Z^{51}$ to $Z^{53}$ each, independently of one another, denote $-CH_2-CH_2-$, $-CH_2-O-$, -CH=CH-, -C=C-, -COO- or a single bond, preferably $-CH_2-CH_2-$, $-CH_2-O-$ or a single bond and particularly preferably a single bond,

i and j each, independently of one another, denote 0 or 1,

(i + j) preferably denotes 0, 1 or 2, more preferably 0 or 1, wherein the respective rings, and preferably the phenylene rings, optionally may each be substituted by one or two alkyl groups, preferably by methyl and/or ethyl groups, preferably by one methyl group, and

e) one or more dielectrically negative compounds selected from the group of formulae VI to IX:

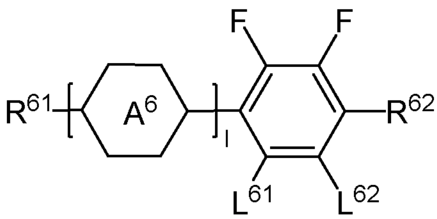

VI

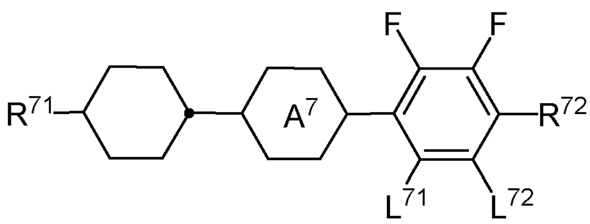

VII

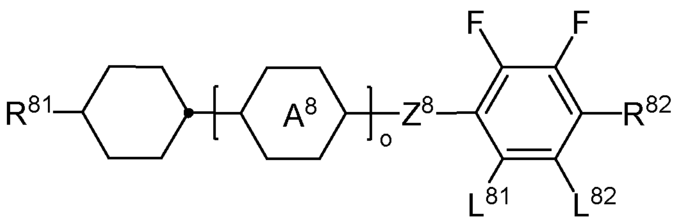

VIII

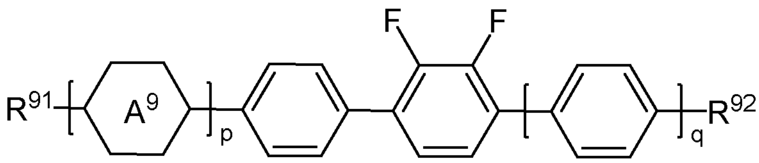

IX

wherein

$R^{61}$ denotes an unsubstituted alkyl radical having 1 to 7 C atoms, preferably a straight-chain alkyl radical, more preferably an *n*-alkyl radical, most preferably propyl or pentyl, an unsubstituted alkenyl radical having 2 to 7 C atoms, preferably a straight-chain alkenyl radical, particularly preferably having 2 to 5 C atoms, an unsubstituted alkoxy radical having 1 to 6 C atoms, an unsubstituted alkenyloxy radical having 2 to 6 C atoms or $C_{3-5}$-cycloalkyl-$(CH_2)_{0-1}$,

$R^{52}$ denotes an unsubstituted alkyl radical having 1 to 7 C atoms, an unsubstituted alkoxy radical having 1 to 6 C atoms, $C_{3-5}$-cycloalkyloxy or an unsubstituted alkenyloxy radical having 2 to 6 C atoms, and

$L^{61}$, $L^{62}$ independently H or methyl, preferably H,

I denotes 0 or 1,

$R^{71}$ denotes an unsubstituted alkyl radical having 1 to 7 C atoms, preferably a straight-chain alkyl radical, more preferably an *n*-alkyl radical, most preferably propyl or pentyl, an unsubstituted alkenyl radical having 2 to 7 C atoms, preferably a straight-chain alkenyl radical, particularly preferably having 2 to 5 C atoms or $C_{3-5}$-cycloalkyl-$(CH_2)_{0-1}$,

$R^{72}$ denotes an unsubstituted alkyl radical having 1 to 7 C atoms, preferably having 2 to 5 C atoms, an unsubstituted alkoxy radical having 1 to 6 C atoms, preferably having 1, 2, 3 or 4 C atoms, or an unsubstituted alkenyloxy radical having 2 to 6 C atoms, preferably having 2, 3 or 4 C atoms, and

$L^{71}$, $L^{72}$ independently H or methyl, preferably H,

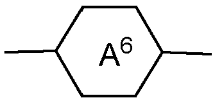

and

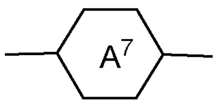

independently denote

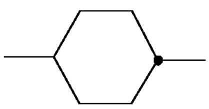

,

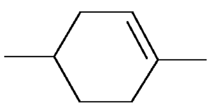

or

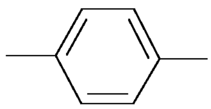

,

$R^{81}$ denotes an unsubstituted alkyl radical having 1 to 7 C atoms, preferably a straight-chain alkyl radical, more preferably an *n*-alkyl radical, most preferably propyl or pentyl, an unsubstituted alkenyl radical having 2 to 7 C atoms, preferably a straight-chain alkenyl radical, particularly preferably having 2 to 5 C atoms or $C_{3-5}$-cycloalkyl-$(CH_2)_{0-1}$,

$R^{82}$ denotes an unsubstituted alkyl radical having 1 to 7 C atoms, preferably having 2 to 5 C atoms, an unsubstituted alkoxy radical having 1 to 6 C atoms, preferably having 1, 2, 3 or 4 C atoms, an unsubstituted alkenyloxy radical having 2 to 6 C atoms, preferably having 2, 3 or 4 C atoms, or $C_{3-5}$-cycloalkyloxy,

$L^{81}$, $L^{82}$ independently H or methyl, preferably H,

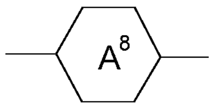

denotes

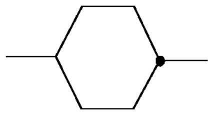 , 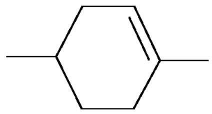 or 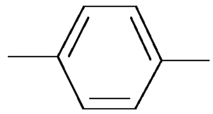 ,

preferably

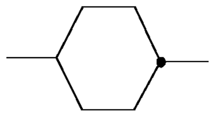 or 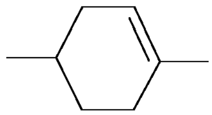 ,

more preferably

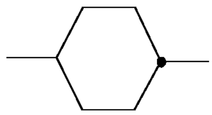 ,

$Z^8$ denotes -(C=O)-O-, $-CH_2-O-$, $-CF_2-O-$ or $-CH_2-CH_2-$, preferably -(C=O)-O- or $-CH_2-O-$, and

o denotes 0 or 1,

$R^{91}$ and $R^{92}$ independently of one another have the meaning given for $R^{72}$ above,

$R^{91}$ preferably denotes an alkyl radical having 2 to 5 C atoms, preferably having 3 to 5 C atoms,

$R^{92}$ preferably denotes an alkyl or alkoxy radical having 2 to 5 C atoms, more preferably an alkoxy radical having 2 to 4 C atoms, or an alkenyloxy radical having 2 to 4 C atoms.

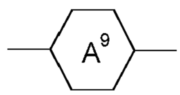

denotes

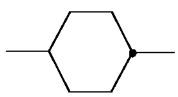 or 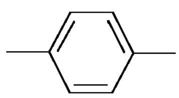 ,

p and q independently of each other denote 0 or 1, and

(p + q) preferably denotes 0 or 1,

in case

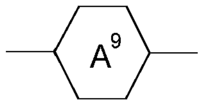

denotes

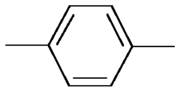 ,

preferably p = q = 1,

f) one or more compounds of formula X, preferably in a concentration in the range from 1 % to 30 %, more preferably in the range from 2 % to 20 %, particularly preferably in the range from 3 % to 10 %,

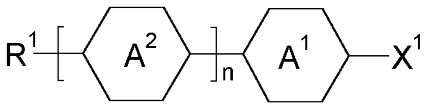 X

in which

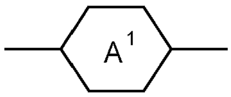

denotes

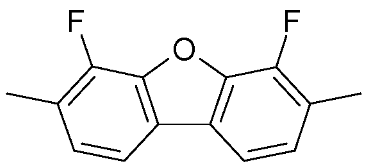

or

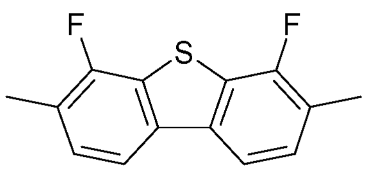 ,

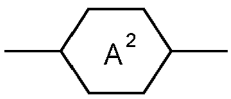

denotes, in each occurrence independently of one another,

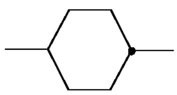 , 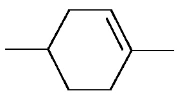 , 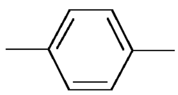 ,

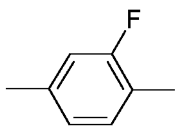 or 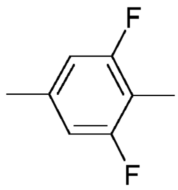 ,

preferably

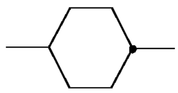 or 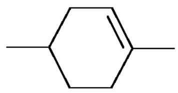 ,

n denotes 1 or 2, preferably 1,

$R^1$ denotes alkyl, alkoxy, fluorinated alkyl or fluorinated alkoxy, preferably having 1 to 7 C atoms, alkenyl, alkenyloxy, alkoxyalkyl or fluorinated alkenyl having 2 to 7 C atoms, $C_{3-5}$-cycloalkyl, $C_{3-5}$-cycloalkyl-alkyl, $C_{3-5}$-cycloalkyl-alkyloxy, preferably alkyl, alkoxy, alkenyl or alkenyloxy, more preferably alkyl, alkenyl, alkoxy or alkenyloxy, and, most preferably alkyl, and

$X^1$ denotes F, Cl, fluorinated alkyl, fluorinated alkenyl, fluorinated alkoxy or fluorinated alkenlyoxy, the latter four groups preferably having 1 to 4 C atoms, preferably F, Cl, $CF_3$ or $OCF_3$,

wherein the respective rings, and preferably the phenylene rings, optionally may each be substituted by one or two alkyl groups, preferably by methyl and/or ethyl groups, preferably by one methyl group, and
g) again optionally, preferably obligatory, either alternatively or additionally, one or more compounds of formula XI:

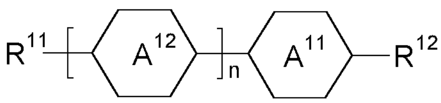

XI

in which

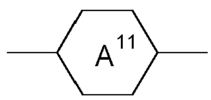

denotes

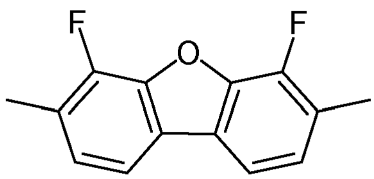 , 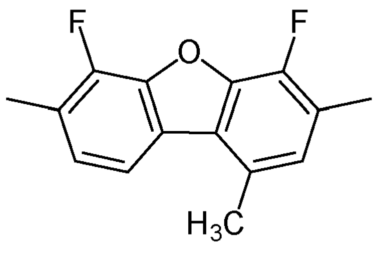

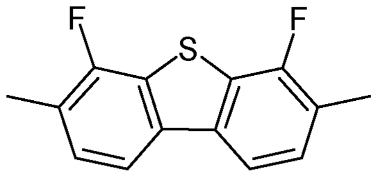,

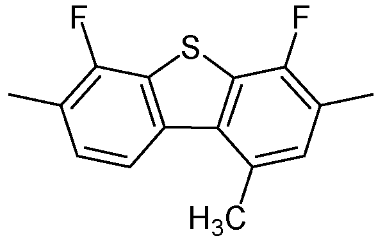

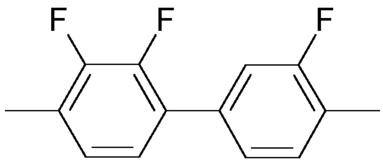,

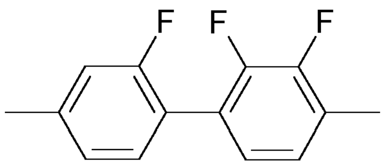,

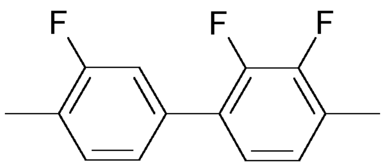,

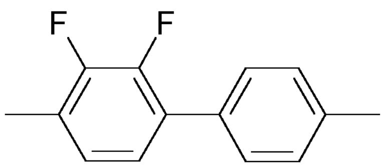,

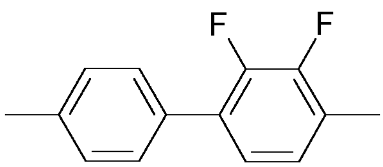,

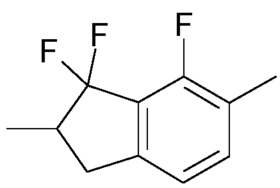

or

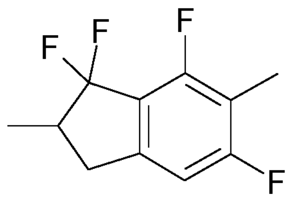

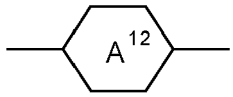

denotes

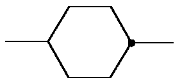 , 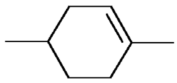 , 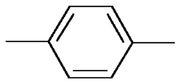 ,

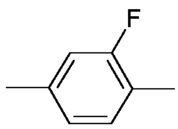 or 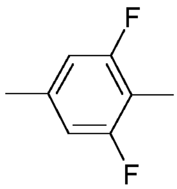 ,

preferably

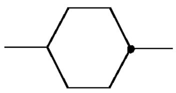 , 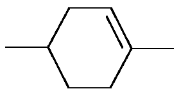 , 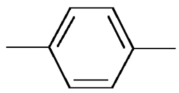

or

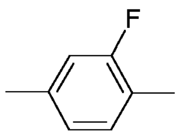 ,

n denotes 0 or 1,

$R^{11}$ and $R^{12}$ independently of each other denote alkyl, alkoxy, fluorinated alkyl or fluorinated alkoxy, preferably having 1 to 7 C atoms, wherein one $CH_2$ group may be replaced by a 1,2-cyclopropyl group, by a 1,3-cyclopentyl group or by a 1,3-cyclopentenylene group, alkenyl, alkenyloxy, alkoxyalkyl or fluorinated alkenyl having 2 to 7 C atoms and preferably alkyl, alkoxy, alkenyl or alkenyloxy, most preferably alkyl, alkoxy or alkenyloxy,

wherein the respective rings, and preferably the phenylene rings, optionally may each be substituted by one or two alkyl groups, preferably by methyl and/or ethyl groups, preferably by one methyl group,
and from which the compounds of formula X are excluded.

Optionally the media according to the present application comprise one or more compounds of more compounds of formula L, preferably in a concentration in the range from 1 % to 40 %, more preferably in the range from 2 % to 30 %, particularly preferably in the range from 3 % to 20 %,

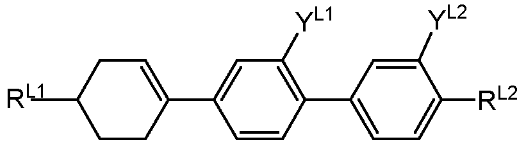

L

in which

$R^{L1}$ and $R^{L2}$, independently of one another, denote, an alkyl radical having 1 to 15 C atoms, wherein one or more $CH_2$ groups, preferably one $CH_2$ group, in these radicals may each be replaced, independently of one another, by -C≡C-, $-CF_2O-$, $-OCF_2-$, -O-, -(CO)-O-, -O-(C=O)-, cyclo-propylene, 1,3-cyclobutylene, 1,3-cyclopentylene, 1,3-cyclo-pentenylene, preferably by cyclopropylene or 1,3-cyclopentylene, preferably one $CH_2$ group may be replaced by a 1,2-cyclopropyl group, by a 1,3-cyclopentyl group or by a 1,3-cyclopentenylene group, alkenyl, alkenyloxy, alkoxyalkyl or fluorinated alkenyl having 2 to 7 C atoms and preferably alkyl or alkenyl, wherein one $-CH_2-$ group may be replaced by cyclo-propylene, 1,3-cyclobutylene, 1,3-cyclopentylene, 1,3-cyclo-pentenylene, preferably by cyclopropylene or 1,3-cyclopentenylene, in such a way that O atoms are not linked directly to one another, and in which one or more H atoms may be replaced by halogen, and

$Y^{L1}$ and $Y^{L2}$, identically or differently, denote H, F or Cl, preferably at least one of $Y^{L1}$ and $Y^{L2}$ is H, preferably $Y^{L2}$ is H, and most preferably $Y^{L1}$ and $Y^{L2}$ are H.

The liquid-crystalline media in accordance with the present application preferably have a nematic phase.

Throughout this application and especially for the definition of $R^1$, $R^2$, $R^3$, $R^{41}$, $R^{51}$, $R^{L1}$, etc. and $R^{L2}$ alkyl means an alkyl group, which may be straight-chain or branched. Each of these radicals is preferably straight-chain and preferably has 1, 2, 3, 4, 5, 6, 7 or 8 C atoms and is accordingly preferably methyl, ethyl, *n*-propyl, *n*-butyl, *n*-pentyl, *n*-hexyl or *n*-heptyl.

In case alkyl means a branched alkyl group it preferably means 2-alkyl, 2-methylalkyl or 2-(2-ethyl)-alkyl, preferably 2-butyl (=1-methylpropyl), 2-methylbutyl, 2-methylpentyl, 3-methylpentyl, 2-ethylhexyl, 2-propylpentyl, in particular 2-methylbutyl, 2-methylbutoxy 4-methylhexyl, 2-hexyl, 2-octyl, 2-nonyl, 2-decyl and 2-dodecyl. Most preferred of these groups are 2-hexyl and 2-octyl.

Respective branched groups which lead to chiral compounds are also called chiral groups in this application. Particularly preferred chiral groups are 2-alkyl, 2-alkoxy, 2-methylalkyl, 2-methylalkoxy, 2-fluoroalkyl, 2-fluoroalkoxy, 2-(2-ethin)-alkyl, 2-(2-ethin)-alkoxy, 1,1,1-trifluoro-2-alkyl and 1,1,1-trifluoro-2-alkoxy.

Particularly preferred chiral groups are 2-butyl (=1-methylpropyl), 2-methylbutyl, 2-methylpentyl, 3-methylpentyl, 2-ethylhexyl, 2-propylpentyl, in particular 2-methylbutyl, 2-methylbutoxy, 2-methylpentoxy, 3-methylpentoxy, 2-ethylhexoxy, 1-methylhexoxy, 2-octyloxy, 2-oxa-3-methylbutyl, 3-oxa-4-methylpentyl, 4-methylhexyl, 2-hexyl, 2-octyl, 2-nonyl, 2-decyl, 2-dodecyl, 6-methoxyoctoxy, 6-methyloctoxy, 6-methyloctanoyloxy, 5-methylheptyloxycarbonyl, 2-methylbutyryloxy, 3-methylvaleroyloxy, 4-methylhexanoyloxy, 2-chlorpropionyloxy, 2-chloro-3-methylbutyryloxy, 2-chloro-4-methylvaleryloxy, 2-chloro-3-methylvaleryloxy, 2-methyl-3-oxapentyl, 2-methyl-3-oxahexyl, 1-methoxypropyl-2-oxy, 1-ethoxypropyl-2-oxy, 1-propoxypropyl-2-oxy, 1-butoxypropyl-2-oxy, 2-fluorooctyloxy, 2-fluorodecyloxy, 1,1,1-trifluoro-2-octyloxy, 1,1,1-trifluoro-2-octyl, 2-fluoromethyloctyloxy for example. Very preferred are 2-hexyl, 2-octyl, 2-octyloxy, 1,1,1-trifluoro-2-hexyl, 1,1,1-trifluoro-2-octyl and 1,1,1-trifluoro-2-octyloxy.

Throughout this application and especially for the definition of $R^D$ alkenyl means an alkenyl group, which may be straight-chain or branched and preferably is straight chain and preferably has 2, 3, 4, 5, 6 or 7 or 8 C atoms. Preferabl it is vinyl, 1-*E*-alkenyl or 3-*E*-alkenyl, most preferably it is vinyl, 1-*E*-propenyl, 1-*E*-butenyl, 1-*E*-pentenyl, 3-butenyl oder 3-*E*-pentenyl.

The compounds of the general formula Py, T and II to X are prepared by methods known per se, as described in the literature (for example in the standard works, such as Houben-Weyl, Methoden der organischen Chemie [Methods of Organic Chemistry], Georg-Thieme-Verlag, Stuttgart), to be precise under reaction conditions which are known and are suitable for the said reactions. Use can be made here of variants which are known per se, but are not mentioned here in greater detail.

The compounds of formula Py may be synthesized as disclosed in EP 2137154 A2 and as in the synthesis examples.

The compounds of general formula T are preferably synthesized as disclosed in WO 2012/013281 A1.

The invention furthermore relates to a liquid-crystal display containing a liquid-crystalline medium according to the invention, in particular an IPS or FFS display, particularly preferably a FFS or SG-FFS display.

The invention furthermore relates to a liquid-crystal display of the IPS or FFS type comprising a liquid-crystal cell consisting of two substrates, where at least one substrate is transparent to light and at least one substrate has an

electrode layer, and a layer, located between the substrates, of a liquid-crystalline medium comprising a polymerised component and a low-molecular-weight component, where the polymerised component is obtainable by polymerisation of one or more polymerisable compounds in the liquid-crystalline medium between the substrates of the liquid-crystal cell, preferably with application of an electrical voltage and where the low-molecular-weight component is a liquid-crystal mixture according to the invention as described above and below.

The displays in accordance with the present invention are preferably addressed by an active matrix (active matrix LCDs, AMDs for short), preferably by a matrix of thin-film transistors (TFTs). However, the liquid crystals according to the invention can also be used in an advantageous manner in displays having other known addressing means.

The invention furthermore relates to a process for the preparation of a liquid-crystalline medium according to the invention by mixing one or more compounds of formula Py or its subformulae with one or more low-molecular-weight liquid-crystalline compounds, or a liquid-crystal mixture and optionally with further liquid-crystalline compounds and/or additives.

The following meanings apply above and below:

The term "FFS" is, unless indicated otherwise, used to represent FFS and SG-FFS displays.

The term "mesogenic group" is known to the person skilled in the art and is described in the literature, and denotes a group which, due to the anisotropy of its attracting and repelling interactions, essentially contributes to causing a liquid-crystalline (LC) phase in low-molecular-weight or polymeric substances. Compounds containing mesogenic groups (mesogenic compounds) do not necessarily have to have a liquid-crystalline phase themselves. It is also possible for mesogenic compounds to exhibit liquid-crystalline phase behaviour only after mixing with other compounds and/or after polymerisation. Typical mesogenic groups are, for example, rigid rod- or disc-shaped units. An overview of the terms and definitions used in connection with mesogenic or liquid-crystalline compounds is given in Pure Appl. Chem. 73(5), 888 (2001) and C. Tschierske, G. Pelzl, S. Diele, Angew. Chem. 2004, 116, 6340-6368.

The term "spacer group" or "spacer" for short, also referred to as "Sp" above and below, is known to the person skilled in the art and is described in the literature, see, for example, Pure Appl. Chem. 73(5), 888 (2001) and C. Tschierske, G. Pelzl, S. Diele, Angew. Chem. 2004, 116, 6340-6368. Unless indicated otherwise, the term "spacer group" or "spacer" above and below denotes a flexible group which connects the mesogenic group and the polymerisable group(s) to one another in a polymerisable mesogenic compound.

For the purposes of this invention, the term "liquid-crystalline medium" is intended to denote a medium which comprises a liquid-crystal mixture and one or more polymerisable compounds (such as, for example, reactive mesogens). The term "liquid-crystal mixture" (or "host mixture") is intended to denote a liquid-crystalline mixture which consists exclusively of unpoly-merisable, low-molecular-weight compounds, preferably of two or more liquid-crystalline compounds and optionally further additives, such as, for example, chiral dopants or stabilisers.

Particular preference is given to liquid-crystal mixtures and liquid-crystalline media which have a nematic phase, in particular at room temperature.

In a preferred embodiment of the present invention, the liquid-crystal medium comprises one or more compounds, preferably having a dielectric anisotropy of greater than 20, selected from formula Py-A and more preferably from the group of the compounds of the formulae Py-1 to Py-5:

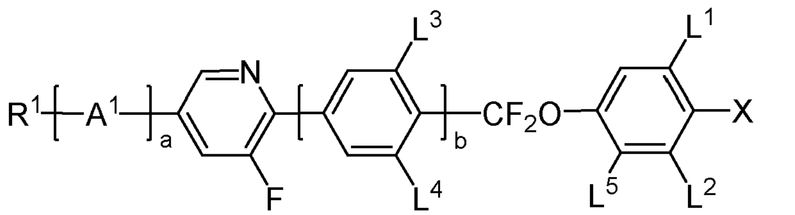

Py-A

alternatively

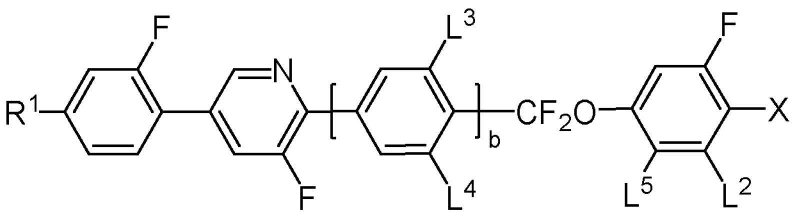

Py-1

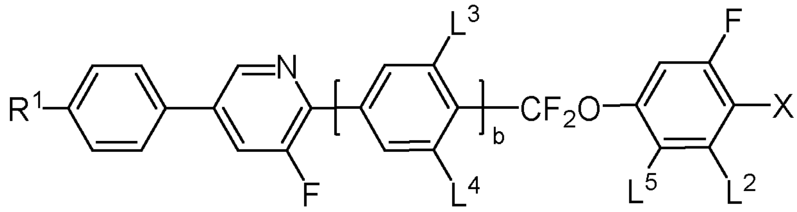 Py-2

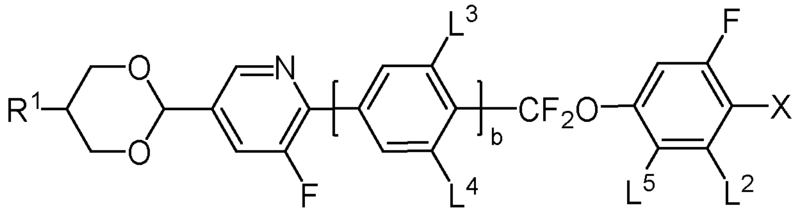 Py-3

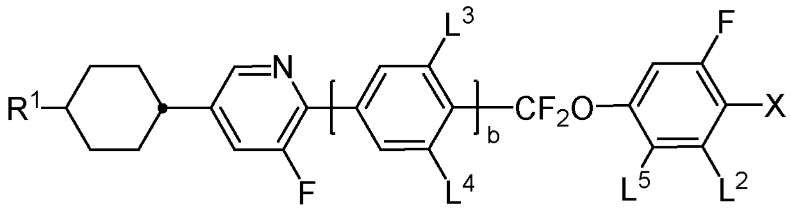 Py-4

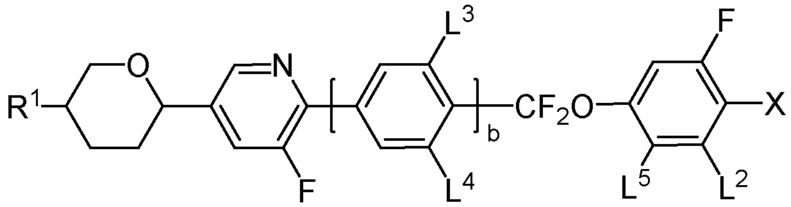 Py-5

wherein the variables are defined as in formula Py above and below.

Particular preference is given here to compounds of formula Py or Py-A in which a + b is 1, 2 or 3. In particular, b is 1 and a is 1. The group $A^1$ here preferably denotes

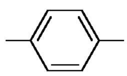 or 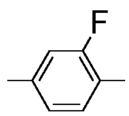 ,

particularly preferably

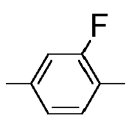 .

In the formulae Py, Py-A and Py-1 to Py-5 compounds are preferred, wherein independently:

b is 1,
$L^2$ is F,
$L^3$ is H or F, preferably F, and $L^4$ is F,
$L^5$ is H,
$R^1$ is an alkyl radical having 1 to 7 C atoms, wherein one or more $CH_2$ groups, including terminal C atoms, in this radical may each be replaced, independently of one another, by -C≡C-, -CH=CH-,

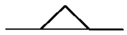 , 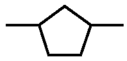 ,

or -O- in such a way that O atoms are not linked directly to one another, and in which, in addition, one or more H atoms may be replaced by F, preferably an alkyl radical having 2 to 7 C atoms.

In a preferred embodiment of the present invention the liquid crystalline medium comprises one or more compounds selected from the group of the compounds of the formulae T-1 to T-54:

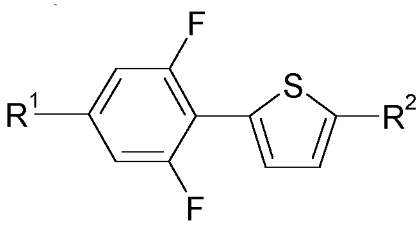

T-1

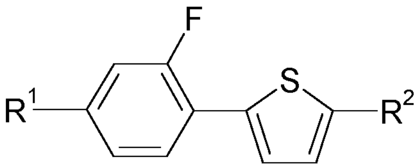

T-2

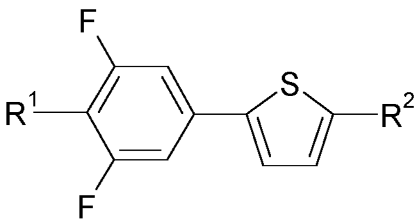

T-3

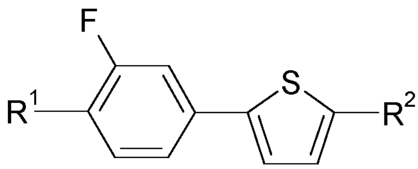

T-4

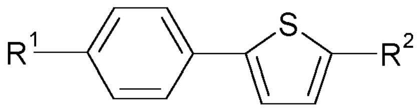

T-5

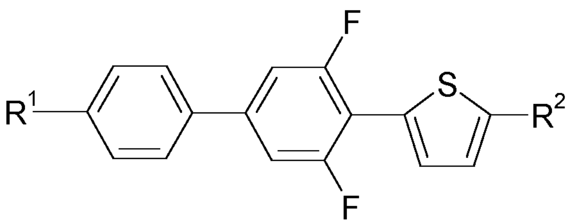

T-6

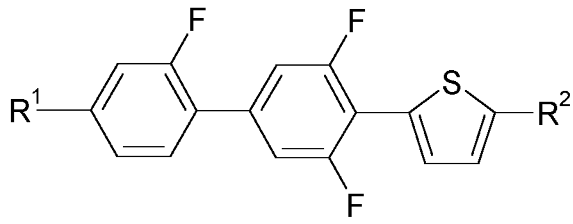

T-7

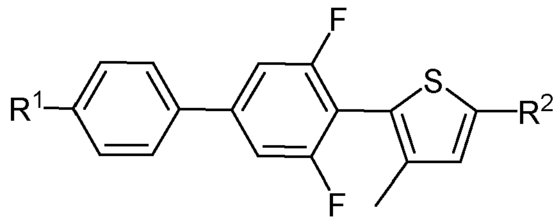

T-8

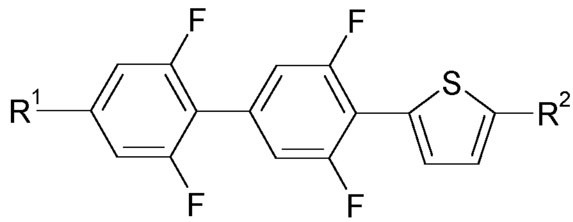

T-9

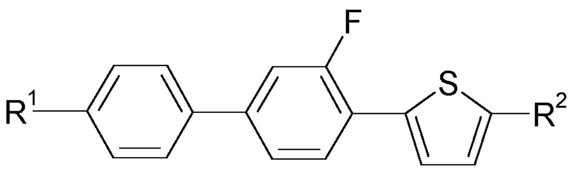

T-10

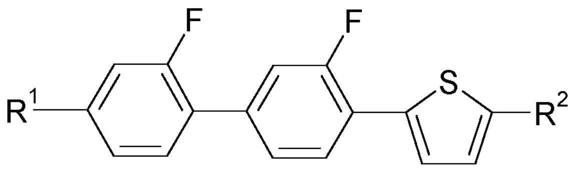

T-11

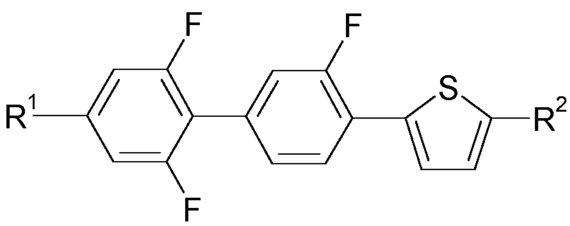

T-12

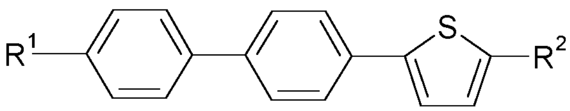

T-13

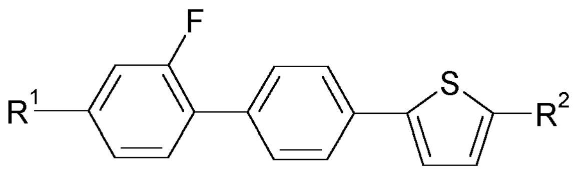

T-14

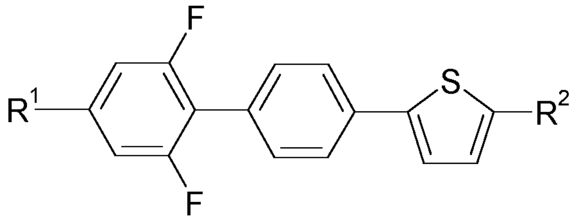

T-15

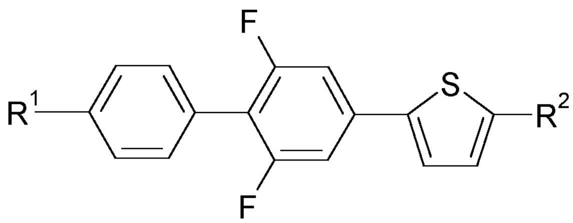

T-16

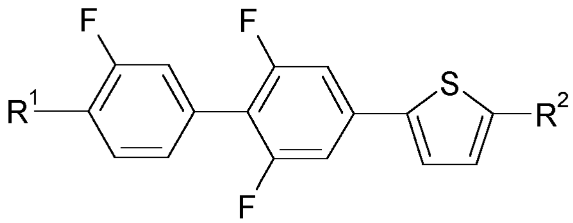

T-17

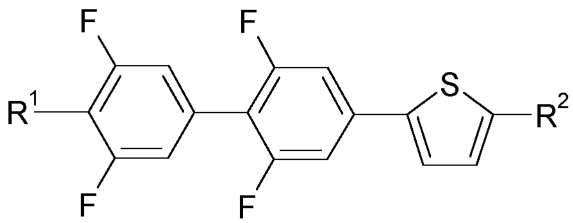

T-18

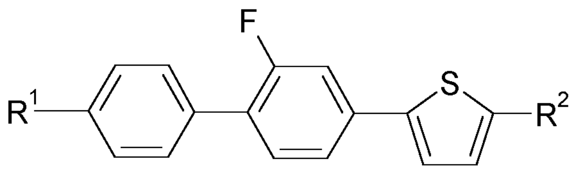

T-19

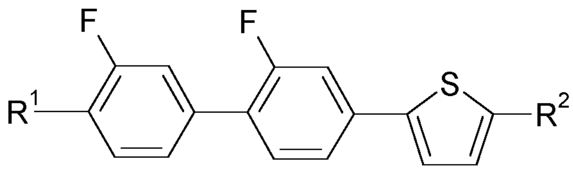

T-20

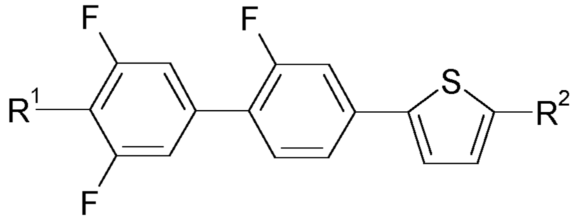

T-21

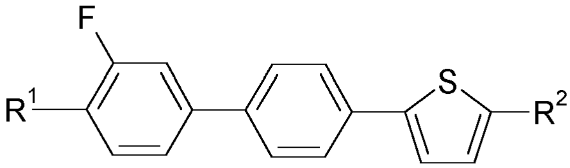

T-22

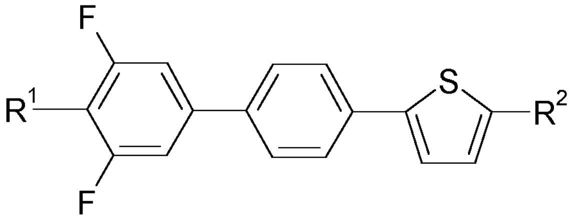

T-23

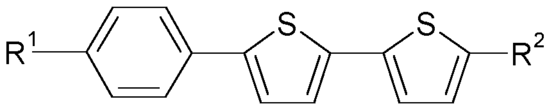

T-24

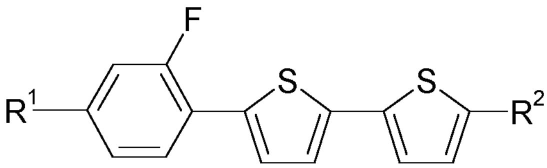

T-25

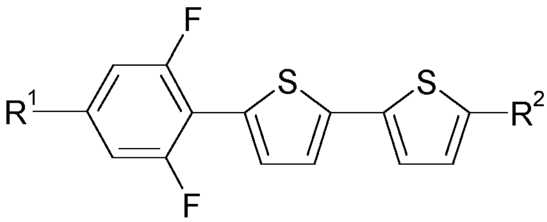

T-26

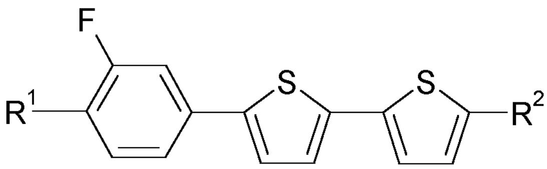

T-27

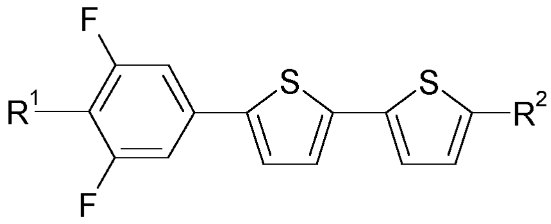

T-28

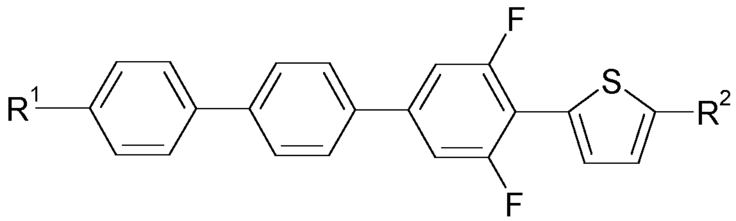

T-29

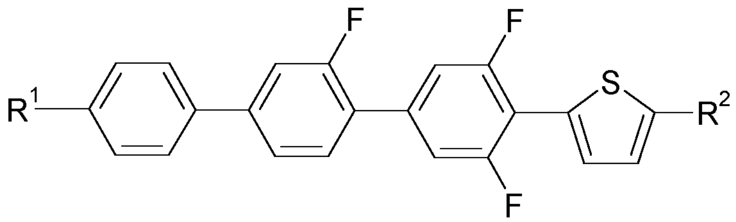

T-30

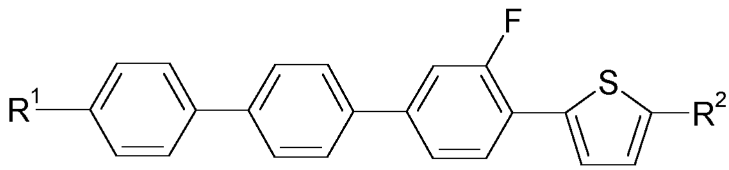

T-31

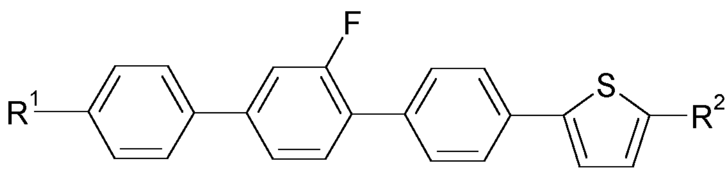

T-32

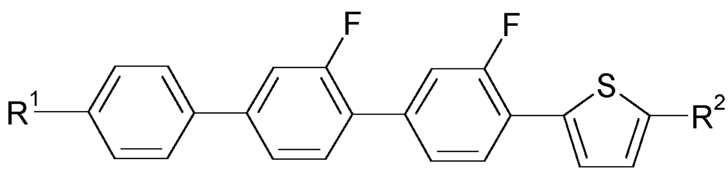

T-33

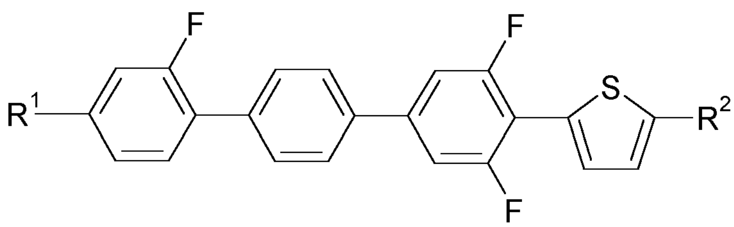

T-34

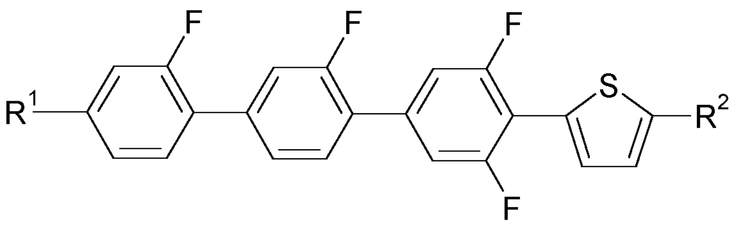

T-35

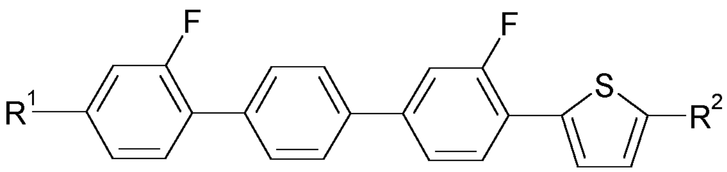

T-36

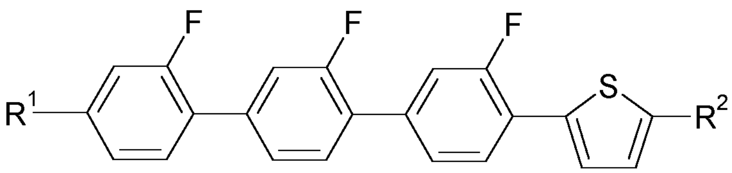

T-37

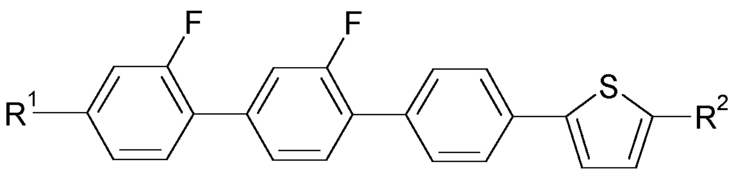

T-38

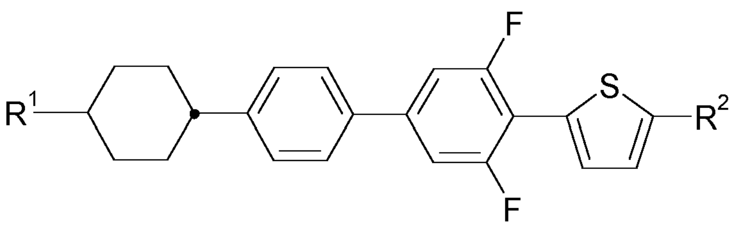

T-39

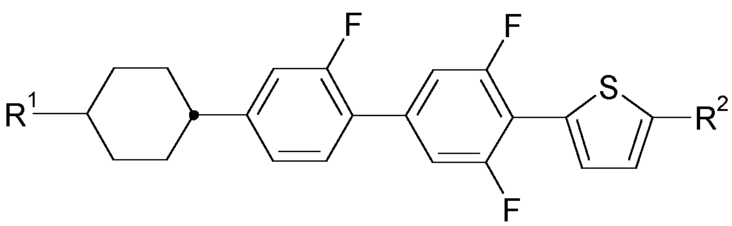

T-40

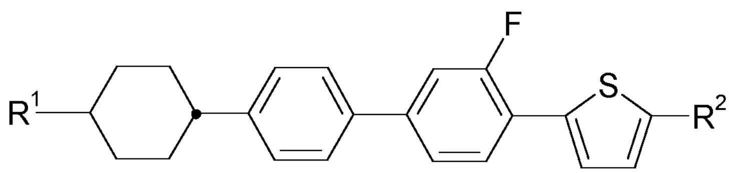

T-41

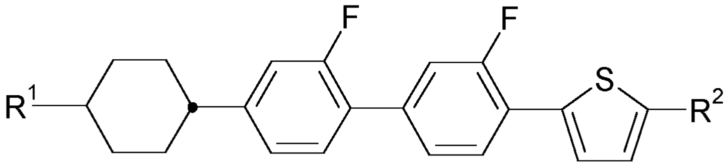

T-42

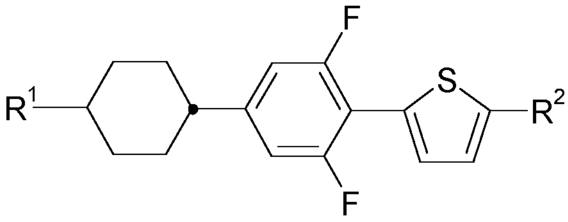

T-43

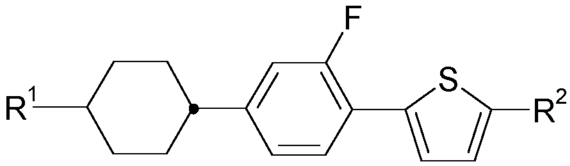

T-44

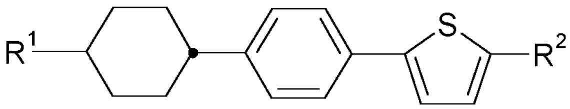

T-45

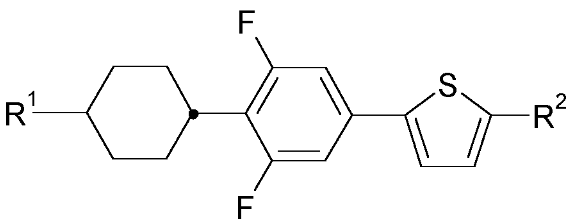

T-46

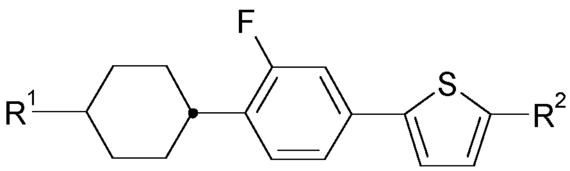

T-47

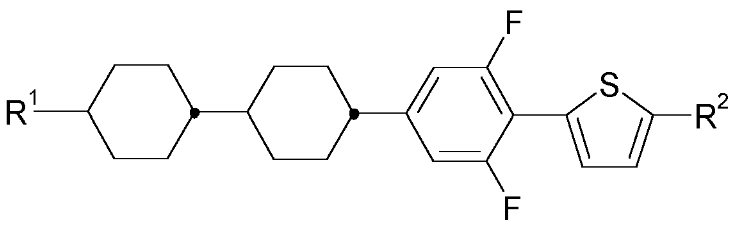

T-48

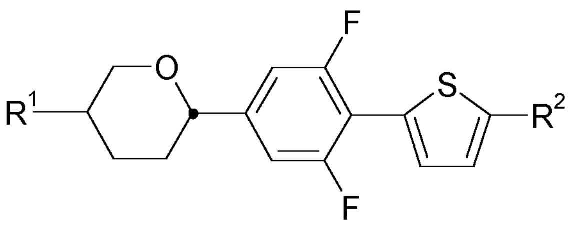

T-49

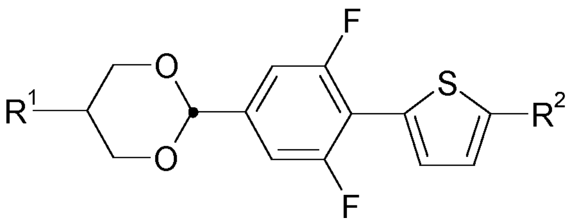

T-50

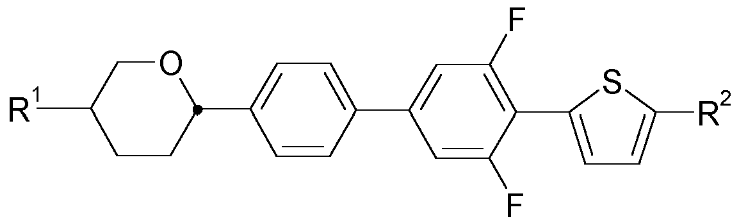

T-51

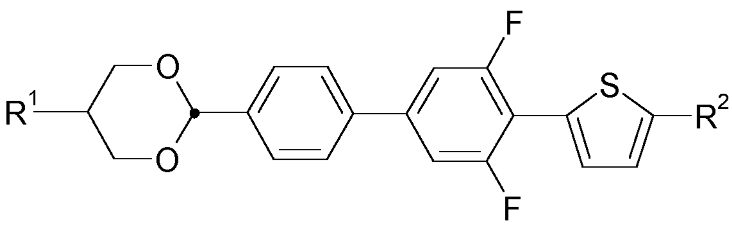

T-52

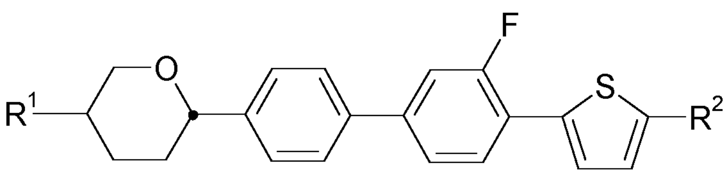

T-53

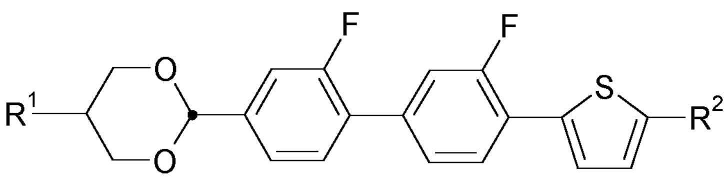

T-54

wherein $R^1$, $R^2$ are defined as for formula T above and below. Among the formulae, the compounds of formulae T-6, T-9 and T-38 are mostly preferred.

In the formula T and its more defined formulae above and below the group $R^1$ preferably is straight-chain or branched alkyl having 1 to 12 C atoms, in which, in addition, one or more $CH_2$ groups, including terminal C atoms, may each be replaced, independently of one another, by -CH=CH-, -C≡C-,

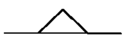 , 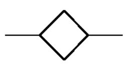 , 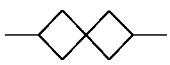 , 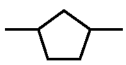 ,

-O-, -S-, -CO-, -CO-O-, -O-CO-, in such a way that O or S atoms are not linked directly to one another, and in which, in addition, one or more H atoms may be replaced by F or Cl, more preferably alkyl with 1 to 7 C atoms or alkenyl with 2 to 7 C atoms, cyclopentylmethyl or cyclopentyl.

In the formula T and its more defined formulae above and below the group $R^2$ preferably is straight-chain or branched alkyl having 1 to 12 C atoms, in which, in addition, one or more $CH_2$ groups, including terminal C atoms, may each be replaced, independently of one another, by -CH=CH-, -C≡C-,

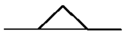 , 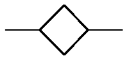 , 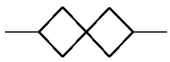 , 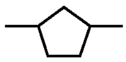 ,

-O-, -S-, -CO-, -CO-O-, -O-CO-, in such a way that O or S atoms are not linked directly to one another, a group $-CF_3$, $-OCF_3$ or F, more preferably methyl, ethyl, n-propyl, n-butyl, $CF_3$ or F, most preferably methyl, ethyl or propyl.

In a preferred embodiment of the present invention, the liquid-crystal medium comprises one or more, preferably dielectrically positive, compounds, preferably having a dielectric anisotropy of greater than 3, selected from the group of the compounds of the formulae II-1 and II-2:

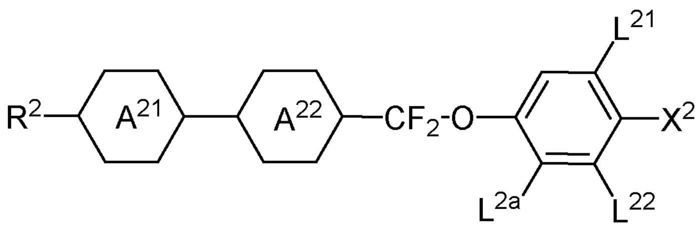

II-1

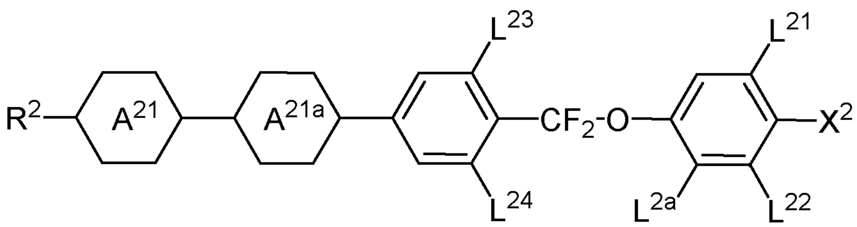

II-2

in which the parameters have the respective meanings indicated above under formula II, and $L^{23}$ and $L^{24}$, independently of one another, denote H or F, preferably $L^{24}$ denotes F, and

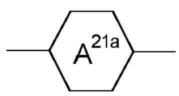

has one of the meanings given for

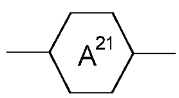

and, in the case of formulae II-1 and II-2, $X^2$ preferably denotes F or $OCF_3$, particularly preferably F, and, in the case of formula II-2,

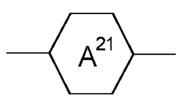

and

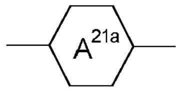

, independently of one another, preferably denote

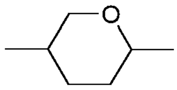

or

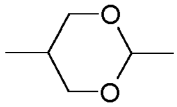

,

and/or selected from the group of the compounds of the formulae III-1 and III-2:

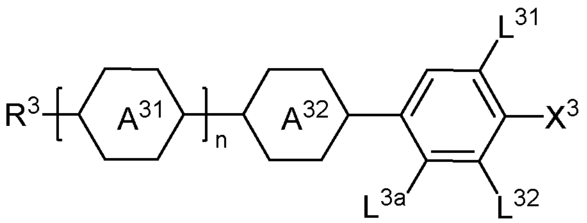

III-1

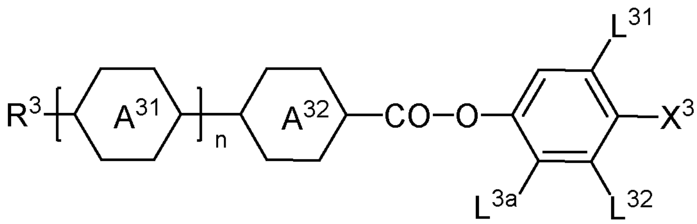

III-2

in which the parameters have the meanings given under formula III,

and the media in accordance with the present invention may comprise, alternatively or in addition to the compounds of the formulae III-1 and/or III-2, one or more compounds of the formula III-3

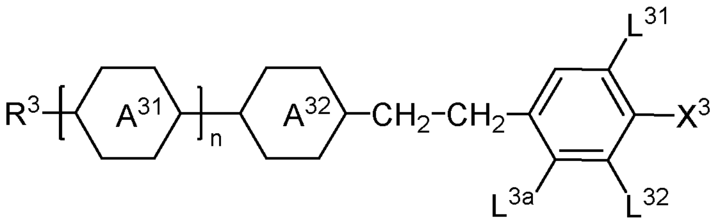

III-3

in which the parameters have the respective meanings indicated above, and the parameters $L^{31}$ and $L^{32}$, independently of one another and of the other parameters, denote H or F.

The liquid-crystal medium preferably comprises compounds selected from the group of the compounds of the formulae II-1 and II-2 in which $L^{21}$ and $L^{22}$ and/or $L^{23}$ and $L^{24}$ both denote F.

In a preferred embodiment, the liquid-crystal medium comprises compounds selected from the group of the compounds of the formulae II-2 in which $L^{21}$, $L^{22}$, $L^{23}$ and $L^{24}$ all denote F.

The liquid-crystal medium preferably comprises one or more compounds of the formula II-1. The compounds of the formula II-1 are preferably selected from the group of the compounds of the formulae II-1 a to II-1 e, preferably one or more compounds of formulaeII-1a and/or II-1b and/or II-1d, preferably of formula II-1a and/or II-1d or II-1b and/or II-1d, most preferably of formula II-1d:

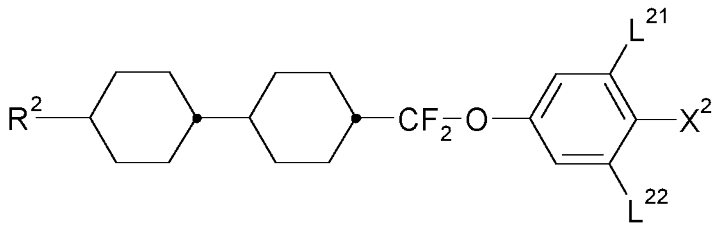

II-1a

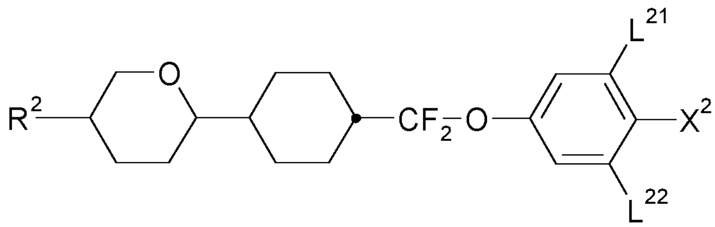

II-1b

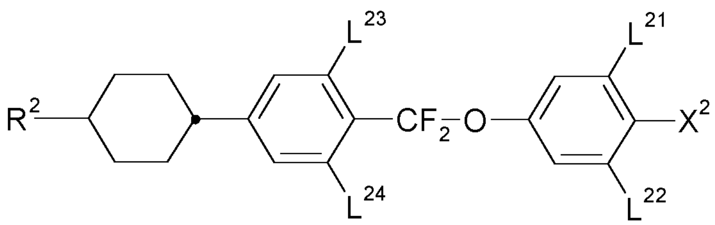

II-1c

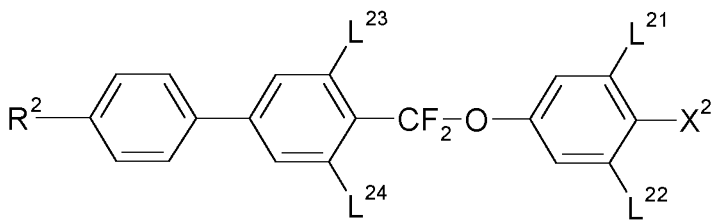

II-1d

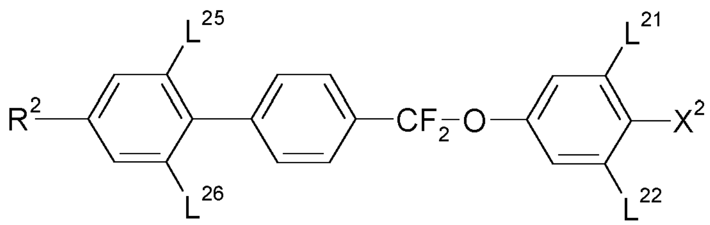

II-1e

in which the parameters have the respective meanings indicated above, and $L^{25}$ and $L^{26}$, independently of one another and of the other parameters, denote H or F, and preferably
in the formulae II-1a and II-1b,
$L^{21}$ and $L^{22}$ both denote F,
in the formulae II-1c and II-1d,
$L^{21}$ and $L^{22}$ both denote F and/or $L^{23}$ and $L^{24}$ both denote F, and
in formula II-1e,
$L^{21}$, $L^{22}$ and $L^{23}$ denote F
and wherein the respective rings, and preferably the phenylene rings, optionally may each be substituted by one or two alkyl groups, preferably by methyl and/or ethyl groups, preferably by one methyl group.

The liquid-crystal medium preferably comprises one or more compounds of the formula II-2, which are preferably selected from the group of the compounds of the formulae II-2a to II-2k, preferably one or more compounds each of

formulae II-2a and/or II-2h and/or II-2j:

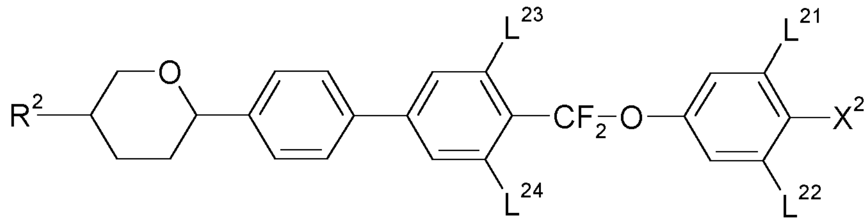

II-2a

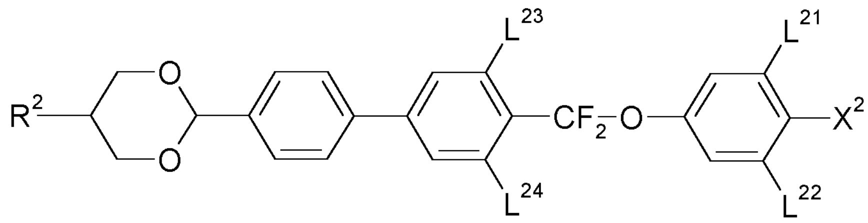

II-2b

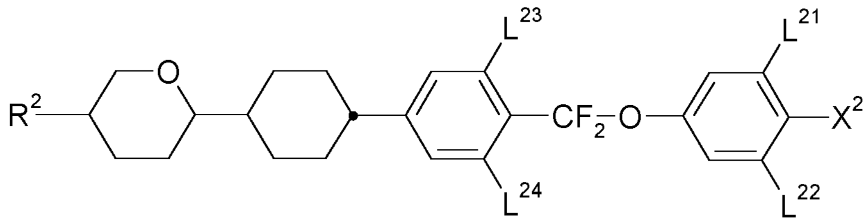

II-2c

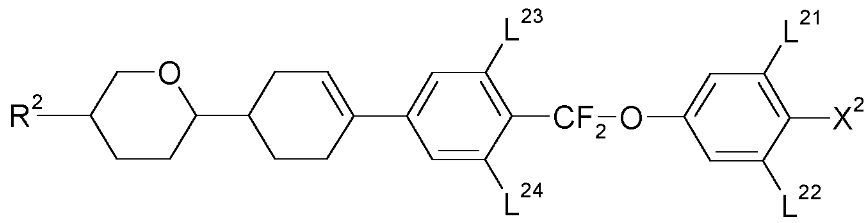

II-2d

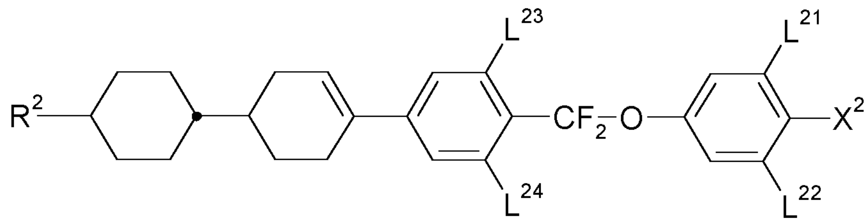

II-2e

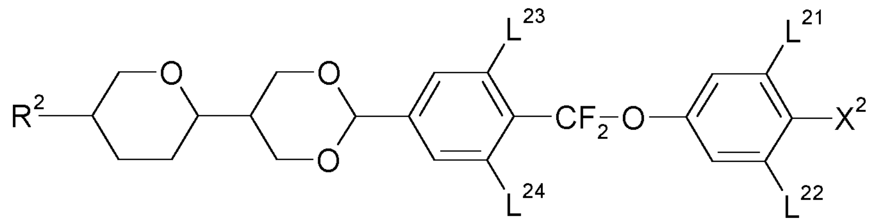

II-2f

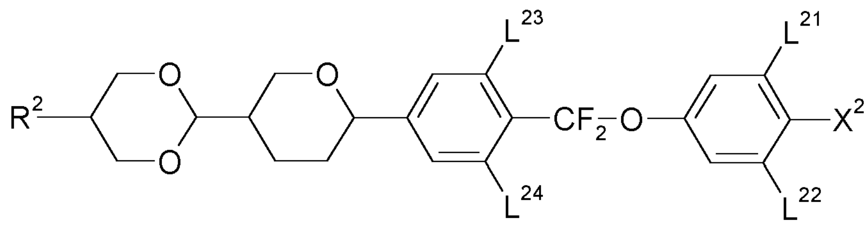

II-2g

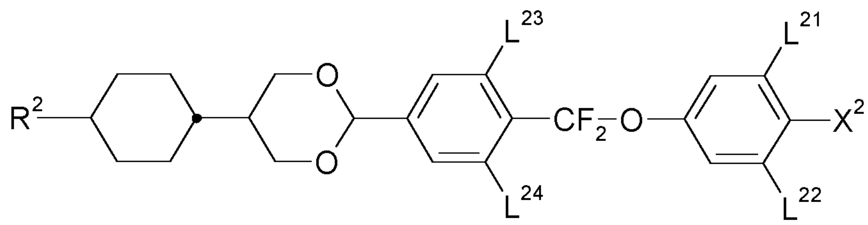

II-2h

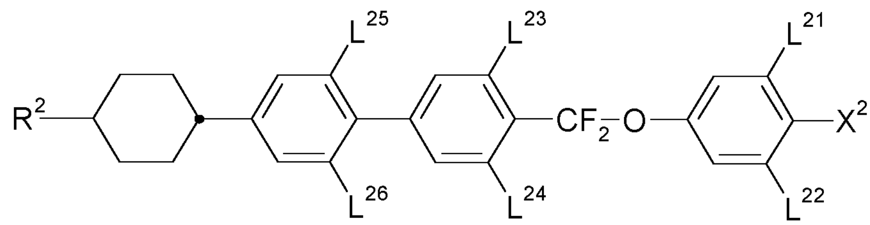

II-2i

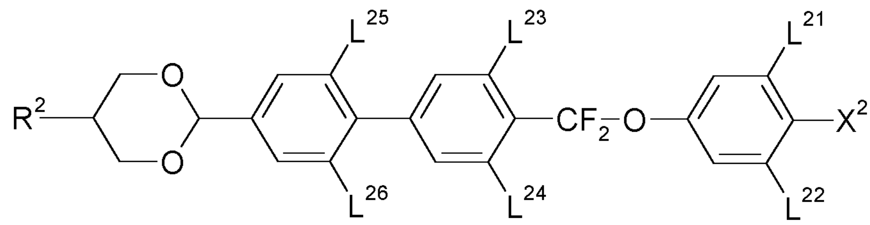

II-2j

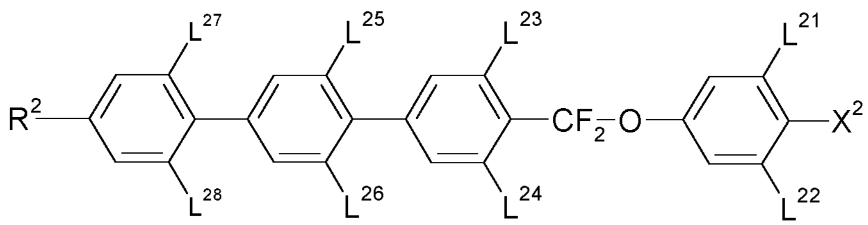

II-2k

in which the parameters have the respective meanings indicated above, and $L^{25}$ to $L^{28}$, independently of one another, denote H or F, preferably $L^{27}$ and $L^{28}$ both denote H, particularly preferably $L^{26}$ denotes H,

and wherein the respective rings, and preferably the phenylene rings, optionally may each be substituted by one or two alkyl groups, preferably by methyl and/or ethyl groups, preferably by one methyl group.

The liquid-crystal medium preferably comprises compounds selected from the group of the compounds of the formulae II-2a to II-2k in which $L^{21}$ and $L^{22}$ both denote F and/or $L^{23}$ and $L^{24}$ both denote F.

In a preferred embodiment, the liquid-crystal medium comprises compounds selected from the group of the compounds of the formulae II-2a to II-2k in which $L^{21}$, $L^{22}$, $L^{23}$ and $L^{24}$ all denote F.

Especially preferred compounds of the formula II-2 are the compounds of the following formulae, particularly preferred of formulae II-2a-1 and/or II-2h-1 and/or II-2k-2:

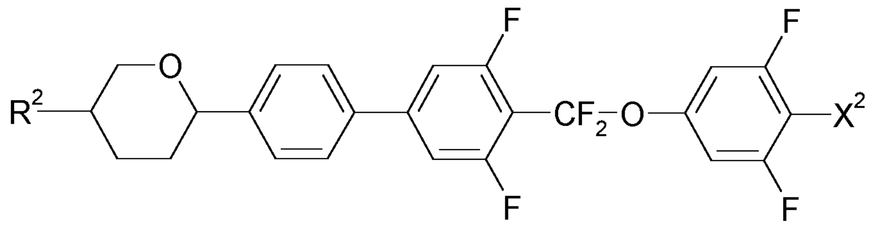

II-2a-1

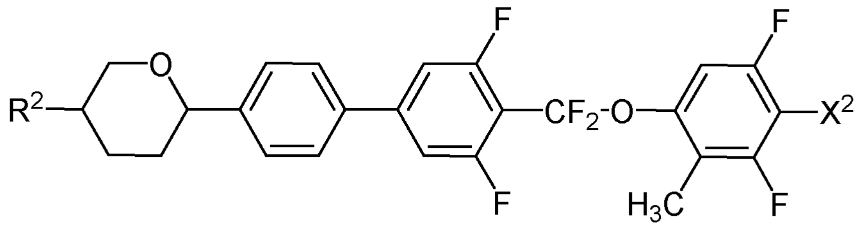

II-2a-2

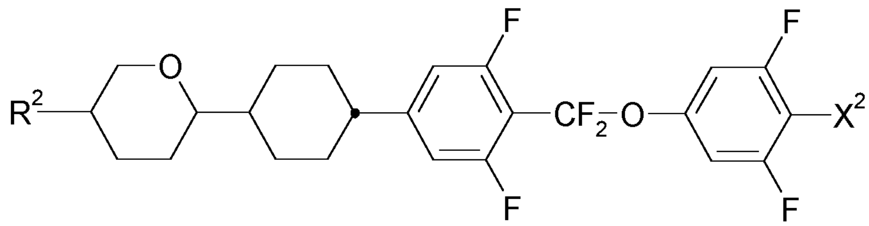

II-2c-1

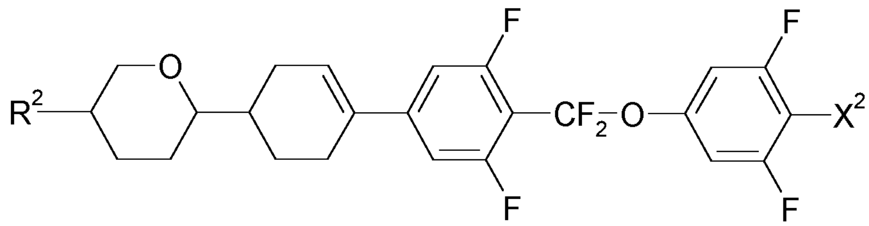

II-2d-1

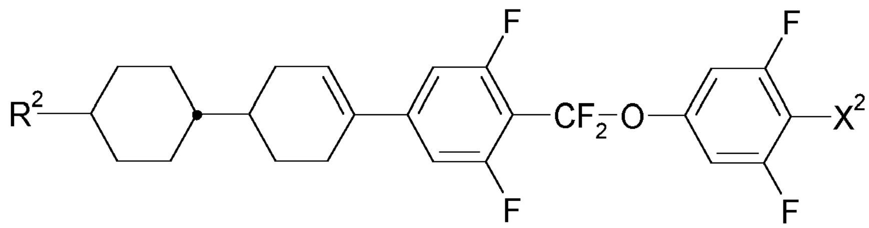

II-2e-1

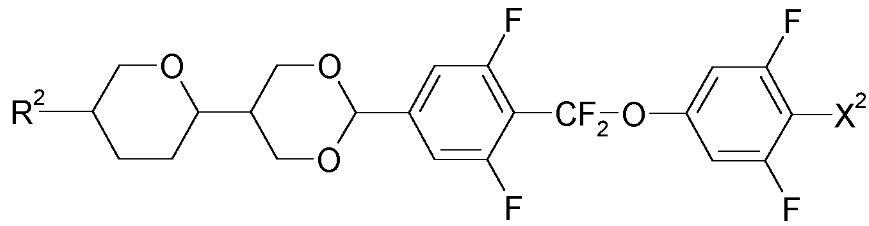

II-2f-1

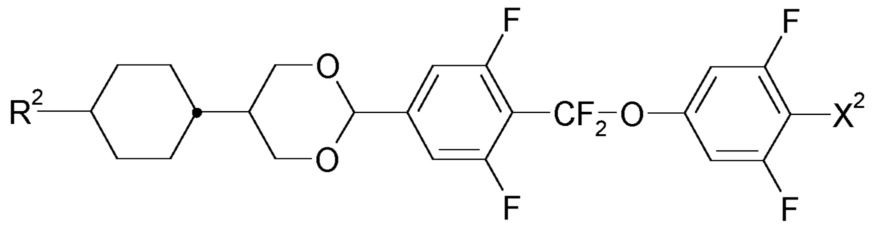

II-2h-1

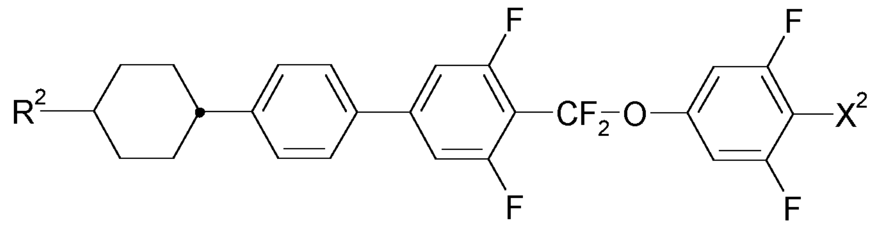

II-2i-1

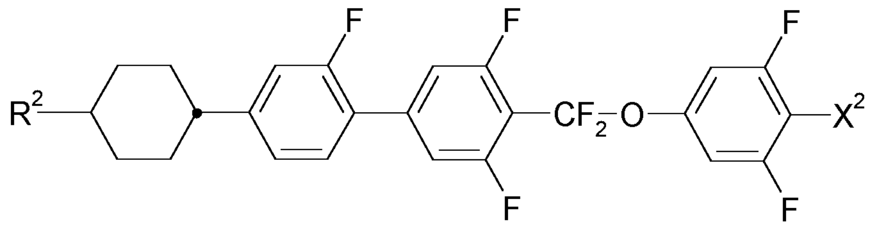

II-2i-2

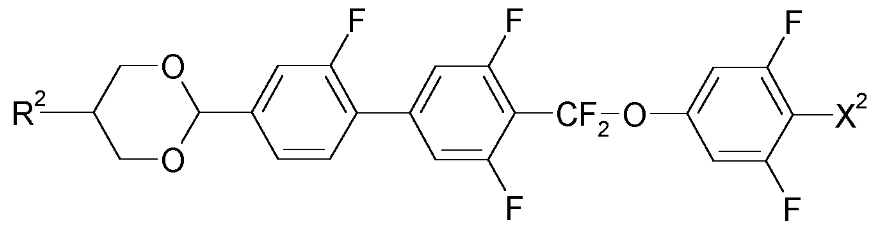

II-2j-1

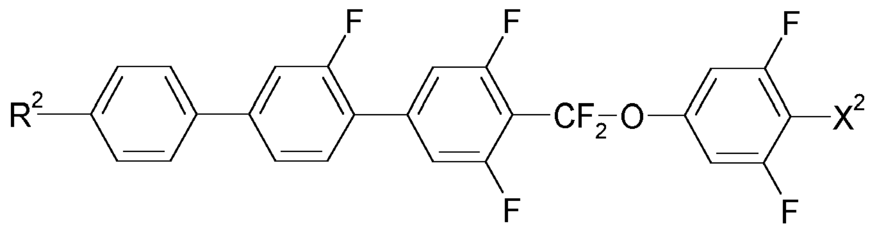

II-2k-1

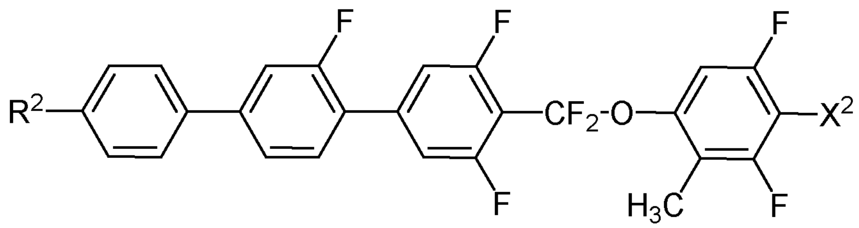

II-2k-2

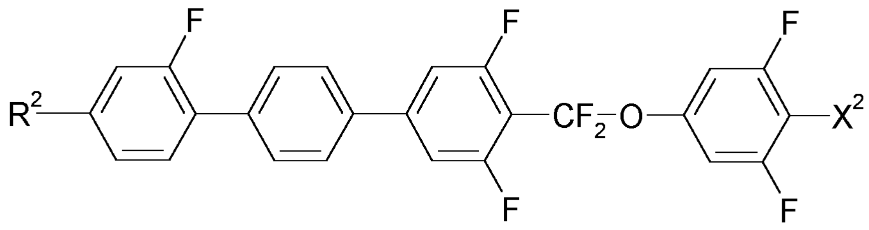

II-2k-2

in which $R^2$ and $X^2$ have the meanings indicated above, and $X^2$ preferably denotes F.

The liquid-crystal medium preferably comprises one or more compounds of the formula III-1. The compounds of the formula III-1 are preferably selected from the group of the compounds of the formulae III-1a to III-1j, preferably from formulae III-1d, III-1g, III-1h, III-1j, III-1k and III-1 m:

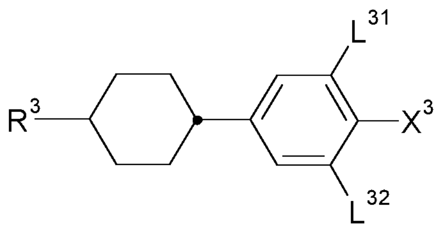

III-1a

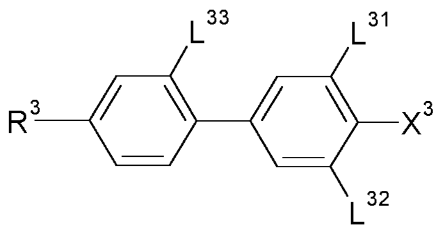

III-1b

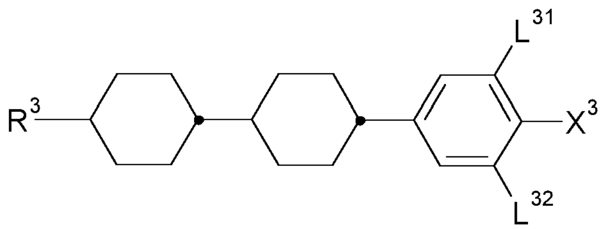

III-1c

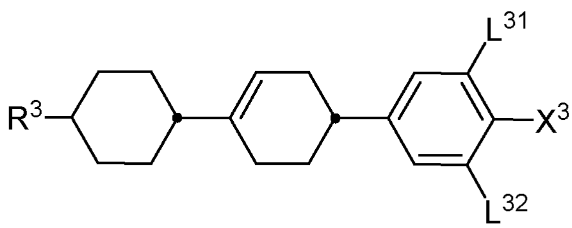

III-1d

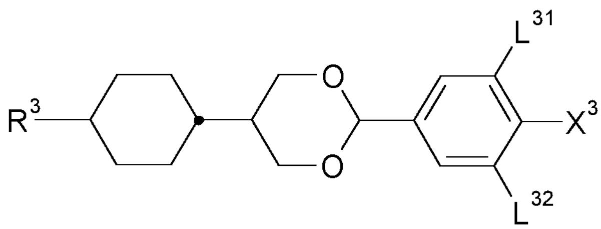

III-1e

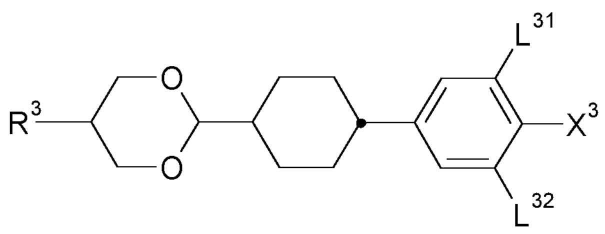

III-1f

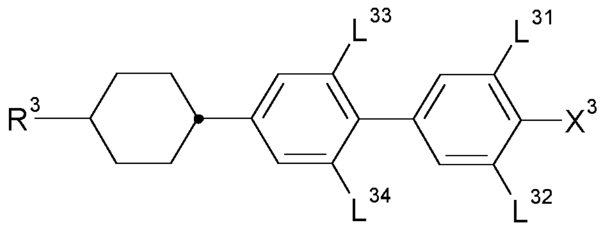

III-1g

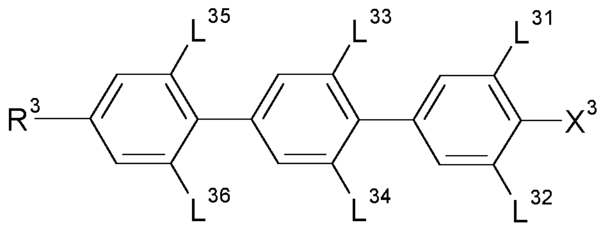

III-1h

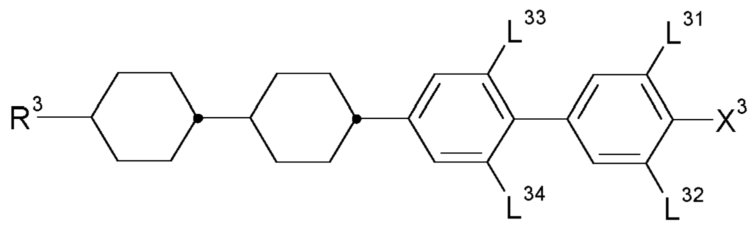

III-1i

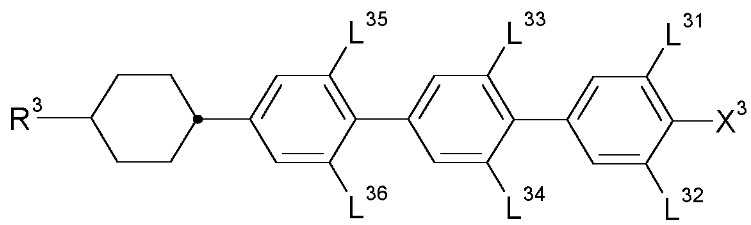

III-1j

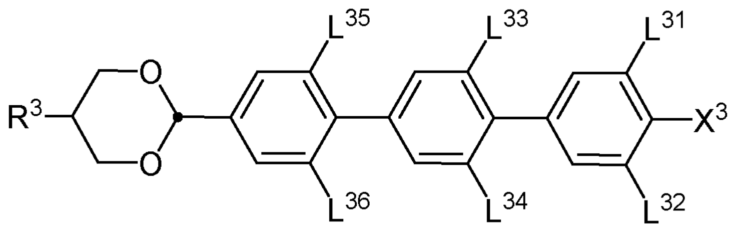

III-1k

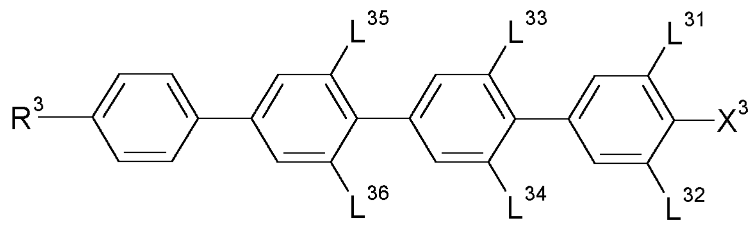

III-1m

in which the parameters have the meanings given above and in which

$X^3$ denotes F, Cl, halogenated alkyl or alkoxy having 1 to 3 C atoms or halogenated alkenyl or alkenyloxy having 2 or 3 C atoms, F, Cl, $-OCF_3$, $-OCHF_2$, $-O-CH_2CF_3$, $-O-CH=CF_2$, $-O-CH=CH_2$ or $-CF_3$, preferably denotes F, $-OCF_3$, or $-CF_3$,

$L^{33}$ and $L^{34}$, independently of one another, denote H or F,

$L^{35}$ and $L^{36}$, independently of one another, denote H or F, preferably H.

The liquid-crystal medium preferably comprises one or more compounds of the formula III-1c, which are preferably selected from the group of the compounds of the formulae III-1c-1 to III-1c-5, preferably of formulae III-1c-1 and/or III-1c-2, most preferably of formula III-1c-1:

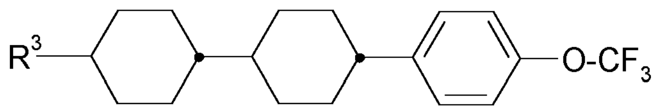

III-1c-1

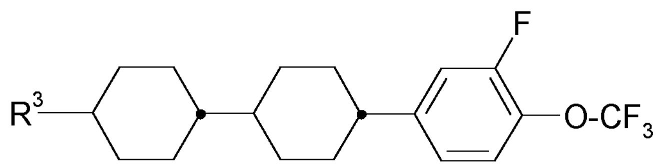

III-1c-2

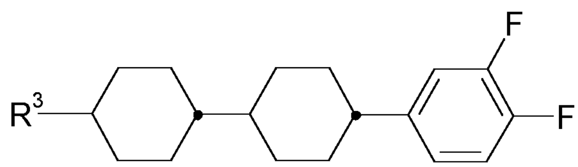

III-1c-3

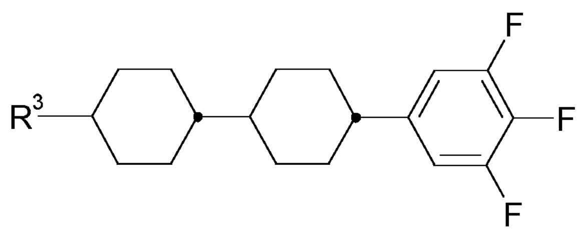

III-1c-4

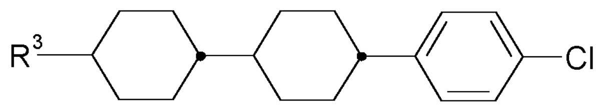

III-1c-5

in which $R^3$ has the meaning indicated above and wherein the respective rings, and preferably the phenylene rings, optionally may each be substituted by one or two alkyl groups, preferably by methyl and/or ethyl groups, preferably by

one methyl group.

The liquid-crystal medium preferably comprises one or more compounds of the formula III-1d, which are preferably selected from the group of the compounds of the formulae III-1d-1 to III-1d-2, preferably of formulae III-1d-1:

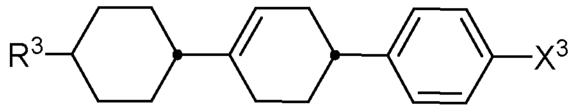

III-1d-1

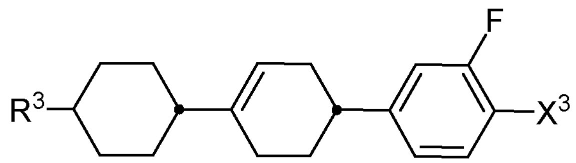

III-1d-2

in which

$R^3$ is defined as above, and $X^3$ is $CF_3$, F or $OCF_3$, preferably $CF_3$.

The liquid-crystal medium preferably comprises one or more compounds of the formula III-1f, which are preferably selected from the group of the compounds of the formulae III-1g-1 to III-1g-6, preferably of formulae III-1f-1 and/or III-1g-2 and/or III-1g-3 and /or III-1g-6, more preferably of formula III-1g-3 and/or III-1g-6, more preferably of formula III-1g-6:

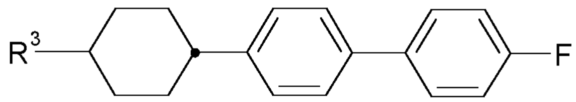

III-1g-1

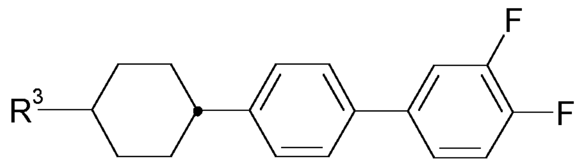

III-1g-2

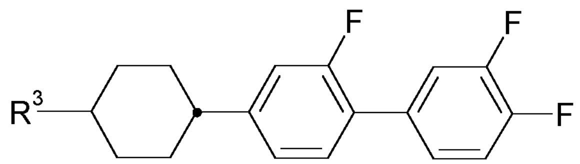

III-1g-3

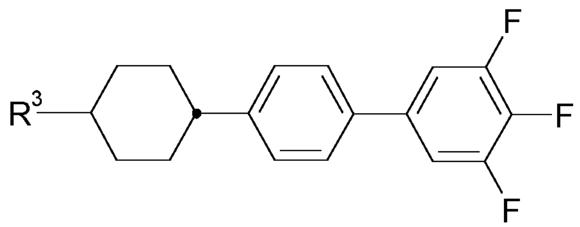

III-1g-4

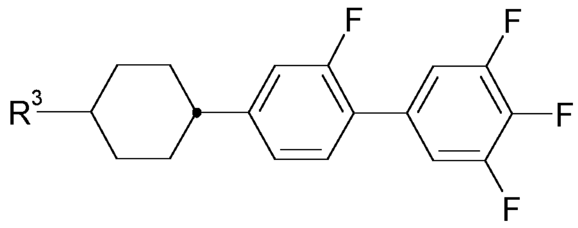

III-1g-5

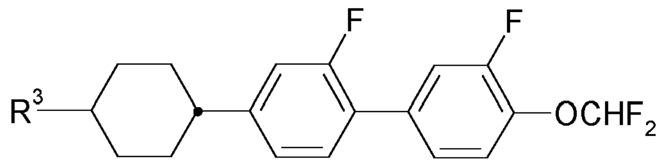

III-1g-6

in which $R^3$ has the meaning indicated above and wherein the respective rings, and preferably the phenylene rings, optionally may each be substituted by one or two alkyl groups, preferably by methyl and/or ethyl groups, preferably by one methyl group.

The liquid-crystal medium preferably comprises one or more compounds of the formula III-1g, which are preferably selected from the group of the compounds of the formulae III-1h-1 to III-1h-5, preferably of formula III-1h-3:

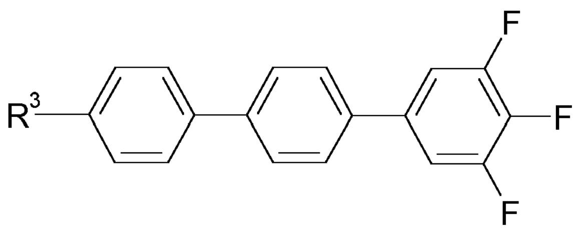

III-1h-1

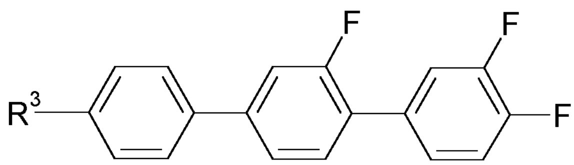

III-1h-2

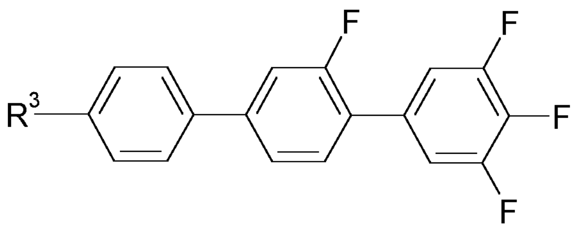

III-1h-3

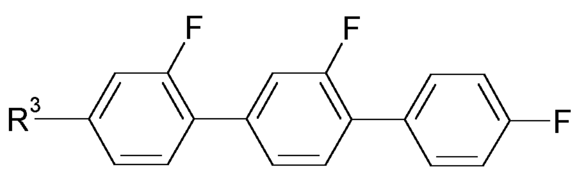

III-1h-4

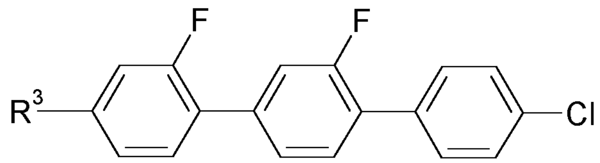

III-1h-5

in which $R^3$ has the meaning indicated above and wherein the respective rings, and preferably the phenylene rings, optionally may each be substituted by one or two alkyl groups, preferably by methyl and/or ethyl groups, preferably by one methyl group.

The liquid-crystal medium preferably comprises one or more compounds selected from the formulae III-1j, III-1k and III-1m, which are preferably selected from the group of the compounds of the formulae III-1j-1, III-1k-1, III-1m-1, preferably of the formula III-1i-1:

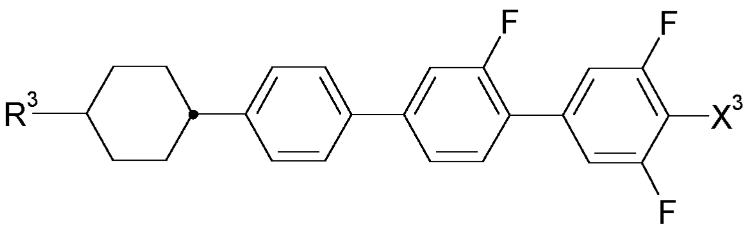

III-1j-1

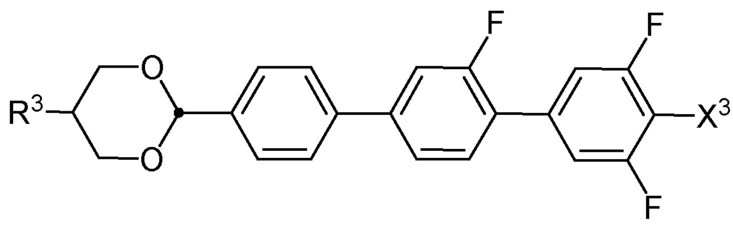

III-1k-1

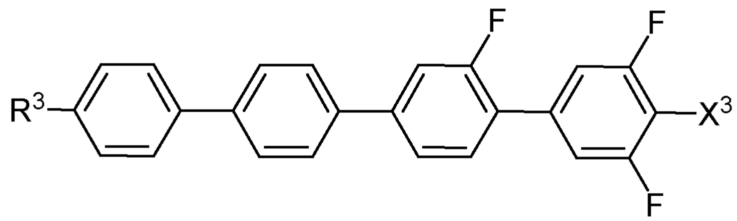

III-1m-1

in which the parameters have the meanings given above, and $X^3$ preferably denotes F or $-OCF_3$.

The liquid-crystal medium preferably comprises one or more compounds of the formula III-1k, which are preferably selected from the group of the compounds of the formulae III-1m-1 and III-1m-2, preferably of the formula III-1m-1:

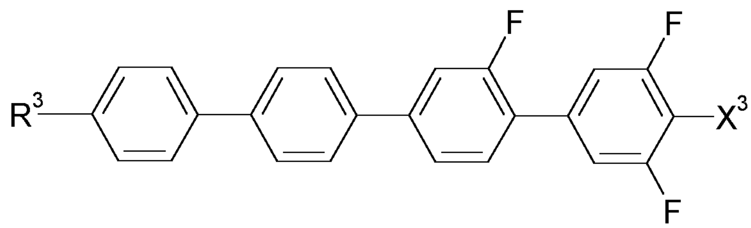

III-1m-1

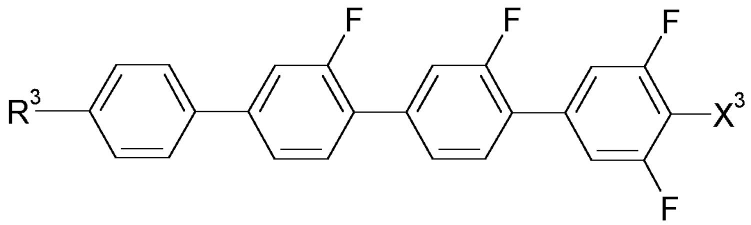

III-1m-2

in which the parameters have the meanings given above and wherein the respective rings, and preferably the phenylene rings, optionally may each be substituted by one or two alkyl groups, preferably by methyl and/or ethyl groups, preferably by one methyl group.

The liquid-crystalline media in accordance with the present invention preferably comprise one or more dielectrically neutral compounds having a dielectric anisotropy in the range from -1.5 to 3, preferably selected from the group of the compounds of the formulae VI, VII, VIII and IX.

In the present application, the elements all include their respective isotopes. In particular, one or more H in the compounds may be replaced by D, and this is also particularly preferred in some embodiments. An incrased degree of deuteration of the corresponding compounds enables, for example, detection and recognition of the compounds.

In the present application,

alkyl particularly preferably denotes straight-chain alkyl, in particular $CH_3$-, $C_2H_5$-, *n*-$C_3H_7$-, *n*-$C_4H_9$- or *n*-$C_5H_{11}$-, and

alkenyl particularly preferably denotes $CH_2$=CH-, *E*-$CH_3$-CH=CH-, $CH_2$=CH-$CH_2$-$CH_2$-, *E*-$CH_3$-CH=CH-$CH_2$-$CH_2$- or *E*-(*n*-$C_3H_7$)-CH=CH-.

In a further preferred embodiment, the medium comprises one or more compounds of formula IV, preferably of formula IVa

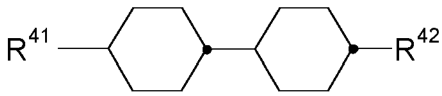

IVa

in which

$R^{41}$ denotes an unsubstituted alkyl radical having 1 to 7 C atoms or an unsubstituted alkenyl radical having 2 to 7 C atoms, preferably an *n*-alkyl radical, particularly preferably having 2, 3, 4 or 5 C atoms, and

$R^{42}$ denotes an unsubstituted alkyl radical having 1 to 7 C atoms, an unsubstituted alkenyl radical having 2 to 7 C atoms, or an unsubstituted alkoxy radical having 1 to 6 C atoms, both preferably having 2 to 5 C atoms, an unsubstituted alkenyl radical preferably having 2, 3 or 4 C atoms, more preferably a vinyl radical or 1-propenyl radical and in particular a vinyl radical.

In a particularly preferred embodiment, the medium comprises one or more compounds of formula IV selected from the group of the compounds of the formulae IV-1 to IV-4, preferably of formula IV-1,

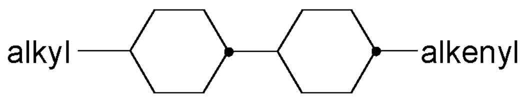

IV-1

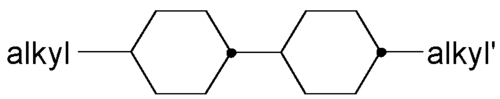

IV-2

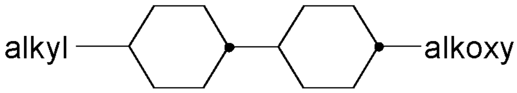

IV-3

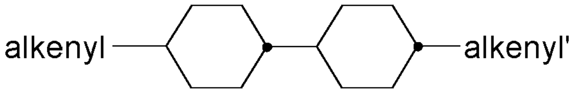

IV-4

in which

alkyl and alkyl', independently of one another, denote alkyl having 1 to 7 C atoms, preferably having 2 to 5 C atoms,

alkenyl and alkenyl', independently of one another, denote alkenyl having 2 to 5 C atoms, preferably having 2 to 4 C atoms, particularly preferably 2 C atoms,

alkenyl' preferably denotes alkenyl having 2 to 5 C atoms, preferably having 2 to 4 C atoms, particularly preferably having 2 to 3 C atoms, and

alkoxy denotes alkoxy having 1 to 5 C atoms, preferably having 2 to 4 C atoms.

In a particularly preferred embodiment, the media according to the invention comprise one or more compounds of formula IV-1 and/or one or more compounds of formula IV-2.

In a further preferred embodiment, the medium comprises one or more compounds of formula IV, selected from the group of the compounds of the formulae IV-2 and IV-3,

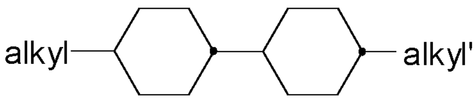

IV-2

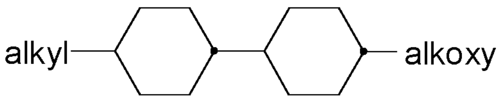

IV-3

in which

alkyl and alkyl', independently of one another, denote alkyl having 1 to 7 C atoms, preferably having 2 to 5 C atoms,

alkoxy denotes alkoxy having 1 to 5 C atoms, preferably having 2 to 4 C atoms.

In a further preferred embodiment, the medium comprises one or more compounds of formula V. In this further preferred embodiment, the medium comprises one or more compounds of formula V selected from the group of the compounds of the formulae V-1 to V-5, preferably one or more of formulae V-1, V-3, V-4 and V-5,

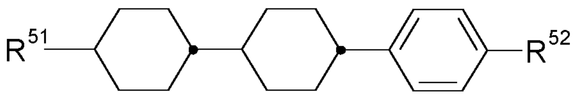

V-1

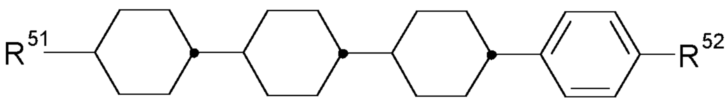

V-2

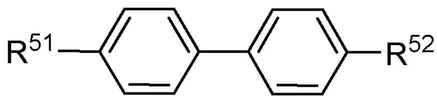

V-3

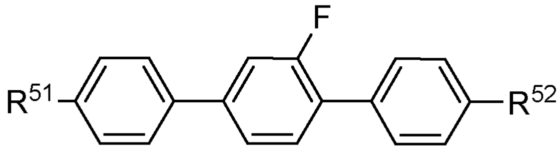

V-4

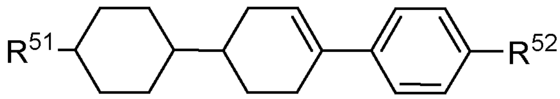

V-5

in which the parameters have the meanings given above under formula V, and preferably

$R^{51}$ denotes alkyl having 1 to 7 C atoms or alkenyl having 2 to 7 C atoms, and

$R^{52}$ denotes alkyl having 1 to 7 C atoms, alkenyl having 2 to 7 C atoms or alkoxy having 1 to 6 C atoms, preferably alkyl or alkenyl.

In a further preferred embodiment the medium comprises one or more compounds of the formula V-1 in which at least one of the $R^{51}$ and $R^{52}$ radicals is alkenyl having 2 to 6 carbon atoms, preferably those selected from the following formula:

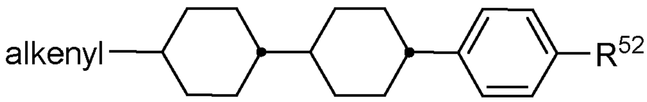

V-1a

wherein alkenyl preferably denotes alkenyl having 2 to 5 C atoms, preferably having 2 to 4 C atoms, particularly preferably vinyl or 3-buten-1-yl, and $R^{52}$ is defined as above, and preferably is methyl or ethyl.

In a further preferred embodiment the medium comprises one or more compounds of the formula V-3 in which at least one of the $R^{51}$ and $R^{52}$ radicals is alkenyl having 2 to 6 carbon atoms, preferably those selected from the following formulae:

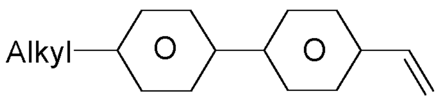

V-3a

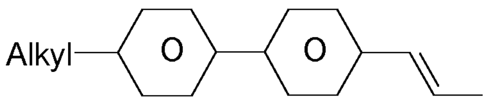

V-3b

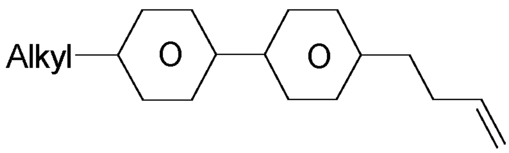

V-3c

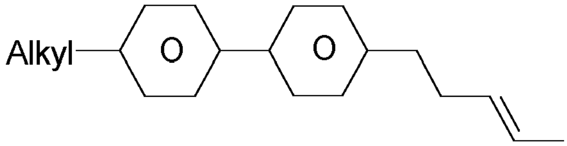

V-3d

in which "Alkyl" has the definition given above, and is preferably methyl or ethyl. Particular preference is given to compounds of the formula V-3d.

In a further preferred embodiment, the medium comprises one or more compounds of formula V-4 selected from the group of the compounds of the formulae V-4a to V-4c,

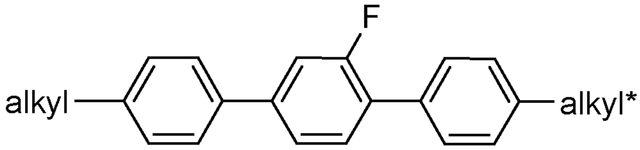

V-4a

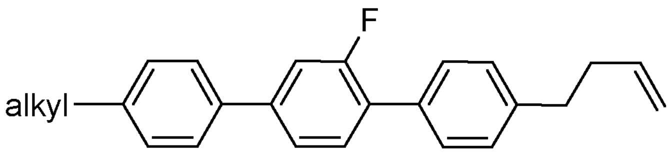

V-4b

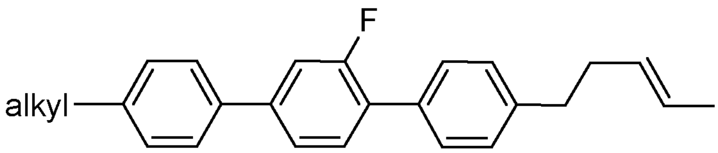

V-4c

in which

alkyl and alkyl* are each independently straight-chain alkyl radical having 1 to 6 carbon atoms, especially methyl, ethyl, n-propyl and pentyl.

The liquid crystalline medium preferably comprises two, three or more compounds selected from the group of compounds of formulae V-4a, V-4b and V-4c.

In a further preferred embodiment, the medium comprises one or more compounds of formula V-5 selected from the group of the compounds of the formulae V-5a to V-5c, preferably V-5a:

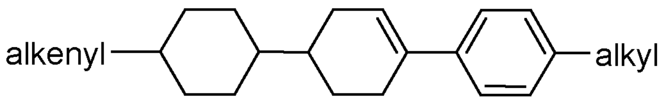

V-5a

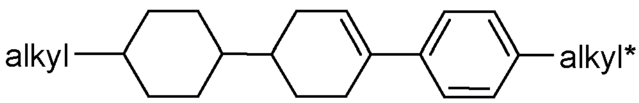

V-5b

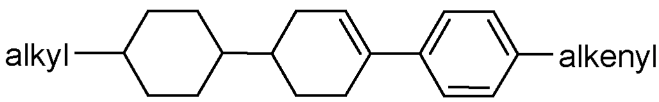

V-5c

in which

alkyl and alkyl* are each independently straight-chain alkyl radical having 1 to 6 carbon atoms, especially methyl, ethyl or n-propyl, and

alkenyl preferably denotes alkenyl having 2 to 5 C atoms, preferably having 2 to 4 C atoms, particularly preferably vinyl.

The media according to the invention preferably comprise the following compounds in the total concentrations indicated:

1 - 30 % by weight of one or more compounds selected from the group of the compounds of formula Py and

2 - 60 % by weight of one or more compounds of formula II, preferably selected from the group of the compounds of the formulae II-1 and II-2 and/or

1 - 60 % by weight of one or more compounds of formula III, and/or

20 - 80 % by weight of one or more compounds of formula IV, and/or

0 - 50 % by weight of one or more compounds of formula V, and/or

0 - 20 % by weight of one or more compounds of formula VI, and/or

0 - 20 % by weight of one or more compounds of formula VII, and/or

0 - 20 % by weight of one or more compounds of formula VIII, preferably selected from the group of the compounds of the formulae VIII-1 and VIII-2 and/or

0 - 20 % by weight of one or more compounds of formula IX, and/ or

0 - 30 % by weight of one or more compounds selected from the group of the compounds of formula X

where the total content of all compounds of formula Py, T, of formulae II to IX and of formula X, which are present in the medium, preferably is 95 % or more, more preferably 97 % or more and, most preferably, 100 %, but always not more than 100 %.

The latter condition holds for all media according to the present application.

In a further preferred embodiment, the media in accordance with the present invention in addition to the compounds of formula Py or the preferred subformulae thereof, preferably comprise one or more dielectrically neutral compounds selected from the group of compounds of formulae IV and V preferably in a total concentration in the range from 5 % or more to 90 % or less, preferably from 10 % or more to 80 % or less, particularly preferably from 20 % or more to 70 % or less.

The medium according to the invention in a particularly preferred embodiment comprises

one or more compounds of formula Py in a total concentration in the range from 3 % or more to 50 % or less, preferably in the range from 5 % or more to 30 % or less, and

one or more compounds of formula T in a total concentration in the range from 1 % or more to 40 % or less, preferably in the range from 5 % or more to 25 % or less, and

one or more compounds of formula II in a total concentration in the range from 5 % or more to 50 % or less, preferably in the range from 10 % or more to 40 % or less, and/or

one or more compounds of formula III in a total concentration in the range from 5 % or more to 50 % or less, preferably in the range from 10 % or more to 40 % or less, and/or

one or more compounds of formula IV-1 and IV-2 in a total concentration in the range from 5 % or more to 55 % or less, preferably in the range from 25 % or more to 50 % or less, and/or

one or more compounds of formula V in a total concentration in the range from 3 % or more to 30 % or less, preferably in the range from 20 % or more to 30 % or less.

Preferably the concentration of the compounds of formula Py in the media according to the invention is in the range from 1 % or more to 20 % or less, more preferably from 1.5 % or more to 20 % or less, most preferably from 2 % or more to 10 % or less.

In a preferred embodiment of the present invention the concentration of the compounds of formula II in the media is in the range from 3 % or more to 60 % or less, more preferably from 5 % or more to 55 % or less, more preferably from 10 % or more to 50 % or less and, most preferably, from 15 % or more to 45 % or less.

In a preferred embodiment of the present invention the concentration of the compounds of formula T and V altogether in the media is in the range from 30 % or more to 55 % or less, more preferably from 35 % or more to 45 % or less.

In a preferred embodiment of the present invention the concentration of the compounds of formula IV-1, IV-2 and V-1 altogether in the media is in the range from 45 % or more to 75 % or less, more preferably from 55 % or more to 70 % or less.

In a preferred embodiment of the present invention the concentration of the compounds of formula III-1d and V-5 altogether in the media is in the range from 4 % or more to 25 % or less, more preferably from 8 % or more to 20 % or less.

The present invention also relates to electro-optical displays or electro-optical components which contain liquid-crystalline media according to the invention. Preference is given to electro-optical displays which are based on the FFS, IPS, VA or ECB effect, preferably on the IPS or FFS effect, and in particular those which are addressed by means of an active-matrix addressing device.

Accordingly, the present invention likewise relates to the use of a liquid-crystalline medium according to the invention in an electro-optical display or in an electro-optical component, and to a process for the preparation of the liquid-crystalline media according to the invention, characterised in that one or more compounds of formula Py are mixed with one or more additional mesogenic compounds and optionally one ore more additives.

Besides compounds of the formulae Py, T, II to X and L, other constituents may also be present, for example in an amount of up to 45 %, but preferably up to 35 %, in particular up to 10 %, of the mixture as a whole.

The media according to the invention may optionally also comprise dielectrically negative compounds, whose total concentration is preferably 20 % or less, more preferably 10 % or less, based on the entire medium.

In a preferred embodiment, the liquid-crystal media according to the invention comprise in total, based on the mixture as a whole,

1 % or more to 30 % or less, preferably 1.5 % or more to 20 % or less, particularly preferably 2 % or more to 10 % or less, of the compound of formula Py, and/or

1 % or more to 35 % or less, preferably 2 % or more to 30 % or less, particularly preferably 5 % or more to 25 % or less, of the compound of formula T, and/or

3 % or more to 50 % or less, preferably 4 % or more to 45 % or less, particularly preferably 5 % or more to 40 % or less, of compounds of formulae II and/or III, and/or

40 % or more to 70 % or less, preferably 50 % or more to 65 % or less, particularly preferably 55 % or more to 65 % or less, of compounds of formulae IV and/ or V, and/or

0 % or more to 30 % or less 0 % or more to 20 % or less, preferably 0 % or more to 15 % or less of compounds of the formulae VI and/or VII and/or VIII and/or IX.

The liquid-crystal media in accordance with the present invention may comprise one or more chiral compounds.

Particularly preferred embodiments of the present invention meet one or more of the following conditions, where the acronyms (abbreviations) are explained in Tables A to C and illustrated by examples in Table D.

Preferably the media according to the present invention fulfil one or more of the following conditions.

i. The liquid-crystalline medium has a birefringence of 0.060 or more, particularly preferably 0.070 or more.

ii. The liquid-crystalline medium has a birefringence of 0.200 or less, particularly preferably 0.180 or less.

iii. The liquid-crystalline medium has a birefringence in the range from 0.090 or more to 0.180 or less.

iv. The liquid-crystalline medium comprises one or more particularly preferred compounds of formula Py, preferably selected from the (sub-) formulae Py-A or Py-1 to Py-5.

v. The liquid-crystalline medium comprises one or more particularly preferred compounds of formula T, preferably

selected from the (sub-) formulae T-1 to T-53, most preferably of (sub-)formula T-6.

vi. The total concentration of the compounds of formula II in the mixture as a whole is 0.5 % or more, preferably 1 % or more, and preferably 14 % or less, particularly preferably 9 % or less, and very particularly preferably in the range from 1 % or more to 6 % or less.

vii. The liquid-crystalline medium comprises one or more compounds of formula IV selected from the group of the compounds of the following formulae: CC-n-V and/or CC-n-Vm and/or CC-n-nVm and/or CC-V-V and/or CC-V-Vn and/or CC-nV-Vn, particularly preferably CC-3-V, preferably in a concentration of up to 60 % or less, particularly preferably up to 50 % or less, and optionally additionally to CC-3-V the compound(s) CC-3-V1, preferably in a concentration of up to 15 % or less, and/or CC-3-2V1, preferably in a concentration of up to 15 % or less, and/or CC-4-V, preferably in a concentration of up to 40 % or less, particularly preferably up to 30 % or less, and/or CC-5-V, preferably in a concentration of up to 20 % or less.

viii. The media comprise the compound of formula CC-n-V, preferably CC-3-V, preferably in a concentration of 1 % or more to 60 % or less, more preferably in a concentration of 20 % or more to 55 % or less.

ix. The liquid-crystalline medium comprises one or more compounds of formula IV, preferably of the formulae IV-1 and/or IV-2, preferably in a total concentration of 20 % or more, in particular 30 % or more, and very particularly preferably 40 % or more, and is preferably in the range from 46 % to 55 % of compounds of formula IV-1.

x. The total concentration of the compounds of formula V in the mixture as a whole is 15 % or more, preferably 20 % or more, and is preferably in the range from 15 % or more to 40 % or less, particularly preferably in the range from 20 % or more to 30 % or less.

xi. The total concentration of the compounds of formula V-3 in the mixture as a whole preferably is 5 % or more to 25 % or less, preferably 5 % or more to 15 % or less.

xii. The total concentration of the compounds of formula V-4, preferably V-4a to V-4c, in the mixture as a whole preferably is 3 % or more to 30 % or less, preferably 10 % or more to 25 % or less.

xiii. The total concentration of the compounds selected from formula V-5, preferably V-5-a, and III-1d, preferably III-1d-1, in the mixture as a whole preferably is 2 % or more to 20 % or less, preferably 4 % or more to 15 % or less.

The invention furthermore relates to an electro-optical display having active-matrix addressing based on the IPS, FFS or UB-FFS effect, characterised in that it contains, as dielectric, a liquid-crystalline medium in accordance with the present invention.

The liquid-crystal mixture preferably has a nematic phase range having a width of at least 70 degrees.

The rotational viscosity $\gamma_1$ is preferably 350 mPa·s or less, preferably 250 mPa·s or less and, in particular, 150 mPa·s or less.

The mixtures according to the invention are suitable for all IPS and FFS-TFT applications using dielectrically positive liquid crystalline media, such as, e.g. XB-FFS.

The liquid-crystalline media according to the invention preferably virtually completely consist of 4 to 15, in particular 5 to 12, and particularly preferably 10 or less, compounds. These are preferably selected from the group of the compounds of the formulae Py, T, II, III, IV, V, VI, VII, VIII and IX.

The liquid-crystalline media according to the invention may optionally also comprise more than 18 compounds. In this case, they preferably comprise 18 to 25 compounds.

In a preferred embodiment, the liquid-crystal media according to the invention predominantly comprise, preferably essentially consist of and, most preferably, virtually completely consist of compounds, which do not comprise a cyano group.

In a preferred embodiment, the liquid-crystal media according to the invention comprise compounds selected from the group of the compounds of the formulae Py, T, II, III, and IV and V, preferably selected from the group of the compounds of the formulae Py-1, T-1, II-1, II-2, III-1, III-2, IV, and V; they preferably consist predominantly, particularly preferably essentially and very particularly preferably virtually completely of the compounds of the said formulae.

The liquid-crystal media according to the invention preferably have a nematic phase from in each case at least -10°C or less to 70°C or more, particularly preferably from -20°C or less to 80°C or more, very particularly preferably from -30°C or less to 85°C or more and most preferably from -40°C or less to 90°C or more.

The expression "have a nematic phase" here means on the one hand that no smectic phase and no crystallisation

are observed at low temperatures at the corresponding temperature and on the other hand that no clearing occurs on heating out of the nematic phase. The investigation at low temperatures is carried out in a flow viscometer at the corresponding temperature and checked by storage in test cells having a cell thickness corresponding to the electro-optical application for at least 100 hours. If the storage stability at a temperature of -20°C in a corresponding test cell is 1,000 h or more, the medium is regarded as stable at this temperature. At temperatures of -30°C and -40°C, the corresponding times are 500 h and 250 h respectively. At high temperatures, the clearing point is measured in capillaries by conventional methods.

In a preferred embodiment, the liquid-crystal media according to the invention are characterised by optical anisotropy values in the moderate to low range. The birefringence values are preferably in the range from 0.075 or more to 0.130 or less, particularly preferably in the range from 0.085 or more to 0.120 or less and very particularly preferably in the range from 0.090 or more to 0.115 or less.

In this embodiment, the liquid-crystal media according to the invention have a positive dielectric anisotropy $\Delta\varepsilon$, which preferably is in the range from 2.0 or more to 20 or less, more preferably to 15 or less, more preferably from 2.0 or more to 10 or less, particularly preferably from 2.0 or more to 9.0 or less and very particularly preferably from 2.5 or more to 8.0 or less.

The liquid-crystal media according to the invention preferably have relatively low values for the threshold voltage (Vn) in the range from 1.0 V or more to 5.0 V or less, preferably to 2.5 V or less, preferably from 1.2 V or more to 2.2 V or less, particularly preferably from 1.3 V or more to 2.0 V or less.

In addition, the liquid-crystal media according to the invention have high values for the VHR in liquid-crystal cells.

In general, liquid-crystal media having a low addressing voltage or threshold voltage here have a lower VHR than those having a higher addressing voltage or threshold voltage, and vice versa.

These preferred values for the individual physical properties are preferably also in each case maintained by the media according to the invention in combination with one another.

In the present application, the term "compounds", also written as "compound(s)", means both one and also a plurality of compounds, unless explicitly indicated otherwise.

For the present invention, the following definitions apply in connection with the specification of the constituents of the compositions, unless indicated otherwise in individual cases:

- "comprise": the concentration of the constituents in question in the composition is preferably 5% or more, particularly preferably 10% or more, very particularly preferably 20% or more,

- "predominantly consist of": the concentration of the constituents in question in the composition is preferably 50% or more, particularly preferably 55% or more and very particularly preferably 60% or more,

- "essentially consist of": the concentration of the constituents in question in the composition is preferably 80% or more, particularly preferably 90% or more and very particularly preferably 95% or more, and

- "virtually completely consist of": the concentration of the constituents in question in the composition is preferably 98% or more, particularly preferably 99% or more and very particularly preferably 100.0%.

This applies both to the media as compositions with their constituents, which can be groups of compounds as well as individual compounds, and also to the groups of compounds with their respective constituents, the compounds. Only in relation to the concentration of an individual compound relative to the medium as a whole does the term comprise mean: the concentration of the compound or compounds in question is preferably 1% or more, particularly preferably 2% or more, very particularly preferably 4% or more.

For the present invention, "≤" means less than or equal to, preferably less than, and "≥" means greater than or equal to, preferably greater than.

For the present invention

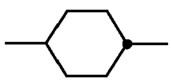
,
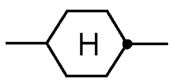
and
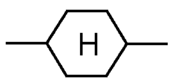

denote *trans-1,4-cyclohexylene,*

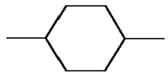

denotes a mixture of both cis- and trans-1,4-cyclohexylene and

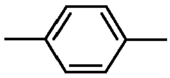 and 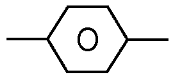

denote 1,4-phenylene.

Throughout this application 1,3-cyclopentenylene is a moiety selected from the group of the formulae

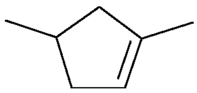 , 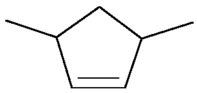 , 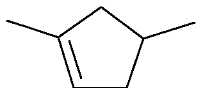 ,

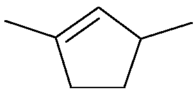 and 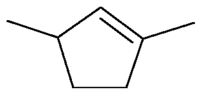 ,

preferably

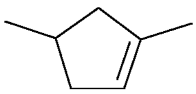 , 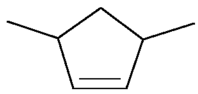 or 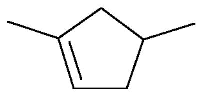 ,

most preferably

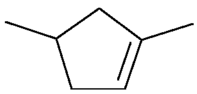 or 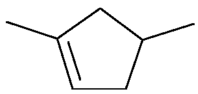 ,

For the present invention, the expression "dielectrically positive compounds" means compounds having a $\Delta\varepsilon$ of > 1.5, the expression "dielectrically neutral compounds" means compounds having $-1.5 \leq \Delta\varepsilon \leq 1.5$ and the expression "dielectrically negative compounds" means compounds having $\Delta\varepsilon < -1.5$. The dielectric anisotropy of the compounds is determined here by dissolving 10% of the compounds in a liquid-crystalline host and determining the capacitance of the resultant mixture in each case in at least one test cell having a cell thickness of 20 $\mu$m with homeotropic and with homogeneous surface alignment at 1 kHz. The measurement voltage is typically 0.5 V to 1.0 V, but is always lower than the capacitive threshold of the respective liquid-crystal mixture investigated.

The host mixture used for dielectrically positive and dielectrically neutral compounds is ZLI-4792 and that used for dielectrically negative compounds is ZLI-2857, both from Merck KGaA, Germany. The values for the respective compounds to be investigated are obtained from the change in the dielectric constant of the host mixture after addition of the compound to be investigated and extrapolation to 100% of the compound employed. The compound to be investigated is dissolved in the host mixture in an amount of 10%. If the solubility of the substance is too low for this purpose, the concentration is halved in steps until the investigation can be carried out at the desired temperature.

The liquid-crystal media according to the invention may, if necessary, also comprise further additives, such as, for example, stabilisers and/or pleo-chroitic, e.g. dichroitic, dyes and/or chiral dopants in the usual amounts. The amount of these additives employed is preferably in total 0 % or more to 10 % or less, based on the amount of the entire mixture, particularly preferably 0.1 % or more to 6 % or less. The concentration of the individual compounds employed is preferably 0.1 % or more to 3 % or less. The concentration of these and similar additives is generally not taken into account when specifying the concentrations and concentration ranges of the liquid-crystal compounds in the liquid-crystal media.

In a preferred embodiment, the liquid-crystal media according to the invention comprise a polymer precursor

which comprises one or more reactive compounds, preferably reactive mesogens, and, if necessary, also further additives, such as, for example, polymerisation initiators and/or polymerisation moderators, in the usual amounts. The amount of these additives employed is in total 0 % or more to 10 % or less, based on the amount of the entire mixture, preferably 0.1 % or more to 2 % or less. The concentration of these and similar additives is not taken into account when specifying the concentrations and concentration ranges of the liquid-crystal compounds in the liquid-crystal media.

The compositions consist of a plurality of compounds, preferably 3 or more to 30 or fewer, particularly preferably 6 or more to 20 or fewer and very particularly preferably 10 or more to 16 or fewer compounds, which are mixed in a conventional manner. In general, the desired amount of the compounds used in lesser amount is dissolved in the compounds making up the principal constituent of the mixture. This is advantageously carried out at elevated temperature. If the selected temperature is above the clearing point of the principal constituent, completion of the dissolution operation is particularly easy to observe. However, it is also possible to prepare the liquid-crystal mixtures in other conventional ways, for example using pre-mixes or from a so-called "multi-bottle system".

The mixtures according to the invention exhibit very broad nematic phase ranges having clearing points of 65°C or more, very favourable values for the capacitive threshold, relatively high values for the holding ratio and at the same time very good low-temperature stabilities at -30°C and -40°C. Furthermore, the mixtures according to the invention are distinguished by low rotational viscosities $\gamma_1$.

It goes without saying to the person skilled in the art that the media according to the invention for use in VA, IPS, FFS or PALC displays may also comprise compounds in which, for example, H, N, O, Cl, F have been replaced by the corresponding isotopes.

The structure of the FFS liquid-crystal displays according to the invention corresponds to the usual geometry, as described, for example, in US 2002/0041354 A1.

The liquid-crystal phases according to the invention can be modified by means of suitable additives in such a way that they can be employed in any type of, for example, IPS and FFS LCD display that has been disclosed to date.

Table E below indicates possible dopants which can be added to the mixtures according to the invention. If the mixtures comprise one or more dopants, it is (they are) employed in amounts of 0.01 % to 4 %, preferably 0.1 % to 1.0%.

Stabilisers which can be added, for example, to the mixtures according to the invention, preferably in amounts of 0.01 % to 6 %, in particular 0.1 % to 3 %, are shown below in Table F.

For the purposes of the present invention, all concentrations are, unless explicitly noted otherwise, indicated in per cent by weight and relate to the corresponding mixture as a whole or mixture constituents, again a whole, unless explicitly indicated otherwise. In this context the term "the mixture" describes the liquid crystalline medium.

All temperature values indicated in the present application, such as, for example, the melting point T(C,N), the smectic (S) to nematic (N) phase transition T(S,N) and the clearing point T(N,I), are indicated in degrees Celsius (°C) and all temperature differences are correspondingly indicated in differential degrees (° or degrees), unless explicitly indicated otherwise.

For the present invention, the term "threshold voltage" relates to the capacitive threshold ($V_0$), also known as the Freedericks threshold, unless explicitly indicated otherwise.

All physical properties are and have been determined in accordance with "Merck Liquid Crystals, Physical Properties of Liquid Crystals", status Nov. 1997, Merck KGaA, Germany, and apply for a temperature of 20°C, and $\Delta n$ is determined at 436 nm, 589 nm and at 633 nm, and $\Delta\varepsilon$ at 1 kHz, unless explicitly indicated otherwise in each case.

The electro-optical properties, for example the threshold voltage ($V_0$) (capacitive measurement), are, as is the switching behaviour, determined in test cells produced at Merck Japan. The measurement cells have soda-lime glass substrates and are constructed in an ECB or VA configuration with polyimide alignment layers (SE-1211 with diluent **26 (mixing ratio 1:1), both from Nissan Chemicals, Japan), which have been rubbed perpendicularly to one another and effect homeotropic alignment of the liquid crystals. The surface area of the transparent, virtually square ITO electrodes is 1 $cm^2$.

Unless indicated otherwise, a chiral dopant is not added to the liquid-crystal mixtures used, but the latter are also particularly suitable for applications in which doping of this type is necessary.

The rotational viscosity is determined using the rotating permanent magnet method and the flow viscosity in a modified Ubbelohde viscometer. For liquid-crystal mixtures ZLI-2293, ZLI-4792 and MLC-6608, all products from Merck KGaA, Darmstadt, Germany, the rotational viscosity values determined at 20°C are 161 mPa s, 133 mPa·s and 186 mPa·s respectively, and the flow viscosity values (v) are 21 $mm^2 \cdot s^{-1}$, 14 $mm^2 \cdot s^{-1}$ and 27 $mm^2 \cdot s^{-1}$, respectively.

The dispersion of the materials may for practical purposes be conveniently characterized in the following way, which is used throughout this application unless explicitly stated otherwise. The values of the birefringence are determined at a temperature of 20°C at several fixed wavelengths using a modified Abbé refractometer with homeotropically aligning surfaces on the sides of the prisms in contact with the material. The birefringence values are determined at the specific wavelength values of 436 nm (respective selected spectral line of a low pressure mercury lamp), 589 nm (sodium "D" line) and 633 nm (wavelength of a HE-Ne laser (used in combination with an attenuator/diffusor in order to prevent damage to the eyes of the observers. In the following table $\Delta n$ is given at 589 nm and $\Delta(\Delta n)$ is given as

$$\Delta(\Delta n) = \Delta n(436\ nm) - \Delta n(633\ nm).$$

The following symbols are used, unless explicitly indicated otherwise:

| | |
|---|---|
| $V_0$ | threshold voltage, capacitive [V] at 20°C, |
| $n_e$ | extraordinary refractive index measured at 20°C and 589 nm, |
| $n_0$ | ordinary refractive index measured at 20°C and 589 nm, |
| $\Delta n$ | optical anisotropy measured at 20°C and 589 nm, |
| $\lambda$ | wavelength $\lambda$ [nm], |
| $\Delta n(\lambda)$ | optical anisotropy measured at 20°C and wavelength $\lambda$, |
| $\Delta(\Delta n)$ | change in optical anisotropy defined as: $\Delta n(20°C, 436\ nm) - \Delta n(20°C, 633\ nm)$, |
| $\Delta(\Delta n^*)$ | "relative change in optical anisotropy" defined as: $\Delta(\Delta n)/\Delta n(20°C, 589\ nm)$, |
| $\varepsilon\perp$ | dielectric susceptibility perpendicular to the director at 20°C and 1 kHz, |
| $\varepsilon\|$ | dielectric susceptibility parallel to the director at 20°C and 1 kHz, |
| $\Delta_\varepsilon$ | dielectric anisotropy at 20°C and 1 kHz, |
| T(N,I) or clp. | clearing point [°C], |
| $\nu$ | flow viscosity measured at 20°C [$mm^2 \cdot s^{-1}$], |
| $\gamma_1$ | rotational viscosity measured at 20°C [mPa·s], |
| $K_{11}$ | elastic constant, "splay" deformation at 20°C [pN], |
| $K_{22}$ | elastic constant, "twist" deformation at 20°C [pN] ($K_{22} \approx \frac{1}{2} K_{11}$), |
| $K_{33}$ | elastic constant, "bend" deformation at 20°C [pN], |
| $K_{av.}$ | average eleastic constant at 20°C [pN] defined here as |

$$K_{av.} \equiv (^3/_2\ K_{11} + K_{33}) / 3 \approx (K_{11} + K_{22} + K_{33}) / 3,$$

| | |
|---|---|
| LTS | low-temperature stability of the phase, determined in test cells, |
| VHR | voltage holding ratio, |
| $\Delta$VHR | decrease in the voltage holding ratio, and |
| $S_{rel}$ | relative stability of the VHR, |

The following examples explain the present invention without limiting it. However, they show the person skilled in the art preferred mixture concepts with compounds preferably to be employed and the respective concentrations thereof and combinations thereof with one another. In addition, the examples illustrate the properties and property combinations that are accessible.

For the present invention and in the following examples, the structures of the liquid-crystal compounds are indicated by means of acronyms, with the transformation into chemical formulae taking place in accordance with Tables A to C below. All radicals $C_nH_{2n+1}$, $C_mH_{2m+1}$ and $C_lH_{2l+1}$ or $C_nH_{2n}$, $C_mH_{2m}$ and $C_lH_{2l}$ are straight-chain alkyl radicals or alkylene radicals, in each case having n, m and l C atoms respectively. Preferably n, m and l are independently of each other 1, 2, 3, 4, 5, 6, or 7. Table A shows the codes for the ring elements of the nuclei of the compound, Table B lists the bridging units, and Table C lists the meanings of the symbols for the left- and right-hand end groups of the molecules. The acronyms are composed of the codes for the ring elements with optional linking groups, followed by a first hyphen and the codes for the left-hand end group, and a second hyphen and the codes for the right-hand end group. Table D shows illustrative structures of compounds together with their respective abbreviations.

**Table A: Ring elements**

| | | | |
|---|---|---|---|
| **C** | 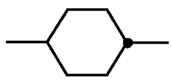 | | |
| **D** | 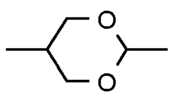 | **DI** | 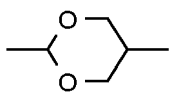 |

(continued)

| | | | |
|---|---|---|---|
| **A** | 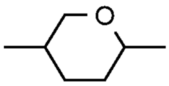 | **AI** | 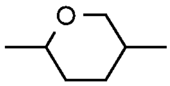 |
| **P** | 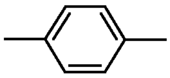 | | |
| **G** | 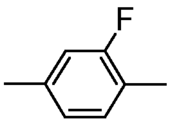 | **GI** | |
| **U** | 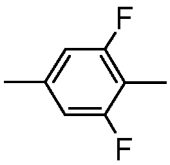 | **UI** | 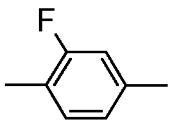 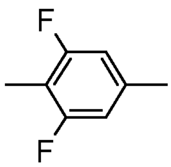 |
| **Y** | 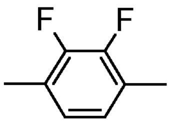 | | |
| **P(F, Cl)Y** | 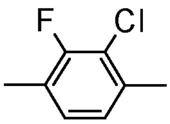 | **P(Cl,F)Y** | 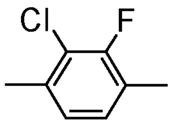 |
| **np** | 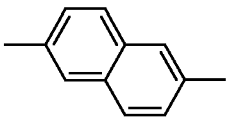 | | |
| **n3f** | 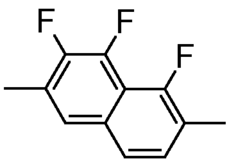 | **nN3fl** | 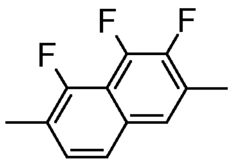 |
| **th** | 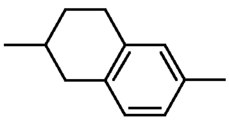 | **thl** | 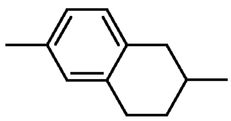 |
| **tH2f** | 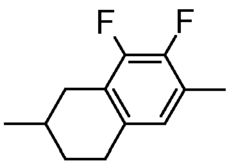 | **tH2fl** | 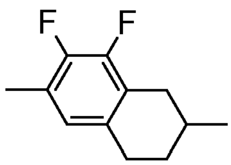 |
| **o2f** | 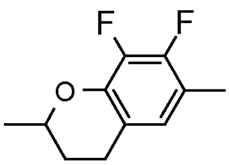 | **o2fl** | 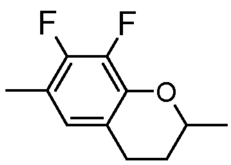 |

(continued)

| | | | |
|---|---|---|---|
| **dh** | 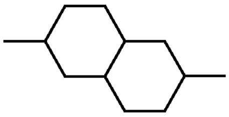 | nf | 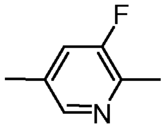 |
| **B** | 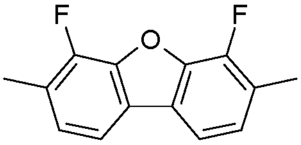 | **B(S)** | 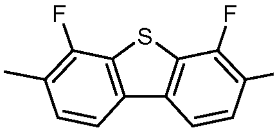 |
| **O** | 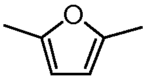 | **S** | 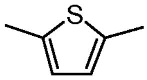 |
| **K** | 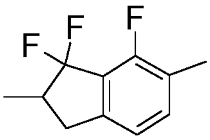 | **Kl** | 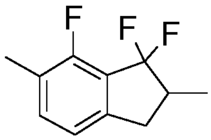 |
| **L** | 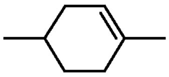 | **Ll** | 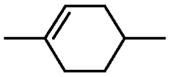 |
| **F** | 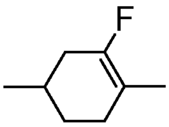 | **Fl** | 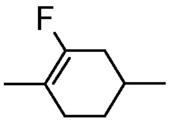 |
| **Bh** | 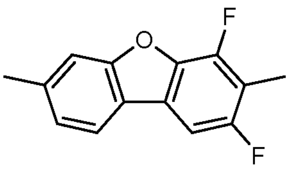 | **Bh(S)** | 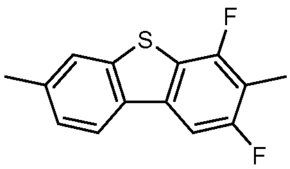 |
| **Bf** | 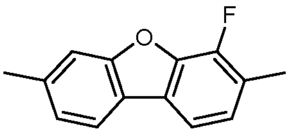 | **Bf(S)** | 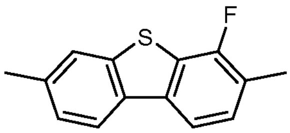 |
| **Bfi** | 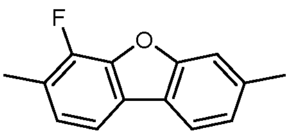 | **Bfi(S)** | 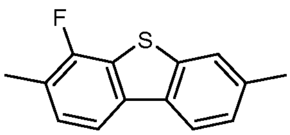 |

**Table B: Bridging units**

| | | | |
|---|---|---|---|
| **E** | $-CH_2-CH_2-$ | | |
| **V** | -CH=CH- | | |
| **T** | $-C\equiv C-$ | | |
| **W** | $-CF_2-CF_2$ | | |
| **B** | -CF=CF- | | |
| **Z** | -CO-O- | **ZI** | -O-CO- |
| **X** | -CF=CH- | **XI** | -CH=CF- |
| **O** | $-CH_2-O-$ | **OI** | $-O-CH_2$ |
| **Q** | $-CF_2-O-$ | **QI** | $-O-CF_2$ |

**Table C: End groups**

| On the left individually or in combination | | On the right individually or in combination | |
|---|---|---|---|
| **-n-** | $CnH_{2n+1}$- | **-n** | -$C_nH_{2n+1}$ |
| **-nO-** | $CnH_{2n+1}$-O- | **-On** | -O- $C_nH_{2n+1}$ |
| **-V-** | $CH_2$=CH- | **-V** | -CH=$CH_2$ |
| **-nV-** | $C_nH_{2n+1}$-CH=CH- | **-nV** | -$C_nH_{2n}$-CH=$CH_2$ |
| **-Vn-** | $CH_2$=CH- $C_nH_{2n}$- | **-Vn** | -CH=CH-$C_nH_{2n+1}$ |
| **-nVm-** | $C_nH_{2n+1}$-CH=CH-$C_mH_{2m}$- | **-nVm** | - $C_nH_{2n}$-CH=CH-$C_mH_{2m+1}$ |
| **-N-** | N≡C- | **-N** | -C≡N |
| **-S-** | S=C=N- | **-S** | -N=C=S |
| **-F-** | F- | **-F** | -F |
| **-CL-** | Cl- | **-CL** | -Cl |
| **-M-** | $CFH_2$- | **-M** | -$CFH_2$ |
| **-D-** | $CF_2$H- | **-D** | -$CF_2$H |
| **-T-** | $CF_3$- | **-T** | -$CF_3$ |
| **-MO-** | $CFH_2$O - | **-OM** | -O$CFH_2$ |
| **-DO-** | $CF_2$HO - | **-OD** | -O$CF_2$H |
| **-TO-** | $CF_3$O - | **-OT** | -O$CF_3$ |
| **-A-** | H-C≡C- | **-A** | -C≡C-H |
| **-nA-** | $C_nH_{2n+1}$-C≡C- | **-An** | -C≡C-$C_nH_{2n+1}$ |
| **-NA-** | N≡C-C≡C- | **-AN** | -C≡C-C≡N |
| **-cp-** | cyclopentyl | **-cp** | cyclopentyl |
| **-cpr-** | cyclopropyl | **-cpr** | cyclopropyl |
| **On the left only in combination** | | **On the right only in combination** | |
| **...n...** | -$C_nH_{2n}$- | **-...n...** | -$C_nH_{2n}$- |
| **-...M...-** | -CFH- | **-...M...** | -CFH- |
| **-...0...-** | -$CF_2$- | **-...D...** | -$CF_2$ |
| **-...V...-** | -CH=CH- | **-...V...** | -CH=CH- |
| **-...Z...-** | -CO-O- | **-...Z...** | -CO-O- |
| **-...ZI...-** | -O-CO- | **-...ZI...** | -O-CO- |
| **-...K...-** | -CO- | **-...K...** | -CO- |
| **-...W...-** | -CF=CF- | **-...W...** | -CF=CF- |
| **-...O...-** | -O- | **-...O...-** | -O- |

in which n and m are each integers, and the three dots "..." are placeholders for other abbreviations from this table.

Besides the compounds of formula B, the mixtures according to the invention preferably comprise one or more compounds of the compounds mentioned below.

The following abbreviations are used: (n, m, k and l are, independently of one another, each an integer, preferably 1 to 9 preferably 1 to 7, k and l possibly may be also 0 and preferably are 0 to 4, more preferably 0 or 2 and most preferably 2, n preferably is 1, 2, 3, 4 or 5, in the combination "-nO-" it preferably is 1, 2, 3 or 4, preferably 2 or 4, m preferably is 1, 2, 3, 4 or 5, in the combination "-Om" it preferably is 1, 2, 3 or 4, more preferably 2 or 4. The combination "-IVm" preferably is "2V1".)

**Table D**

Exemplary, preferred dielectrically positive compounds

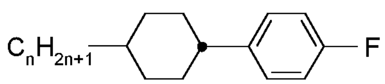

**CP-n-F**

(continued)

Exemplary, preferred dielectrically positive compounds

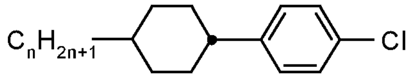

**CP-n-CL**

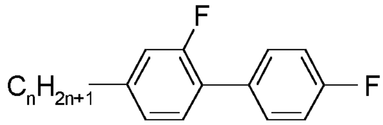

**GP-n-F**

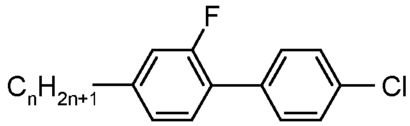

**GP-n-CL**

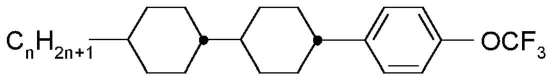

**CCP-n-OT**

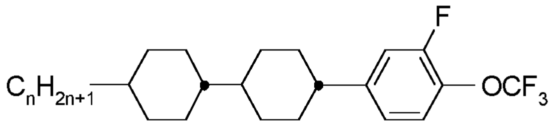

**CCG-n-OT**

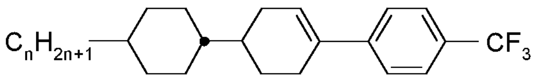

**CLP-n-T**

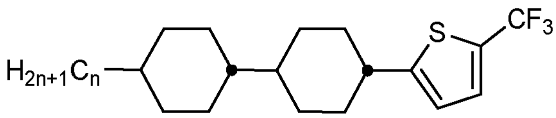

**CCS-n-T**

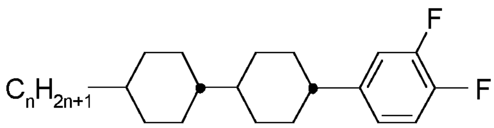

**CCG-n-F**

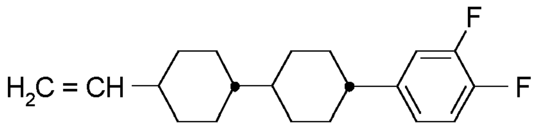

**CCG-V-F**

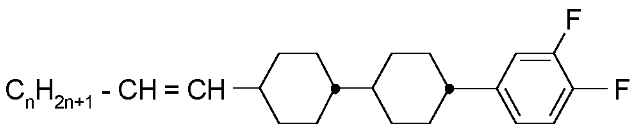

**CCG-nV-F**

(continued)

Exemplary, preferred dielectrically positive compounds

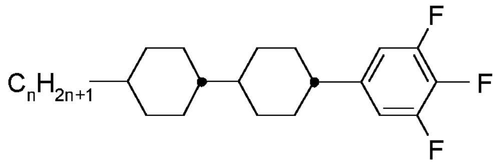

**CCU-n-F**

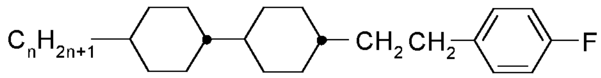

**CCEP-n-F**

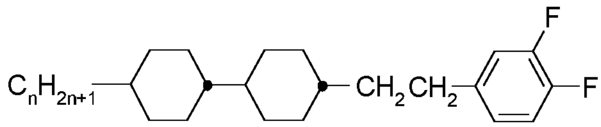

**CCEU-n-F**

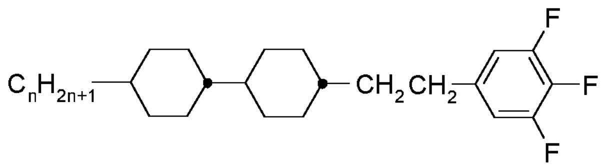

**CCEU-n-F**

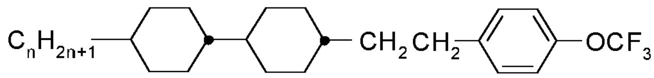

**CCEP-n-OT**

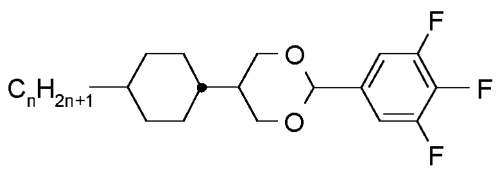

**CDU-n-F**

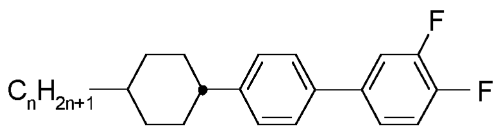

**CPG-n-F**

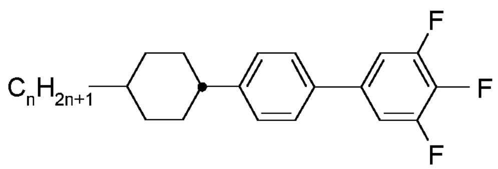

**CPU-n-F**

(continued)

Exemplary, preferred dielectrically positive compounds

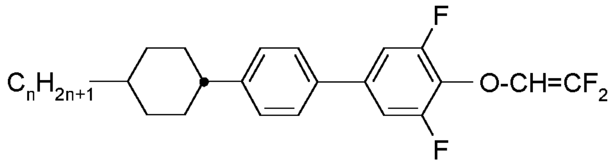

**CPU-n-OXF**

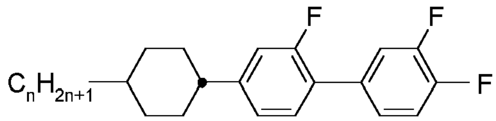

**CGG-n-F**

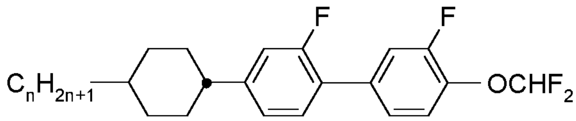

**CGG-n-OD**

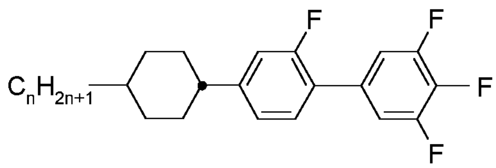

**CGU-n-F**

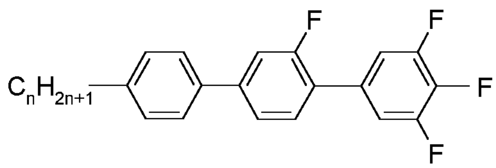

**PGU-n-F**

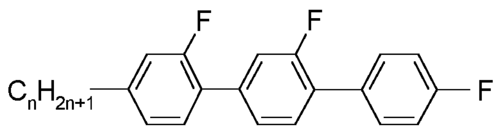

**GGP-n-F**

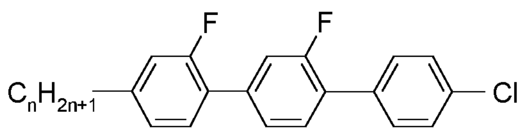

**GGP-n-CL**

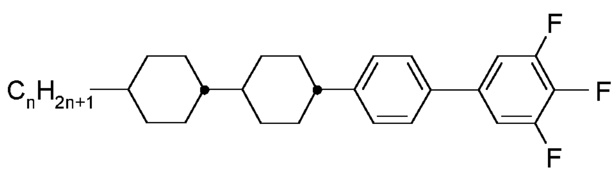

**CCPU-n-F**

(continued)

Exemplary, preferred dielectrically positive compounds

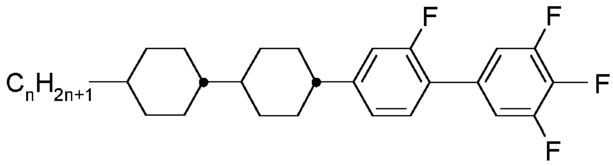

**CCGU-n-F**

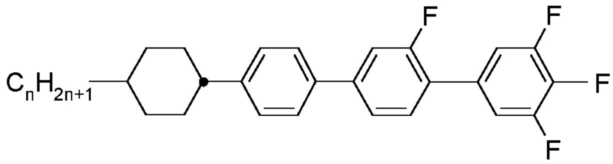

**CPGU-n-F**

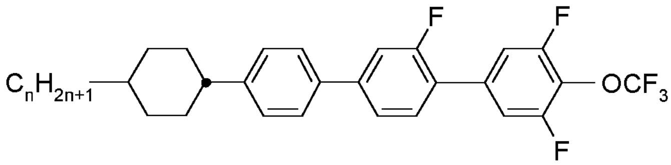

**CPGU-n-OT**

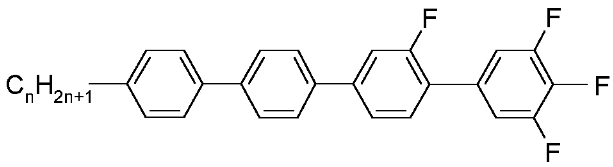

**PPGU-n-F**

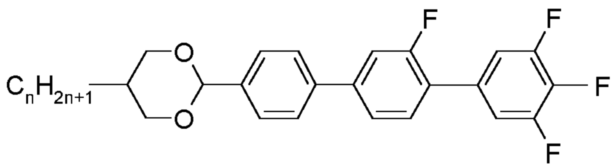

**DPGU-n-F**

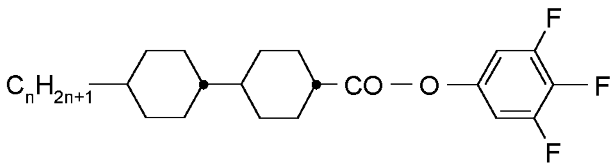

**CCZU-n-F**

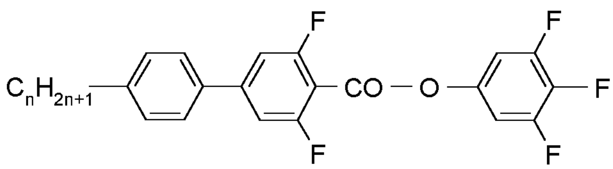

**PUZU-n-F**

(continued)

Exemplary, preferred dielectrically positive compounds

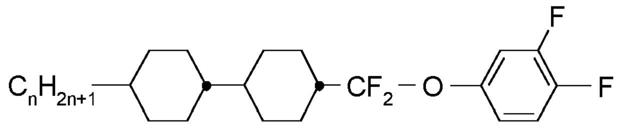

**CCQG-n-F**

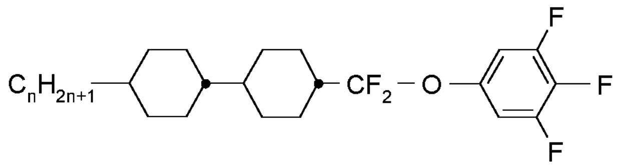

**CCQU-n-F**

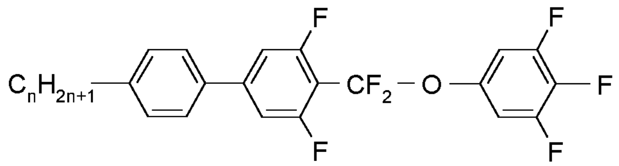

**PUQU-n-F**

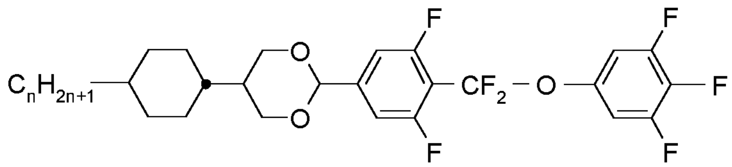

**CDUQU-n-F**

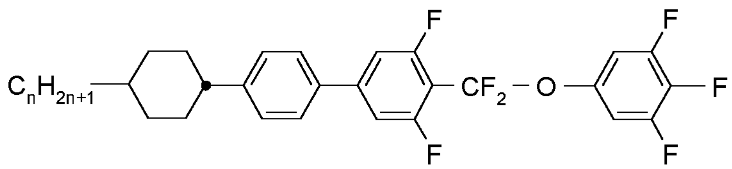

**CPUQU-n-F**

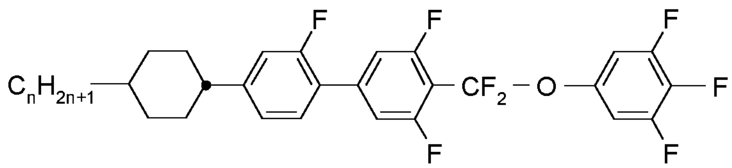

**CGUQU-n-F**

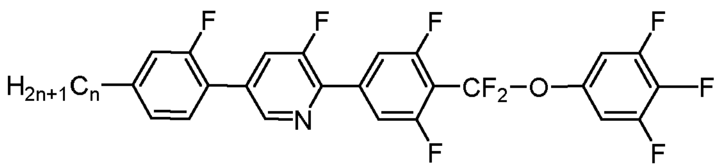

**GnfUQU-n-F**

(continued)

Exemplary, preferred dielectrically positive compounds

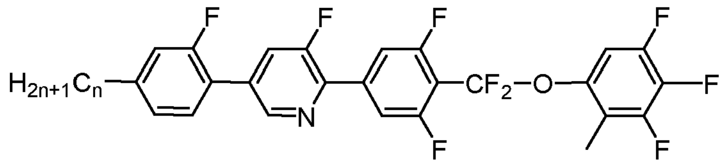

**GnfUQU(Me)-n-F**

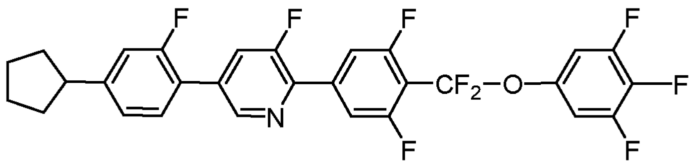

**GnfUQU-cp-F**

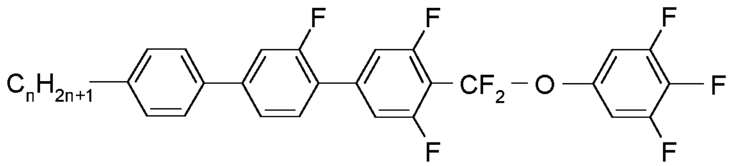

**PGUQU-n-F**

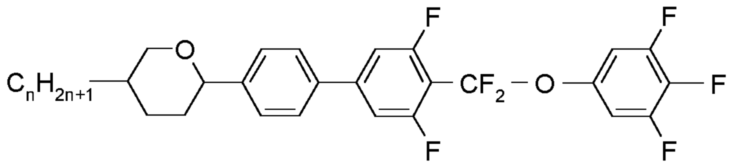

**APUQU-n-F**

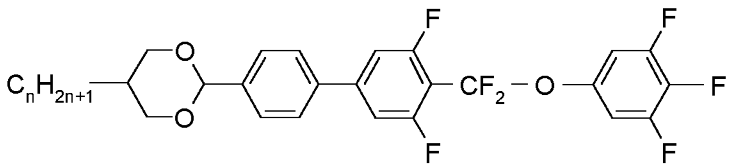

**DPUQU-n-F**

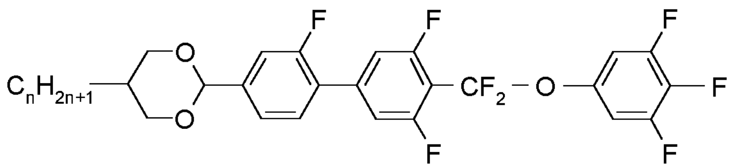

**DGUQU-n-F**

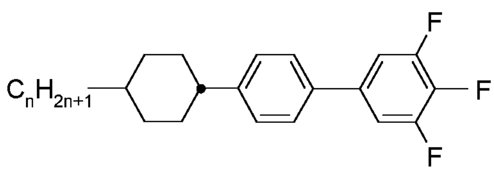

**CPU-n-F**

(continued)

Exemplary, preferred dielectrically positive compounds

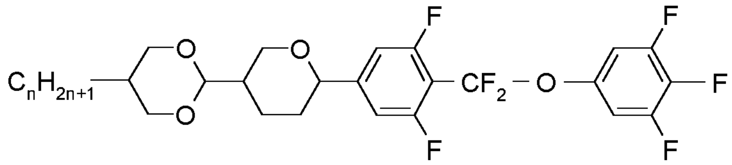

**DAUQU-n-F**

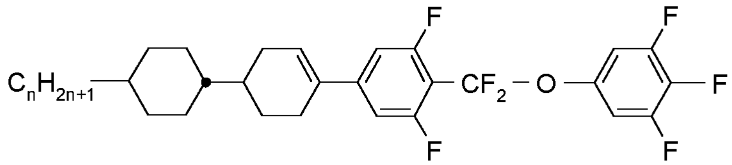

**CLUQU-n-F**

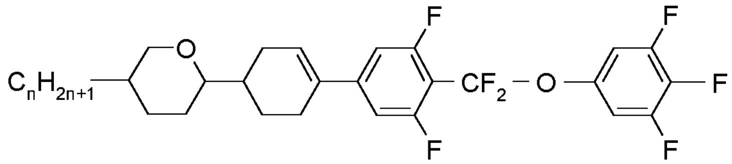

**ALUQU-n-F**

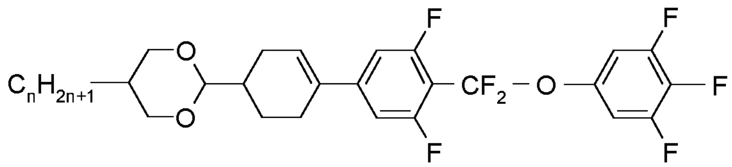

**DLUQU-n-F**

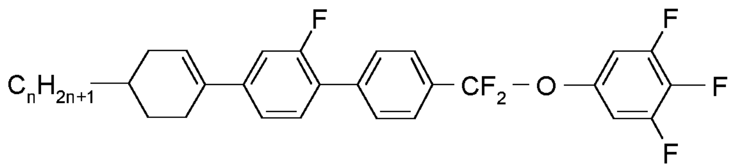

**LGPQU-n-F**

Exemplary, preferred dielectrically neutral compounds

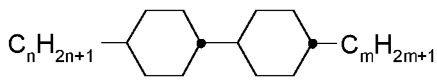

**CC-n-m**

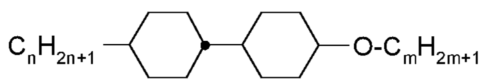

**CC-n-Om**

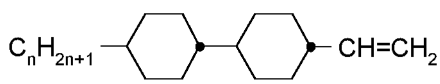

**CC-n-V**

(continued)

Exemplary, preferred dielectrically positive compounds

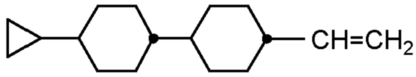

**CC-cpr-V**

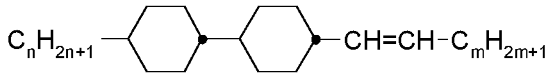

**CC-n-Vm**

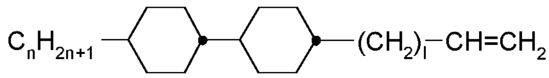

**CC-n-IV**

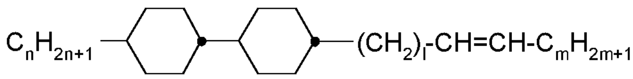

**CC-n-IVm**

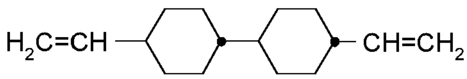

**CC-V-V**

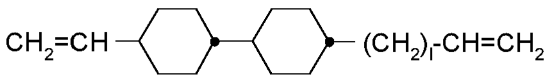

**CC-V-IV**

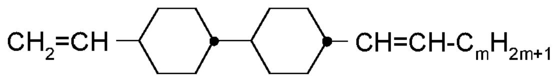

**CC-V-Vm**

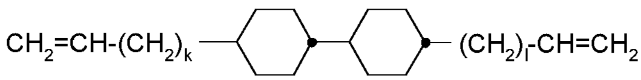

**CC-Vk-IV**

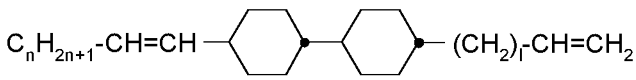

**CC-nV-IV**

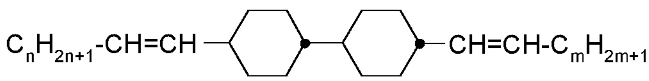

**CC-nV-Vm**

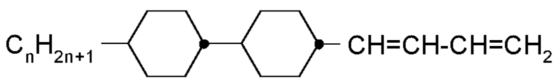

**CC-n-VV**

(continued)

Exemplary, preferred dielectrically positive compounds

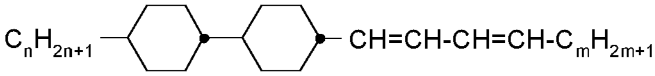

**CC-n-VVm**

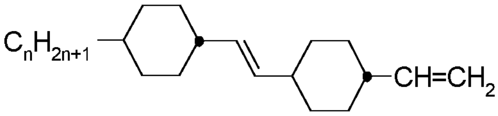

**CVC-n-V**

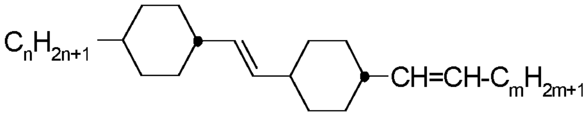

**CVC-n-Vm**

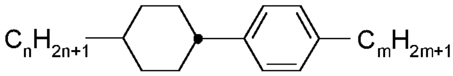

**CP-n-m**

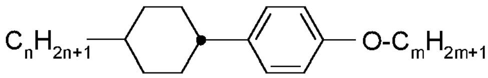

**CP-n-Om**

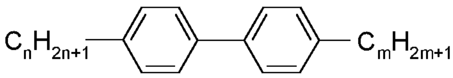

**PP-n-m**

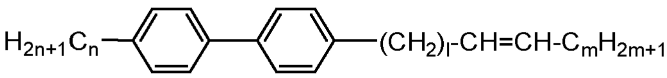

**PP-n-lVm**

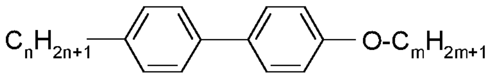

**PP-n-Om**

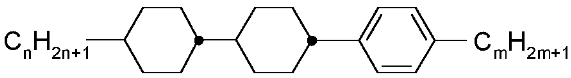

**CCP-n-m**

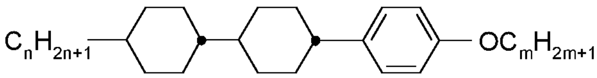

**CCP-n-Om**

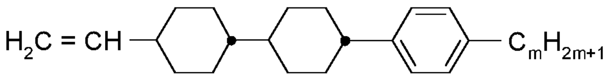

**CCP-V-m**

(continued)

Exemplary, preferred dielectrically positive compounds

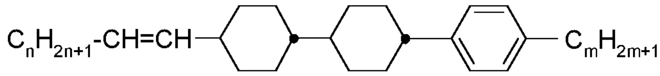

**CCP-nV-m**

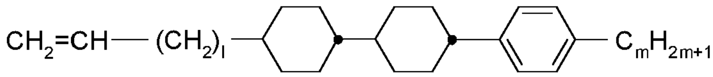

**CCP-Vl-m**

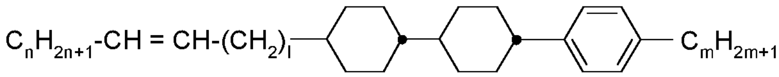

**CCP-nVl-m**

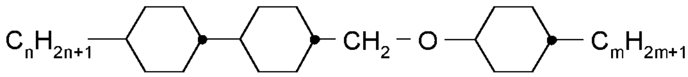

**CCOC-n-m**

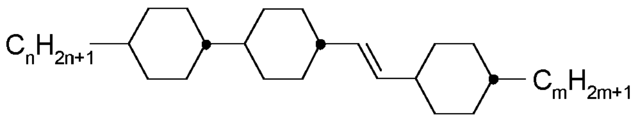

**CCVC-n-m**

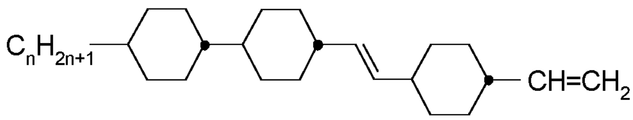

**CCVC-n-V**

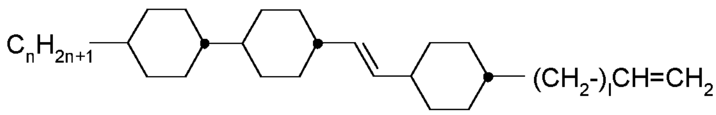

**CCVC-n-IV**

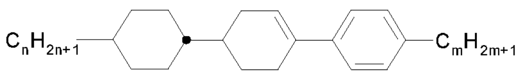

**CLP-n-m**

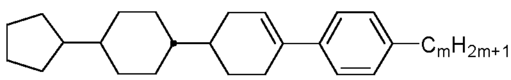

**CLP-cp-m**

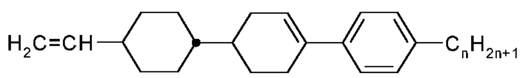

**CLP-V-n**

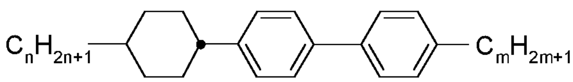

**CPP-n-m**

(continued)

Exemplary, preferred dielectrically positive compounds

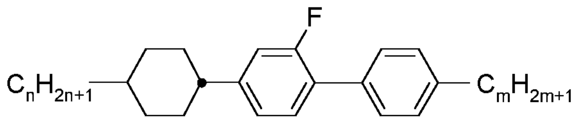

**CPP-n-m**

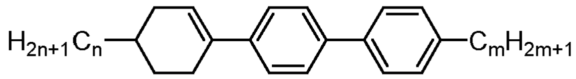

**LPP-n-m**

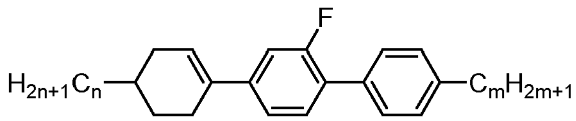

**LGP-n-m**

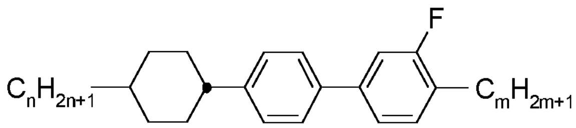

**CPG-n-m**

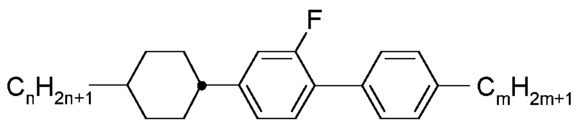

**CGP-n-m**

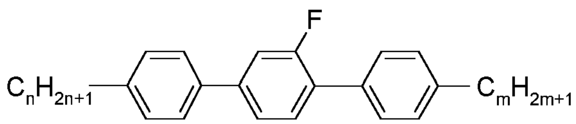

**PGP-n-m**

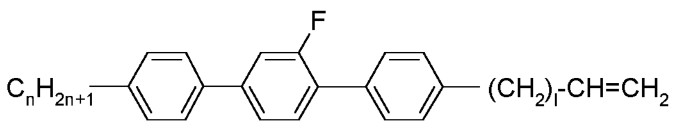

**PGP-n-lV**

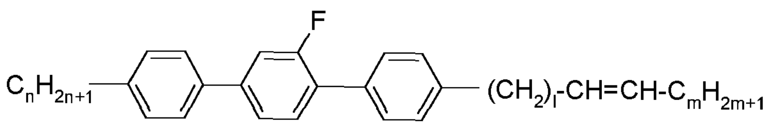

**PGP-n-lVm**

(continued)

Exemplary, preferred dielectrically positive compounds

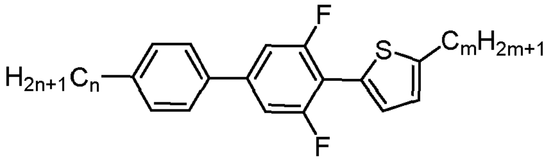

**PUS-n-m**

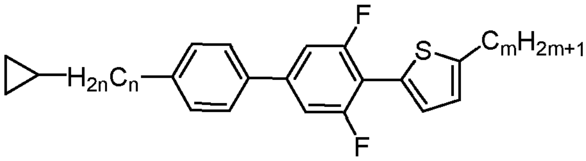

**PUS-cprn-m**

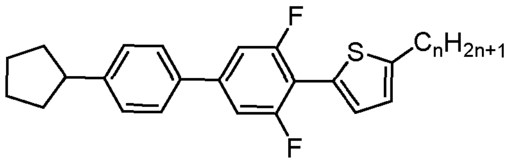

**PUS-cp-n**

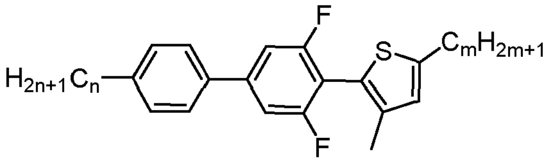

**PUS(Me)-n-m**

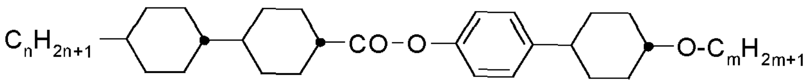

**CCZPC-n-m**

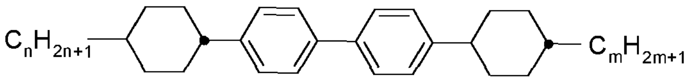

**CPPC-n-m**

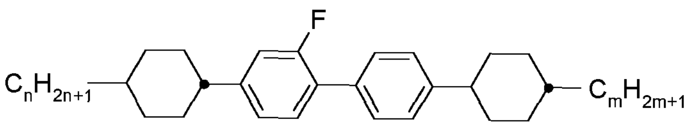

**CGPC-n-m**

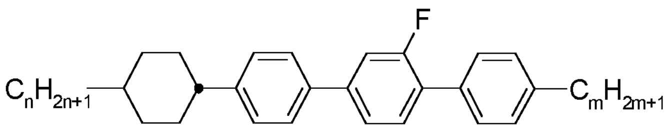

**CPGP-n-m**

(continued)

Exemplary, preferred dielectrically positive compounds

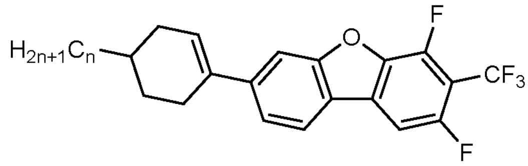

**LBh-n-T**

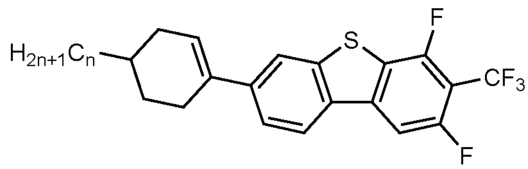

**LBh(S)-n-T**

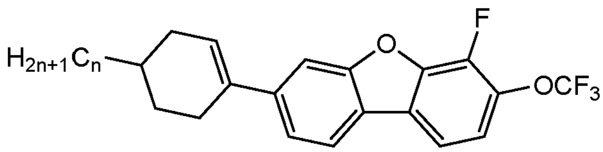

**LBf-n-OT**

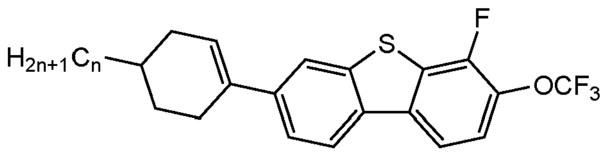

**LBf(S)B-n-OT**

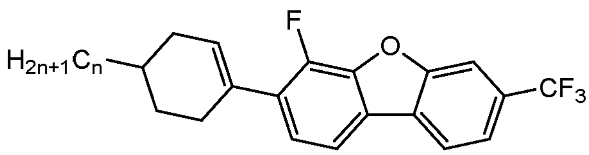

**LBfi-n-T**

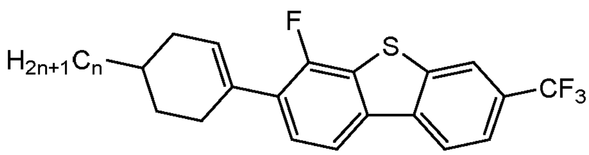

**LBfi(S)B-n-T**

Exemplary compounds having a high $\varepsilon\perp$:

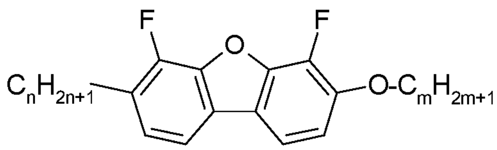

**B-n-Om**

(continued)

Exemplary, preferred dielectrically positive compounds

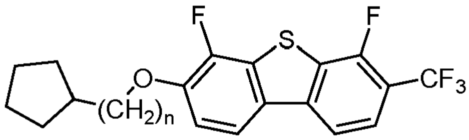

**B(S)-cpnO-T**

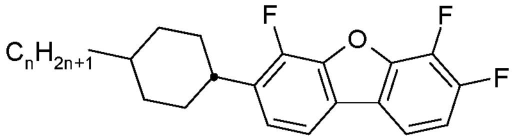

**CB-n-F**

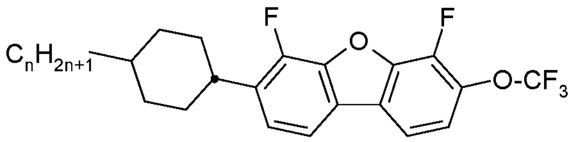

**CB-n-OT**

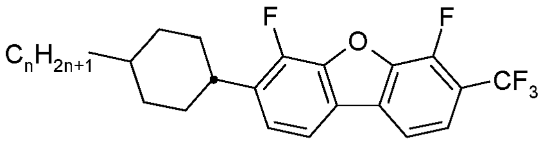

**CB-n-T**

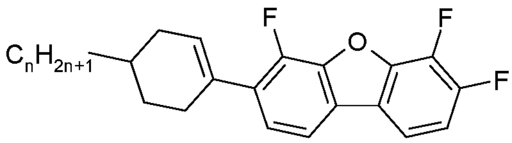

**LB-n-F**

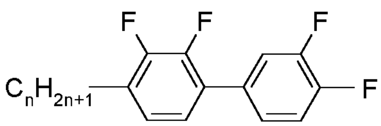

**YG-n-F**

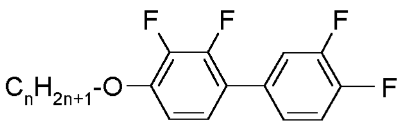

**YG-nO-F**

Exemplary, preferred dielectrically negative compounds

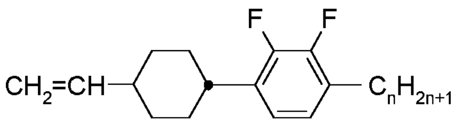

**CY-V-n**

(continued)

Exemplary, preferred dielectrically positive compounds

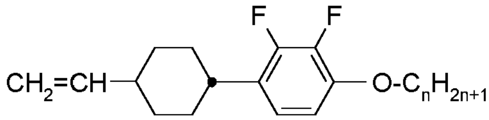

**CY-V-On**

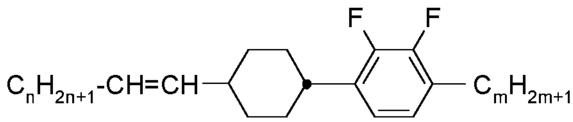

**CY-nV-m**

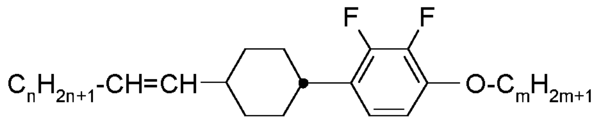

**CY-nV-Om**

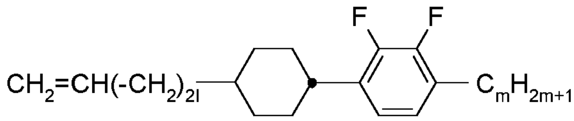

**CY-Vl-m**

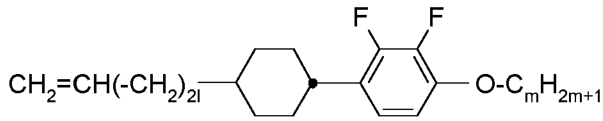

**CY-Vl-Om**

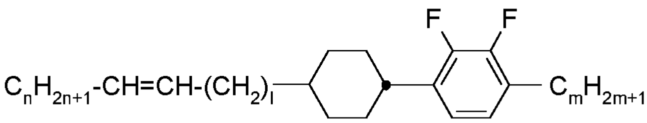

**CY-nVl-m**

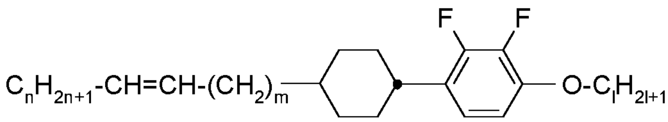

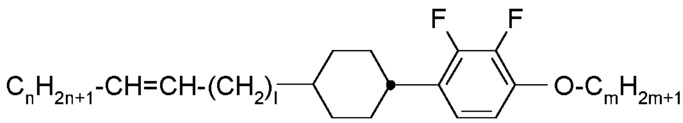

**CY-nVl-Om**

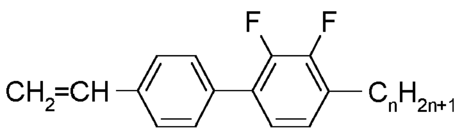

**PY-V-n**

(continued)

Exemplary, preferred dielectrically positive compounds

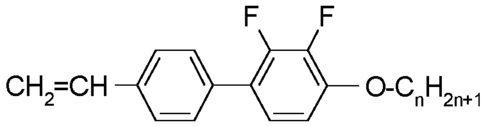

**PY-V-On**

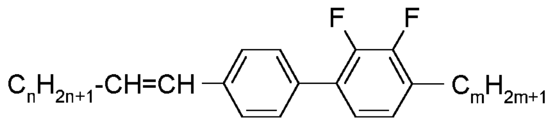

**PY-nV-m**

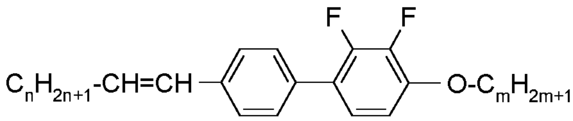

**PY-nV-Om**

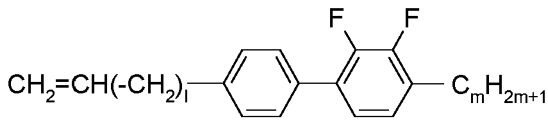

**PY-Vl-m**

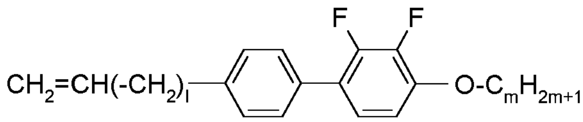

**PY-Vl-Om**

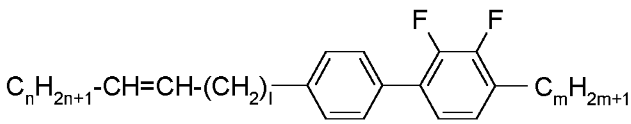

**PY-nVl-m**

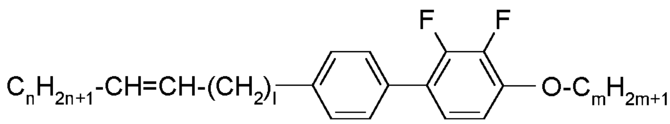

**PY-nVl-Om**

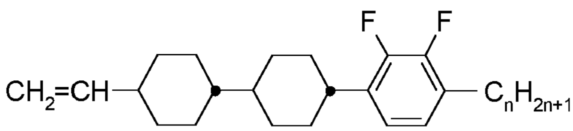

**CCY-V-n**

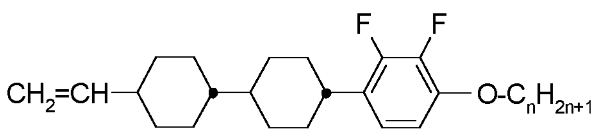

**CCY-V-On**

(continued)

Exemplary, preferred dielectrically positive compounds

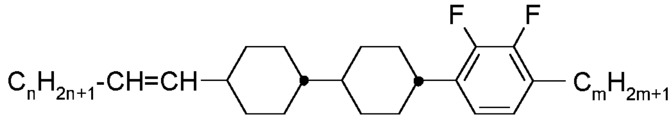

**CCY-nV-m**

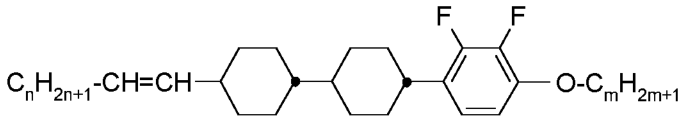

**CCY-nV-Om**

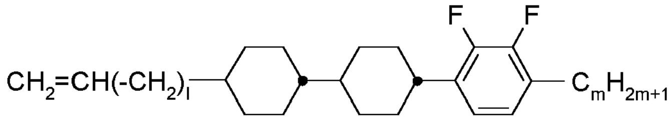

**CCY-Vl-m**

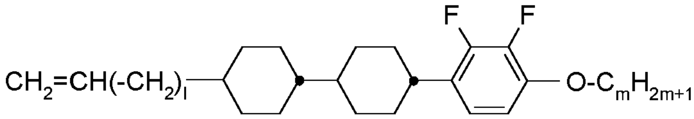

**CCY-Vl-Om**

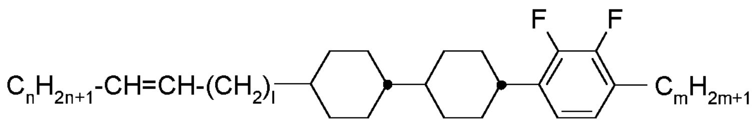

**CCY-nVl-m**

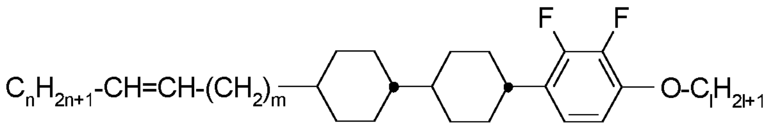

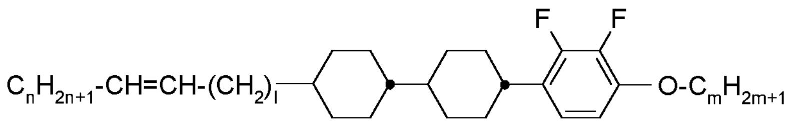

**CCY-nVl-Om**

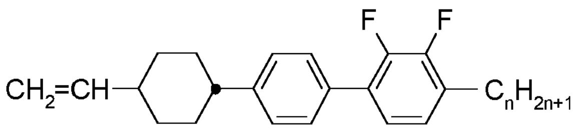

**CPY-V-n**

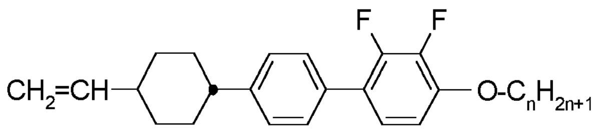

**CPY-V-On**

(continued)

Exemplary, preferred dielectrically positive compounds

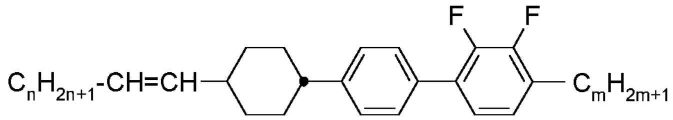

**CPY-nV-m**

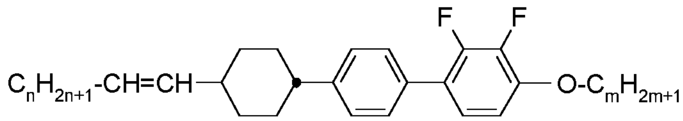

**CPY-nV-Om**

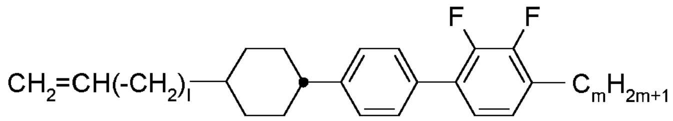

**CPY-Vl-m**

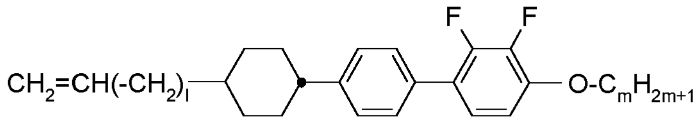

**CPY-Vl-Om**

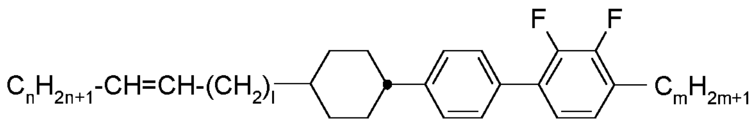

**CPY-nVl-k**

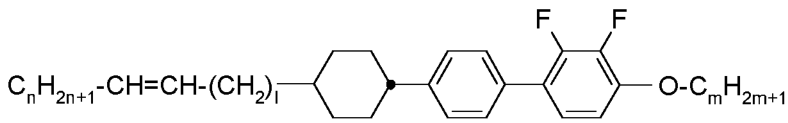

**CPY-nVl-Om**

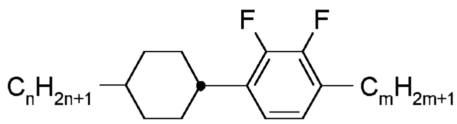

**CY-n-m**

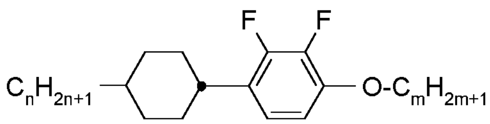

**CY-n-Om**

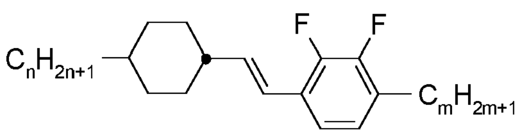

**CVY-n-m**

(continued)

Exemplary, preferred dielectrically positive compounds

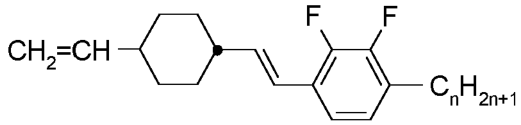

**CVY-V-n**

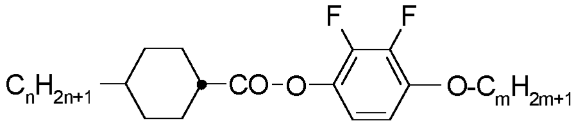

**CZY-n-Om**

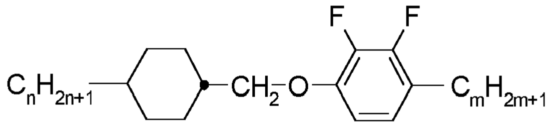

**COY-n-m**

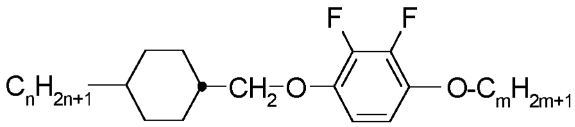

**COY-n-Om**

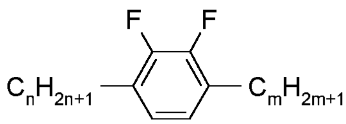

**Y-n-m**

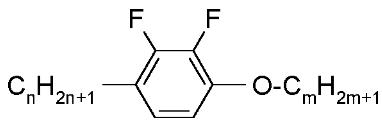

**Y-n-Om**

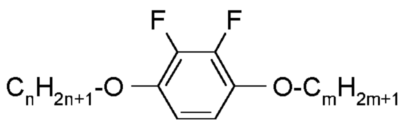

**Y-nO-Om**

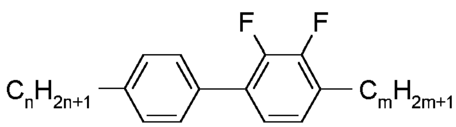

**PY-n-m**

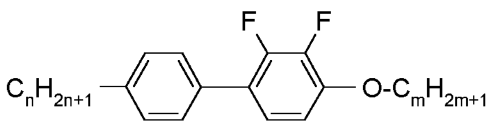

**PY-n-Om**

(continued)

Exemplary, preferred dielectrically positive compounds

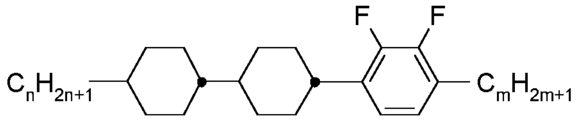

**CCY-n-m**

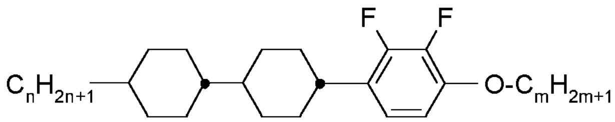

**CCY-n-Om**

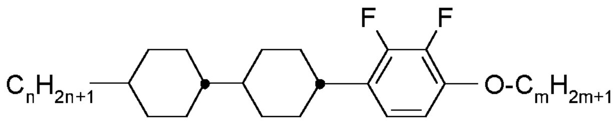

**CCY-cp-Om**

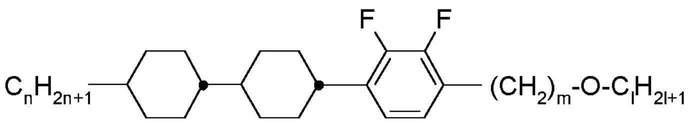

**CCY-n-mOl**

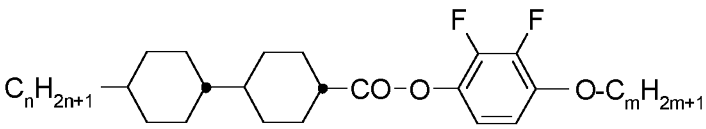

**CCZY-n-Om**

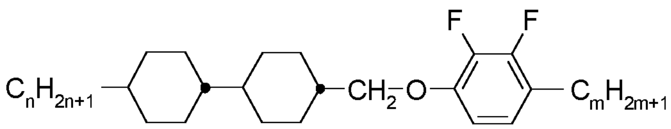

**CCOY-n-m**

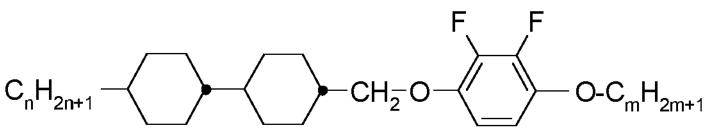

**CCOY-n-Om**

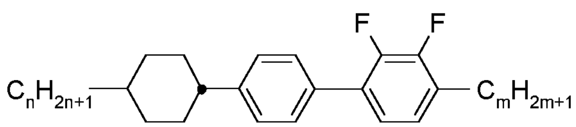

**CPY-n-m**

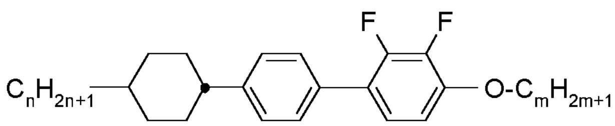

**CPY-n-Om**

(continued)

Exemplary, preferred dielectrically positive compounds

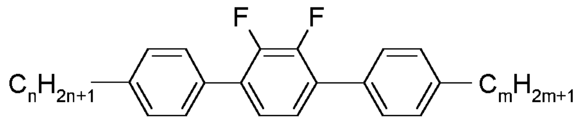

**PYP-n-m**

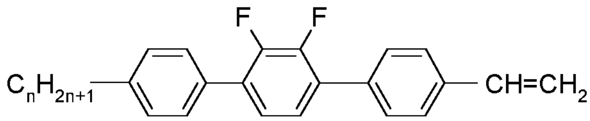

**PYP-n-V**

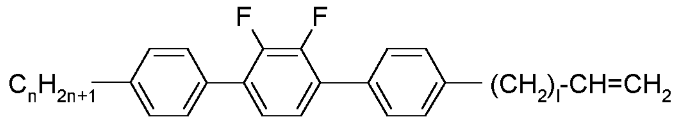

**PYP-n-lV**

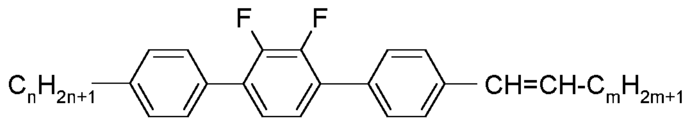

**PYP-n-Vm**

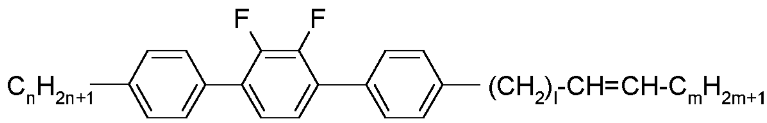

**PYP-n-lVm**

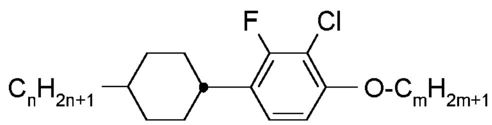

**CP(F,Cl)-n-Om**

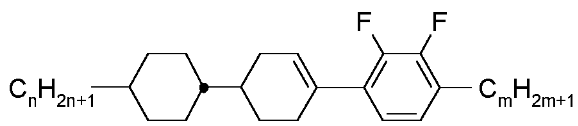

**CLY-n-m**

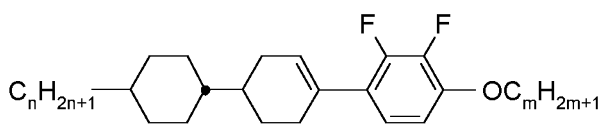

**CLY-n-Om**

Table E shows chiral dopants which are preferably employed in the mixtures according to the invention.

## Table E

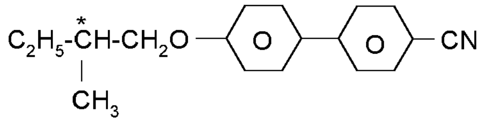

C 15

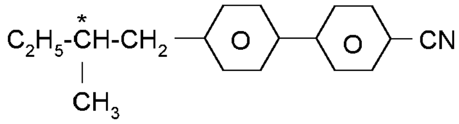

**CB 15**

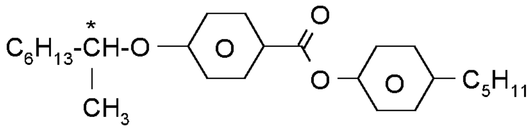

**CM 21**

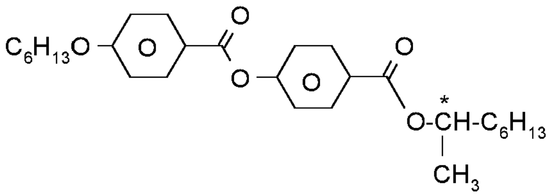

**R S-811 / S-811**

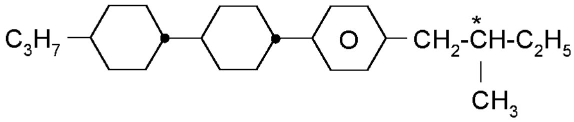

**CM 44**

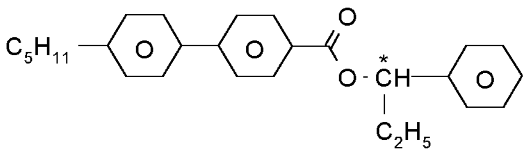

**CM 45**

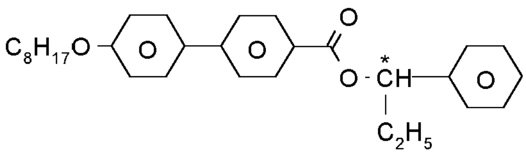

**CM 47**

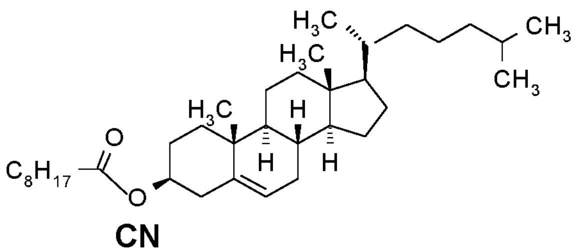

**CN**

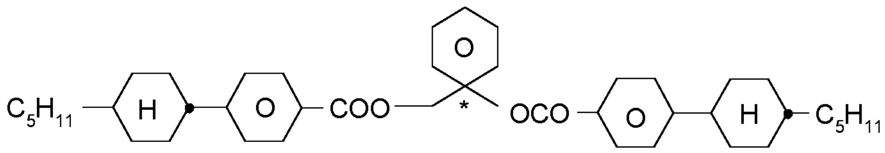

**R-1011 / S-1011**

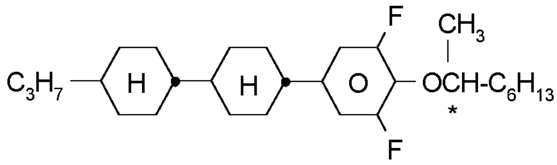

**R-2011 / S-2011**

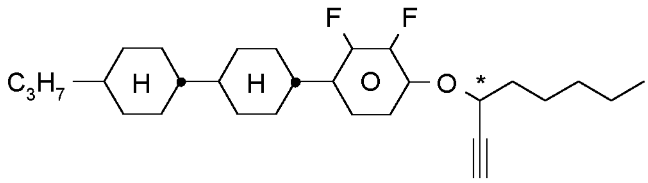

**R-3011 / S-3011**

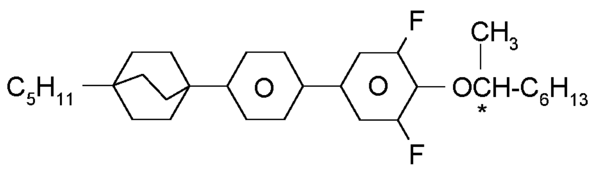

**R-4011 / S-4011**

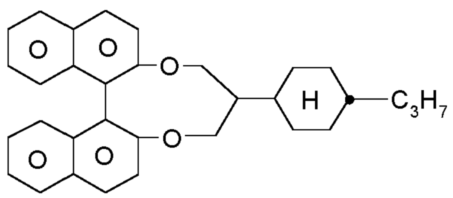

**R-5011 / S-5011**

In a preferred embodiment of the present invention, the media according to the invention comprise one or more compounds selected from the group of the compounds from Table E.

Table F shows stabilisers which can preferably be employed in addition in the mixtures according to the invention. The parameter n here denotes an integer in the range from 1 to 12. In particular, the phenol derivatives shown can be employed as additional stabilisers since they act as antioxidants.

## Table F

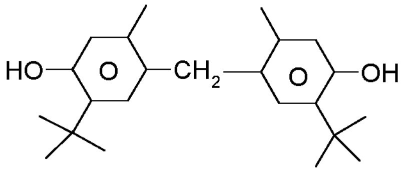

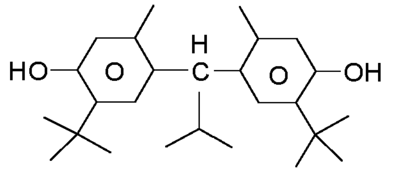

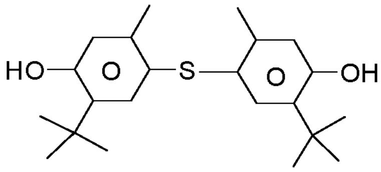

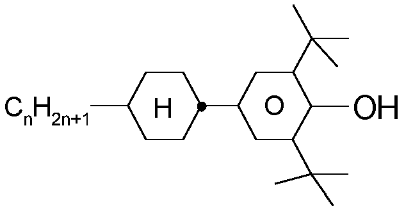

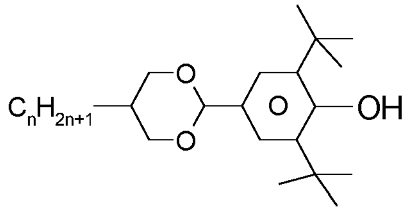

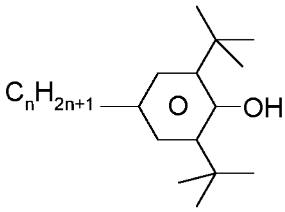

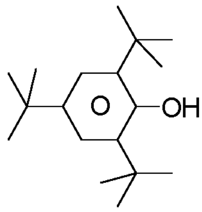

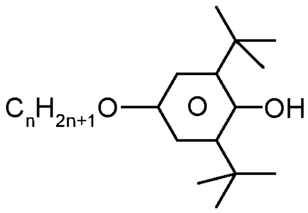

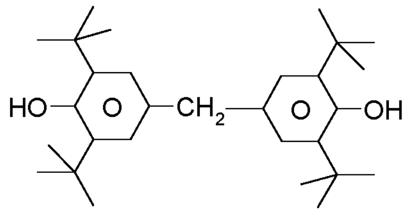

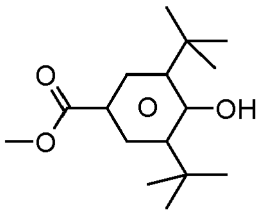

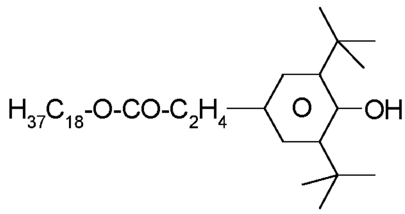

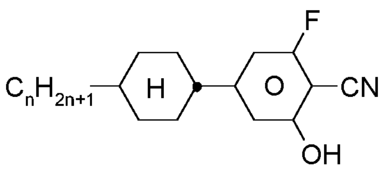

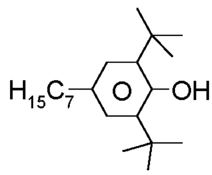

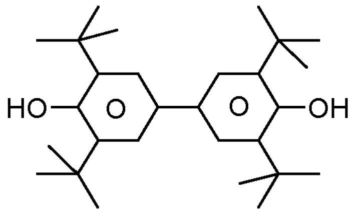

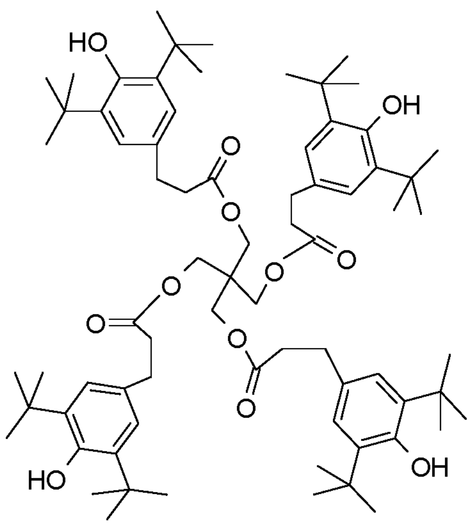

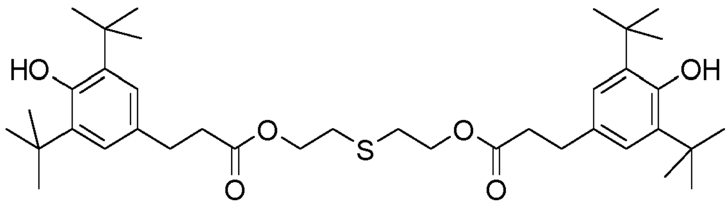

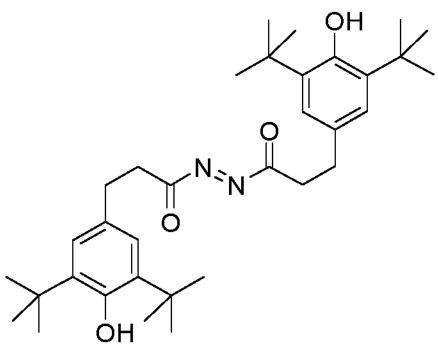

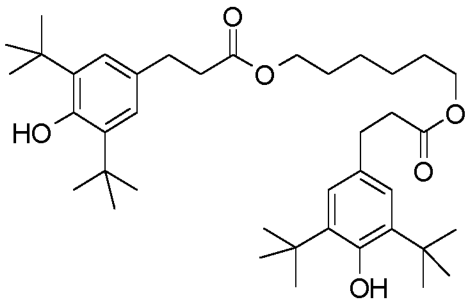

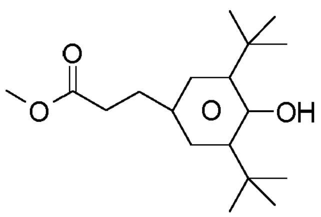

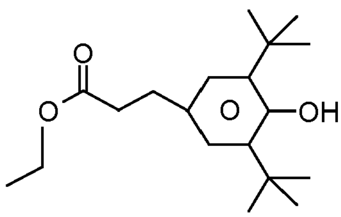

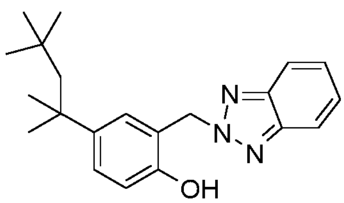

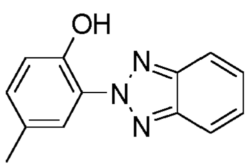

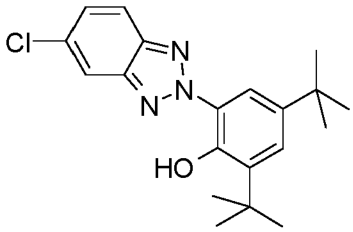

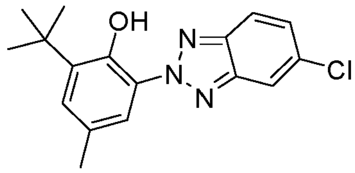

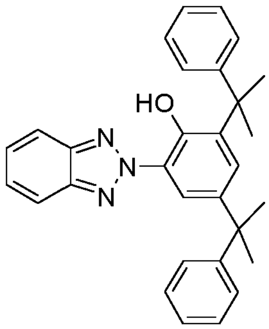

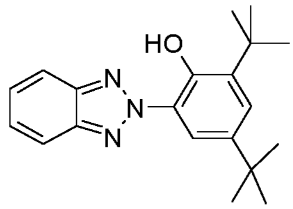

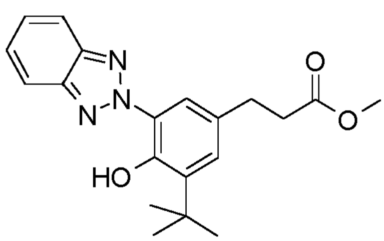

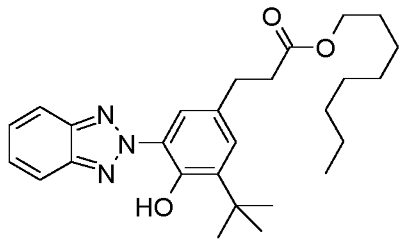

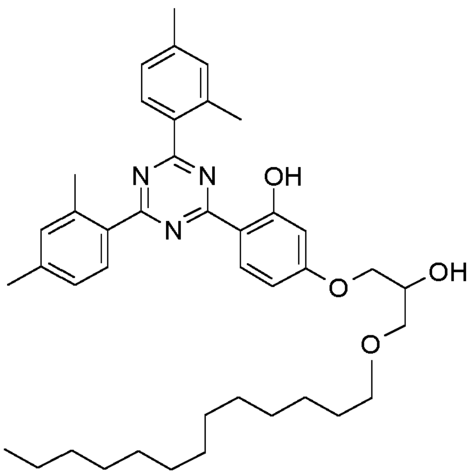

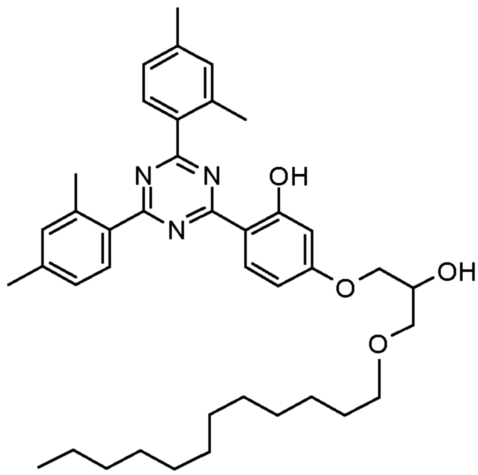

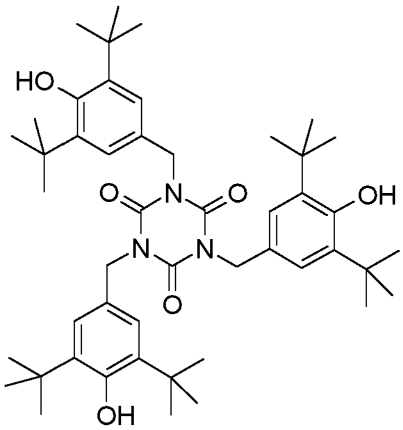

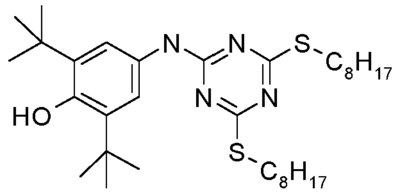

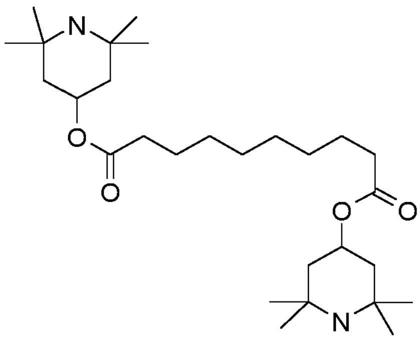

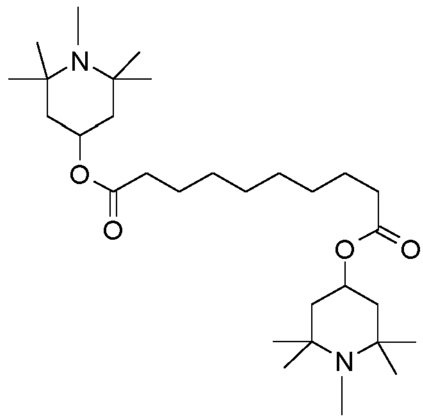

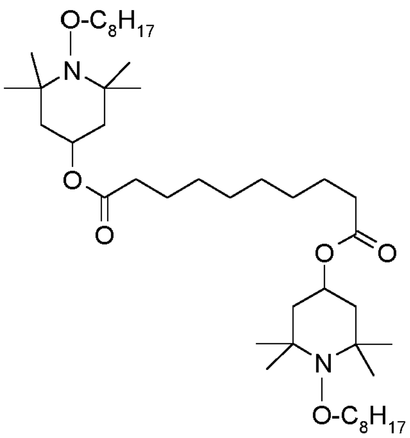

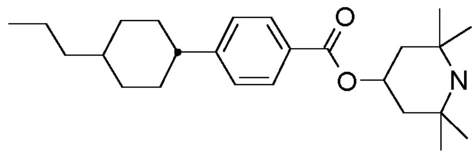

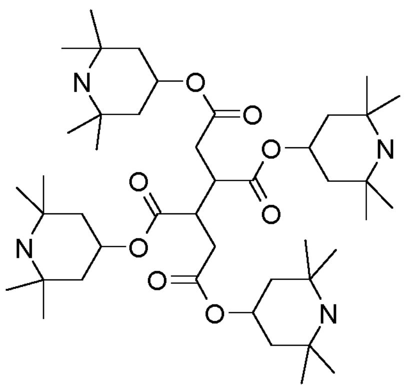

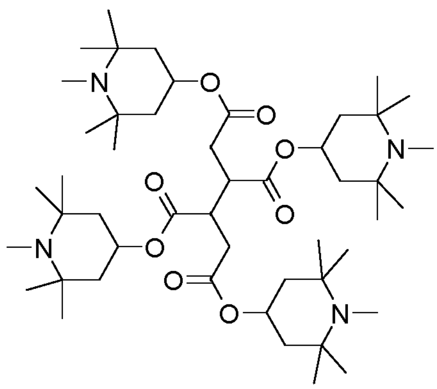

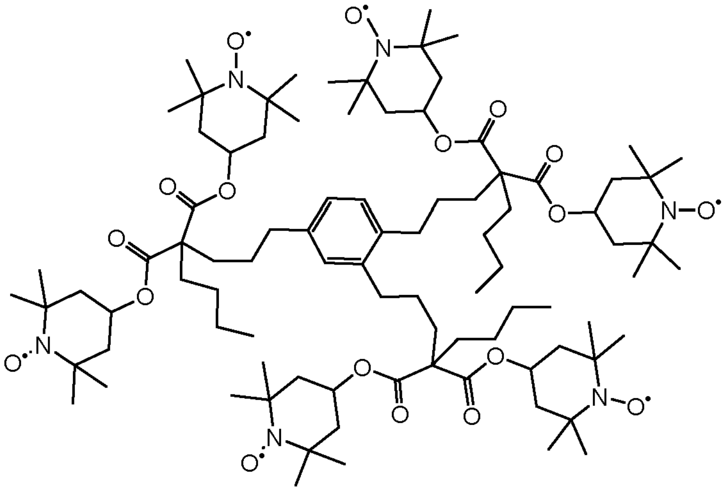

In a preferred embodiment of the present invention, the media according to the invention comprise one or more compounds selected from the group of the compounds from Table F, in particular one or more compounds selected from the group of the compounds of the following two formulae

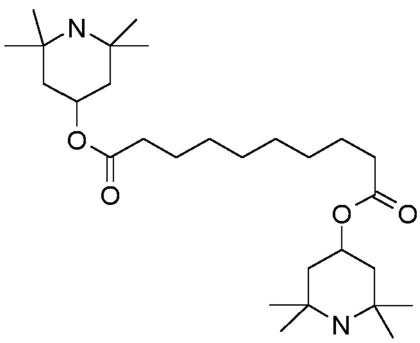

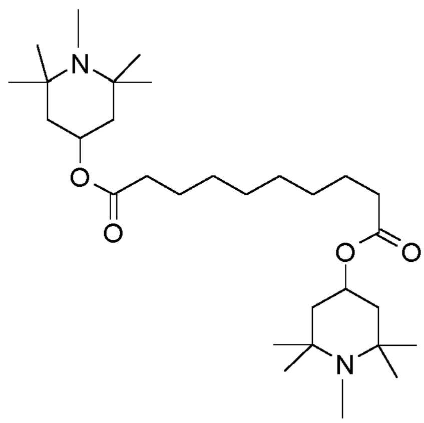

Examples

The following examples explain the present invention without restricting it in any way. However, the physical properties make it clear to the person skilled in the art what properties can be achieved and in what ranges they can be modified. In particular, the combination of the various properties which can preferably be achieved is thus well defined for the person skilled in the art.

Synthesis Examples

A synthesis of fluoropyridines is disclosed in WO 2008/128622 A1 and in the following examples.

Synthesis Example 1: Synthesis of GnfUQU-3-F

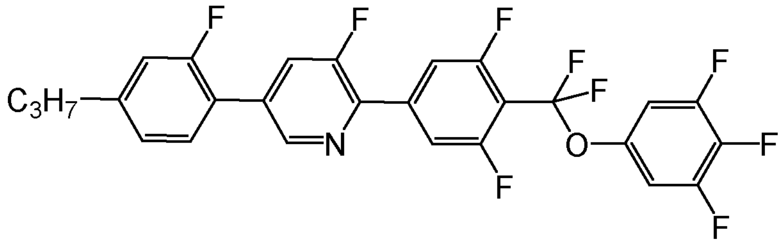

Step 1.1

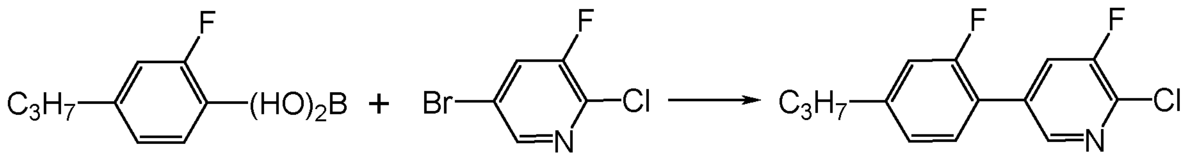

0.7 g of tetrakis(triphenylphosphine)palladium, 9.1 g (50 mmol) of the boronic acid and 10.5 g (50 mmol) of the pyridine derivative are added to a mixture of 100 ml of toluene and 50 ml of 2 N sodium carbonate solution. After 60 h at 60°C, the reaction mixture is diluted with MTB ether. The organic phase is evaporated. The residue is filtered through silica gel (n-heptane).

Step 1.2

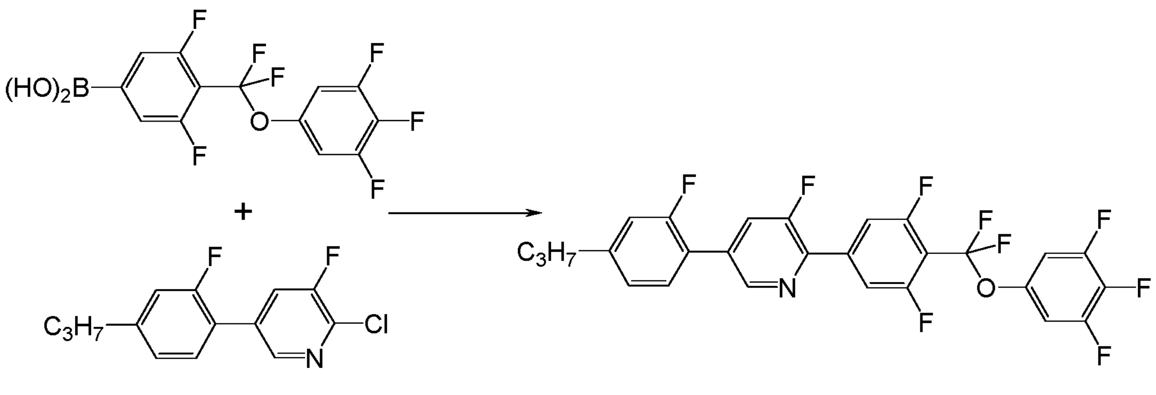

12.6 g (45 mmol) of sodium metaborate octahydrate are initially introduced in 23 ml of water, and 25 ml of THF, 0.1 ml (0.7 mmol) of hydrazinium hydroxide and 0.7 g (1 mmol) of bis(triphenylphosphine)palladium(II) chloride are added, and the mixture is stirred at RT for 5 min. A solution of 10.6 g (30 mmol) of the boronic acid and 8.0 g (30 mmol) of the chloropyridine is subsequently added to the batch. After 16 h under reflux, the reaction mixture is diluted with MTB ether. The organic phase is evaporated. The residue is filtered through silica gel (n-heptane). The final purification of the product is carried out by crystallisation from heptane.

Phases: C 70 SmA (64) N 118 I
$\Delta\varepsilon$: 41

Δn: 0.21
$\gamma_1$ : 241 mPa·s

Further Compound Examples

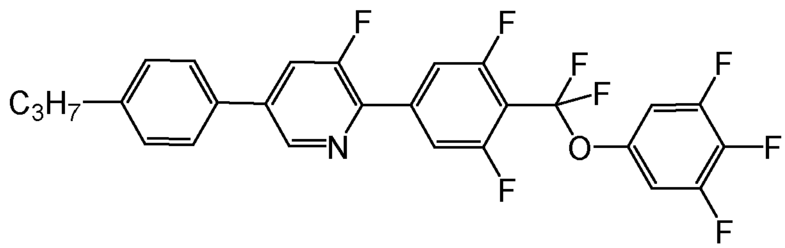

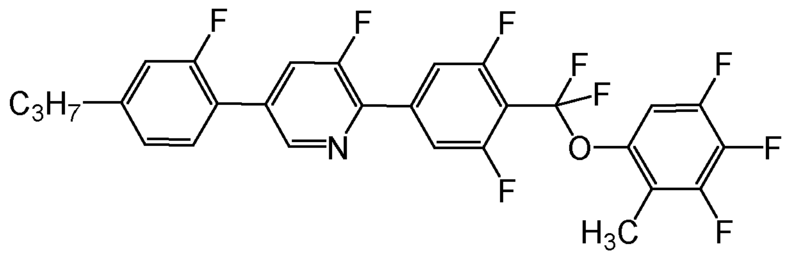

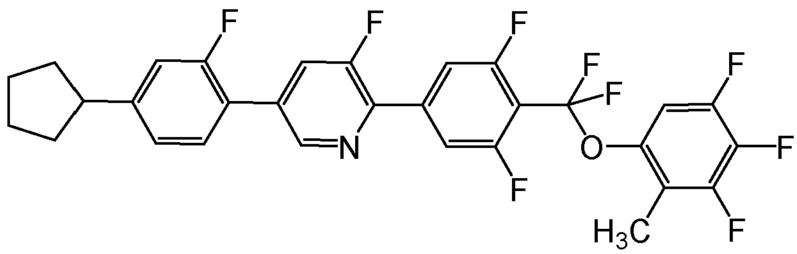

In the following table the following abbreviations for the end groups are used

| | |
|---|---|
| *c*-$C_3H_5$ | 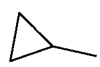 |
| *c*-$C_3H_5CH_2$ | 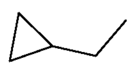 |
| *c*-$C_4H_7$ | 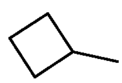 |
| *c*-$C_5H_7$ | 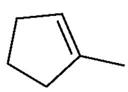 |
| *c*-$C_5H_9$ | 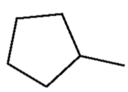 |

The physical properties are given at a temperature of 20°C and $\gamma_1$ is given in mPa·s.

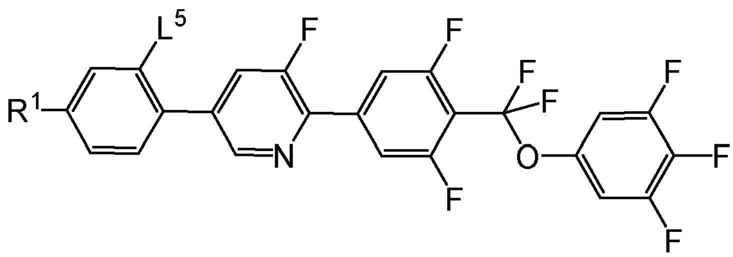

| Ex. | $R^1$ | $L^5$ | Phase Range; properties |
|---|---|---|---|
| 1 | $CH_3$ | H | |
| 2 | $C_2H_5$ | H | |
| 3 | *n*-$C_3H_7$ | H | Tg -51 K 60 SmA 85 N 128 I |
| 4 | *n*-$C_4H_9$ | H | |
| 5 | *n*-$C_5H_{11}$ | H | |
| 6 | *n*-$C_6H_{13}$ | H | |
| 7 | *n*-$C_7H_{15}$ | H | |
| 8 | *n*-$C_8H_{17}$ | H | |
| 9 | *c*-$C_3H_5$ | H | |
| 10 | *c*-$C_3H_5$-$CH_2$ | H | |
| 11 | *c*-$C_4H_7$ | H | |
| 12 | *c*-$C_5H_7$ | H | |
| 13 | *c*-$C_5H_9$ | H | |
| 14 | *c*-$C_5H_9CH_2$ | H | |
| 15 | $CH_2$=CH | H | |
| 16 | $CH_3CH$=CH | H | |
| 17 | $CH_2$=CH($CH_2$)$_2$ | H | |
| 18 | $CH_3CH$=CH($CH_2$)$_2$ | H | |
| 19 | $CH_3$ | F | |
| 20 | $C_2H_5$ | F | |
| 21 | *n*-$C_3H_7$ | F | K 70 SmA (64) N 118 I |
| 22 | *n*-$C_4H_9$ | F | |
| 23 | *n*-$C_5H_{11}$ | F | |
| 24 | *n*-$C_6H_{13}$ | F | |
| 25 | *n*-$C_7H_{15}$ | F | |
| 26 | *n*-$C_8H_{17}$ | F | |
| 27 | *c*-$C_3H_5$ | F | |
| 28 | *c*-$C_3H_5$-$CH_2$ | F | |

(continued)

| Ex. | $R^1$ | $L^5$ | Phase Range; properties |
|---|---|---|---|
| 29 | *c*-$C_4H_7$ | F | |
| 30 | *c*-$C_5H_7$ | F | |
| 31 | *c*-$C_5H_9$ | F | |
| 32 | *c*-$C_5H_9CH_2$ | F | |
| 33 | $CH_2$=CH | F | |
| 34 | $CH_3$CH=CH | F | |
| 35 | $CH_2$=CH($CH_2$)$_2$ | F | |
| 36 | $CH_3$CH=CH($CH_2$)$_2$ | F | |

Mixture Examples

In the following exemplary mixtures are disclosed. All % values are % by weight.

Mixture Example 1

The following mixture (M-1) is prepared and investigated.

| Compound | % | Properties | |
|---|---|---|---|
| CC-3-2V1 | 5.0 | T(N,I) [°C]: | 79.2 |
| CC-3-V | 35.0 | $\Delta$n [589 nm, 20°C]: | 0.1340 |
| CC-3-V1 | 8.0 | $n_e$ [589 nm, 20°C]: | 1.6271 |
| CCP-V2-1 | 6.25 | $n_o$ [589 nm, 20°C]: | 1.4931 |
| CLP-3-T | 8.0 | $\Delta\varepsilon$ [1 kHz, 20°C]: | 3.5 |
| GnfUQU-3-F | 4.0 | $\varepsilon_{\parallel}$ [1 kHz, 20°C]: | 6.1 |
| PGP-1-2V | 2.5 | $\varepsilon_{\perp}$ [1 kHz, 20°C]: | 2.6 |
| PGUQU-3-F | 3.25 | $\gamma_1$ [mPa s, 20°C]: | 57 |
| PP-1-2V1 | 10.0 | $K_{11}$ [pN, 20°C]: | 18.5 |
| PPGU-3-F | 0.5 | $K_{33}$ [pN, 20°C]: | 15.7 |
| PUS-3-2 | 17.5 | | |
| $\Sigma$ | 100.0 | | |

This mixture, mixture M-1 is characterized by a very good contrast and fast switching in a FFS display. It has a an average elastic constant ($K_{av.}$ = ($K_{11}$ + ½ $K_{11}$ + $K_{33}$) / 3) of 14.4 pN and a response time parameter ($\gamma_1/K_{11}$) of 3.1 mPa.s / pN.

Mixture Examples 1.1 to 1.4

Alternatively, 0.05 % of the compounds of one of the formulae

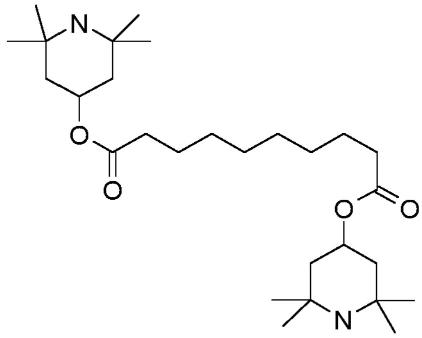

,

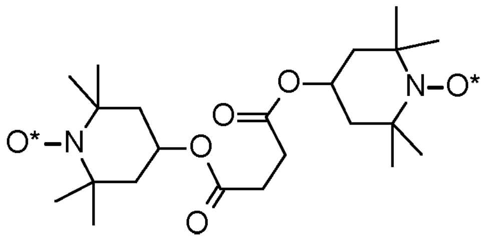

wherin the two O atoms bonded to the N atoms indicate radicals, and

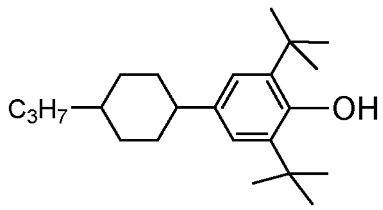

,

and

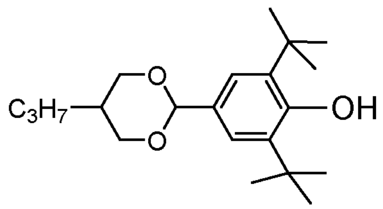

,

respectively
are added to the mixture M-1. The resultant mixtures M-1.1, M-1.2, M-1.3 and M-1.4 respectively are characterized by an improved stability against severe conditions, especially against exposure to light.

Comparative Mixture Example 1

The following mixture (C-1) is prepared and investigated.

| Compound | % | Properties | |
|---|---|---|---|
| CC-3-V | 47.0 | T(N,I) [°C]: | 75.3 |
| CC-3-V1 | 4.5 | Δn [589 nm, 20°C]: | 0.1344 |
| CLP-3-T | 7.0 | $n_e$ [589 nm, 20°C]: | 1.6290 |
| PGP-1-2V | 5.5 | $n_0$ [589 nm, 20°C]: | 1.4946 |
| PGP-2-2V | 10.0 | $\Delta_\varepsilon$ [1 kHz, 20°C]: | 3.0 |

(continued)

| Compound | % | Properties | |
|---|---|---|---|
| PGU-2-F | 1.0 | $\varepsilon_{\parallel}$ [1 kHz, 20°C]: | 5.7 |
| PGUQU-3-F | 4.0 | $\varepsilon_{\perp}$ [1 kHz, 20°C]: | 2.7 |
| PGUQU-4-F | 2.5 | $\gamma_1$ [mPa s, 20°C]: | 52 |
| PP-1-2V1 | 7.5 | $K_{11}$ [pN, 20°C]: | 15.9 |
| PPGU-3-F | 1.0 | $K_{33}$ [pN, 20°C]: | 13.7 |
| PUS-3-2 | 10.0 | | |
| $\Sigma$ | 100.0 | | |

Comparative Mixture Example 2

The following mixture (C-2) is prepared and investigated.

| Compound | % | Properties | |
|---|---|---|---|
| CC-3-2V1 | 2.5 | T(N,I) [°C]: | 82.3 |
| CC-3-V | 33.5 | $\Delta n$ [589 nm, 20°C]: | 0.1341 |
| CC-3-V1 | 8.0 | $n_e$ [589 nm, 20°C]: | 1.6281 |
| CCP-V-1 | 11.0 | $n_o$ [589 nm, 20°C]: | 1.4940 |
| CLP-3-T | 7.5 | $\Delta_{\varepsilon}$ [1 kHz, 20°C]: | 3.5 |
| PGP-1-2V | 4.25 | $\varepsilon_{\parallel}$ [1 kHz, 20°C]: | 6.0 |
| PGUQU-3-F | 4.0 | $\varepsilon_{\perp}$ [1 kHz, 20°C]: | 2.6 |
| PGUQU-4-F | 3.0 | $\gamma_1$ [mPa s, 20°C]: | 61 |
| PGUQU-5-F | 2.25 | $K_{11}$ [pN, 20°C]: | 17.8 |
| PP-1-2V1 | 9.0 | $K_{33}$ [pN, 20°C]: | 15.8 |
| PUS-3-2 | 15.0 | | |
| $\Sigma$ | 100.0 | | |

Table 1: Comparison of average elastic constant $K_{avg}$. and response time parameter $\gamma_1$ /$K_{11}$ of mixtures C-1, C-2 and M-1

| Mixture | Comment | $K_{Avg}$. | $\gamma_1/K_{11}$ |
|---|---|---|---|
| C-1 | Comparison | 12.5 pN | 3.3 mPas/pN |
| C-2 | Comparison | 14.2 pN | 3.4 mPas/pN |
| M-1 | Invention | 14.4 pN | 3.2 mPas/pN |

Mixture Example 2

The following mixture (M-2) is prepared and investigated.

| Compound | % | Properties | |
|---|---|---|---|
| CC-3-2V1 | 6.0 | T(N,I) [°C]: | 82.1 |
| CC-3-V | 36.0 | $\Delta n$ [589 nm, 20°C]: | 0.1363 |
| CC-3-V1 | 8.0 | $n_e$ [589 nm, 20°C]: | 1.6297 |
| CLP-3-T | 7.0 | $n_o$ [589 nm, 20°C]: | 1.4934 |
| CLP-V-1 | 4.0 | $\Delta_{\varepsilon}$ [1 kHz, 20°C]: | 3.7 |
| GnfUQU-3-F | 6.0 | $\varepsilon_{\parallel}$ [1 kHz, 20°C]: | 6.4 |

(continued)

| Compound | % | Properties | |
|---|---|---|---|
| PGP-2-2V | 14.0 | $\varepsilon_{\perp}$ [1 kHz, 20°C]: | 2.7 |
| PGUQU-3-F | 1.5 | $\gamma_1$ [mPa s, 20°C]: | 58 |
| PP-1-2V1 | 7.0 | $K_{11}$ [pN, 20°C]: | 18.3 |
| PPGU-3-F | 0.5 | $K_{33}$ [pN, 20°C]: | 15.5 |
| PUS-3-2 | 10.0 | | |
| Σ | 100.0 | | |

Mixture Example 3

The following mixture (M-3) is prepared and investigated.

| Compound | % | Properties | |
|---|---|---|---|
| CC-3-2V1 | 5.0 | T(N,I) [°C]: | 76.1 |
| CC-3-V | 39.0 | $\Delta n$ [589 nm, 20°C]: | 0.1356 |
| CC-3-V1 | 8.0 | $n_e$ [589 nm, 20°C]: | 1.6289 |
| CLP-3-T | 7.0 | $n_o$ [589 nm, 20°C]: | 1.4933 |
| DGUQU-4-F | 2.5 | $\Delta_{\varepsilon}$ [1 kHz, 20°C]: | 3.3 |
| GnfUQU-3-F | 4.0 | $\varepsilon_{\parallel}$ [1 kHz, 20°C]: | 6.0 |
| PGP-1-2V | 10.5 | $\varepsilon_{\perp}$ [1 kHz, 20°C]: | 2.7 |
| PP-1-2V1 | 8.0 | $\gamma_1$ [mPa s, 20°C]: | 54 |
| PPGU-3-F | 0.5 | $K_{11}$ [pN, 20°C]: | 17.0 |
| PUS-3-2 | 15.5 | $K_{33}$ [pN, 20°C]: | 14.9 |
| Σ | 100.0 | | |

Mixture Example 4

The following mixture (M-4) is prepared and investigated.

| Compound | % | Properties | |
|---|---|---|---|
| CC-3-2V1 | 5.0 | T(N,I) [°C]: | 75.9 |
| CC-3-V | 39.0 | $\Delta n$ [589 nm, 20°C]: | 0.1343 |
| CC-3-V1 | 8.0 | $n_e$ [589 nm, 20°C]: | 1.6274 |
| CLP-3-T | 8.0 | $n_o$ [589 nm, 20°C]: | 1.4931 |
| DGUQU-4-F | 3.0 | $\Delta_{\varepsilon}$ [1 kHz, 20°C]: | 3.2 |
| GnfUQU-3-F | 3.0 | $\varepsilon_{\parallel}$ [1 kHz, 20°C]: | 5.9 |
| PGP-1-2V | 9.0 | $\varepsilon_{\perp}$ [1 kHz, 20°C]: | 2.7 |
| PP-1-2V1 | 8.0 | $\gamma_1$ [mPa s, 20°C]: | 55 |
| PPGU-3-F | 0.5 | $K_{11}$ [pN, 20°C]: | 17.4 |
| PUS-3-2 | 16.5 | $K_{33}$ [pN, 20°C]: | 14.8 |
| Σ | 100.0 | | |

Mixture Example 5

The following mixture (M-5) is prepared and investigated.

| Compound | % | Properties | |
|---|---|---|---|
| CC-3-V | 28.0 | T(N,I) [°C]: | 82.8 |

(continued)

| Compound | % | Properties | |
|---|---|---|---|
| CC-3-V1 | 8.0 | Δn [589 nm, 20°C]: | 0.1538 |
| CCP-V-1 | 5.0 | $n_e$ [589 nm, 20°C]: | 1.6553 |
| CCP-V2-1 | 5.0 | $n_o$ [589 nm, 20°C]: | 1.5015 |
| CLP-3-T | 3.0 | $\Delta_\varepsilon$ [1 kHz, 20°C]: | 4.0 |
| CLP-V-1 | 3.0 | $\varepsilon_\parallel$ [1 kHz, 20°C]: | 6.7 |
| DGUQU-4-F | 3.5 | $\varepsilon_\perp$ [1 kHz, 20°C]: | 2.7 |
| PGP-2-2V | 6.5 | $\gamma_1$ [mPa s, 20°C]: | 67 |
| PP-1-2V1 | 12.0 | $K_{11}$ [pN, 20°C]: | 17.8 |
| PPGU-3-F | 1.0 | $K_{33}$ [pN, 20°C]: | 15.0 |
| PUS-3-2 | 20.0 | LTS bulk [h, -20°C]: | 0 |
| GnfUQU-3-F | 5.0 | | |
| Σ | 100.0 | | |

Mixture Example 6

The following mixture (M-6) is prepared and investigated.

| Compound | % | Properties | |
|---|---|---|---|
| CC-3-V | 30.0 | T(N,I) [°C]: | 85.1 |
| CC-3-V1 | 8.0 | Δn [589 nm, 20°C]: | 0.1535 |
| CCH-35 | 3.0 | $n_e$ [589 nm, 20°C]: | 1.6510 |
| CCP-30CF3 | 5.0 | $n_o$ [589 nm, 20°C]: | 1.4975 |
| CCP-V2-1 | 5.0 | $\Delta_\varepsilon$ [1 kHz, 20°C]: | 3.6 |
| GnfUQU-3-F | 7.5 | $\varepsilon_\parallel$ [1 kHz, 20°C]: | 6.4 |
| PGP-1-2V | 2.0 | $\varepsilon_\perp$ [1 kHz, 20°C]: | 2.8 |
| PGP-2-2V | 12.0 | $\gamma_1$ [mPa s, 20°C]: | 66 |
| PGP-2-3 | 6.0 | $K_{11}$ [pN, 20°C]: | 17.3 |
| PP-1-2V1 | 8.0 | $K_{33}$ [pN, 20°C]: | 15.3 |
| PPGU-3-F | 1.0 | | |
| PUS-3-2 | 12.5 | | |
| Σ | 100.0 | | |

Mixture Example 7

The following mixture (M-7) is prepared and investigated.

| Compound | % | Properties | |
|---|---|---|---|
| CC-3-V | 29.5 | T(N,I) [°C]: | 85.1 |
| CC-3-V1 | 8.0 | Δn [589 nm, 20°C]: | 0.1542 |
| CCH-35 | 3.0 | $n_e$ [589 nm, 20°C]: | 1.6549 |
| CCP-V-1 | 5.0 | $n_o$ [589 nm, 20°C]: | 1.5007 |
| CCP-V2-1 | 5.0 | $\Delta_\varepsilon$ [1 kHz, 20°C]: | 3.5 |
| GnfUQU-3-F | 8.0 | $\varepsilon_\parallel$ [1 kHz, 20°C]: | 6.3 |
| PGP-1-2V | 2.0 | $\varepsilon_\perp$ [1 kHz, 20°C]: | 2.8 |
| PGP-2-2V | 12.0 | $\gamma_1$ [mPa s, 20°C]: | 70 |
| PGP-2-3 | 6.0 | $K_{11}$ [pN, 20°C]: | 16.3 |

(continued)

| Compound | % | Properties | |
|---|---|---|---|
| PP-1-2V1 | 8.0 | $K_{33}$ [pN, 20°C]: | 15.3 |
| PPGU-3-F | 1.0 | | |
| PUS-3-2 | 12.5 | | |
| Σ | 100.0 | | |

Mixture Example 8

The following mixture (M-8) is prepared and investigated.

| Compound | % | Properties | |
|---|---|---|---|
| CC-3-V | 27.0 | T(N,I) [°C]: | 85.3 |
| CC-3-V1 | 8.0 | $\Delta n$ [589 nm, 20°C]: | 0.1525 |
| CCH-34 | 2.0 | $n_e$ [589 nm, 20°C]: | 1.6508 |
| CCH-35 | 5.0 | $n_o$ [589 nm, 20°C]: | 1.4983 |
| CCP-30CF3 | 2.0 | $\Delta_\varepsilon$ [1 kHz, 20°C]: | 3.4 |
| CCP-V-1 | 2.0 | $\varepsilon_\parallel$ [1 kHz, 20°C]: | 6.2 |
| CCP-V2-1 | 5.0 | $\varepsilon_\perp$ [1 kHz, 20°C]: | 2.8 |
| GnfUQU-3-F | 7.5 | $\gamma_1$ [mPa s, 20°C]: | 67 |
| PGP-1-2V | 2.0 | $K_{11}$ [pN, 20°C]: | 17.3 |
| PGP-2-2V | 12.0 | $K_{33}$ [pN, 20°C]: | 15.3 |
| PGP-2-3 | 6.0 | | |
| PP-1-2V1 | 8.0 | | |
| PPGU-3-F | 1.0 | | |
| PUS-3-2 | 12.5 | | |
| Σ | 100.0 | | |

Mixture Example 9

The following mixture (M-9) is prepared and investigated.

| Compound | % | Properties | |
|---|---|---|---|
| CC-3-V | 28.0 | T(N,I) [°C]: | 84.8 |
| CC-3-V1 | 8.0 | $\Delta n$ [589 nm, 20°C]: | 0.1534 |
| CCH-35 | 5.0 | $n_e$ [589 nm, 20°C]: | 1.6535 |
| CCP-V-1 | 5.5 | $n_o$ [589 nm, 20°C]: | 1.5001 |
| CCP-V2-1 | 5.0 | $\Delta_\varepsilon$ [1 kHz, 20°C]: | 3.6 |
| GnfUQU-3-F | 8.0 | $\varepsilon_\parallel$ [1 kHz, 20°C]: | 6.3 |
| PGP-1-2V | 2.5 | $\varepsilon_\perp$ [1 kHz, 20°C]: | 2.8 |
| PGP-2-2V | 12.0 | $\gamma_1$ [mPa s, 20°C]: | 66 |
| PP-1-2V1 | 8.0 | $K_{11}$ [pN, 20°C]: | 16.5 |
| PPGU-3-F | 1.0 | $K_{33}$ [pN, 20°C]: | 15.3 |
| PUS-3-2 | 17.0 | | |
| Σ | 100.0 | | |

Mixture Example 10

The following mixture (M-10) is prepared and investigated.

| Compound | % | Properties | |
|---|---|---|---|
| CC-3-V | 46.0 | T(N,I) [°C]: | 74.3 |
| CC-3-V1 | 7.5 | $\Delta n$ [589 nm, 20°C]: | 0.1326 |
| CLP-3-T | 6.5 | $n_e$ [589 nm, 20°C]: | 1.6261 |
| PGP-2-2V | 15.0 | $n_o$ [589 nm, 20°C]: | 1.4935 |
| GnfUQU-3-F | 5.5 | $\Delta_\varepsilon$ [1 kHz, 20°C]: | 3.1 |
| PP-1-2V1 | 6.5 | $\varepsilon_\parallel$ [1 kHz, 20°C]: | 5.7 |
| PPGU-3-F | 1.0 | $\varepsilon_\perp$ [1 kHz, 20°C]: | 2.7 |
| PUS-3-2 | 12.0 | $\gamma_1$ [mPa s, 20°C]: | 50 |
| $\Sigma$ | 100.0 | | |

Mixture Example 11

The following mixture (M-11) is prepared and investigated.

| Compound | % | Properties | |
|---|---|---|---|
| CC-3-V | 42.0 | T(N,I) [°C]: | 75.8 |
| CC-3-V1 | 8.0 | $\Delta n$ [589 nm, 20°C]: | 0.1332 |
| CCH-35 | 3.0 | $n_e$ [589 nm, 20°C]: | 1.6260 |
| CLP-3-T | 7.0 | $n_o$ [589 nm, 20°C]: | 1.4928 |
| GnfUQU-3-F | 5.5 | $\Delta_\varepsilon$ [1 kHz, 20°C]: | 3.1 |
| PGP-1-2V | 3.0 | $\varepsilon_\parallel$ [1 kHz, 20°C]: | 5.8 |
| PGP-2-2V | 12.0 | $\varepsilon_\perp$ [1 kHz, 20°C]: | 2.7 |
| PP-1-2V1 | 7.5 | $\gamma_1$ [mPa s, 20°C]: | 53 |
| PPGU-3-F | 1.0 | $K_{11}$ [pN, 20°C]: | 16.4 |
| PUS-3-2 | 11.0 | $K_{33}$ [pN, 20°C]: | 13.8 |
| $\Sigma$ | 100.0 | | |

Mixture Example 12

The following mixture (M-12) is prepared and investigated.

| Compound | % | Properties | |
|---|---|---|---|
| CC-3-2V1 | 5.5 | T(N,I) [°C]: | 82.6 |
| CC-3-V | 33.5 | $n_e$ [589 nm, 20°C]: | 1.6265 |
| CC-3-V1 | 7.5 | $n_o$ [589 nm, 20°C]: | 1.4935 |
| CCP-V2-1 | 10.0 | $\Delta n$ [589 nm, 20°C]: | 0.1330 |
| CLP-3-T | 6.5 | $\varepsilon_\parallel$ [1 kHz, 20°C]: | 6.1 |
| GnfUQU-3-F | 4.0 | $\varepsilon_\perp$ [1 kHz, 20°C]: | 2.6 |
| PGP-1-2V | 4.0 | $\Delta_\varepsilon$ [1 kHz, 20°C]: | 3.5 |
| PGUQU-3-F | 4.0 | $\gamma_1$ [mPa s, 20°C]: | 63 |
| PP-1-2V1 | 9.5 | $K_1$ [pN, 20°C]: | 18.5 |
| PPGU-3-F | 0.5 | $K_3$ [pN, 20°C]: | 16.4 |
| PUS-3-2 | 15.0 | | |
| $\Sigma$ | 100.0 | | |

Mixture Example 13

The following mixture (M-13) is prepared and investigated.

| Compound | % | Properties | |
|---|---|---|---|
| CC-3-2V1 | 5.0 | T(N,I) [°C]: | 84.5 |
| CC-3-V | 33.5 | $n_e$ [589 nm, 20°C]: | 1.6274 |
| CC-3-V1 | 8.0 | $n_o$ [589 nm, 20°C]: | 1.4932 |
| CCP-V2-1 | 10.0 | Δn [589 nm, 20°C]: | 0.1342 |
| CLP-3-T | 7.0 | $\varepsilon_{\|\|}$ [1 kHz, 20°C]: | 6.2 |
| GnfUQU-3-F | 5.0 | $\varepsilon_{\perp}$ [1 kHz, 20°C]: | 2.6 |
| PGP-1-2V | 5.0 | $\Delta_{\varepsilon}$ [1 kHz, 20°C]: | 3.6 |
| PGUQU-3-F | 2.5 | $\gamma_1$ [mPa s, 20°C]: | 63 |
| PP-1-2V1 | 8.0 | $K_1$ [pN, 20°C]: | 18.7 |
| PPGU-3-F | 0.5 | $K_3$ [pN, 20°C]: | 16.5 |
| PUS-3-2 | 15.5 | LTS bulk [h, -20°C]: | 1000 |
| Σ | 100.0 | | |

Mixture Example 14

The following mixture (M-14) is prepared and investigated.

| Compound | % | Properties | |
|---|---|---|---|
| CC-3-2V1 | 5.5 | T(N,I) [°C]: | 78 |
| CC-3-V | 35.5 | $n_e$ [589 nm, 20°C]: | 1.6272 |
| CC-3-V1 | 8.0 | $n_o$ [589 nm, 20°C]: | 1.4934 |
| CCP-V2-1 | 5.5 | Δn [589 nm, 20°C]: | 0.1338 |
| CLP-3-T | 7.0 | $\varepsilon_{\|\|}$ [1 kHz, 20°C]: | 6.0 |
| GnfUQU-3-F | 5.0 | $\varepsilon_{\perp}$ [1 kHz, 20°C]: | 2.6 |
| PGP-1-2V | 5.0 | Δε [1 kHz, 20°C]: | 3.4 |
| PGUQU-3-F | 2.0 | $\gamma_1$ [mPa s, 20°C]: | 57 |
| PP-1-2V1 | 10.5 | $K_1$ [pN, 20°C]: | 17.9 |
| PPGU-3-F | 0.5 | $K_3$ [pN, 20°C]: | 15.7 |
| PUS-3-2 | 15.5 | | |
| Σ | 100.0 | | |

Mixture Example 15

The following mixture (M-15) is prepared and investigated.

| Compound | % | Properties | |
|---|---|---|---|
| CC-3-2V1 | 5.5 | T(N,I) [°C]: | 94.7 |
| CC-3-V | 20.5 | $n_e$ [589 nm, 20°C]: | 1.6297 |
| CC-3-V1 | 8.0 | $n_o$ [589 nm, 20°C]: | 1.4950 |
| CC-5-V | 5.0 | Δn [589 nm, 20°C]: | 0.1347 |
| CCP-V-1 | 15.0 | $\varepsilon_{\|\|}$ [1 kHz, 20°C]: | 6.5 |
| CCP-V2-1 | 5.0 | $\varepsilon_{\perp}$ [1 kHz, 20°C]: | 2.6 |
| CLP-3-T | 7.0 | $\Delta_{\varepsilon}$ [1 kHz, 20°C]: | 3.9 |
| CPGP-5-2 | 1.0 | $\gamma_1$ [mPa s, 20°C]: | 76 |

(continued)

| Compound | % | Properties | |
|---|---|---|---|
| GnfUQU-3-F | 7.5 | $K_1$ [pN, 20°C]: | 20.2 |
| PGP-1-2V | 5.0 | $K_3$ [pN, 20°C]: | 19.0 |
| PP-1-2V1 | 9.0 | LTS bulk [h, -20°C]: | 1000 |
| PPGU-3-F | 0.5 | | |
| PUS-3-2 | 11.0 | | |
| Σ | 100.0 | | |

**Claims**

1. Liquid-crystalline medium having a nematic phase **characterized in that** it comprises one or more compounds of formula Py

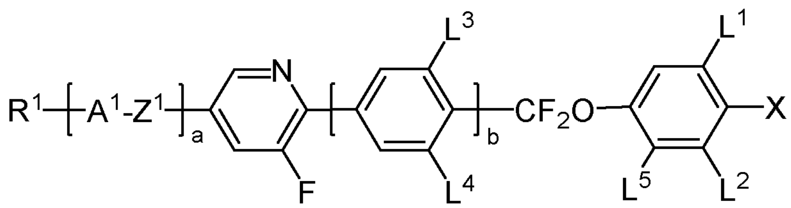

Py

in which

$R^1$ an alkyl radical having 1 to 15 C atoms, wherein one or more $CH_2$ groups, including terminal C atoms, in this radical may each be replaced, independently of one another, by -C≡C-, -CH=CH-,

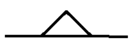 , 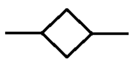 , 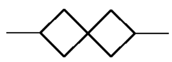 , 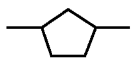 ,

-O-, -S-, -(CO)-O-, -O-(CO)- in such a way that O or S atoms are not linked directly to one another, and in which, in addition, one or more H atoms may be replaced by F or Cl, or H,

$A^1$ on each appearance, independently of one another,

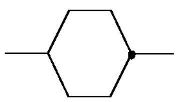 , 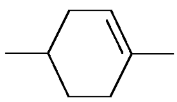 , 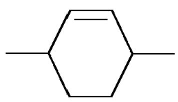 ,

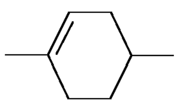 , 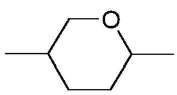 , 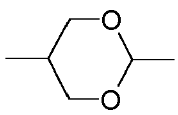 ,

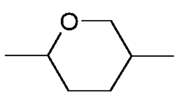 , 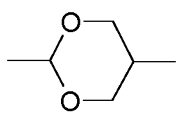 ,

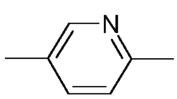 , 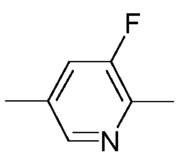 , 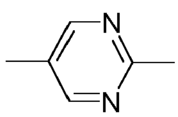 ,

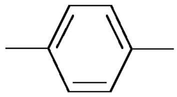, 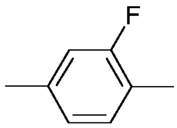, 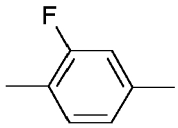,

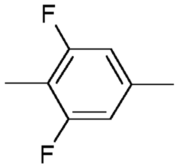 or 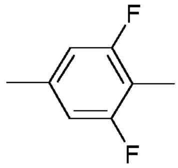,

$Z^1$ independently of one another, identically or differently, denotes a single bond, $-CH_2O-$, -(CO)O-, $-CF_2O-$, $-CH_2CH_2CF_2O-$, $-CF_2CF_2-$, $-CH_2CF2-$, $-CH_2CH_2-$, $-(CH_2)_4-$, -CH=CH-, -CH=CF-, -CF=CF- or -C=C-, where asymmetrical bridges may be oriented to both sides,
$L^1$, $L^2$, $L^3$, $L^4$ independently H, F or Cl,
$L^5$ H, $CH_3$ or $CH_2CH_3$, preferably H,
X F, Cl, $-CF_3$, $-OCF_3$, -CN or -SCN,
a denotes 0, 1 or 2, preferably 1,
b denotes 0, 1 or 2, preferably 1, and

where a + b is $\leq 3$,
and one or more additional compounds of the formula T

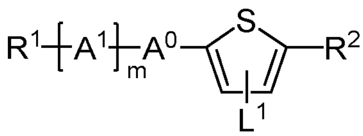

T

in which the individual radicals have the following meanings:

$R^1$ and $R^2$ denote H, F, Cl, Br, -CN, -NCS or straight-chain or branched alkyl having 1 to 12 C atoms, in which, in addition, one or more $CH_2$ groups, including terminal C atoms, may each be replaced, independently of one another, by -CH=CH-, -C≡C-,

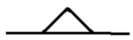, 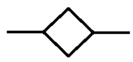,

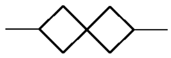, 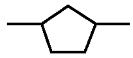,

-O-, -S-, -CO-, -CO-O-, -O-CO-, in such a way that O or S atoms are not linked directly to one another, and in which, in addition, one or more H atoms may be replaced by F or Cl,
$L^1$ H or $CH_3$,
$A^0$ denotes

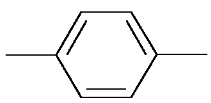, 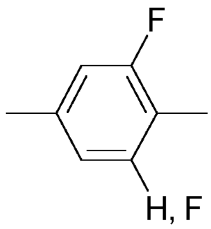, 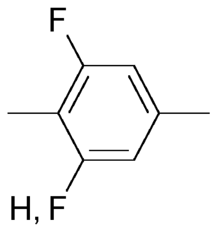,

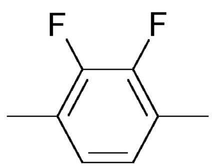 or 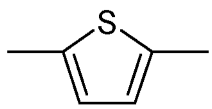

$A^1$, independently of one another, denotes phenylene-1,4-diyl, in which, in addition, one or two CH groups may be replaced by N and one or more H atoms may be replaced by halogen, CN, $CH_3$, $CHF_2$, $CH_2F$, $OCH_3$, $OCHF_2$ or $OCF_3$, cyclohexane-1,4-diyl, in which, in addition, one or two non-adjacent $CH_2$ groups may be replaced, independently of one another, by O and/or S and one or more H atoms may be replaced by F, cyclohexene-1,4-diyl, bicyclo[1.1.1]pentane-1,3-diyl, bicyclo[2.2.2]octane-1,4-diyl, spiro[3.3]heptane-2,6-diyl, tetrahydropyran-2,5-diyl or 1,3-dioxane-2,5-diyl,
m in each case, independently of one another, denotes 0, 1 or 2.

**2.** Liquid-crystalline medium according to Claim 1, wherein in formula Py

$A^1$ denotes on each appearance, independently of one another,

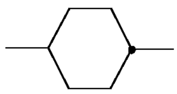, 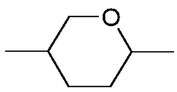, 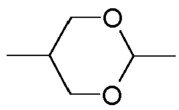,

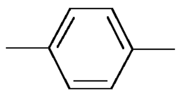, 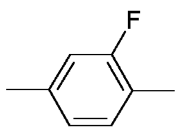 or 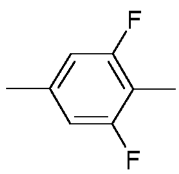.

**3.** Liquid-crystalline medium according to Claim 1 or 2, **characterized in that** it comprises one or more compounds selected from the group of compounds of formulae II and III.

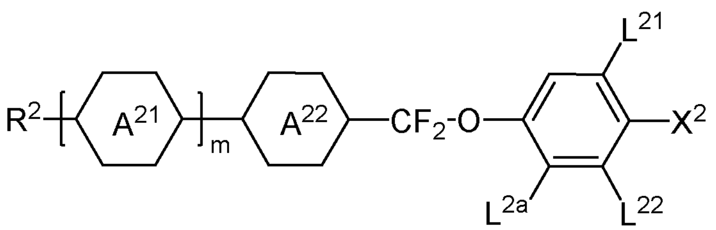

II

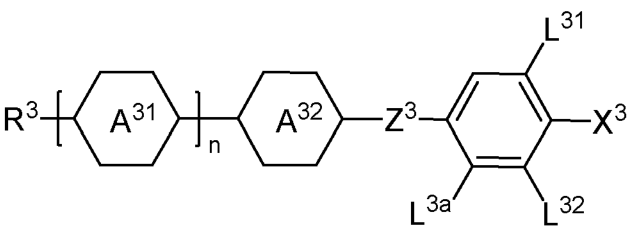

III

in which

$R^2$ an alkyl radical having 1 to 15 C atoms, wherein one or more $CH_2$ groups, including terminal C atoms, in this radical may each be replaced, independently of one another, by -C=C-, -CH=CH-,

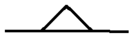 , 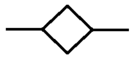 ,

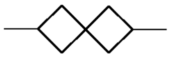 , 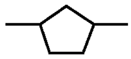 ,

-O-, -S-, -(CO)-O-, -O-(CO)- in such a way that O or S atoms are not linked directly to one another, and in which, in addition, one or more H atoms may be replaced by F or Cl, or H,

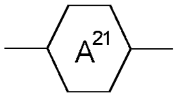 and 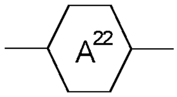

on each appearance, independently of one another, are

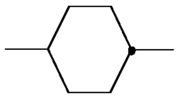 , 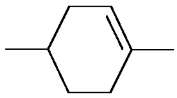 , 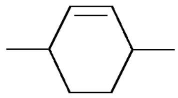 ,

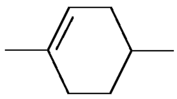 , 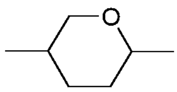 , 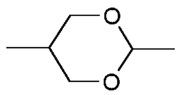 ,

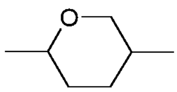 , 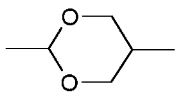 ,

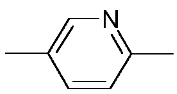 , 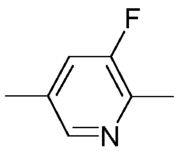 , 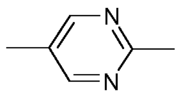 ,

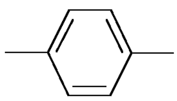 , 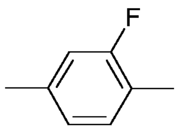 , 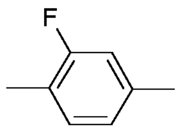 ,

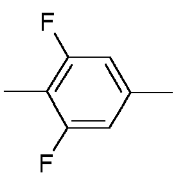 or 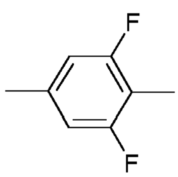 ,

wherein the rings optionally may each be substituted by one or two alkyl groups,
$L^{21}$ and $L^{22}$ denote H or F,
$L^{2a}$ H or $CH_3$,
$X^2$ denotes halogen, halogenated alkyl or alkoxy having 1 to 3 C atoms or halogenated alkenyl or alkenyloxy having 2 or 3 C atoms,
m denotes 0, 1, 2 or 3,
$R^3$ an alkyl radical having 1 to 15 C atoms, wherein one or more $CH_2$ groups, including terminal C atoms, in

this radical may each be replaced, independently of one another, by -C=C-, -CH=CH-,

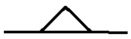 , 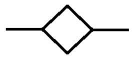 ,

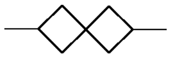 , 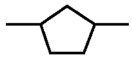 ,

-O-, -S-, -(CO)-O-, -O-(CO)- in such a way that O or S atoms are not linked directly to one another, and in which, in addition, one or more H atoms may be replaced by F or Cl,

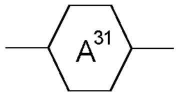 and 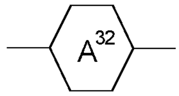

on each appearance, independently of one another, are

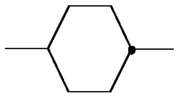 , 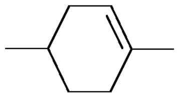 , 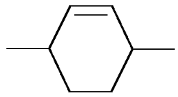 ,

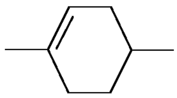 , 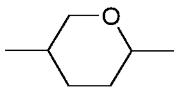 , 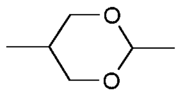 ,

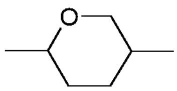 , 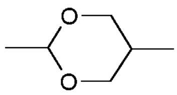 , 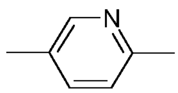 ,

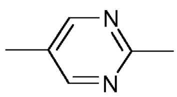 , 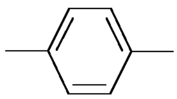 , 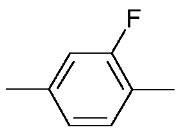 ,

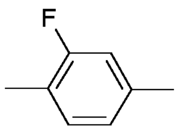 , 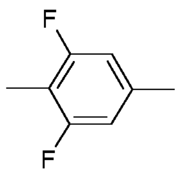 or 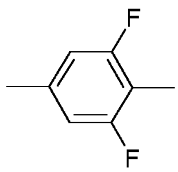 ,

wherein the rings optionally may each be substituted by one or two alkyl groups,
$L^{31}$ and $L^{32}$, independently of one another, denote H or F,
$L^{3a}$ H or $CH_3$,
$X^3$ denotes halogen, halogenated alkyl or alkoxy having 1 to 3 C atoms or halogenated alkenyl or alkenyloxy having 2 or 3 C atoms, F, Cl, $-OCF_3$, $-OCHF_2$, $-O-CH_2CF_3$, $-O-CH=CF_2$, $-O-CH=CH_2$ or $-CF_3$,
$Z^3$ denotes $-CH_2CH_2-$, $-CF_2CF_2-$, -COO-, trans-CH=CH-, *trans*-CF=CF-, $-CH_2O-$ or a single bond, and
n denotes 0, 1, 2 or 3.

4. Liquid-crystalline medium according to one or more of Claims 1 to 3, **characterized in that** it comprises one or more dielectrically neutral compounds selected from the group of formulae IV and V:

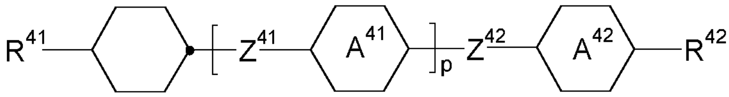

IV

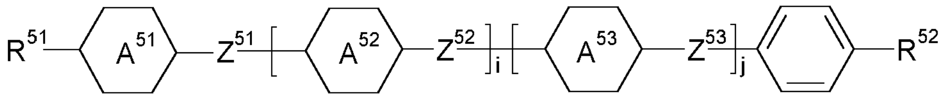

V

in which

$R^{41}$ and $R^{42}$, independently of one another, an alkyl radical having 1 to 15 C atoms, wherein one or more $CH_2$ groups, including terminal C atoms, in this radical may each be replaced, independently of one another, by -C≡C-, -CH=CH-,

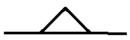 , 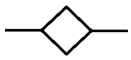 , 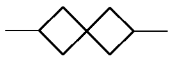 , 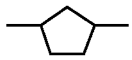 ,

-O-, -S-, -(CO)-O-, -O-(CO)- in such a way that O or S atoms are not linked directly to one another, and in which, in addition, one or more H atoms may be replaced by F or Cl,

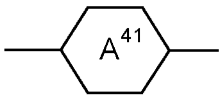 and 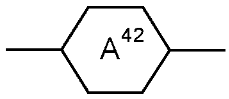

independently of one another and, if

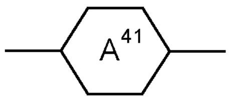

occurs twice, also these independently of one another, denote

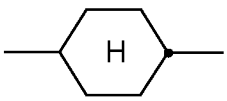 , 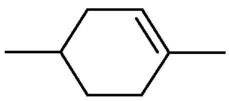 , 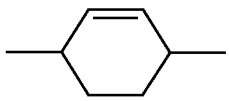 ,

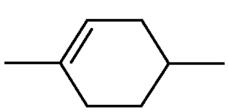 , 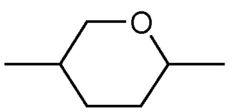 , 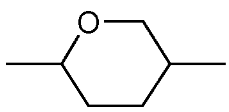 ,

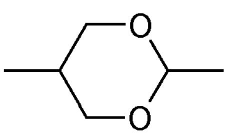 , 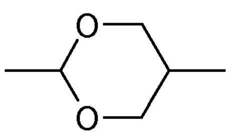 , 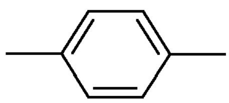 ,

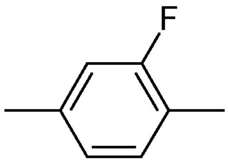, 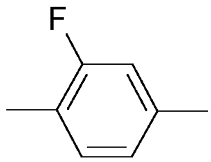, 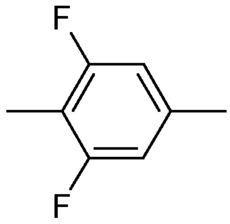,

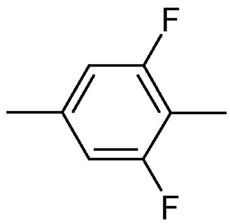 or 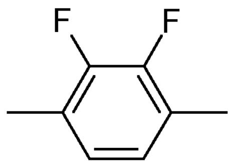,

wherein the rings optionally may each be substituted by one or two alkyl groups,
$Z^{41}$ and $Z^{42}$, independently of one another and, if $Z^{41}$ occurs twice, also these independently of one another, denote $-CH_2CH_2-$, -COO-, *trans*-CH=CH-, *trans*-CF=CF-, $-CH_2O-$, $-CF_2O-$, -C=C- or a single bond,
p denotes 0, 1 or 2,
$R^{51}$ and $R^{52}$, independently of one another, have one of the meanings given for $R^{41}$ and $R^{42}$

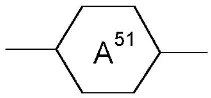 to 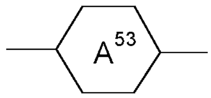

if present, each, independently of one another, denote

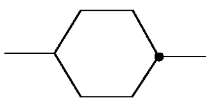, 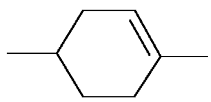, 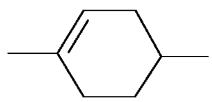,

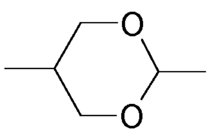, 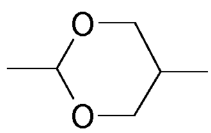, 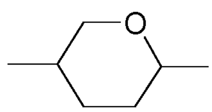,

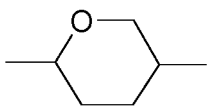, 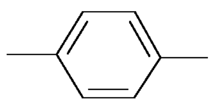,

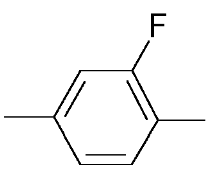 or 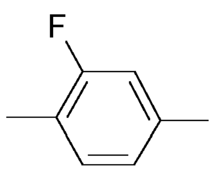,

wherein the rings optionally may each be substituted by one or two alkyl groups,
$Z^{51}$ to $Z^{53}$ each, independently of one another, denote $-CH_2-CH_2-$, $-CH_2-O-$, -CH=CH-, -C≡C-, -COO- or a single bond, and
i and j each, independently of one another, denote 0 or 1.

5. Liquid-crystalline medium according to one or more of claims 1 to 4, **characterised in that** it comprises one or more compounds selected from the group consisting of the following formulae T-1 to T-54:

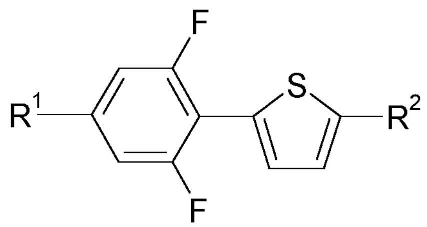

T-1

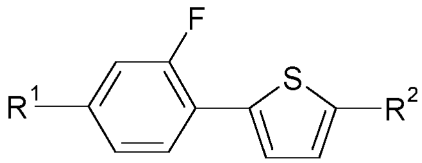

T-2

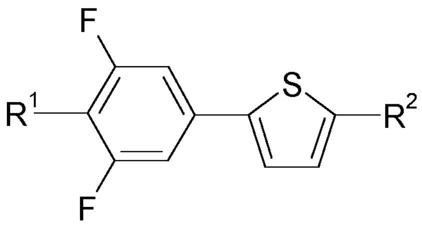

T-3

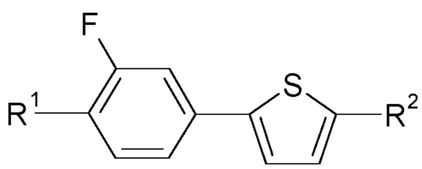

T-4

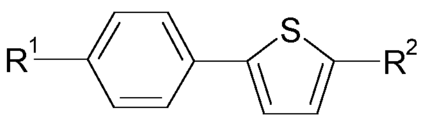

T-5

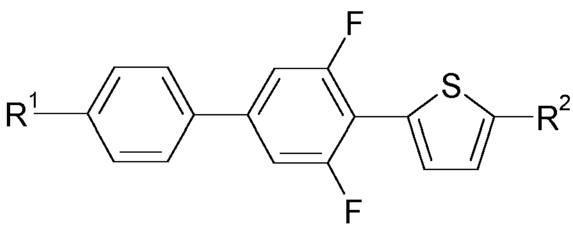

T-6

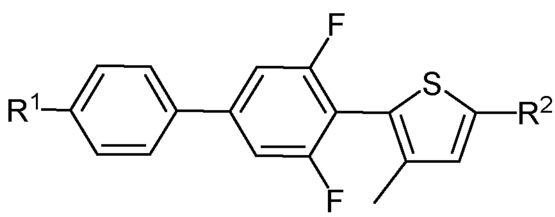

T-7

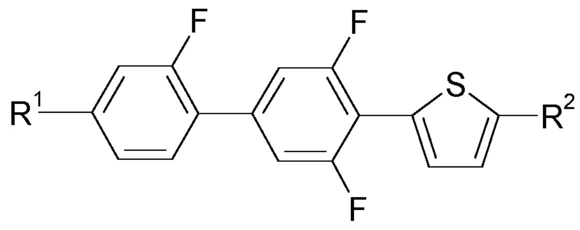

T-8

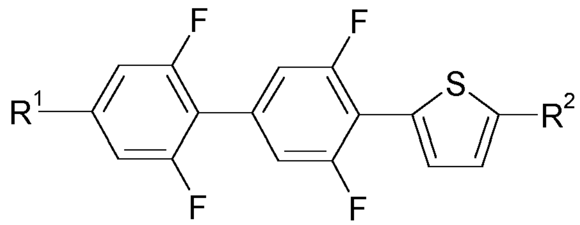

T-9

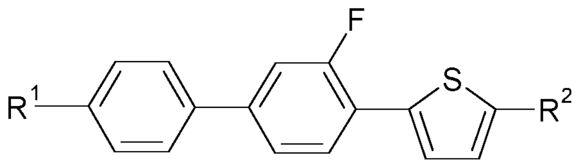

T-10

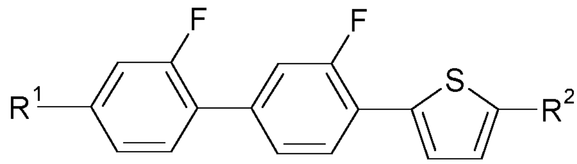

T-11

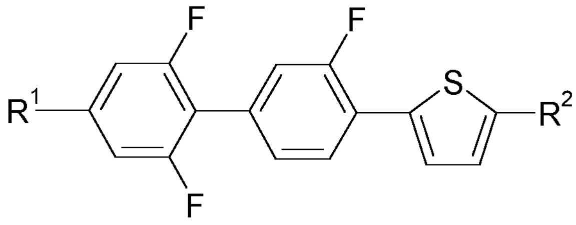

T-12

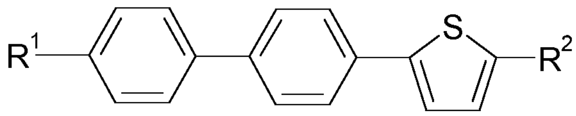

T-13

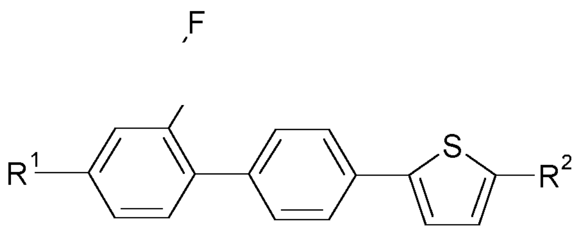

T-14

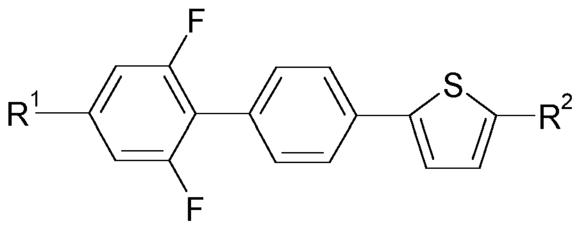

T-15

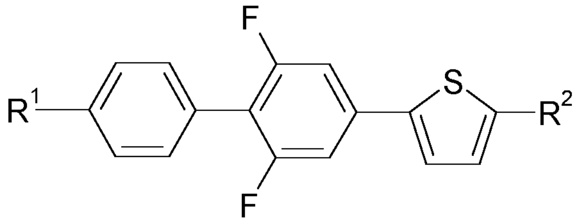

T-16

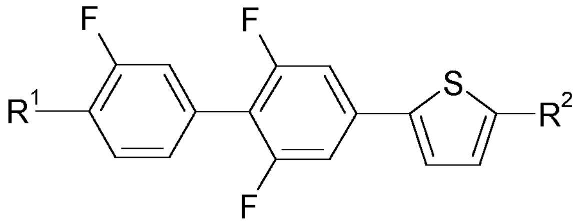

T-17

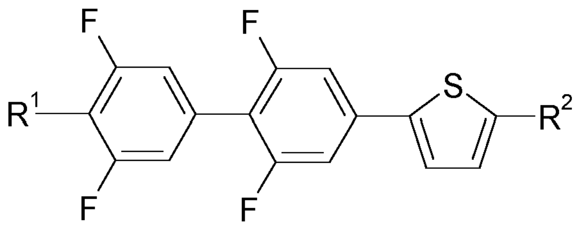

T-18

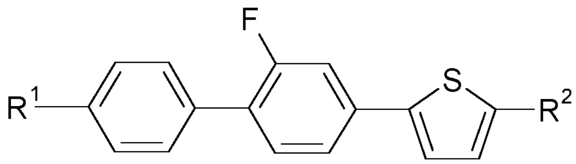

T-19

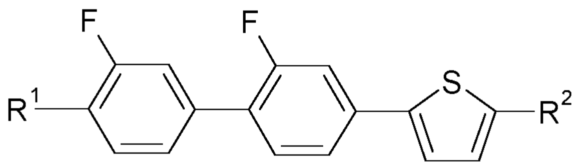

T-20

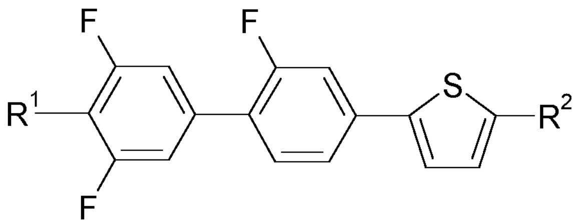

T-21

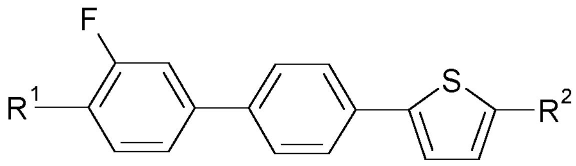

T-22

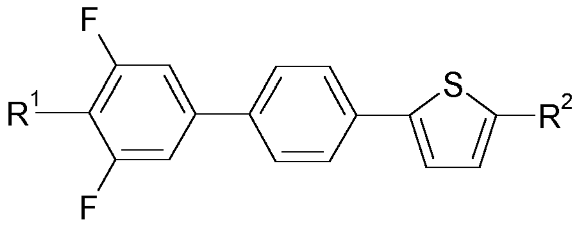

T-23

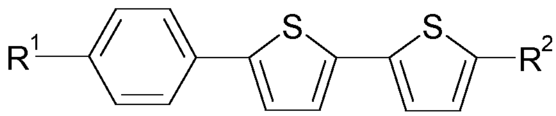

T-24

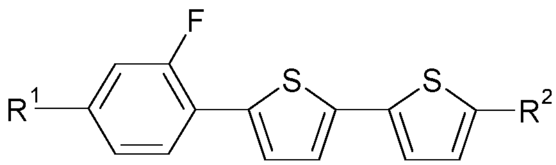

T-25

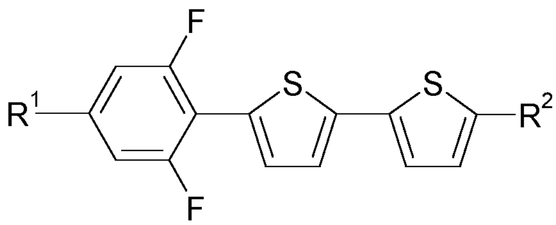

T-26

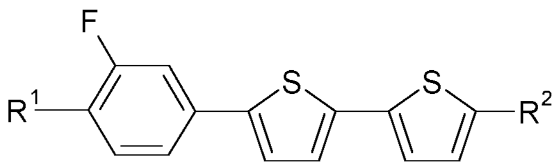

T-27

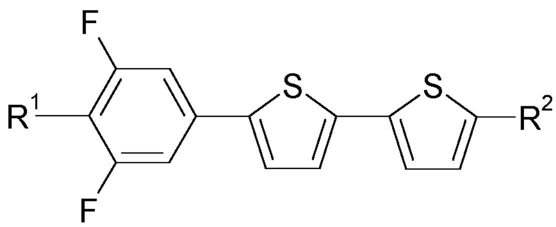

T-28

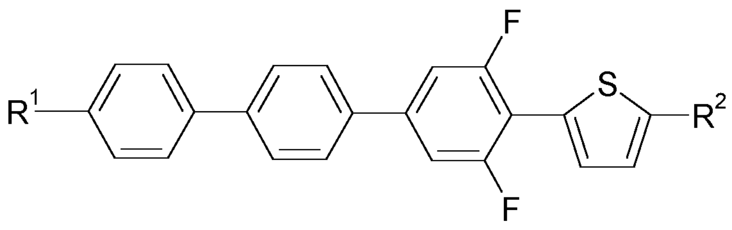

T-29

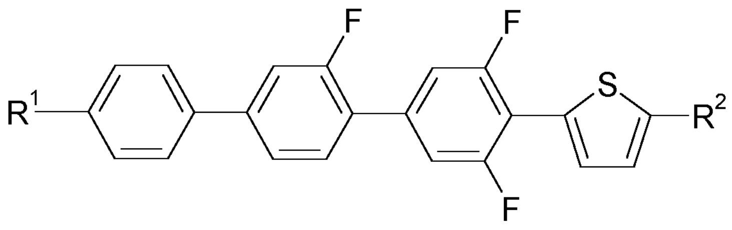

T-30

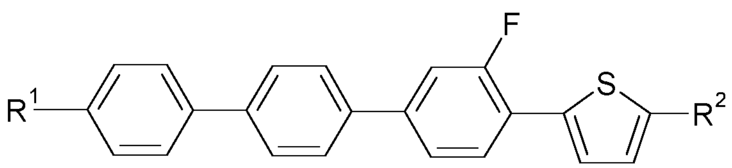

T-31

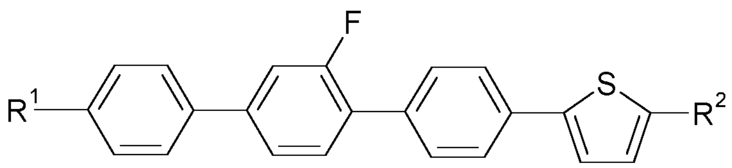

T-32

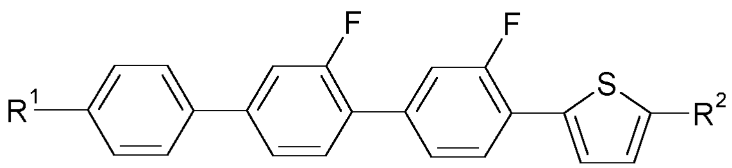

T-33

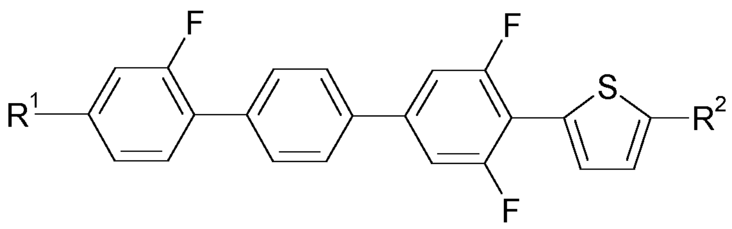

T-34

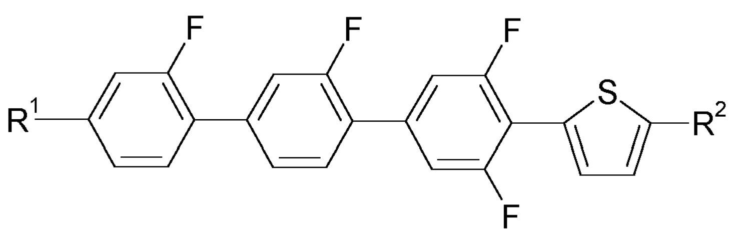

T-35

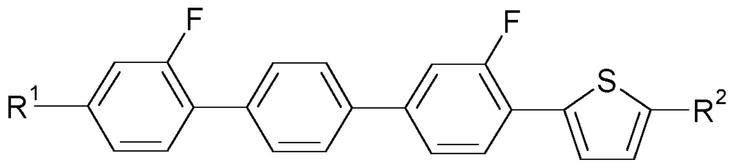

T-36

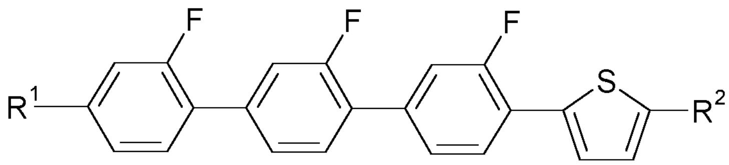

T-37

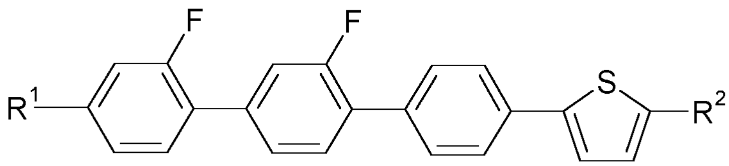

T-38

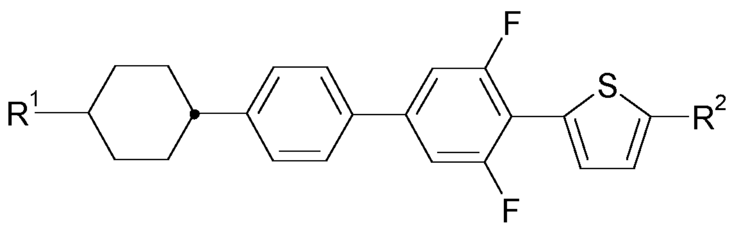

T-39

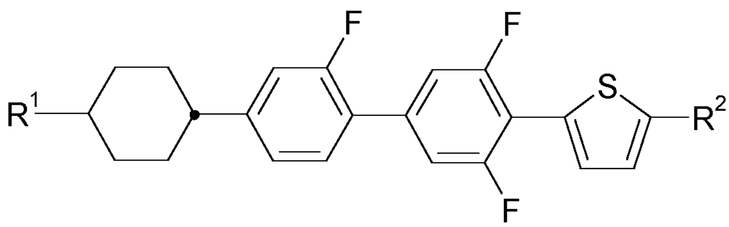

T-40

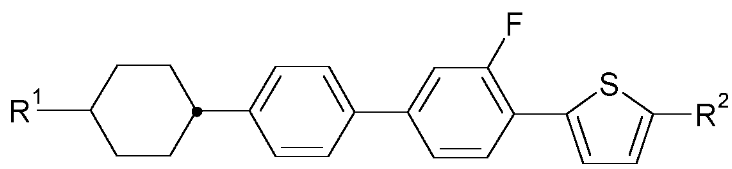

T-41

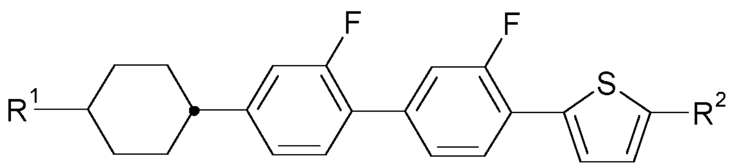

T-42

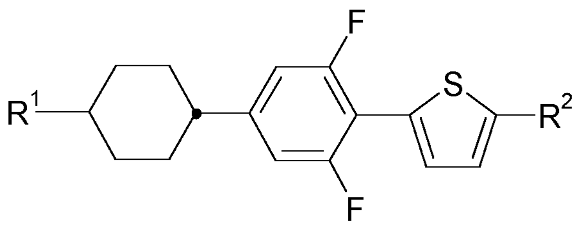

T-43

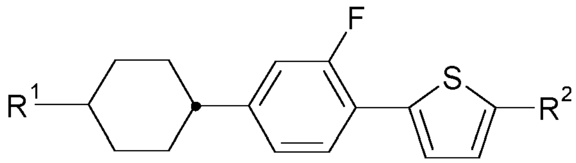

T-44

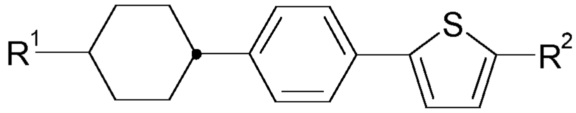

T-45

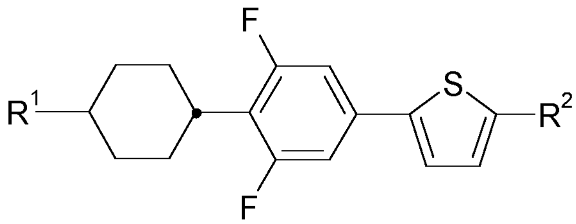

T-46

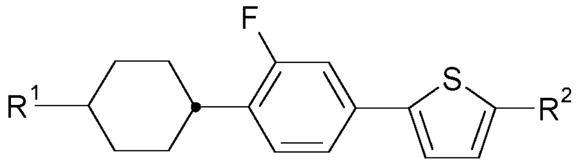

T-47

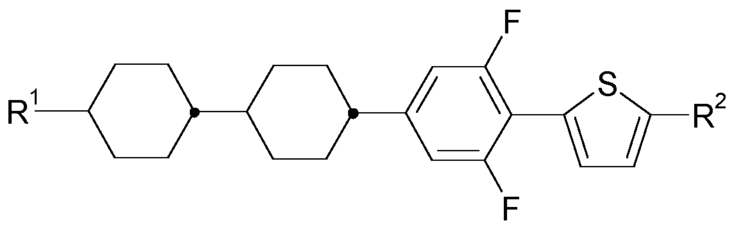

T-48

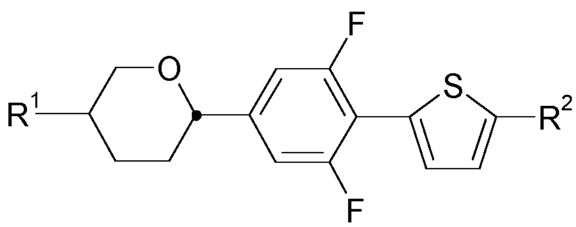

T-49

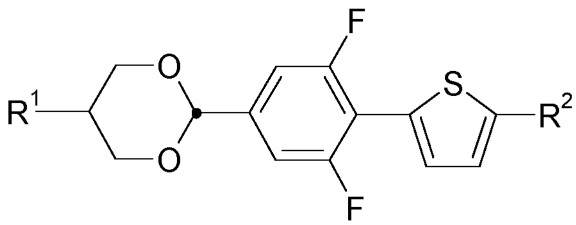

T-50

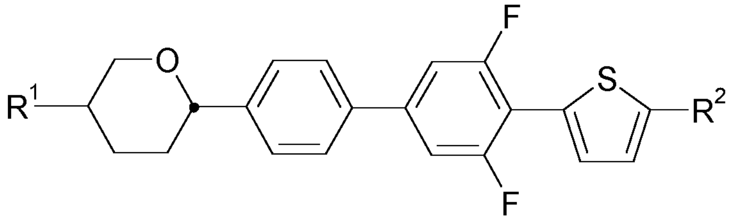

T-51

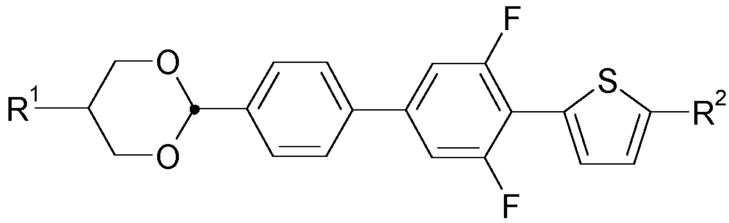

T-52

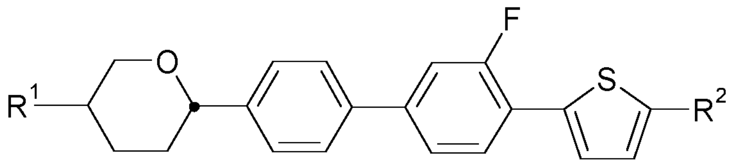

T-53

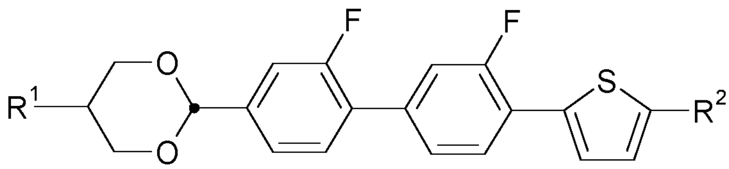

T-54

in which $R^1$ and $R^2$ have the meanings indicated in Claim 1.

**6.** Liquid-crystalline medium according to one or more of Claims 1 to 5, **characterized in that** it additionally comprises one or more compounds selected from the formulae III-1j, III-1k and III-1m:

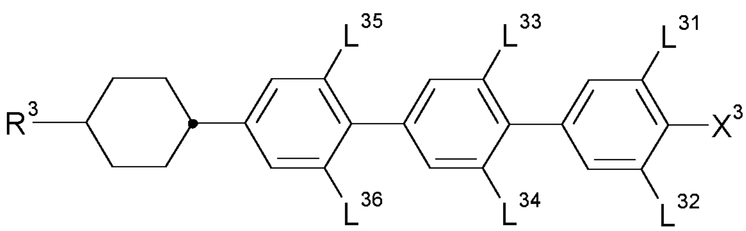

III-1j

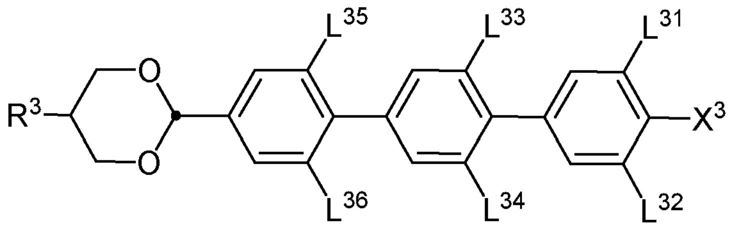

III-1k

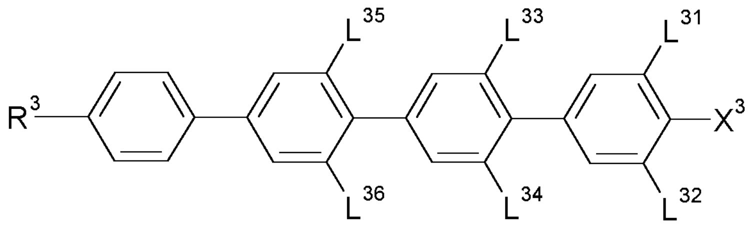

III-1m

in which

$R^3$ denotes alkyl, alkoxy, fluorinated alkyl or fluorinated alkoxy having 1 to 7 C atoms, alkenyl, alkenyloxy, alkoxyalkyl or fluorinated alkenyl having 2 to 7 C atoms,
$X^3$ denotes F, Cl, halogenated alkyl or alkoxy having 1 to 3 C atoms or halogenated alkenyl or alkenyloxy having 2 or 3 C atoms, F, Cl, $-OCF_3$, $-OCHF_2$, $-O-CH_2CF_3$, $-O-CH=CF_2$, $-O-CH=CH_2$ or $-CF_3$,
$L^{33}$ and $L^{34}$, independently of one another, denote H or F, and
$L^{35}$ and $L^{36}$, independently of one another, denote H or F.

**7.** Liquid-crystalline medium according to one or more of Claims 1 to 6, which additionally comprises one or more compounds selected from the group of the compounds of the formulae IV-1 to IV-4,

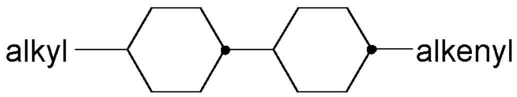

IV-1

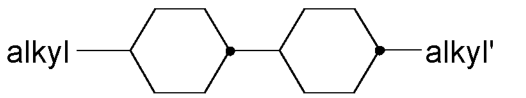

IV-2

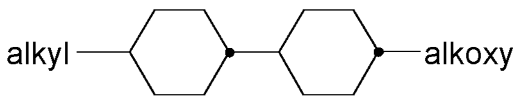

IV-3

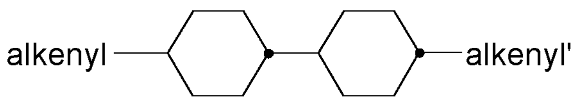

IV-4

in which

alkyl and alkyl', independently of one another, denote alkyl having 1 to 7 C atoms,
alkenyl and alkenyl', independently of one another, denote alkenyl having 2 to 5 C atoms, and
alkoxy denotes alkoxy having 1 to 5 C atoms.

**8.** Liquid-crystalline medium according to at least one of Claims 1 to 7, **characterized in that** the total concentration of the compounds of formula Py in the medium as a whole is 1 % or more but less than 30%.

9. Liquid-crystalline medium according to at least one of Claims 1 to 8, **characterized in that** it additionally comprises one or more chiral compounds and/or stabilizers.

10. Electro-optical display or electro-optical component, **characterized in that** it comprises a liquid-crystalline medium according to at least one of Claims 1 to 9.

11. Display according to Claim 10, **characterized in that** it is based on the IPS- or FFS mode.

12. Display according to Claim 10 or 11, **characterized in that** it contains an active-matrix addressing device.

13. Use of a medium according to at least one of Claims 1 to 9 in an electro-optical display, in an electro-optical component or energy-saving applications.

14. Process for the preparation of a liquid-crystalline medium according to one or more of Claims 1 to 9, **characterized in that** one or more compounds of formula Py are mixed with one or more additional mesogenic compounds and optionally one or more additives.

**Patentansprüche**

1. Flüssigkristallines Medium mit einer nematischen Phase, **dadurch gekennzeichnet, dass** es eine oder mehrere Verbindungen der Formel Py

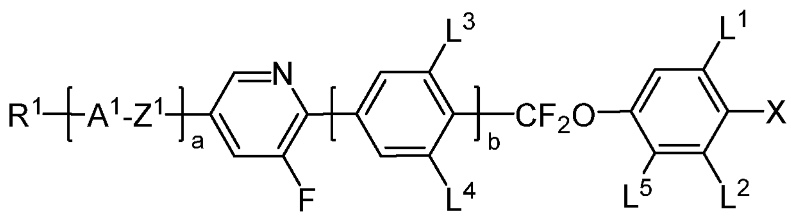

Py

worin

$R^1$ einen Alkylrest mit 1 bis 15 C-Atomen, wobei in diesem Rest eine oder mehrere $CH_2$-Gruppen, einschließlich endständiger C-Atome, jeweils unabhängig voneinander so durch -C≡C-, -CH=CH-,

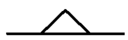, 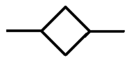, 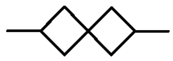, 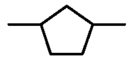,

-O-, -S-, -(CO)-O-, -O-(CO)- ersetzt sein können, dass O- oder S-Atome nicht direkt miteinander verknüpft sind, und worin zusätzlich ein oder mehrere H-Atome durch F oder Cl ersetzt sein können, oder H bedeutet,

$A^1$ bei jedem Auftreten unabhängig voneinander

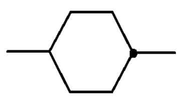, 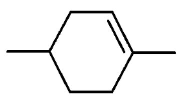, 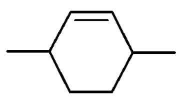,

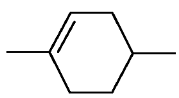, 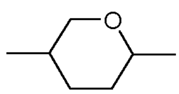, 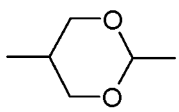,

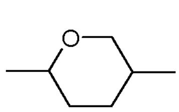, 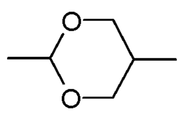,

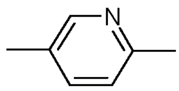, 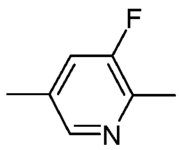, 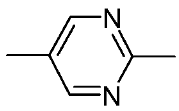,

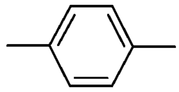, 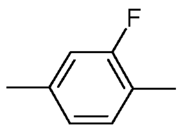, 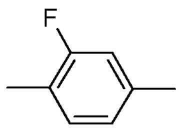,

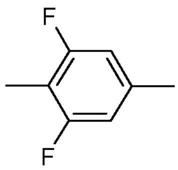 oder 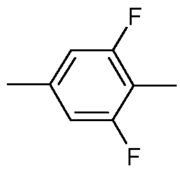

bedeutet,

$Z^1$ unabhängig voneinander gleich oder verschieden eine Einfachbindung, $-CH_2O-$, -(CO)O-, $-CF_2O-$, $-CH_2CH_2CF_2O-$, $-CF_2CF_2-$, $-CH_2CF_2-$, $-CH_2CH_2-$, $-(CH_2)_4-$, -CH=CH-, -CH=CF-, -CF=CF- oder -C=C- bedeutet, wo asymmetrische Brücken zu beiden Seiten orientiert sein können,

$L^1$, $L^2$, $L^3$, $L^4$ unabhängig H, F oder Cl bedeuten,

$L^5$ H, $CH_3$ oder $CH_2CH_3$, vorzugsweise H bedeutet,

X F, Cl, $-CF_3$ , $-OCF_3$, -CN oder -SCN bedeutet,

a 0, 1 oder 2, vorzugsweise 1 bedeutet,

b 0, 1 oder 2, vorzugsweise 1 bedeutet, und

wo $a + b \leq 3$ ist,

und eine oder mehrere zusätzliche Verbindungen der Formel T

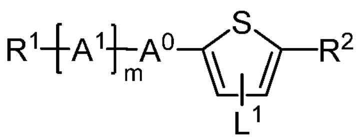 T

worin die einzelnen Reste die folgenden Bedeutungen besitzen:

$R^1$ und $R^2$ bedeuten H, F, Cl, Br, -CN, -NCS oder geradkettiges oder verzweigtes Alkyl mit 1 bis 12 C-Atomen, worin zusätzlich ein oder mehrere $CH_2$-Gruppen, einschließlich endständiger C-Atome, jeweils unabhängig voneinander so durch -CH=CH-, -C≡C-,

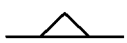, 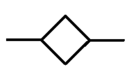, 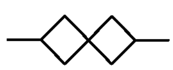,

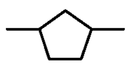,

-O-, -S-, -CO-, -CO-O-, -O-CO- ersetzt sein können, dass O- oder S-Atome nicht direkt miteinander verknüpft sind, und worin zusätzlich ein oder mehrere H-Atome durch F oder Cl ersetzt sein können,

$L^1$ H oder $CH_3$,

$A^0$ bedeutet

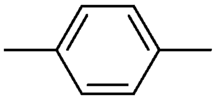, 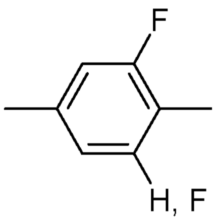, 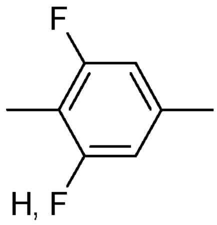,

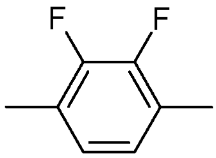 oder 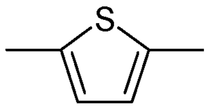,

$A^1$ bedeutet unabhängig voneinander Phenylen-1,4-diyl, worin zusätzlich eine oder zwei CH-Gruppen durch N ersetzt sein können und ein oder mehrere H-Atome durch Halogen, CN, $CH_3$, $CHF_2$, $CH_2F$, $OCH_3$, $OCHF_2$ oder $OCF_3$ ersetzt sein können, Cyclohexan-1,4-diyl, worin zusätzlich eine oder zwei nicht benachbarte $CH_2$-Gruppen unabhängig voneinander durch O und/oder S ersetzt sein können und ein oder mehrere H-Atome durch F ersetzt sein können, Cyclohexen-1,4-diyl, Bicyclo[1.1.1]pentan-1,3-diyl, Bicyclo[2.2.2]octan-1,4-diyl, Spiro[3.3]heptan-2,6-diyl, Tetrahydropyran-2,5-diyl oder 1,3-Dioxan-2,5-diyl,

m bedeutet jeweils unabhängig voneinander 0, 1 oder 2,

enthält.

**2.** Flüssigkristallines Medium nach Anspruch 1, wobei in Formel Py

$A^1$ bei jedem Auftreten unabhängig voneinander

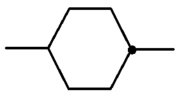, 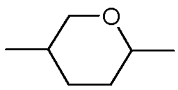, 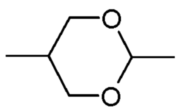,

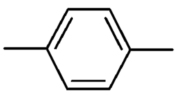, 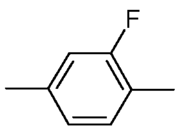 oder 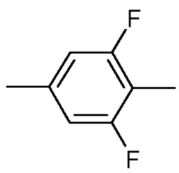

bedeutet.

**3.** Flüssigkristallines Medium nach Anspruch 1 oder 2, **dadurch gekennzeichnet, dass** es eine oder mehrere Verbindungen enthält, die ausgewählt sind aus der Gruppe der Verbindungen der Formeln II und III:

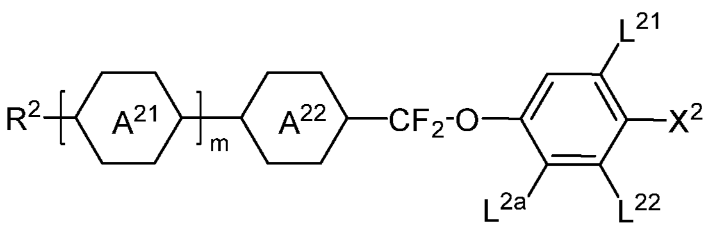 II

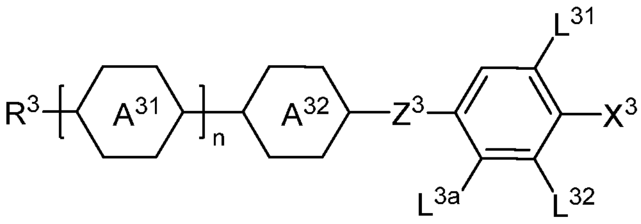 III

worin

$R^2$ einen Alkylrest mit 1 bis 15 C-Atomen, wobei in diesem Rest eine oder mehrere $CH_2$-Gruppen, einschließlich endständiger C-Atome, jeweils unabhängig voneinander so durch -C≡C-, -CH=CH-,

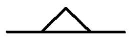 , 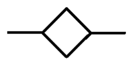 ,

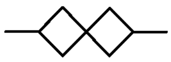 , 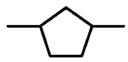 ,

-O-, -S-, -(CO)-O-, -O-(CO)- ersetzt sein können, dass O- oder S-Atome nicht direkt miteinander verknüpft sind, und worin zusätzlich ein oder mehrere H-Atome durch F oder Cl ersetzt sein können, oder H bedeutet,

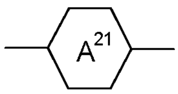 und 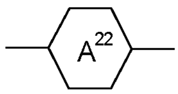

bei jedem Auftreten unabhängig voneinander

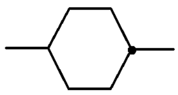 , 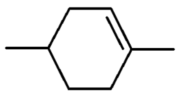 , 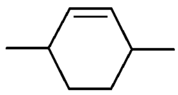 ,

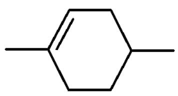 , 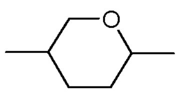 , 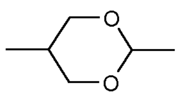 ,

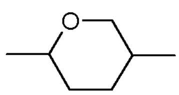 , 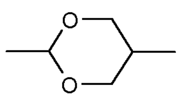 ,

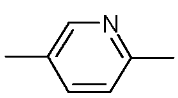 , 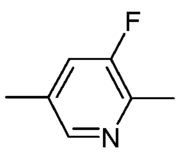 , 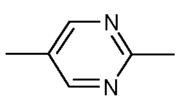 ,

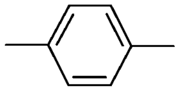 , 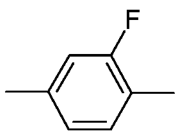 , 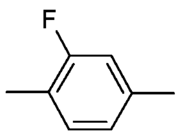 ,

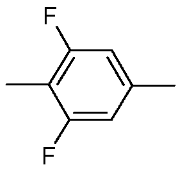
oder
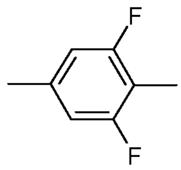

sind, wobei die Ringe gegebenenfalls jeweils durch eine oder zwei Alkylgruppen substituiert sein können,
$L^{21}$ und $L^{22}$ H oder F bedeuten,
$L^{2a}$ H oder $CH_3$ bedeutet,
$X^2$ Halogen, halogeniertes Alkyl oder Alkoxy mit 1 bis 3 C-Atomen oder halogeniertes Alkenyl oder Alkenyloxy mit 2 oder 3 C-Atomen bedeutet,
m 0, 1, 2 oder 3 bedeutet,
$R^3$ einen Alkylrest mit 1 bis 15 C-Atomen bedeutet, wobei in diesem Rest eine oder mehrere $CH_2$-Gruppen, einschließlich endständiger C-Atome, jeweils unabhängig voneinander so durch -C≡C-, -CH=CH-,

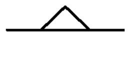 , 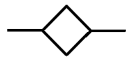 ,

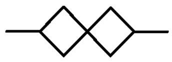 , 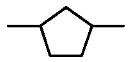 ,

-O-, -S-, -(CO)-O-, -O-(CO)- ersetzt sein können, dass O- oder S-Atome nicht direkt miteinander verknüpft sind, und worin zusätzlich ein oder mehrere H-Atome durch F oder Cl ersetzt sein können,

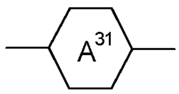
und
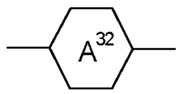

bei jedem Auftreten unabhängig voneinander

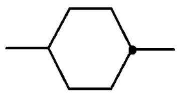 , 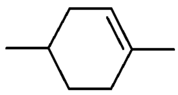 , 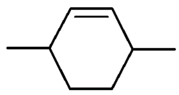 ,

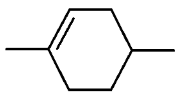 , 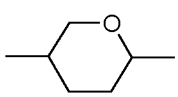 , 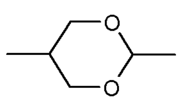 ,

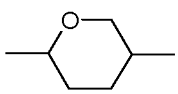 , 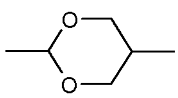 , 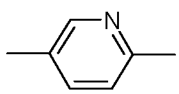 ,

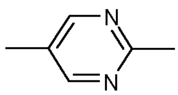 , 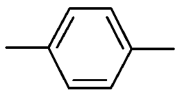 , 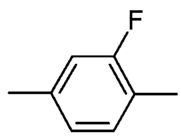 ,

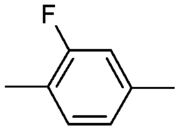, 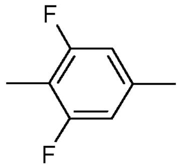 oder 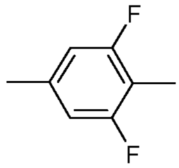

sind, wobei die Ringe gegebenenfalls jeweils durch eine oder zwei Alkylgruppen substituiert sein können,
$L^{31}$ und $L^{32}$ unabhängig voneinander H oder F bedeuten,
$L^{3a}$ H oder $CH_3$ bedeutet,
$X^3$ Halogen, halogeniertes Alkyl oder Alkoxy mit 1 bis 3 C-Atomen oder halogeniertes Alkenyl oder Alkenyloxy mit 2 oder 3 C-Atomen, F, Cl, $-OCF_3$, $-OCHF_2$, $-O-CH_2CF_3$, $-O-CH=CF_2$, $-O-CH=CH_2$ oder $-CF_3$ bedeutet,
$Z^3$ $-CH_2CH_2-$, $-CF_2CF_2-$, -COO-, *trans*-CH=CH-, *trans*-CF=CF-, $-CH_2O-$ oder eine Einfachbindung bedeutet, und
n 0, 1, 2 oder 3 bedeutet.

**4.** Flüssigkristallines Medium nach einem oder mehreren der Ansprüche 1 bis 3, **dadurch gekennzeichnet, dass** es eine oder mehrere dielektrisch neutrale Verbindungen enthält, die ausgewählt sind aus der Gruppe der Formeln IV und V:

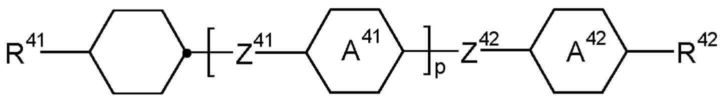 IV

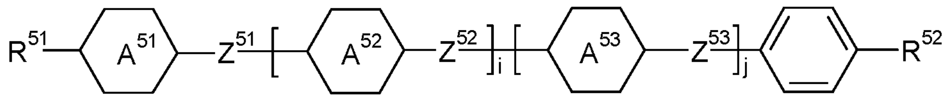 V

worin

$R^{41}$ und $R^{42}$ unabhängig voneinander einen Alkylrest mit 1 bis 15 C-Atomen bedeuten, wobei in diesem Rest eine oder mehrere $CH_2$-Gruppen, einschließlich endständiger C-Atome, jeweils unabhängig voneinander so durch -C=C-, -CH=CH-,

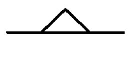, 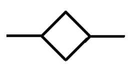, 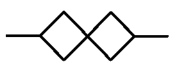, 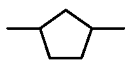,

-O-, -S-, -(CO)-O-, -O-(CO)- ersetzt sein können, dass O- oder S-Atome nicht direkt miteinander verknüpft sind, und worin zusätzlich ein oder mehrere H-Atome durch F oder Cl ersetzt sein können,

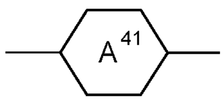 und 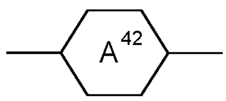

unabhängig voneinander und, wenn

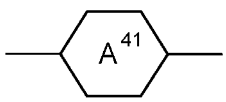

zweimal auftritt, auch diese unabhängig voneinander

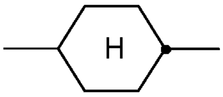 , 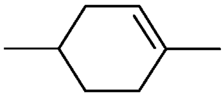 , 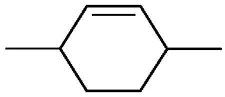 ,

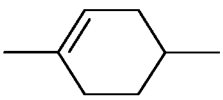 , 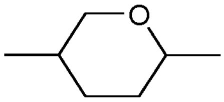 , 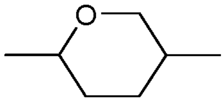 ,

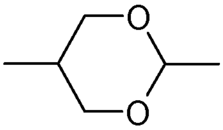 , 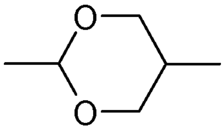 , 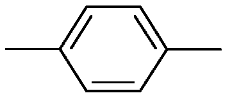 ,

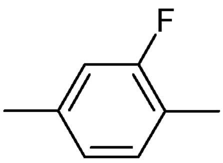 , 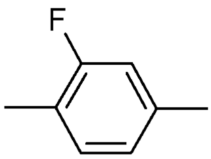 , 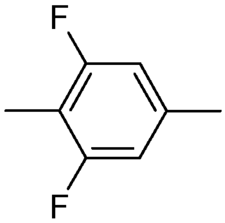 ,

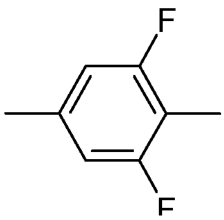 oder 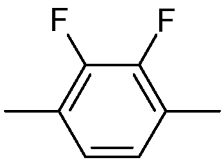

bedeuten, wobei die Ringe gegebenenfalls jeweils durch eine oder zwei Alkylgruppen substituiert sein können,
$Z^{41}$ und $Z^{42}$ unabhängig voneinander und, wenn $Z^{41}$ zweimal auftritt, auch diese unabhängig voneinander $-CH_2CH_2-$, -COO-, *trans*-CH=CH-, *trans*-CF=CF-, $-CH_2O-$, $-CF_2O-$, -C=C- oder eine Einfachbindung bedeuten,
p 0, 1 oder 2 bedeutet,
$R^{51}$ und $R^{52}$ unabhängig voneinander eine der für $R^{41}$ und $R^{42}$ genannten Bedeutungen besitzen,

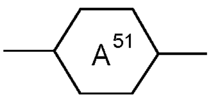 bis 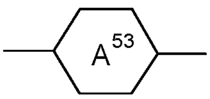 ,

wenn vorhanden, jeweils unabhängig voneinander

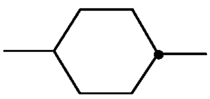 , 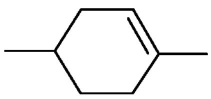 , 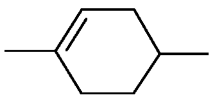 ,

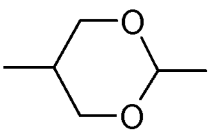 , 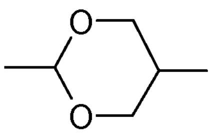 , 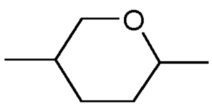 ,

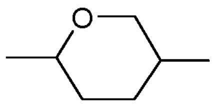 , 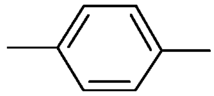 ,

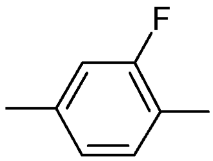 oder 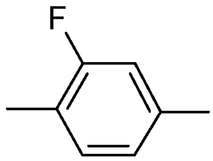

bedeuten, wobei die Ringe gegebenenfalls jeweils durch eine oder zwei Alkylgruppen substituiert sein können, $Z^{51}$ bis $Z^{53}$ jeweils unabhängig voneinander $-CH_2-CH_2-$, $-CH_2-O-$, -CH=CH-, -C=C-, -COO- oder eine Einfachbindung bedeuten, und
i und j jeweils unabhängig voneinander 0 oder 1 bedeuten.

5. Flüssigkristallines Medium nach einem oder mehreren der Ansprüche 1 bis 4, **dadurch gekennzeichnet, dass** es eine oder mehrere Verbindungen enthält, die ausgewählt sind aus der Gruppe bestehend aus den folgenden Formeln T-1 bis T-54:

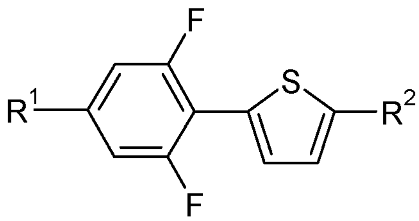

T-1

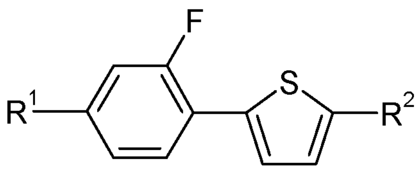

T-2

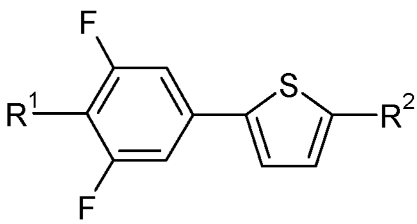

T-3

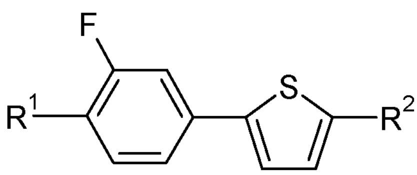

T-4

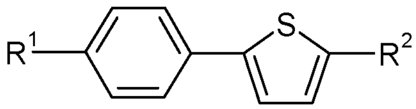

T-5

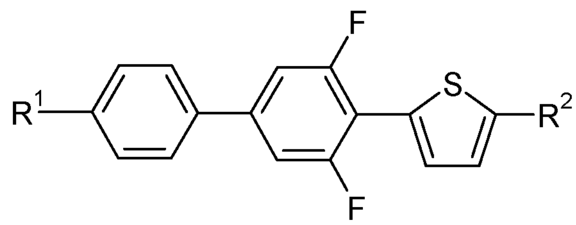

T-6

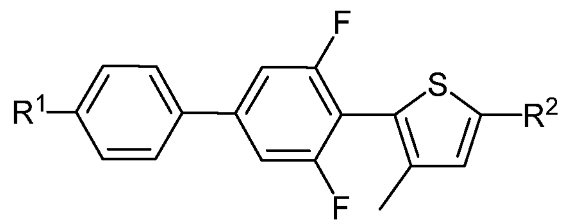

T-7

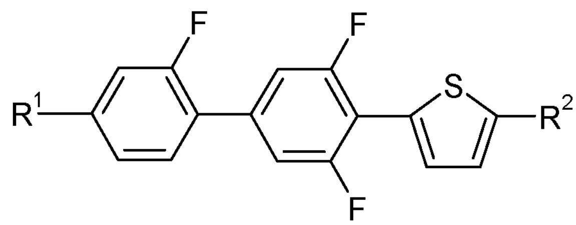

T-8

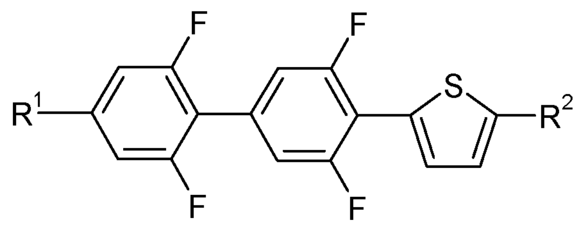

T-9

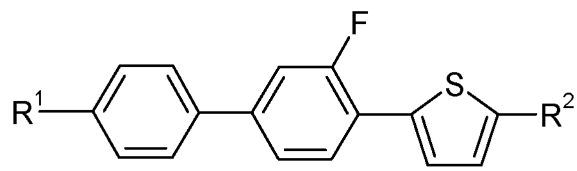

T-10

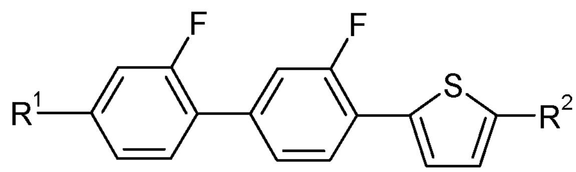

T-11

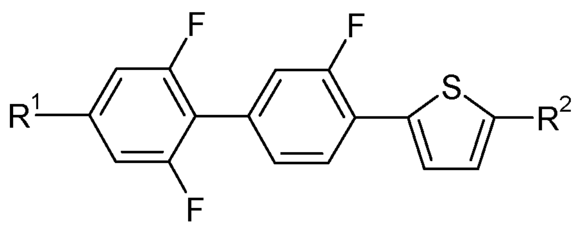

T-12

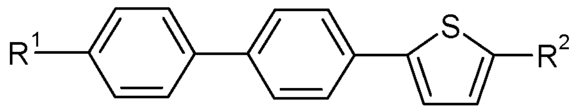

T-13

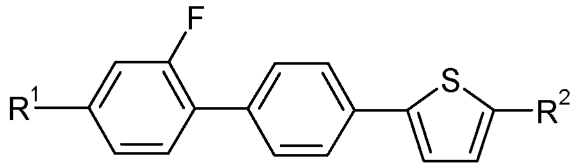

T-14

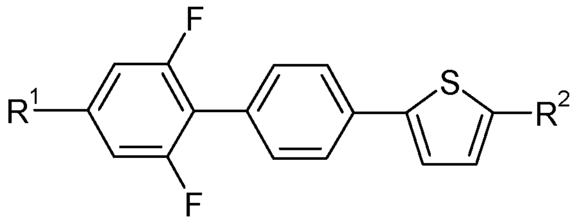

T-15

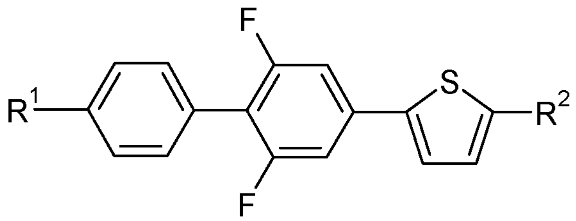

T-16

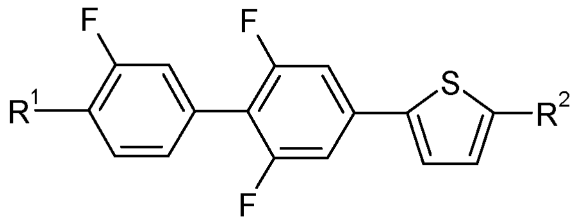

T-17

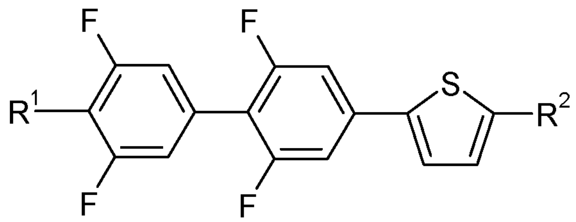

T-18

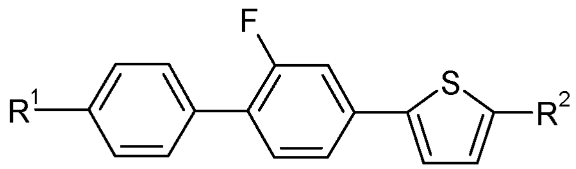

T-19

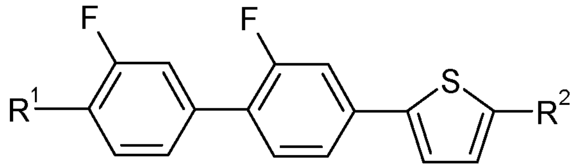

T-20

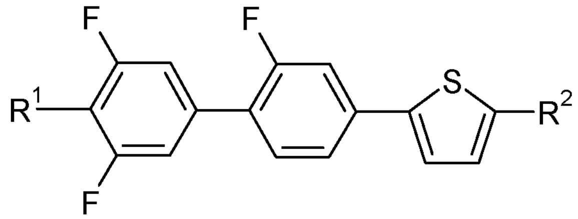

T-21

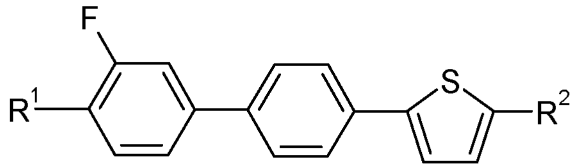

T-22

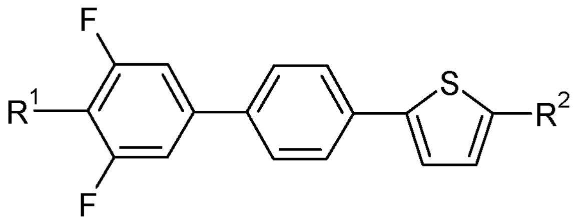

T-23

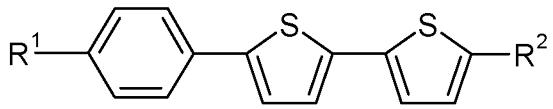

T-24

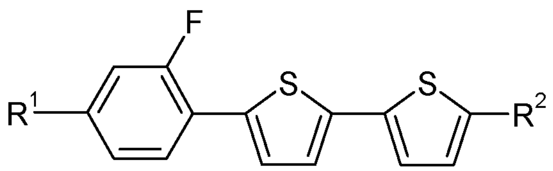

T-25

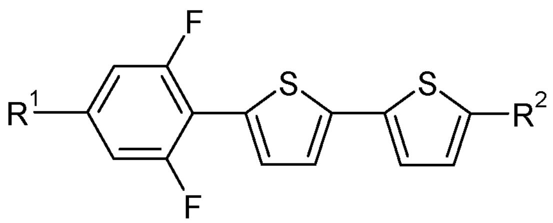

T-26

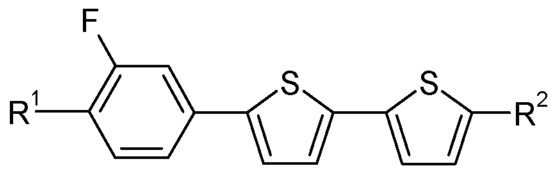

T-27

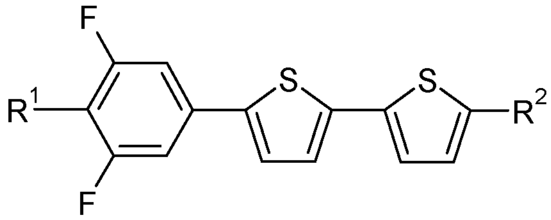

T-28

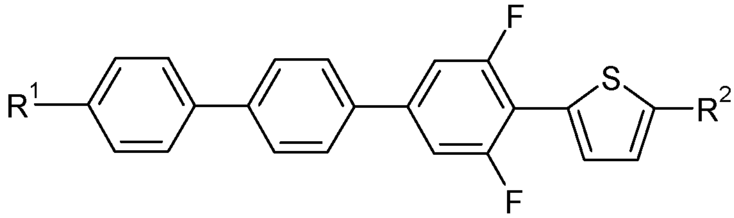

T-29

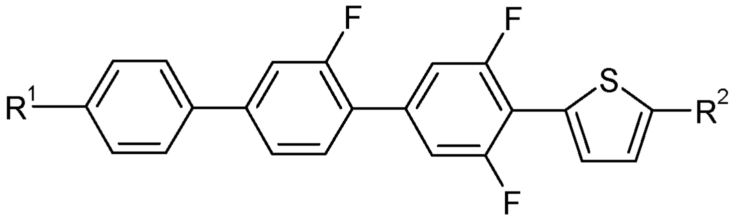

T-30

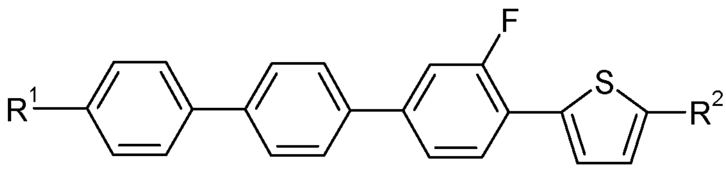

T-31

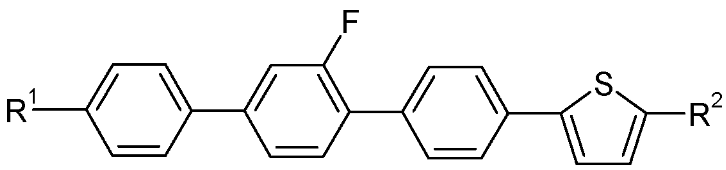

T-32

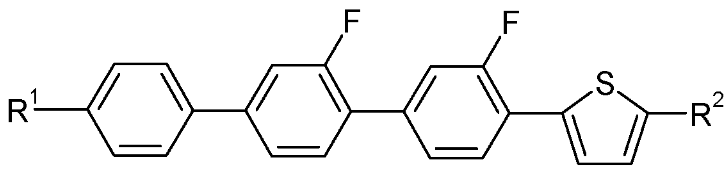

T-33

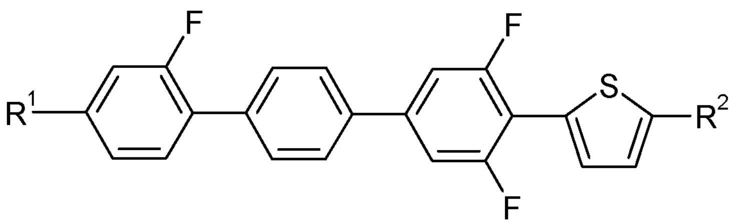

T-34

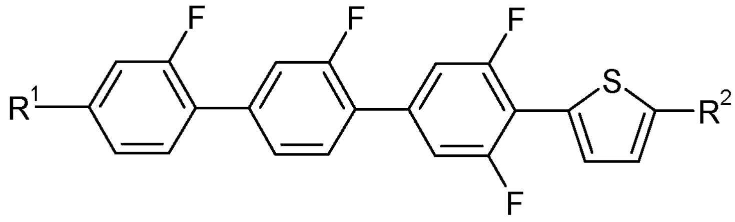

T-35

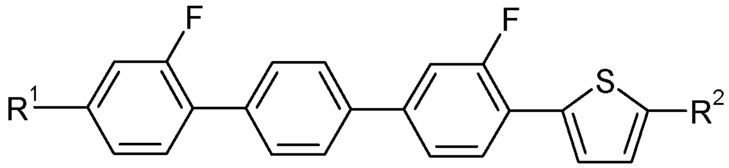

T-36

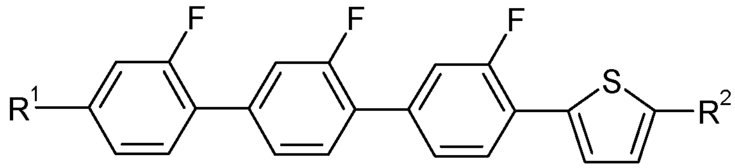

T-37

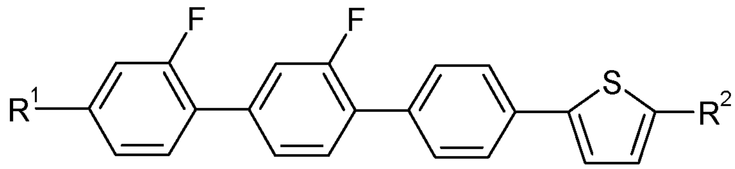

T-38

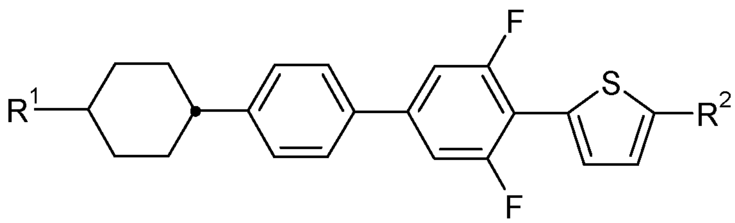

T-39

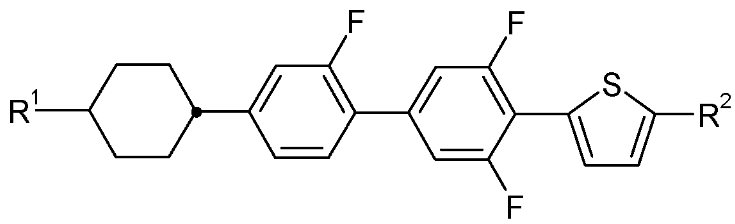

T-40

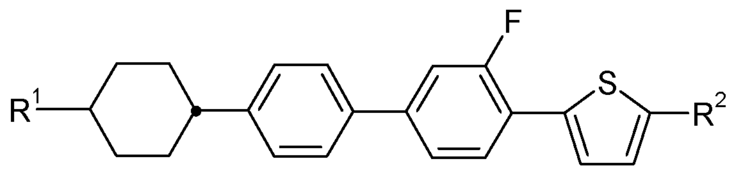

T-41

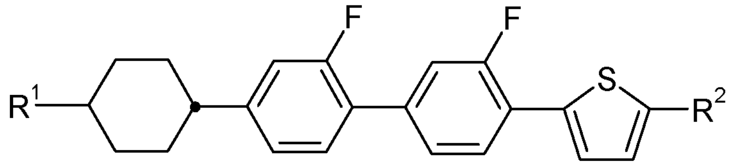

T-42

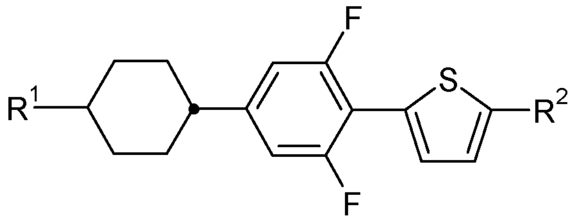

T-43

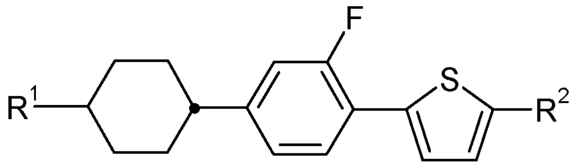

T-44

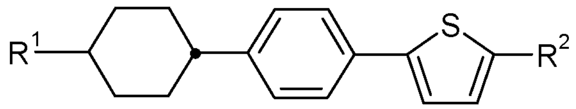

T-45

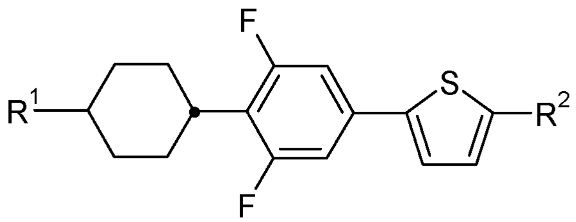

T-46

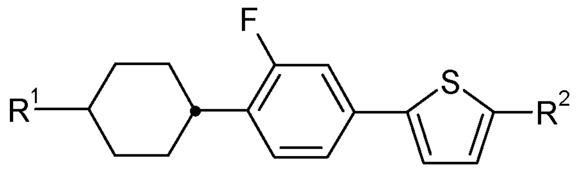

T-47

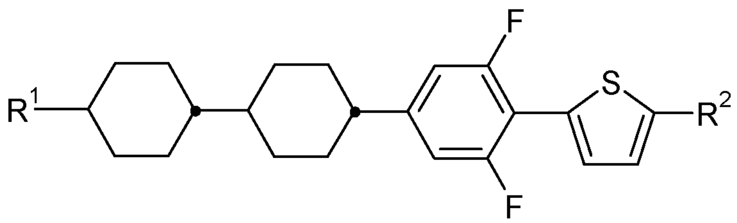

T-48

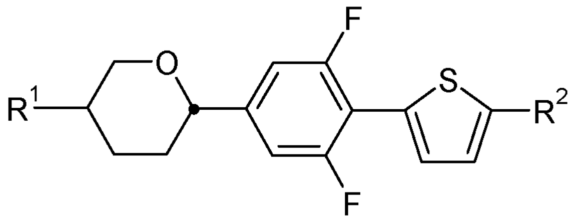

T-49

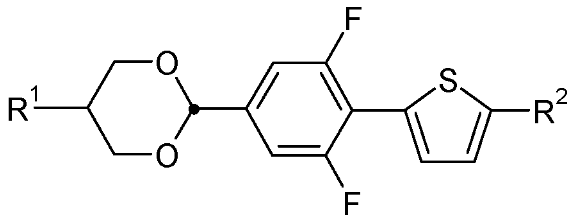

T-50

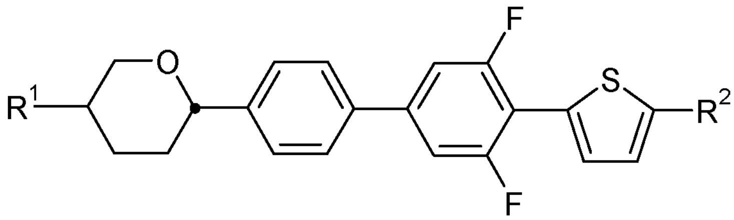

T-51

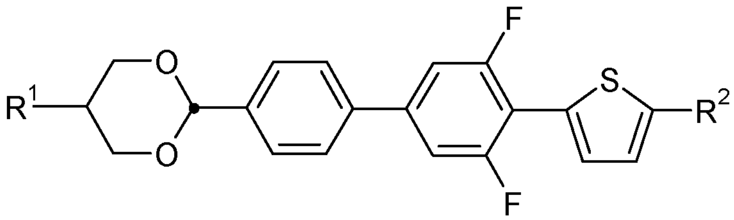

T-52

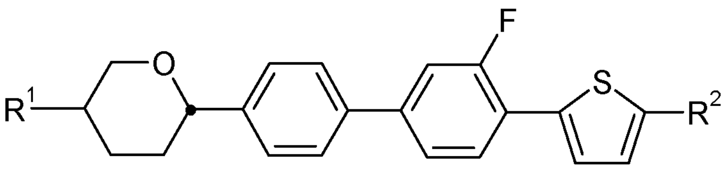

T-53

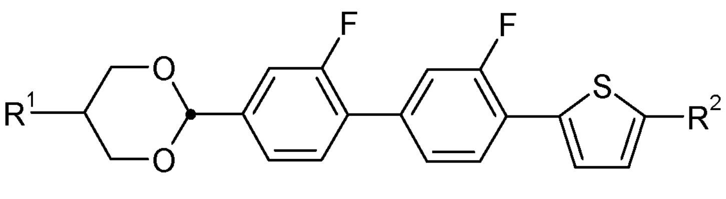

T-54

worin $R^1$ und $R^2$ die in Anspruch 1 angegebenen Bedeutungen besitzen.

**6.** Flüssigkristallines Medium nach einem oder mehreren der Ansprüche 1 bis 5, **dadurch gekennzeichnet, dass** es zusätzlich eine oder mehrere Verbindungen enthält, die ausgewählt sind aus den Formeln III-1j, III-1k und III-1m:

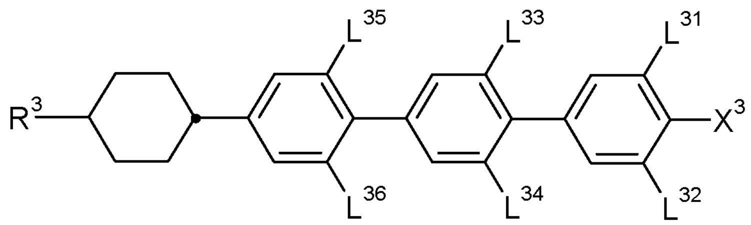

III-1j

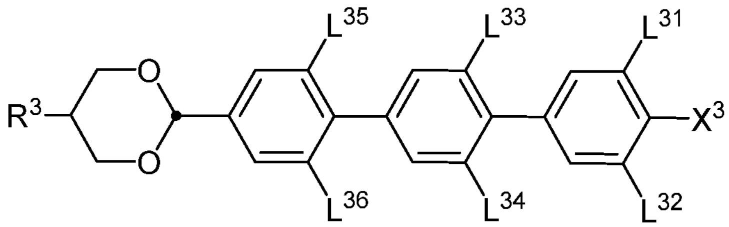

III-1k

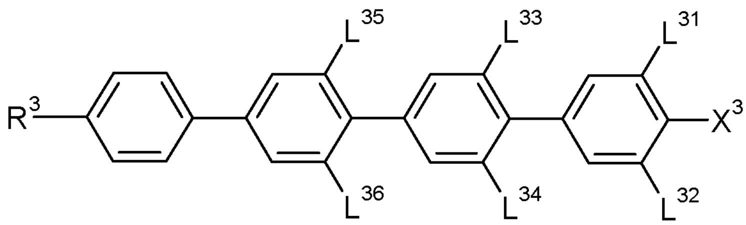

III-1m

worin

$R^3$ Alkyl, Alkoxy, fluoriertes Alkyl oder fluoriertes Alkoxy mit 1 bis 7 C-Atomen, Alkenyl, Alkenyloxy, Alkoxyalkyl oder fluoriertes Alkenyl mit 2 bis 7 C-Atomen bedeutet,
$X^3$ F, Cl, halogeniertes Alkyl oder Alkoxy mit 1 bis 3 C-Atomen oder halogeniertes Alkenyl oder Alkenyloxy mit 2 oder 3 C-Atomen, F, Cl, $-OCF_3$, $-OCHF_2$, $-O-CH_2CF_3$, $-O-CH=CF_2$, $-O-CH=CH_2$ oder $-CF_3$ bedeutet,
$L^{33}$ und $L^{34}$ unabhängig voneinander H oder F bedeuten, und
$L^{35}$ und $L^{36}$ unabhängig voneinander H oder F bedeuten.

**7.** Flüssigkristallines Medium nach einem oder mehreren der Ansprüche 1 bis 6, das zusätzlich eine oder mehrere Verbindungen enthält, die ausgewählt sind aus der Gruppe der Verbindungen der Formeln IV-1 bis IV-4

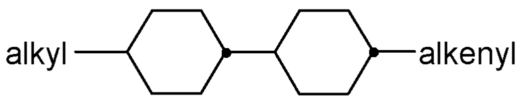

IV-1

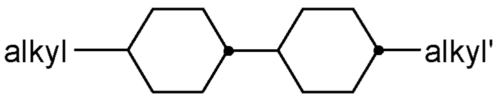

IV-2

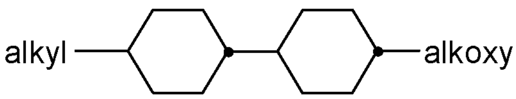

IV-3

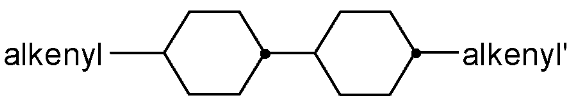

IV-4

worin

alkyl und alkyl' unabhängig voneinander Alkyl mit 1 bis 7 C-Atomen bedeuten,
alkenyl und alkenyl' unabhängig voneinander Alkenyl mit 2 bis 5 C-Atomen bedeuten, und
Alkoxy Alkoxy mit 1 bis 5 C-Atomen bedeutet.

**8.** Flüssigkristallines Medium nach mindestens einem der Ansprüche 1 bis 7, **dadurch gekennzeichnet, dass** die Gesamtkonzentration der Verbindungen der Formel Py im gesamten Medium 1 % oder mehr, aber weniger als 30 % beträgt.

**9.** Flüssigkristallines Medium nach mindestens einem der Ansprüche 1 bis 8, **dadurch gekennzeichnet, dass** es zusätzlich eine oder mehrere chirale Verbindungen und/oder Stabilisatoren enthält.

**10.** Elektrooptische Anzeige oder elektrooptische Komponente, **dadurch gekennzeichnet, dass** sie ein flüssigkristallines Medium nach mindestens einem der Ansprüche 1 bis 9 enthält.

**11.** Anzeige nach Anspruch 10, **dadurch gekennzeichnet, dass** sie auf dem IPS- oder FFS-Modus basiert.

**12.** Anzeige nach Anspruch 10 oder 11, **dadurch gekennzeichnet, dass** sie eine Aktivmatrix-Adressierungsvorrichtung enthält.

**13.** Verwendung eines Mediums nach mindestens einem der Ansprüche 1 bis 9 in einer elektrooptischen Anzeige, in einer elektrooptischen Komponente oder energiesparenden Anwendungen.

**14.** Verfahren zur Herstellung eines flüssigkristallinen Mediums nach einem oder mehreren der Ansprüche 1 bis 9, **dadurch gekennzeichnet, dass** man eine oder mehrere Verbindungen der Formel Py mit einer oder mehreren zusätzlichen mesogenen Verbindungen und gegebenenfalls einem oder mehreren Zusatzstoffen mischt.

## Revendications

**1.** Milieu cristallin liquide ayant une phase nématique, **caractérisé en ce qu'**il comprend un ou plusieurs composés de formule Py

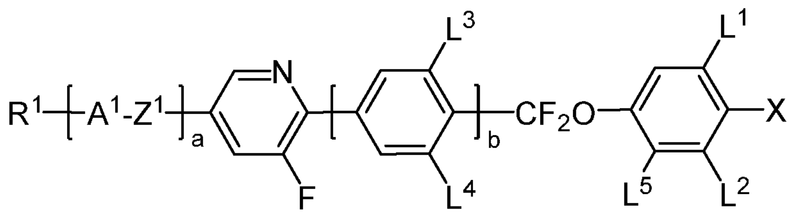

Py

dans laquelle

$R^1$ désigne un radical alkyle ayant de 1 à 15 atomes de C, où un ou plusieurs groupements $CH_2$, y compris des atomes de C terminaux, dans ce radical peuvent chacun être remplacés, indépendamment les uns des autres, par -C=C-, -CH=CH-,

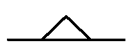 ,

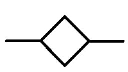 , 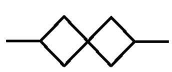 , 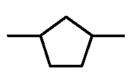 ,

-O-, -S-, -(CO)-O-, -O-(CO)- de telle sorte que les atomes de O ou S ne soient pas liés directement les uns aux autres, et où, de plus, un ou plusieurs atomes de H peuvent être remplacés par F ou Cl, ou H,
$A^1$ à chaque occurrence, indépendamment les uns des autres, désigne

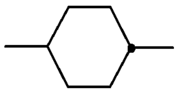 , 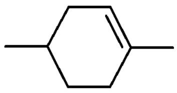 , 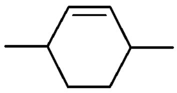 ,

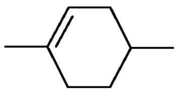 , 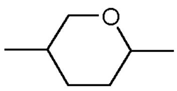 , 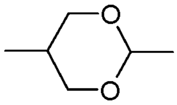 ,

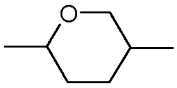 , 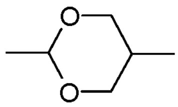 ,

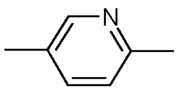 , 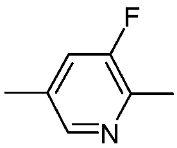 , 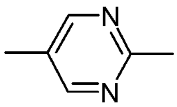 ,

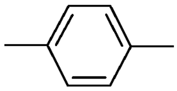 , 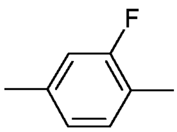 , 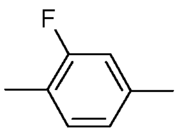 ,

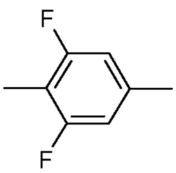 ou 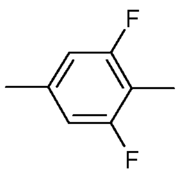 ,

$Z^1$ indépendamment les uns des autres, de manière identique ou différente, désigne une liaison simple, $-CH_2O-$, $-(CO)O-$, $-CF_2O-$, $-CH_2CH_2CF_2O-$, $-CF_2CF_2-$, $-CH_2CF_2-$, $-CH_2CH_2-$, $-(CH_2)_4-$, -CH=CH-, -CH=CF-, -CF=CF- ou -C=C-, où les ponts asymétriques peuvent être orientés des deux côtés,
$L^1$, $L^2$, $L^3$, $L^4$ désignent indépendamment H, F ou Cl,
$L^5$ désigne H, $CH_3$ ou $CH_2CH_3$, préférablement H,
X désigne F, Cl, $-CF_3$, $-OCF_3$, -CN ou -SCN,
a désigne 0, 1 ou 2, préférablement 1,
b désigne 0, 1 ou 2, préférablement 1, et

où $a + b$ est $\leq 3$,

et un ou plusieurs composés supplémentaires de formule T

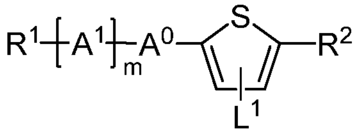 T

dans laquelle les radicaux individuels revêtent les significations suivantes :

$R^1$ et $R^2$ désignent H, F, Cl, Br, -CN, -NCS ou alkyle à chaîne linéaire ou ramifiée ayant de 1 à 12 atomes de C, où, de plus, un ou plusieurs groupements $CH_2$, y compris des atomes de C terminaux, peuvent

chacun être remplacés, indépendamment les uns des autres, par -CH=CH-, -C≡C-,

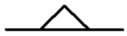 , 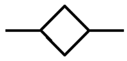 , 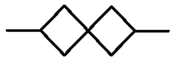 , 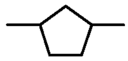 ,

-O-, -S-, -CO-, -CO-O-, -O-CO-, de telle sorte que les atomes de O ou S ne soient pas liés directement les uns aux autres, et où, de plus, un ou plusieurs atomes de H peuvent être remplacés par F ou Cl,

$L^1$ H ou $CH_3$,

$A^0$ désigne

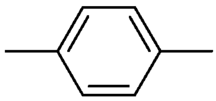 , 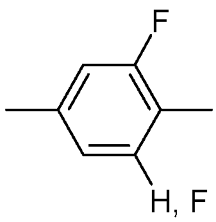 , 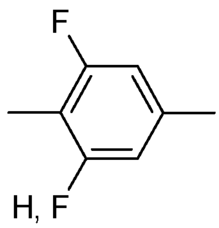 ,

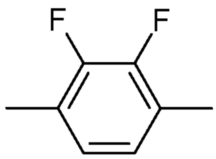 ou 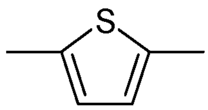 ,

$A^1$, indépendamment les uns des autres, désigne phénylène-1,4-diyle, où, de plus, un ou deux groupements CH peuvent être remplacés par N et un ou plusieurs atomes de H peuvent être remplacés par halogène, CN, $CH_3$, $CHF_2$, $CH_2F$, $OCH_3$, $OCHF_2$ ou $OCF_3$, cyclohexane-1,4-diyle, où, de plus, un ou deux groupements $CH_2$ non adjacents peuvent être remplacés, indépendamment les uns des autres, par O et/ou S et un ou plusieurs atomes de H peuvent être remplacés par F, cyclohexène-1,4-diyle, bicyclo[1.1.1]pentane-1,3-diyle, bicyclo[2.2.2]octane-1,4-diyle, spiro[3.3]heptane-2,6-diyle, tétrahydropyran-2,5-diyle ou 1,3-dioxane-2,5-diyle,

m dans chaque cas, indépendamment les uns des autres, désigne 0, 1 ou 2.

**2.** Milieu cristallin liquide selon la revendication 1, dans lequel, dans la formule Py

$A^1$ désigne à chaque occurrence, indépendamment les uns des autres,

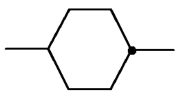 , 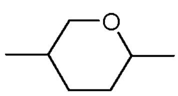 , 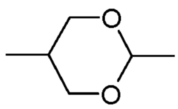 ,

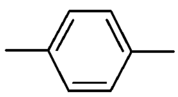 , 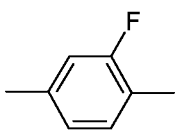 ou 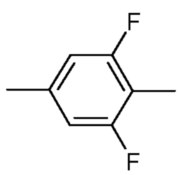 .

**3.** Milieu cristallin liquide selon la revendication 1 ou 2, **caractérisé en ce qu'**il comprend un ou plusieurs composés choisis dans le groupe constitué par les composés de formules II et III :

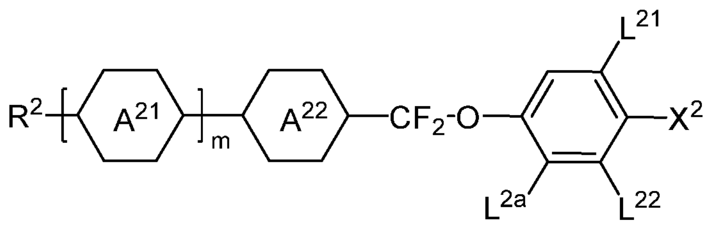

II

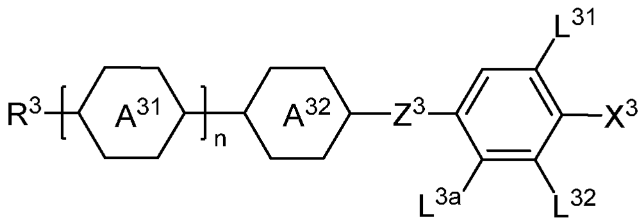

III

dans lesquelles

$R^2$ désigne un radical alkyle ayant de 1 à 15 atomes de C, où un ou plusieurs groupements $CH_2$, y compris des atomes de C terminaux, dans ce radical peuvent chacun être remplacés, indépendamment les uns des autres, par -C=C-, -CH=CH-,

, 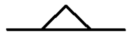 , 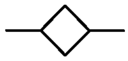 , 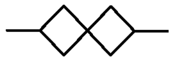 ,

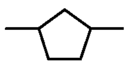 ,

-O-, -S-, -(CO)-O-, -O-(CO)- de telle sorte que les atomes de O ou S ne soient pas liés directement les uns aux autres, et où, de plus, un ou plusieurs atomes de H peuvent être remplacés par F ou Cl, ou H,

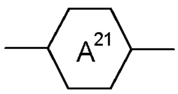 et 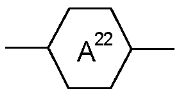

à chaque occurrence, indépendamment l'un de l'autre, sont

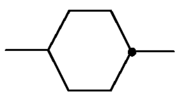 , 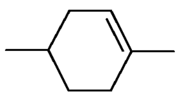 , 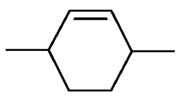 ,

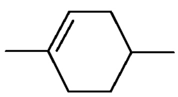 , 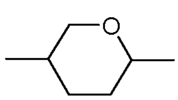 , 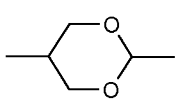 ,

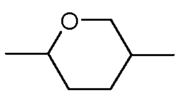 , 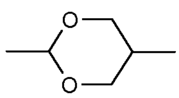 ,

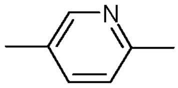, 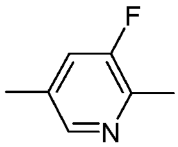, 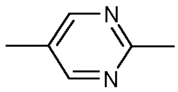,

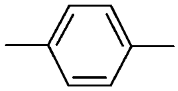, 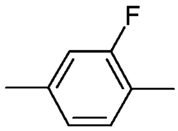, 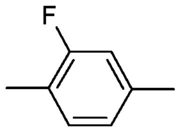,

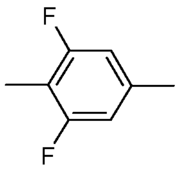 ou 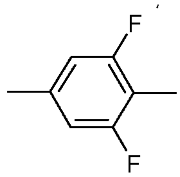,

où les cycles peuvent éventuellement être chacun substitués par un ou deux groupements alkyle,
$L^{21}$ et $L^{22}$ désignent H ou F,
$L^{2a}$ désigne H ou $CH_3$,
$X^2$ désigne halogène, alkyle ou alcoxy halogéné ayant de 1 à 3 atomes de C ou alcényle ou alcényloxy halogéné ayant 2 ou 3 atomes de C,
m désigne 0, 1, 2 ou 3,
$R^3$ désigne un radical alkyle ayant de 1 à 15 atomes de C, où un ou plusieurs groupements $CH_2$, y compris des atomes de C terminaux, dans ce radical peuvent chacun être remplacés, indépendamment les uns des autres, par -C=C-, -CH=CH-,

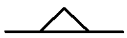, 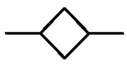, 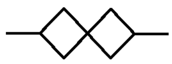,

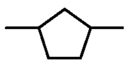,

-O-, -S-, -(CO)-O-, -O-(CO)- de telle sorte que les atomes de O ou S ne soient pas liés directement les uns aux autres, et où, de plus, un ou plusieurs atomes de H peuvent être remplacés par F ou Cl,

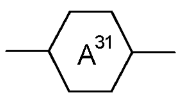 et 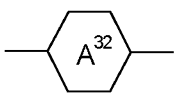

à chaque occurrence, indépendamment l'un de l'autre, sont

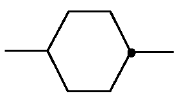, 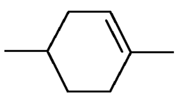, 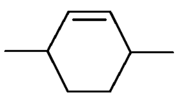,

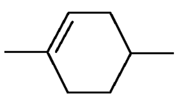, 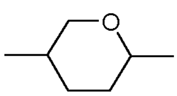, 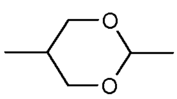,

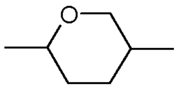, 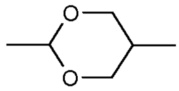, 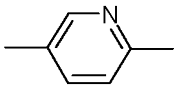,

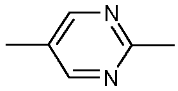, 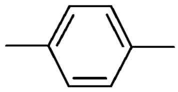, 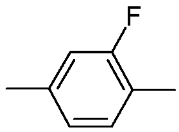,

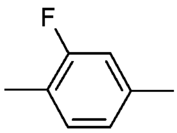, 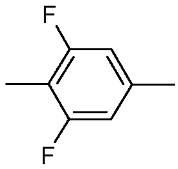 ou 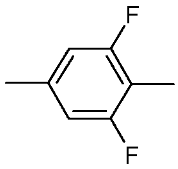,

où les cycles peuvent éventuellement être chacun substitués par un ou deux groupements alkyle,
$L^{31}$ et $L^{32}$, indépendamment l'un de l'autre, désignent H ou F,
$L^{3a}$ désigne H ou $CH_3$,
$X^3$ désigne halogène, alkyle ou alcoxy halogéné ayant de 1 à 3 atomes de C ou alcényle ou alcényloxy halogéné ayant 2 ou 3 atomes de C, F, Cl, $-OCF_3$, $-OCHF_2$, $-O-CH_2CF_3$, $-O-CH=CF_2$, $-O-CH=CH_2$ ou $-CF_3$,
$Z^3$ désigne $-CH_2CH_2-$, $-CF_2CF_2-$, -COO-, *trans*-CH=CH-, *trans*-CF=CF-, $-CH_2O-$ ou une liaison simple, et
n désigne 0, 1, 2 ou 3.

4. Milieu cristallin liquide selon l'une ou plusieurs parmi les revendications 1 à 3, **caractérisé en ce qu'**il comprend un ou plusieurs composés diélectriquement neutres choisis dans le groupe constitué par les formules IV et V :

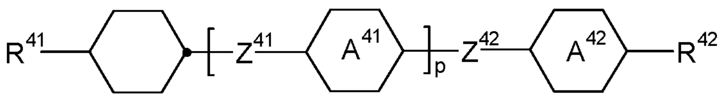 IV

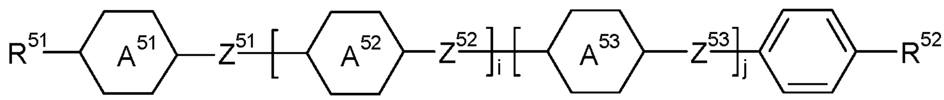 V

dans lesquelles

$R^{41}$ et $R^{42}$, indépendamment l'un de l'autre, désignent un radical alkyle ayant de 1 à 15 atomes de C, où un ou plusieurs groupements $CH_2$, y compris des atomes de C terminaux, dans ce radical peuvent chacun être remplacés, indépendamment les uns des autres, par -C≡C-, -CH=CH-,

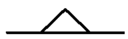, 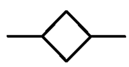,

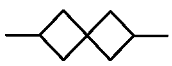, 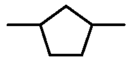,

-O-, -S-, -(CO)-O-, -O-(CO)- de telle sorte que les atomes de O ou S ne soient pas liés directement les uns aux autres, et où, de plus, un ou plusieurs atomes de H peuvent être remplacés par F ou Cl,

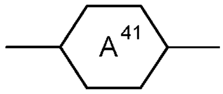 et 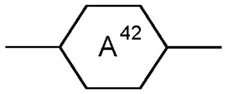

indépendamment l'un de l'autre et, si

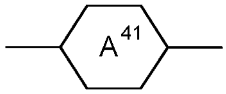

est présent deux fois, de même ceux-ci indépendamment l'un de l'autre, désignent

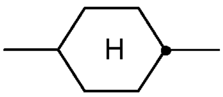 , 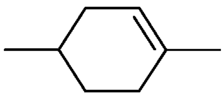 , 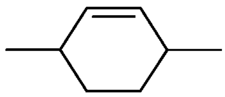 ,

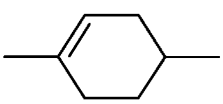 , 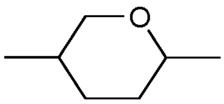 , 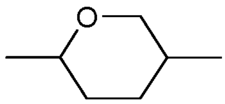 ,

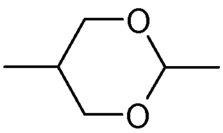 , 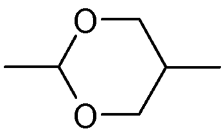 , 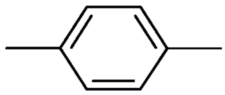 ,

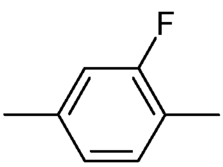 , 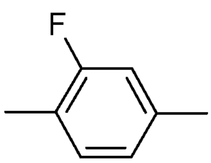 , 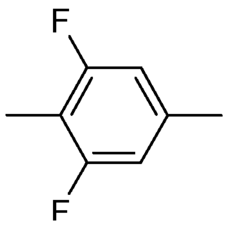 ,

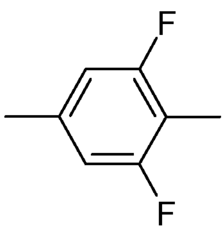 ou 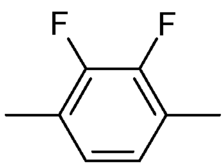 ,

où les cycles peuvent éventuellement être chacun substitués par un ou deux groupements alkyle,
$Z^{41}$ et $Z^{42}$, indépendamment l'un de l'autre et, si $Z^{41}$ est présent deux fois, de même ceux-ci indépendamment l'un de l'autre, désignent $-CH_2CH_2-$, -COO-, *trans*-CH=CH-, *trans*-CF=CF-, $-CH_2O-$, $-CF_2O-$, -C=C- ou une liaison simple,
p désigne 0, 1 ou 2,
$R^{51}$ et $R^{52}$, indépendamment l'un de l'autre, revêtent l'une des significations données pour $R^{41}$ et $R^{42}$

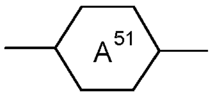 à 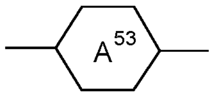.

si présents, désignent chacun, indépendamment les uns des autres

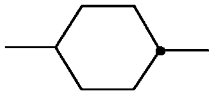, 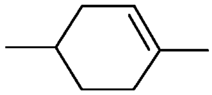, 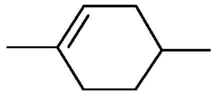,

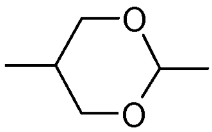, 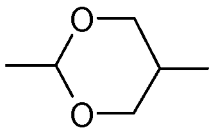, 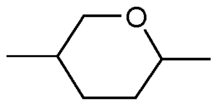,

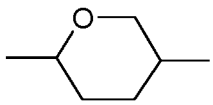, 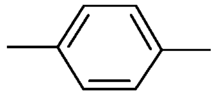,

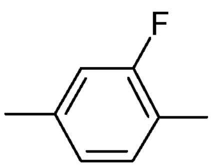 ou 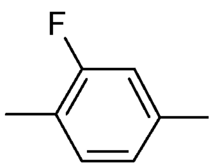,

où les cycles peuvent éventuellement être chacun substitués par un ou deux groupements alkyle,
$Z^{51}$ à $Z^{53}$ désignent chacun, indépendamment les uns des autres, $-CH_2-CH_2-$, $-CH_2-O-$, -CH=CH-, -C=C-, -COO- ou une liaison simple, et
i et j désignent chacun, indépendamment l'un de l'autre, 0 ou 1.

**5.** Milieu cristallin liquide selon l'une ou plusieurs parmi les revendications 1 à 4, **caractérisé en ce qu'**il comprend un ou plusieurs composés choisis dans le groupe constitué par les formules T-1 à T-54 suivantes :

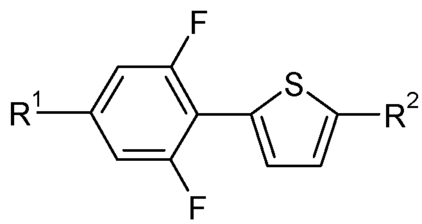

T-1

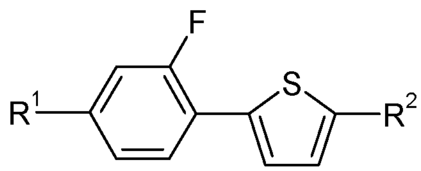

T-2

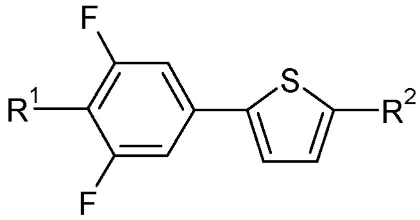

T-3

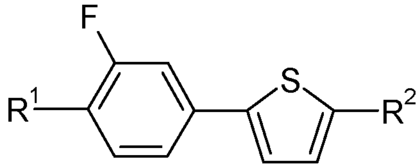

T-4

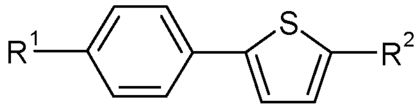

T-5

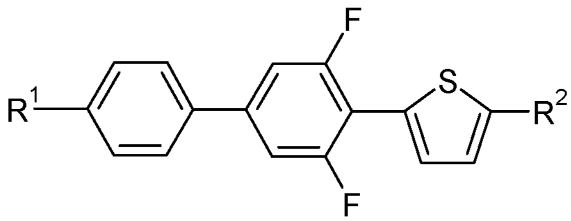

T-6

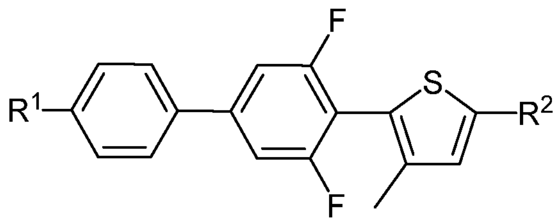

T-7

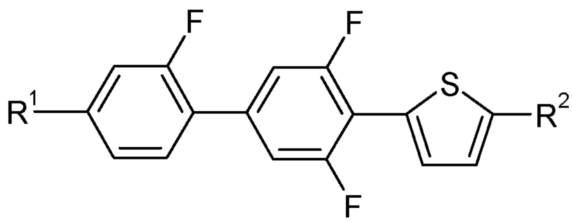

T-8

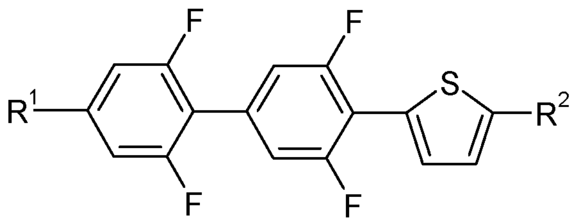

T-9

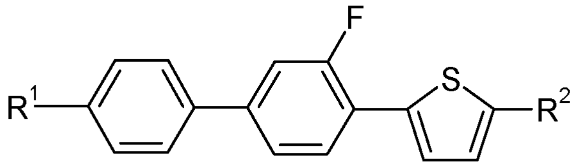

T-10

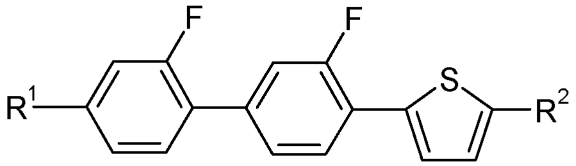

T-11

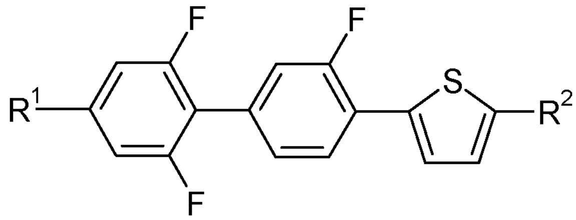

T-12

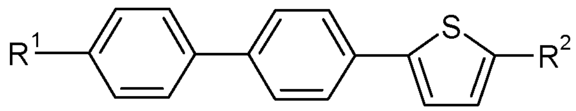

T-13

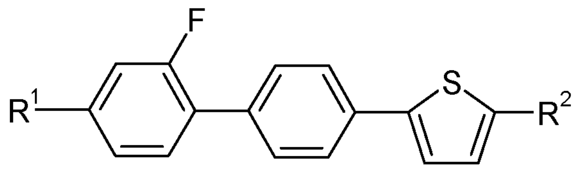

T-14

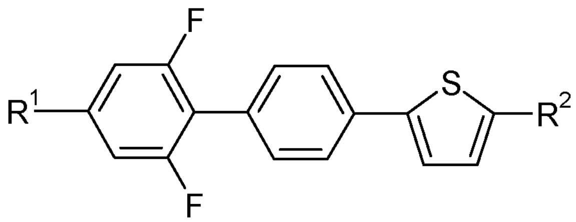

T-15

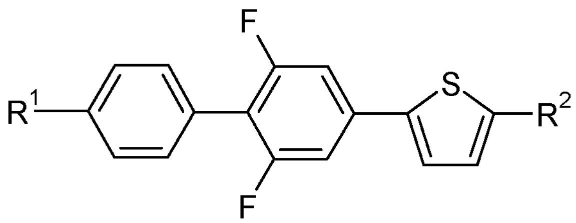

T-16

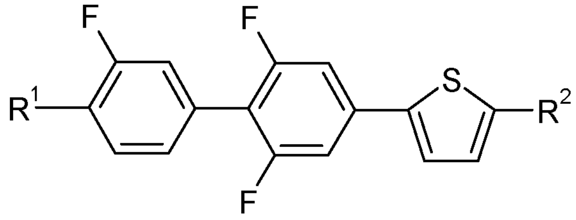

T-17

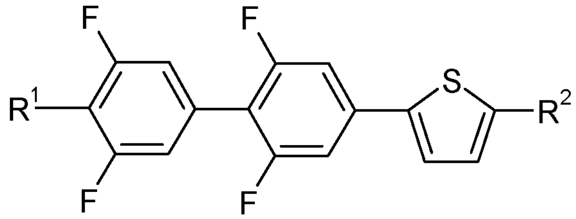

T-18

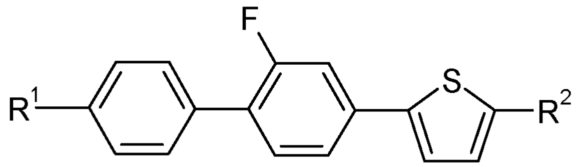

T-19

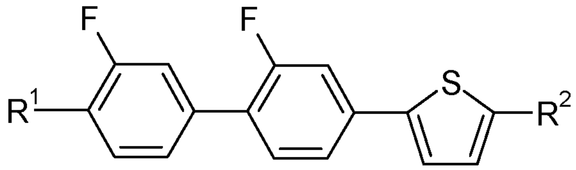

T-20

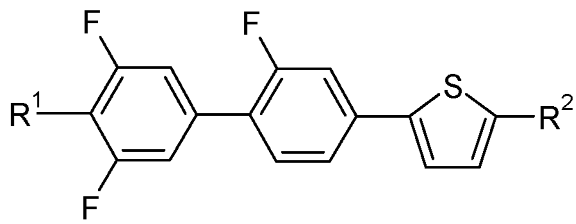

T-21

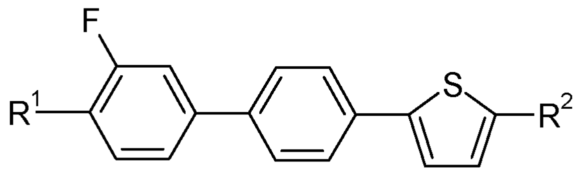

T-22

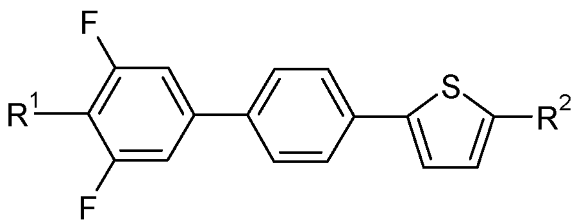

T-23

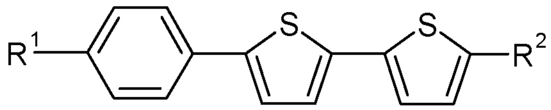

T-24

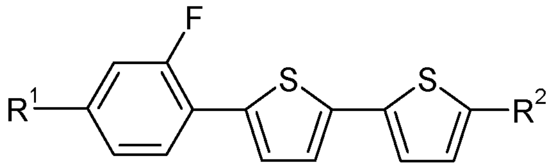

T-25

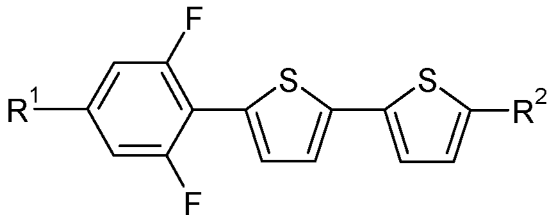

T-26

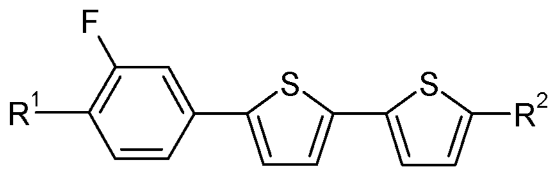

T-27

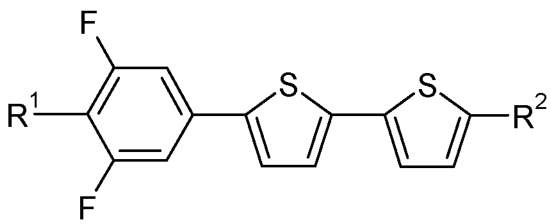

T-28

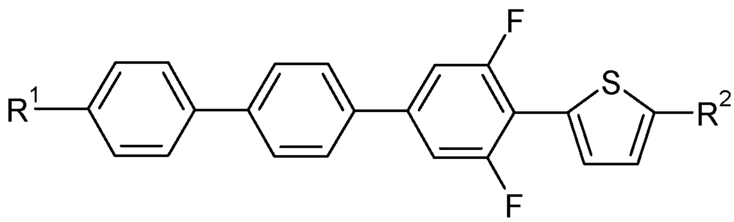

T-29

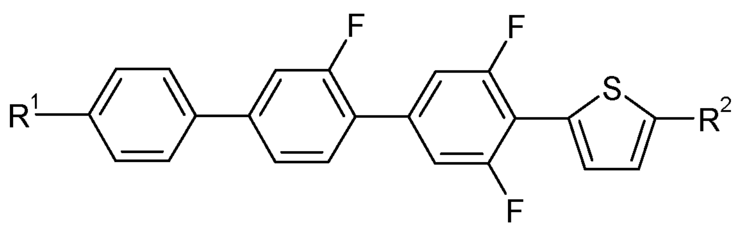

T-30

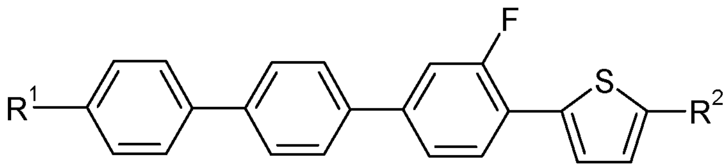

T-31

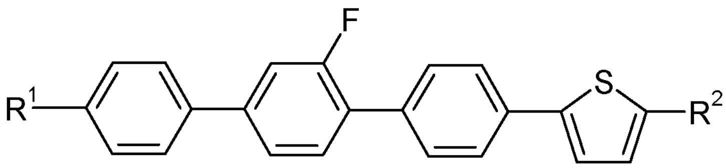

T-32

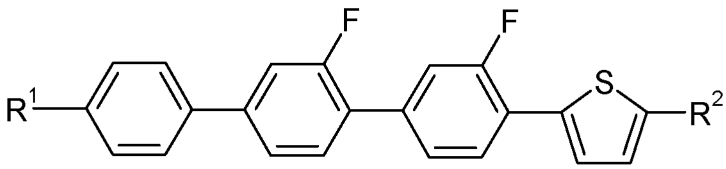

T-33

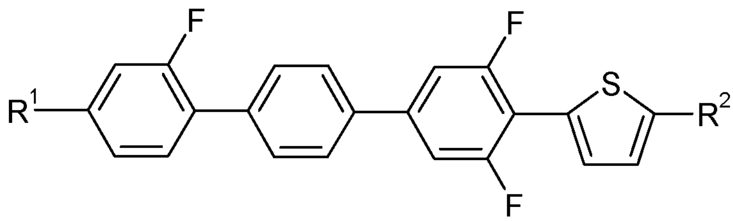

T-34

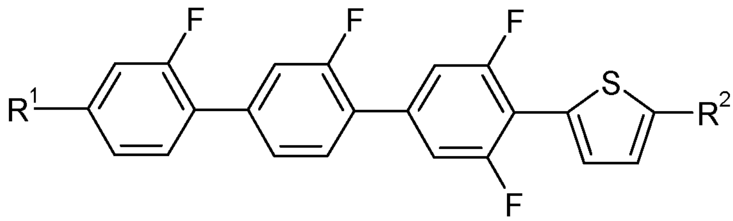

T-35

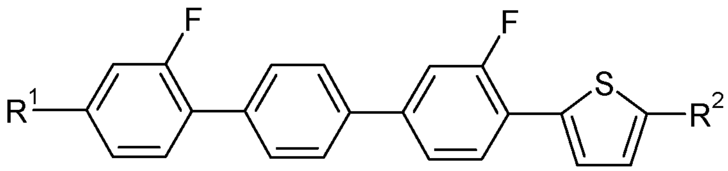

T-36

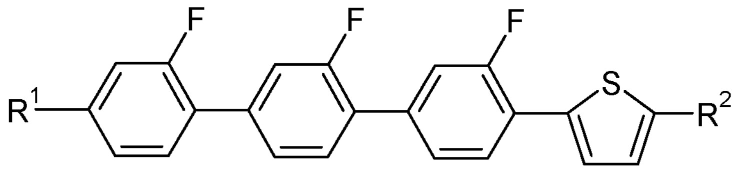

T-37

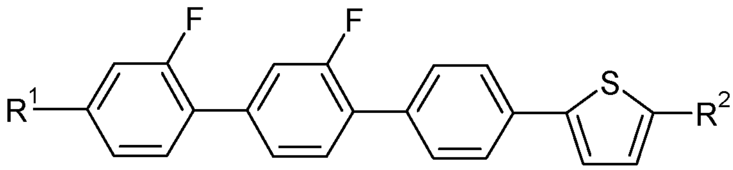

T-38

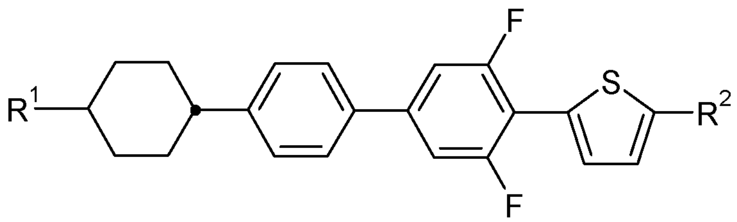

T-39

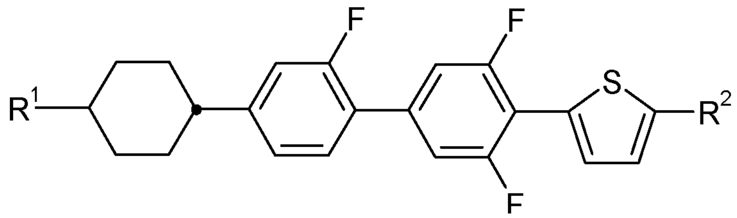

T-40

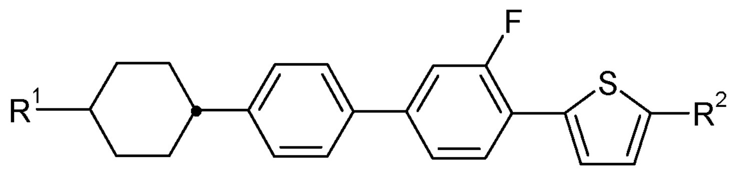

T-41

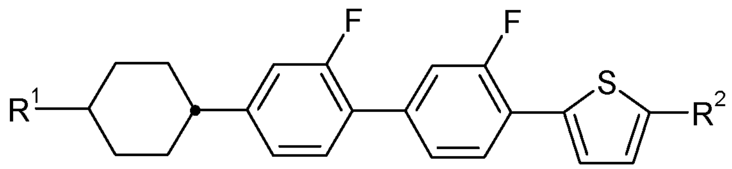

T-42

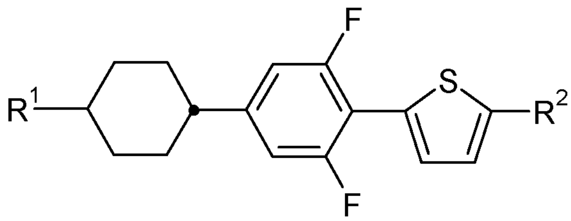

T-43

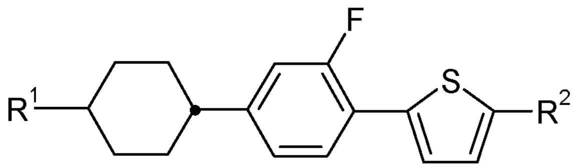

T-44

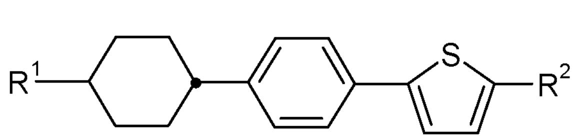

T-45

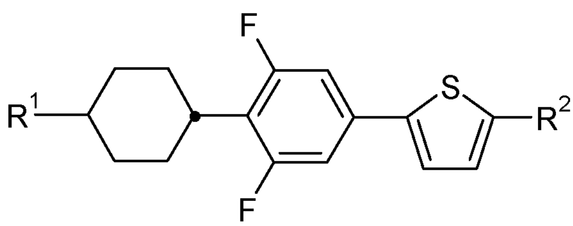

T-46

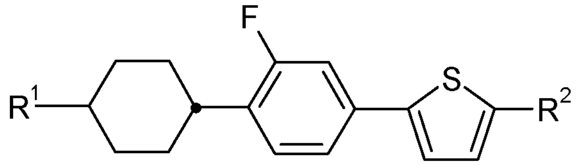

T-47

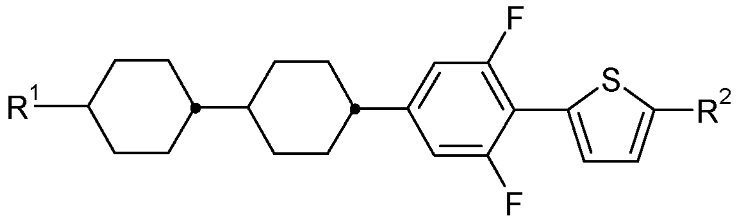

T-48

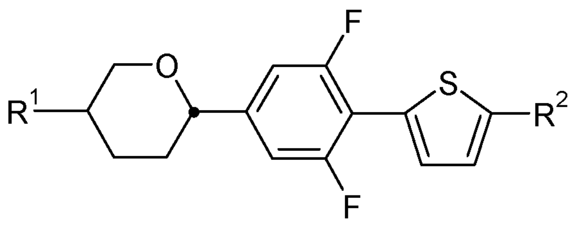

T-49

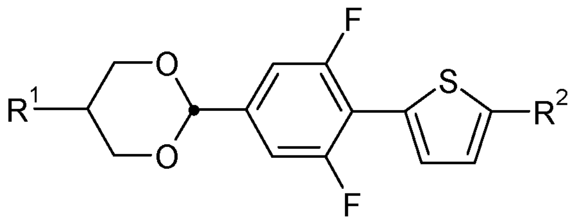

T-50

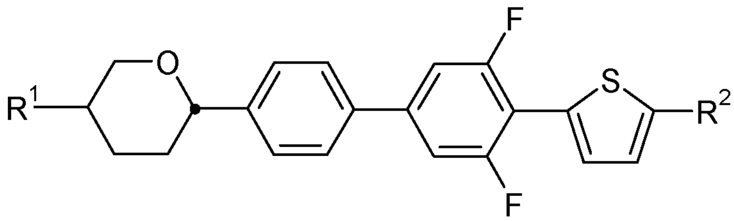

T-51

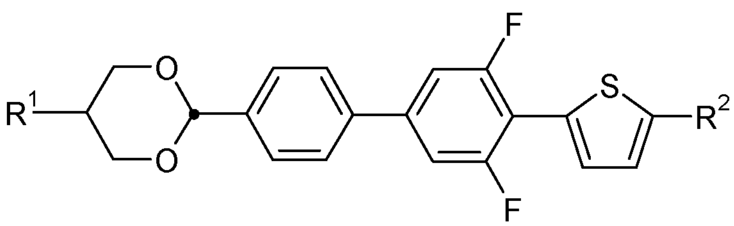

T-52

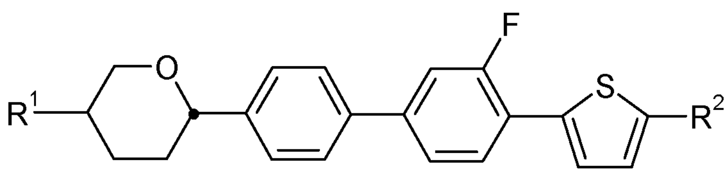

T-53

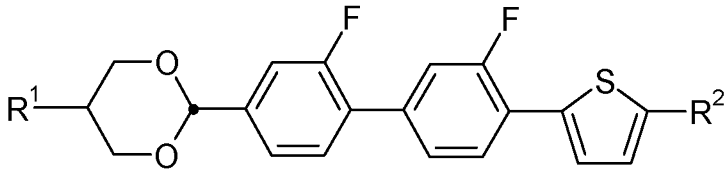

T-54

dans lesquelles $R^1$ et $R^2$ revêtent les significations indiquées selon la revendication 1.

**6.** Milieu cristallin liquide selon l'une ou plusieurs parmi les revendication; 1 à 5, **caractérisé en ce qu'**il comprend en outre un ou plusieurs composés choisis parmi les formules III-1j, III-1k et III-1m :

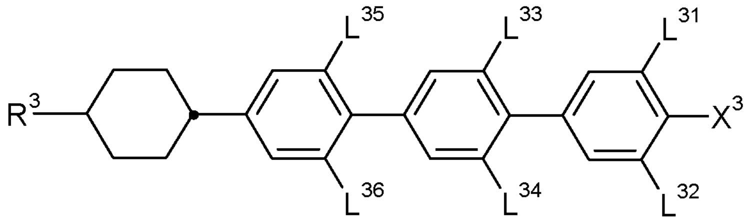

III-1j

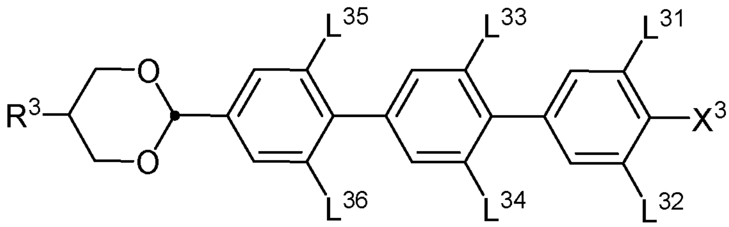

III-1k

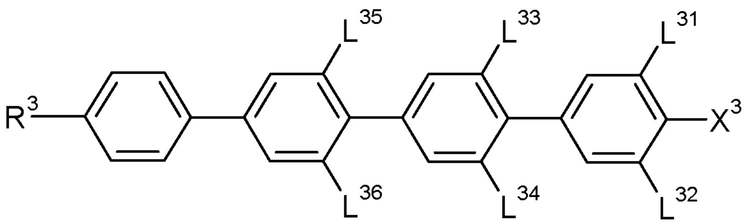

III-1m

dans lesquelles

$R^3$ désigne alkyle, alcoxy, alkyle fluoré ou alcoxy fluoré ayant de 1 à 7 atomes de C, alcényle, alcényloxy, alcoxyalkyle, ou alcényle fluoré ayant de 2 à 7 atomes de C,
$X^3$ désigne F, Cl, alkyle ou alcoxy halogéné ayant de 1 à 3 atomes de C ou alcényle ou alcényloxy halogéné ayant 2 ou 3 atomes de C, F, Cl, $-OCF_3$, $-OCHF_2$, $-O-CH_2CF_3$, $-O-CH=CF_2$, $-O-CH=CH_2$ ou $-CF_3$,
$L^{33}$ et $L^{34}$, indépendamment l'un de l'autre, désignent H ou F, et
$L^{35}$ et $L^{36}$, indépendamment l'un de l'autre, désignent H ou F.

**7.** Milieu cristallin liquide selon l'une ou plusieurs parmi les revendications 1 à 6, comprenant en outre un ou plusieurs composés choisis dans le groupe constitué par les composés de formules IV-1 à IV-4,

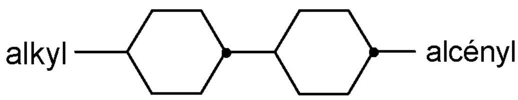

IV-1

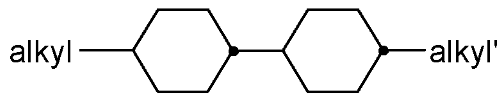

IV-2

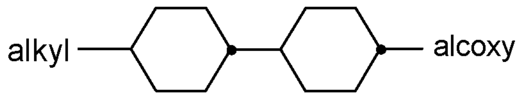

IV-3

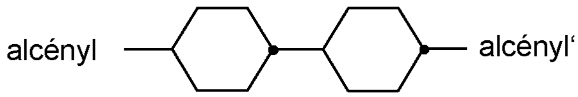

IV-4

dans lesquelles

alkyl et alkyl', indépendamment les uns des autres, désignent alkyle ayant de 1 à 7 atomes de C,
alcényl et alcényl', indépendamment les uns des autres, désignent alcényle ayant de 2 à 5 atomes de C, et
alcoxy désigne alcoxy ayant de 1 à 5 atomes de C.

**8.** Milieu cristallin liquide selon au moins l'une parmi les revendications 1 à 7, **caractérisé en ce que** la concentration totale en composés de formule Py dans le milieu dans son ensemble est de 1 % ou plus mais inférieure à 30%.

**9.** Milieu cristallin liquide selon au moins l'une parmi les revendications 1 à 8, **caractérisé en ce qu'**il comprend en outre un ou plusieurs composés chiraux et/ou stabilisateurs.

**10.** Affichage électro-optique ou composant électro-optique, **caractérisé en ce qu'**il comprend un milieu cristallin liquide selon au moins l'une parmi les revendications 1 à 9.

**11.** Affichage selon la revendication 10, **caractérisé en ce qu'**il est basé sur le mode IPS ou FFS.

**12.** Affichage selon la revendication 10 ou 11, **caractérisé en ce qu'**il contient un dispositif d'adressage à matrice active.

**13.** Utilisation d'un milieu selon au moins l'une parmi les revendications 1 à 9, dans un affichage électro-optique, dans un composant électro-optique ou des applications d'économie d'énergie.

**14.** Procédé de préparation d'un milieu cristallin liquide selon l'une ou plusieurs parmi les revendications 1 à 9, **caractérisé en ce qu'**un ou plusieurs composés de formule Py sont mélangés avec un ou plusieurs composés mésogènes supplémentaires et éventuellement un ou plusieurs additifs.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20130207038 A1
- WO 2008128622 A1
- DE 102013016399 A1
- WO 2012013281 A1
- EP 2137154 A2
- US 20020041354 A1


### Non-patent literature cited in the description


- **TOGASHI, S. ; SEKIGUCHI, K. ; TANABE, H. ; YAMAMOTO, E. ; SORIMACHI, K. ; TAJIMA, E. ; WATANABE, H. ; SHIMIZU, H.** A 210-288 Matrix LCD Controlled by Double Stage Diode Rings. *Proc. Eurodisplay,* September 1984, vol. 84, 141
- **STROMER, M.** Design of Thin Film Transistors for Matrix Addressing of Television Liquid Crystal Displays. *Proc. Eurodisplay,* September 1984, vol. 84, 145
- **YEO, S.D.** An LC Display for the TV Application. *SID 2004 International Symposium, Digest of Technical Papers, XXXV, Book II,* 758, , 759
- **YOSHIDE, H. et al.** MVA LCD for Notebook or Mobile PCs ... *SID 2004 International Symposium, Digest of Technical Papers, XXXV, Book I,* 6-9
- **LIU, C.T. et al.** A 46-inch TFT-LCD HDTV Technology ... *SID 2004 International Symposium, Digest of Technical Papers, XXXV, Book II,* 750-753
- **KIM, SANG SOO.** Super PVA Sets New State-of-the-Art for LCD-TV. *SID 2004 International Symposium, Digest of Technical Papers, XXXV, Book II,* 760-763
- **SHIGETA, MITZUHIRO ; FUKUOKA, HIROFUMI.** Development of High Quality LCDTV. *SID 2004 International Symposium, Digest of Technical Papers, XXXV, Book II,* 754-757
- **SOUK, JUN.** Recent Advances in LCD Technology. *Seminar Lecture Notes, M-6/1 to M-6/26*
- **MILLER, IAN.** LCD-Television. *Seminar Lecture Notes, M-7/1 to M-7/32,* 2004
- **KIM, HYEON KYEONG et al.** A 57-in. Wide UXGA TFT-LCD for HDTV Application. *SID 2004 International Symposium, Digest of Technical Papers, XXXV, Book I,* 106-109
- *Pure Appl. Chem.,* 2001, vol. 73 (5), 888
- **C. TSCHIERSKE ; G. PELZL ; S. DIELE.** *Angew. Chem.,* 2004, vol. 116, 6340-6368
- Merck Liquid Crystals, Physical Properties of Liquid Crystals. Merck KGaA, November 1997